# EUROPEAN PATENT SPECIFICATION

(11) **EP 3 017 044 B1**
(45) Date of publication and mention of the grant of the patent: **26.02.2020**
(21) Application number: 14819490.5
(22) Date of filing: 01.07.2014
(51) Int. Cl.: C12N 15/11, A61K 38/00

(54) **MODULATORS OF GROWTH HORMONE RECEPTOR**
MODULATOREN DES WACHSTUMSHORMONREZEPTORS
MODULATEURS DU RÉCEPTEUR DE L'HORMONE DE CROISSANCE

(30) Priority: 02.07.2013 US 201361842302 P
(43) Date of publication of application: 11.05.2016
(73) Proprietor: Ionis Pharmaceuticals, Inc., Carlsbad, CA 92010 (US)
(72) Inventor: BHANOT, Sanjay, Carlsbad, CA 92010 (US); FREIER, Susan, M., Carlsbad, CA 92010 (US); BUI, Huynh-Hoa, Carlsbad, CA 92010 (US)
(74) Representative: Carpmaels & Ransford LLP
(86) International application number: PCT/US2014/045088
(87) International publication number: WO 2015/002971

(56) References cited:
- EP-A1- 2 849 801
- EP-A1- 2 850 183
- WO-A2-01/23616
- WO-A2-01/77384
- WO-A2-2004/078922
- US-A1- 2006 003 322
- US-A1- 2011 092 572
- US-A1- 2011 178 283
- US-A1- 2011 191 912
- US-A1- 2012 084 885
- US-A1- 2013 059 902
- PELLIGRINI E ET AL: "CENTRAL ADMINISTRATION OF A GROWTH HORMONE (GH) RECEPTOR mRNA ANTISENSE INCREASES GH PULSATILITY AND DECREASES HYPOTHALAMIC SOMATOSATIN EXPRESSION IN RATS", JOURNAL OF NEUROSCIENCE, SOCIETY FOR NEUROSCIENCE, US, vol. 16, no. 24, 1 December 1996 (1996-12-01), pages 8140-8148, XP003006516, ISSN: 0270-6474
- TACHAS G ET AL: "A GH receptor antisense oligonucleotide inhibits hepatic GH receptor expression, lGF-I production and body weight gain in normal mice", JOURNAL OF ENDOCRINOLOGY, SOCIETY FOR ENDOCRINOLOGY, GB, vol. 189, no. 1, 1 April 2006 (2006-04-01) , pages 147-154, XP002442310, ISSN: 0022-0795, DOI: 10.1677/JOE.1.06553
- WILKINSON-BERKA JENNIFER L; LOFTHOUSE SHARI; JAWORSKI KASSIE; NINKOVIC SLAVISA; TACHAS GEORGE; WRAIGHT CHRISTOPHER J: "An antisense oligonucleotide targeting the growth hormone receptor inhibits neovascularization in a mouse model of retinopathy", MOLECULAR VISION, vol. 13, 29 August 2007 (2007-08-29), pages 1529-1538, XP055302167, US ISSN: 1090-0535
- RAN GANG ET AL: "[Effect of rhGH on JAK2-STAT3 signal pathway after GHR was down-regulated by siRNA in gastric cancer cell]", YAO HSUEH HSUEH PAO - ACTA PHARMACEUTICA SINICA, YAOXUE XUEBAO, CN, vol. 48, no. 3, 1 March 2013 (2013-03-01), pages 435-440, XP008182938, ISSN: 0513-4870
- SCHERER L J ET AL: "Approaches for the sequence-specific knockdown of mRNA", NATURE BIOTECHNOLOGY, GALE GROUP INC, US, vol. 21, no. 12, 1 December 2003 (2003-12-01), pages 1457-1465, XP003008517, ISSN: 1087-0156, DOI: 10.1038/NBT915
- VICKERS T A ET AL: "Efficient reduction of target RNAs by small interfering RNA and RNase H-dependent antisense agents: A comparative analysis", JOURNAL OF BIOLOGICAL CHEMISTRY, AMERICAN SOCIETY FOR BIOCHEMISTRY AND MOLECULAR BIOLOGY, US, vol. 278, no. 9, 28 February 2003 (2003-02-28), pages 7108-7118, XP002281434, ISSN: 0021-9258, DOI: 10.1074/JBC.M210326200

## Description

### Field

The present embodiments provide methods, compounds, and compositions for treating, preventing, or ameliorating a disease associated with excess growth hormone using antisense compounds or oligonucleotides targeted to growth hormone receptor (GHR).

### Background

Growth hormone is produced in the pituitary and secreted into the bloodstream where it binds to growth hormone receptor (GHR) on many cell types, causing production of insulin-like growth factor-1 (IGF-1). IGF-1 is produced mainly in the liver, but also in adipose tissue and the kidney, and secreted into the bloodstream. Several disorders, such as acromegaly and gigantism, are associated with elevated growth hormone levels and/or elevated IGF-I levels in plasma and/or tissues.

Excessive production of growth hormone can lead to diseases such as acromegaly or gigantism. Acromegaly and gigantism are associated with excess growth hormone, often caused by a pituitary tumor, and affects 40-50 per million people worldwide with about 15,000 patients in each of the US and Europe and an annual incidence of about 4-5 per million people. Acromegaly and gigantism are initially characterized by abnormal growth of the hands and feet and bony changes in the facial features. Many of the growth related outcomes are mediated by elevated levels of serum IGF-1.

WO 2004/078922 describes compounds, compositions and methods for modulating the expression of growth hormone receptor and/or insulin like growth factor-I (IGF-I).

Pellegrini et al. (J Neurosci. 1996 Dec 15;16(24):8140-8) investigate involvement of centrally expressed rat growth hormone receptors (rGH-R) in the ultradian rhythmicity of pituitary GH secretion.

Tachas et al. (J Endocrinol. 2006 Apr;189(1):147-54) investigate the ability of a modified oligodeoxynucleotide to suppress GH receptor mRNA *in vitro.*

Wilkinson-Berka et al. (Mol Vis. 2007 Aug 29;13:1529-38) investigate whether systemically delivered antisense oligonucleotide could inhibit neovascularization in mice with oxygen induced retinopathy (OIR).

Ran et al. (Yao Xue Xue Bao. 2013 Mar;48(3):435-40) investigate the effect of recombinant human growth hormone (rhGH) on JAK2-STAT3 pathway and the growth of gastric cancer cell lines at different GHR expression status.

### Summary

The invention provides a compound comprising a modified oligonucleotide consisting of 18 to 30 linked nucleosides in length, wherein the modified oligonucleotide has a nucleobase sequence comprising a portion of at least 18 contiguous nucleobases 100% complementary to an equal length portion of nucleobases 153921-153940 of a growth hormone receptor nucleic acid having the nucleobase sequence of SEQ ID NO: 2, wherein the nucleobase sequence of the modified oligonucleotide is at least 90% complementary to SEQ ID NO: 2, wherein the compound reduces the amount or activity of the growth hormone receptor nucleic acid.

The invention further provides a composition comprising the compound of the invention or salt thereof and at least one of a pharmaceutically acceptable carrier or diluent.

Embodiments provided herein relate to methods, compounds, and compositions for treating, preventing, or ameliorating a disease associated with excess growth hormone. Several embodiments provided herein are drawn to antisense compounds or oligonucleotides targeted to growth hormone receptor (GHR). Several embodiments are directed to treatment, prevention, or amelioration of acromegaly with antisense compounds or oligonucleotides targeted to growth hormone receptor (GHR).

### Detailed Description

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

Unless otherwise indicated, the following terms have the following meanings:
"2'-O-methoxyethyl" (also 2'-MOE and 2'-O(CH₂)₂-OCH₃) refers to an O-methoxy-ethyl modification at the 2' position of a furanose ring. A 2'-O-methoxyethyl modified sugar is a modified sugar.
"2'-MOE nucleoside" (also 2'-O-methoxyethyl nucleoside) means a nucleoside comprising a 2'-MOE modified sugar moiety.
"2'-substituted nucleoside" means a nucleoside comprising a substituent at the 2'-position of the furanosyl ring other than H or OH. In certain embodiments, 2' substituted nucleosides include nucleosides with bicyclic sugar modifications.
"3' target site" refers to the nucleotide of a target nucleic acid which is complementary to the 3'-most nucleotide of a particular antisense compound.
"5' target site" refers to the nucleotide of a target nucleic acid which is complementary to the 5'-most nucleotide of a particular antisense compound.
"5-methylcytosine" means a cytosine modified with a methyl group attached to the 5 position. A 5-methylcytosine is a modified nucleobase.
"About" means within ±10% of a value. For example, if it is stated, "the compounds affected at least about 70% inhibition of GHR", it is implied that GHR levels are inhibited within a range of 60% and 80%.
"Administration" or "administering" refers to routes of introducing an antisense compound provided herein to a subject to perform its intended function. An example of a route of administration that can be used includes, but is not limited to parenteral administration, such as subcutaneous, intravenous, or intramuscular injection or infusion.
"Amelioration" refers to a lessening of at least one indicator, sign, or symptom of an associated disease, disorder, or condition. In certain embodiments, amelioration includes a delay or slowing in the progression of one or more indicators of a condition or disease. The severity of indicators may be determined by subjective or objective measures, which are known to those skilled in the art.
"Animal" refers to a human or non-human animal, including, but not limited to, mice, rats, rabbits, dogs, cats, pigs, and non-human primates, including, but not limited to, monkeys and chimpanzees.
"Antisense activity" means any detectable or measurable activity attributable to the hybridization of an antisense compound to its target nucleic acid. In certain embodiments, antisense activity is a decrease in the amount or expression of a target nucleic acid or protein encoded by such target nucleic acid.
"Antisense compound" means an oligomeric compound that is is capable of undergoing hybridization to a target nucleic acid through hydrogen bonding. Examples of antisense compounds include single-stranded and double-stranded compounds, such as, antisense oligonucleotides, siRNAs, shRNAs, ssRNAs, and occupancy-based compounds.
"Antisense inhibition" means reduction of target nucleic acid levels in the presence of an antisense compound complementary to a target nucleic acid compared to target nucleic acid levels in the absence of the antisense compound.
"Antisense oligonucleotide" means a single-stranded oligonucleotide having a nucleobase sequence that permits hybridization to a corresponding region or segment of a target nucleic acid.
"Base complementarity" refers to the capacity for the precise base pairing of nucleobases of an antisense oligonucleotide with corresponding nucleobases in a target nucleic acid (i.e., hybridization), and is mediated by Watson-Crick, Hoogsteen or reversed Hoogsteen hydrogen binding between corresponding nucleobases.
"Bicyclic sugar moiety" means a modified sugar moiety comprising a 4 to 7 membered ring (including but not limited to a furanosyl) comprising a bridge connecting two atoms of the 4 to 7 membered ring to form a second ring, resulting in a bicyclic structure. In certain embodiments, the 4 to 7 membered ring is a sugar ring. In certain embodiments the 4 to 7 membered ring is a furanosyl. In certain such embodiments, the bridge connects the 2'-carbon and the 4'-carbon of the furanosyl.
"Bicyclic nucleic acid" or " BNA" or "BNA nucleosides" means nucleic acid monomers having a bridge connecting two carbon atoms between the 4' and 2 'position of the nucleoside sugar unit, thereby forming a bicyclic sugar. Examples of such bicyclic sugar include, but are not limited to A) α-L-Methyleneoxy (4'-CH₂-O-2') LNA, (B) β-D-Methyleneoxy (4'-CH₂-O-2') LNA, (C) Ethyleneoxy (4'-(CH₂)₂-O-2') LNA, (D) Aminooxy (4'-CH₂-O-N(R)-2') LNA and (E) Oxyamino (4'-CH₂-N(R)-O-2') LNA, as depicted below. 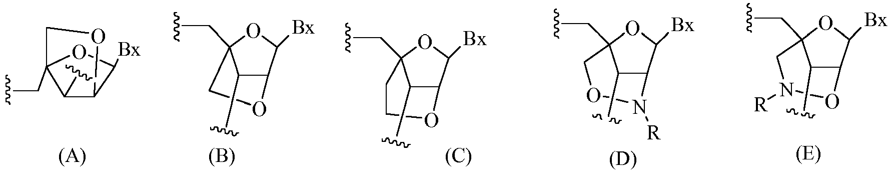
"cEt" or "constrained ethyl" means a bicyclic sugar moiety comprising a bridge connecting the 4'-carbon and the 2'-carbon, wherein the bridge has the formula: 4'-CH(CH₃)-O-2'.
"Constrained ethyl nucleoside" (also cEt nucleoside) means a nucleoside comprising a bicyclic sugar moiety comprising a 4'-CH(CH₃)-O-2' bridge.
"Chemically distinct region" refers to a region of an antisense compound that is in some way chemically different than another region of the same antisense compound. For example, a region having 2'-O-methoxyethyl nucleotides is chemically distinct from a region having nucleotides without 2'-O-methoxyethyl modifications.
"Complementarity" means the capacity for pairing between nucleobases of a first nucleic acid and a second nucleic acid.
"Comprise," "comprises" and "comprising" will be understood to imply the inclusion of a stated step or element or group of steps or elements but not the exclusion of any other step or element or group of steps or elements.
"Contiguous nucleobases" means nucleobases immediately adjacent to each other.
"Deoxyribonucleotide" means a nucleotide having a hydrogen at the 2' position of the sugar portion of the nucleotide. Deoxyribonucleotides may be modified with any of a variety of substituents.
"Designing" or "Designed to" refer to the process of designing an oligomeric compound that specifically hybridizes with a selected nucleic acid molecule.
"Effective amount" means the amount of active pharmaceutical agent sufficient to effectuate a desired physiological outcome in an individual in need of the agent. The effective amount may vary among individuals depending on the health and physical condition of the individual to be treated, the taxonomic group of the individuals to be treated, the formulation of the composition, assessment of the individual's medical condition, and other relevant factors.
"Efficacy" means the ability to produce a desired effect.
"Expression" includes all the functions by which a gene's coded information is converted into structures present and operating in a cell. Such structures include, but are not limited to the products of transcription and translation.
"Fully complementary" or "100% complementary" means each nucleobase of a first nucleic acid has a complementary nucleobase in a second nucleic acid. In certain embodiments, a first nucleic acid is an antisense compound and a target nucleic acid is a second nucleic acid.
"Gapmer" means a chimeric antisense compound in which an internal region having a plurality of nucleosides that support RNase H cleavage is positioned between external regions having one or more nucleosides, wherein the nucleosides comprising the internal region are chemically distinct from the nucleoside or nucleosides comprising the external regions. The internal region may be referred to as the "gap" and the external regions may be referred to as the "wings."
"Growth Hormone Receptor (GHR)" means any nucleic acid or protein of GHR. "GHR nucleic acid" means any nucleic acid encoding GHR. For example, in certain embodiments, a GHR nucleic acid includes a DNA sequence encoding GHR, an RNA sequence transcribed from DNA encoding GHR (including genomic DNA comprising introns and exons), including a non-protein encoding (*i.e.* non-coding) RNA sequence, and an mRNA sequence encoding GHR. "GHR mRNA" means an mRNA encoding a GHR protein.
"GHR specific inhibitor" refers to any agent capable of specifically inhibiting GHR RNA and/or GHR protein expression or activity at the molecular level. For example, GHR specific inhibitors include nucleic acids (including antisense compounds), peptides, antibodies, small molecules, and other agents capable of inhibiting the expression of GHR RNA and/or GHR protein.
"Hybridization" means the annealing of complementary nucleic acid molecules. In certain embodiments, complementary nucleic acid molecules include, but are not limited to, an antisense compound and a nucleic acid target. In certain embodiments, complementary nucleic acid molecules include, but are not limited to, an antisense oligonucleotide and a nucleic acid target.
"Identifying an animal having, or at risk for having, a disease, disorder and/or condition" means identifying an animal having been diagnosed with the disease, disorder and/or condition or identifying an animal predisposed to develop the disease, disorder and/or condition. Such identification may be accomplished by any method including evaluating an individual's medical history and standard clinical tests or assessments.
"Immediately adjacent" means there are no intervening elements between the immediately adjacent elements.
"Individual" means a human or non-human animal selected for treatment or therapy.
"Inhibiting the expression or activity" refers to a reduction, blockade of the expression or activity and does not necessarily indicate a total elimination of expression or activity.
"Internucleoside linkage" refers to the chemical bond between nucleosides.
"Linked deoxynucleoside" means a nucleic acid base (A, G, C, T, U) substituted by deoxyribose linked by a phosphate ester to form a nucleotide.
"Linked nucleosides" means adjacent nucleosides linked together by an internucleoside linkage.
"Mismatch" or "non-complementary nucleobase" refers to the case when a nucleobase of a first nucleic acid is not capable of pairing with the corresponding nucleobase of a second or target nucleic acid.
"Modified internucleoside linkage" refers to a substitution or any change from a naturally occurring internucleoside bond (i.e. a phosphodiester internucleoside bond).
"Modified nucleobase" means any nucleobase other than adenine, cytosine, guanine, thymidine, or uracil. An "unmodified nucleobase" means the purine bases adenine (A) and guanine (G), and the pyrimidine bases thymine (T), cytosine (C) and uracil (U).
"Modified nucleoside" means a nucleoside having, independently, a modified sugar moiety and/or modified nucleobase.
"Modified nucleotide" means a nucleotide having, independently, a modified sugar moiety, modified internucleoside linkage, or modified nucleobase.
"Modified oligonucleotide" means an oligonucleotide comprising at least one modified internucleoside linkage, a modified sugar, and/or a modified nucleobase.
"Modified sugar" means substitution and/or any change from a natural sugar moiety.
"Modulating" refers to changing or adjusting a feature in a cell, tissue, organ or organism. For example, modulating GHR mRNA can mean to increase or decrease the level of GHR mRNA and/or GHR protein in a cell, tissue, organ or organism. A "modulator" effects the change in the cell, tissue, organ or organism. For example, a GHR antisense compound can be a modulator that decreases the amount of GHR mRNA and/or GHR protein in a cell, tissue, organ or organism.
"Monomer" refers to a single unit of an oligomer. Monomers include, but are not limited to, nucleosides and nucleotides, whether naturally occuring or modified.
"Motif" means the pattern of unmodified and modified nucleosides in an antisense compound.
"Natural sugar moiety" means a sugar moiety found in DNA (2'-H) or RNA (2'-OH).
"Naturally occurring internucleoside linkage" means a 3' to 5' phosphodiester linkage.
"Non-complementary nucleobase" refers to a pair of nucleobases that do not form hydrogen bonds with one another or otherwise support hybridization.
"Nucleic acid" refers to molecules composed of monomeric nucleotides. A nucleic acid includes, but is not limited to, ribonucleic acids (RNA), deoxyribonucleic acids (DNA), single-stranded nucleic acids, and double-stranded nucleic acids.
"Nucleobase" means a heterocyclic moiety capable of pairing with a base of another nucleic acid.
"Nucleobase complementarity" refers to a nucleobase that is capable of base pairing with another nucleobase. For example, in DNA, adenine (A) is complementary to thymine (T). For example, in RNA, adenine (A) is complementary to uracil (U). In certain embodiments, complementary nucleobase refers to a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair.
"Nucleobase sequence" means the order of contiguous nucleobases independent of any sugar, linkage, and/or nucleobase modification.
"Nucleoside" means a nucleobase linked to a sugar.
"Nucleotide" means a nucleoside having a phosphate group covalently linked to the sugar portion of the nucleoside.
"Oligomeric compound" means a polymer of linked monomeric subunits which is capable of hybridizing to at least a region of a nucleic acid molecule.
"Oligonucleoside" means an oligonucleotide in which the internucleoside linkages do not contain a phosphorus atom.
"Oligonucleotide" means a polymer of linked nucleosides each of which can be modified or unmodified, independent one from another.
"Parenteral administration" means administration through injection or infusion. Parenteral administration includes subcutaneous administration, intravenous administration, intramuscular administration, intraarterial administration, intraperitoneal administration, or intracranial administration, e.g. intrathecal or intracerebroventricular administration.
"Pharmaceutical composition" means a mixture of substances suitable for administering to an individual. For example, a pharmaceutical composition may comprise one or more active pharmaceutical agents and a sterile aqueous solution.
"Pharmaceutically acceptable salts" means physiologically and pharmaceutically acceptable salts of antisense compounds, i.e., salts that retain the desired biological activity of the parent oligonucleotide and do not impart undesired toxicological effects thereto.
"Phosphorothioate linkage" means a linkage between nucleosides where the phosphodiester bond is modified by replacing one of the non-bridging oxygen atoms with a sulfur atom. A phosphorothioate linkage is a modified internucleoside linkage.
"Portion" means a defined number of contiguous (i.e., linked) nucleobases of a nucleic acid. In certain embodiments, a portion is a defined number of contiguous nucleobases of a target nucleic acid. In certain embodiments, a portion is a defined number of contiguous nucleobases of an antisense compound
"Prevent" refers to delaying or forestalling the onset, development or progression of a disease, disorder, or condition for a period of time from minutes to indefinitely. Prevent also means reducing the risk of developing a disease, disorder, or condition.
"Prophylactically effective amount" refers to an amount of a pharmaceutical agent that provides a prophylactic or preventative benefit to an animal.
"Region" is defined as a portion of the target nucleic acid having at least one identifiable structure, function, or characteristic.
"Ribonucleotide" means a nucleotide having a hydroxy at the 2' position of the sugar portion of the nucleotide. Ribonucleotides may be modified with any of a variety of substituents.
"Segments" are defined as smaller or sub-portions of regions within a target nucleic acid.
"Side effects" means physiological disease and/or conditions attributable to a treatment other than the desired effects. In certain embodiments, side effects include injection site reactions, liver function test abnormalities, renal function abnormalities, liver toxicity, renal toxicity, central nervous system abnormalities, myopathies, and malaise. For example, increased aminotransferase levels in serum may indicate liver toxicity or liver function abnormality. For example, increased bilirubin may indicate liver toxicity or liver function abnormality.
"Sites," as used herein, are defined as unique nucleobase positions within a target nucleic acid.
"Slows progression" means decrease in the development of the said disease.
"Specifically hybridizable" refers to an antisense compound having a sufficient degree of complementarity between an antisense oligonucleotide and a target nucleic acid to induce a desired effect, while exhibiting minimal or no effects on non-target nucleic acids under conditions in which specific binding is desired, i.e., under physiological conditions in the case of *in vivo* assays and therapeutic treatments. "Stringent hybridization conditions" or "stringent conditions" refer to conditions under which an oligomeric compound will hybridize to its target sequence, but to a minimal number of other sequences.
"Subject" means a human or non-human animal selected for treatment or therapy.
"Target" refers to a protein, the modulation of which is desired.
"Target gene" refers to a gene encoding a target.
"Targeting" means the process of design and selection of an antisense compound that will specifically hybridize to a target nucleic acid and induce a desired effect.
"Target nucleic acid," "target RNA," "target RNA transcript" and "nucleic acid target" all mean a nucleic acid capable of being targeted by antisense compounds.
"Target region" means a portion of a target nucleic acid to which one or more antisense compounds is targeted.
"Target segment" means the sequence of nucleotides of a target nucleic acid to which an antisense compound is targeted. "5' target site" refers to the 5'-most nucleotide of a target segment. "3' target site" refers to the 3'-most nucleotide of a target segment.
"Therapeutically effective amount" means an amount of a pharmaceutical agent that provides a therapeutic benefit to an individual.
"Treat" refers to administering a pharmaceutical composition to an animal in order to effect an alteration or improvement of a disease, disorder, or condition in the animal. In certain embodiments, one or more pharmaceutical compositions can be administered to the animal.
"Unmodified" nucleobases mean the purine bases adenine (A) and guanine (G), and the pyrimidine bases thymine (T), cytosine (C) and uracil (U).
"Unmodified nucleotide" means a nucleotide composed of naturally occuring nucleobases, sugar moieties, and internucleoside linkages. In certain aspects of the disclosure, an unmodified nucleotide is an RNA nucleotide (i.e. β-D-ribonucleosides) or a DNA nucleotide (i.e. β-D-deoxyribonucleoside).

### Certain Embodiments and Aspects of the Disclosure

Certain aspects of the disclosure provide methods, compounds and compositions for inhibiting growth hormone receptor (GHR) expression.

Certain embodiments provide antisense compounds targeted to a GHR nucleic acid. In certain aspects of the disclosure, the GHR nucleic acid has the sequence set forth in GENBANK Accession No. NM_000163.4 (incorporated herein as SEQ ID NO: 1), GENBANK Accession No. NT_006576.16 truncated from nucleotides 42411001 to 42714000 (incorporated herein as SEQ ID NO: 2), GENBANK Accession No X06562.1 (incorporated herein as SEQ ID NO: 3), GENBANK Accession No. DR006395.1 (incorporated herein as SEQ ID NO: 4), GENBANK Accession No. DB052048.1 (incorporated herein as SEQ ID NO: 5), GENBANK Accession No. AF230800.1 (incorporated herein as SEQ ID NO: 6), the complement of GENBANK Accession No. AA398260.1 (incorporated herein as SEQ ID NO: 7), GENBANK Accession No. BC136496.1 (incorporated herein as SEQ ID NO: 8), GENBANK Accession No. NM_001242399.2 (incorporated herein as SEQ ID NO: 9), GENBANK Accession No. NM_001242400.2 (incorporated herein as SEQ ID NO: 10), GENBANK Accession No. NM_001242401.3 (incorporated herein as SEQ ID NO: 11), GENBANK Accession No. NM_001242402.2 (incorporated herein as SEQ ID NO: 12), GENBANK Accession No. NM_001242403.2 (incorporated herein as SEQ ID NO: 13), GENBANK Accession No. NM_001242404.2 (incorporated herein as SEQ ID NO: 14), GENBANK Accession No. NM_001242405.2 (incorporated herein as SEQ ID NO: 15), GENBANK Accession No. NM_001242406.2 (incorporated herein as SEQ ID NO: 16), GENBANK Accession No. NM_001242460.1 (incorporated herein as SEQ ID NO: 17), GENBANK Accession NM_001242461.1 (incorporated herein as SEQ ID NO: 18), or GENBANK Accession No. NM_001242462.1 (incorporated herein as SEQ ID NO: 19).

Certain aspects of the disclosure provide a compound comprising a modified oligonucleotide consisting of 10 to 30 linked nucleosides and having a nucleobase sequence comprising at least 8 contiguous nucleobases of any of the nucleobase sequences of SEQ ID NOs: 20-2295.

Certain aspects of the disclosure provide a compound comprising a modified oligonucleotide consisting of 10 to 30 linked nucleosides complementary within nucleotides Certain aspects of the disclosure provide a compound comprising a modified oligonucleotide consisting of 10 to 30 linked nucleosides complementary within nucleotides 153831-154112 of SEQ ID NO: 2, wherein said modified oligonucleotide is at least 90% complementary to SEQ ID NO: 2.

Certain aspects of the disclosure provide a compound comprising a modified oligonucleotide consisting of 10 to 30 linked nucleosides having a nucleobase sequence comprising a portion of at least 8 contiguous nucleobases 100% complementary to an equal length portion of nucleobases 153831-154112 of SEQ ID NO: 2, wherein the nucleobase sequence of the modified oligonucleotide is complementary to SEQ ID NO: 2 153831-154112.

In certain embodiments, an antisense compound or oligonucleotide targeted to a growth hormone receptor nucleic acid target the following nucleotide regions of SEQ ID NO: 2: 153831-154112.

In certain embodiments, antisense compounds or oligonucleotides target a region of a growth hormone receptor nucleic acid. In certain aspects of the disclosure, such compounds or oligonucleotides targeted to a region of a GHR nucleic acid have a contiguous nucleobase portion that is complementary to an equal length nucleobase portion of the region. For example, the portion can be at least an 8, 9, 10, 11, 12, 13, 14, 15, or 16 contiguous nucleobases portion complementary to an equal length portion of a region recited herein. In certain embodiments, such compounds or oligonucleotide target the following nucleotide regions of SEQ ID NO: 2: 153831-154112.

In certain embodiments, any of the foregoing compounds or oligonucleotides comprises at least one modified sugar. In certain aspects, at least one modified sugar comprises a 2'-O-methoxyethyl group. In certain aspects, at least one modified sugar is a bicyclic sugar, such as a 4'-CH(CH3)-O-2' group, a 4'-CH2-O-2' group, or a 4'-(CH2)2-O-2'group. In certain aspects, the modified oligonucleotide comprises at least one modified internucleoside linkage, such as a phosphorothioate internucleoside linkage.

In certain embodiments, any of the foregoing compounds or oligonucleotides comprises at least one modified nucleobase, such as 5-methylcytosine.

In certain embodiments, any of the foregoing compounds or oligonucleotides comprises:
a gap segment consisting of linked deoxynucleosides;
a 5' wing segment consisting of linked nucleosides; and
a 3' wing segment consisting of linked nucleosides;
wherein the gap segment is positioned between the 5' wing segment and the 3' wing segment and wherein each nucleoside of each wing segment comprises a modified sugar.

Certain aspects of the disclosure provide a compound comprising a modified oligonucleotide consisting of 10 to 30 linked nucleosides having a nucleobase sequence comprising the sequence recited in SEQ ID NO: 918, 479, 703, 1800, 1904, 2122, 2127, or 2194.

In certain aspects, the modified oligonucleotide has a nucleobase sequence comprising the sequence recited in SEQ ID NOs: 918, 479 or 703, wherein the modified oligonucleotide comprises
a gap segment consisting of ten linked deoxynucleosides;
a 5' wing segment consisting of five linked nucleosides; and
a 3' wing segment consisting of five linked nucleosides;
wherein the gap segment is positioned between the 5' wing segment and the 3' wing segment, wherein each nucleoside of each wing segment comprises a 2'-O-methoxyethyl sugar; wherein each internucleoside linkage is a phosphorothioate linkage and wherein each cytosine is a 5-methylcytosine.

In certain aspects, the modified oligonucleotide has a nucleobase sequence comprising the sequence recited in SEQ ID NOs: 1800, 1904, 2122, 2127, or 2194, wherein the modified oligonucleotide comprises:
a gap segment consisting of ten linked deoxynucleosides;
a 5' wing segment consisting of 3 linked nucleosides; and
a 3' wing segment consisting of 3 linked nucleosides;
wherein the gap segment is positioned between the 5' wing segment and the 3' wing segment, wherein each nucleoside of each wing segment comprises a 2'-O-methoxyethyl sugar or a constrained ethyl sugar; and wherein each internucleoside linkage is a phosphorothioate linkage.

Certain embodiments provide a compound comprising a modified oligonucleotide consisting of 20 linked nucleosides having a nucleobase sequence consisting of the sequence recited in SEQ ID NO: 703. In certain aspects, the modified oligonucleotide comprises at least one modified sugar. In certain aspects, the at least one modified sugar comprises a 2'-O-methoxyethyl group. In certain aspects, the at least one modified sugar is a bicyclic sugar, such as a 4'-CH(CH3)-O-2' group, a 4'-CH2-O-2' group, or a 4'-(CH2)2-O-2'group. In certain aspects, the modified oligonucleotide comprises at least one modified internucleoside linkage, such as a phosphorothioate internucleoside linkage. In certain aspects, the modified oligonucleotide comprises at least one modified nucleobase, such as a 5-methylcytosine. In certain aspects, the modified oligonucleotide comprises:
a gap segment consisting of linked deoxynucleosides;
a 5' wing segment consisting of linked nucleosides; and
a 3' wing segment consisting of linked nucleosides;
wherein the gap segment is positioned between the 5' wing segment and the 3' wing segment and wherein each nucleoside of each wing segment comprises a modified sugar.

Certain embodiments provide a compound comprising a modified oligonucleotide consisting of 20 linked nucleosides having a nucleobase sequence consisting of the sequence recited in SEQ ID NO: 703, wherein the modified oligonucleotide comprises:
a gap segment consisting of ten linked deoxynucleosides;
a 5' wing segment consisting of five linked nucleosides; and
a 3' wing segment consisting of five linked nucleosides;
wherein the gap segment is positioned between the 5' wing segment and the 3' wing segment; wherein each nucleoside of each wing segment comprises a 2'-O-methoxyethyl sugar; wherein each internucleoside linkage is a phosphorothioate linkage; and wherein each cytosine is a 5-methylcytosine.

In any of the foregoing embodiments, the compound or oligonucleotide can be at least 85%, at least 90%, at least 95%, at least 98%, at least 99%, or 100% complementary to a nucleic acid encoding growth hormone receptor.

In any of the foregoing embodiments, the nucleic acid encoding growth hormone receptor can comprise the nucleotide sequence of any one of SEQ ID NOs: 1-19.

In any of the foregoing embodiments, the compound or oligonucleotide can be single-stranded.

Certain embodiments provide a composition comprising the compound of any of the aforementioned embodiments or salt thereof and at least one of a pharmaceutically acceptable carrier or diluent. In certain aspects, the composition has a viscosity less than about 40 centipoise (cP), less than about 30 centipose (cP), less than about 20 centipose (cP), less than about 15 centipose (cP), or less than about 10 centipose (cP). In certain aspects, the composition having any of the aforementioned viscosities comprises a compound provided herein at a concentration of about 100 mg/mL, about 125 mg/mL, about 150 mg/mL, about 175 mg/mL, about 200 mg/mL, about 225 mg/mL, about 250 mg/mL, about 275 mg/mL, or about 300 mg/mL. In certain aspects, the composition having any of the aforementioned viscosities and/or compound concentrations has a temperature of room temperature or about 20°C, about 21°C, about 22°C, about 23°C, about 24°C, about 25°C, about 26°C, about 27°C, about 28°C, about 29°C, or about 30°C.

Certain aspects of the disclosure provide a method of treating a disease associated with excess growth hormone in a human comprising administering to the human a therapeutically effective amount of the compound or composition of any of the aforementioned embodiments, thereby treating the disease associated with excess growth hormone. In certain aspects, the disease associated with excess growth hormone is acromegaly. In certain aspects, the treatment reduces IGF-1 levels.

Certain aspects of the disclosure provide a method of preventing a disease associated with excess growth hormone in a human comprising administering to the human a therapeutically effective amount of a compound or composition of any of the aforementioned embodiments, thereby preventing the disease associated with excess growth hormone. In certain aspects of the disclosure, the disease associated with excess growth hormone is acromegaly.

Certain aspects of the disclosure provide a method of reducing growth hormone receptor (GHR) levels in a human comprising administering to the human a therapeutically effective amount of the compound or composition of any of the aforementioned embodiments, thereby reducing GHR levels in the human. In certain aspects, the human has a disease associated with excess growth hormone. In certain aspects, the disease associated with excess growth hormone is acromegaly.

In certain aspects, the foregoing methods comprise co-administering the compound or composition and a second agent. In certain aspects, the compound or composition and the second agent are administered concomitantly.

### Antisense compounds

Oligomeric compounds include, but are not limited to, oligonucleotides, oligonucleosides, oligonucleotide analogs, oligonucleotide mimetics, antisense compounds, antisense oligonucleotides, and siRNAs. An oligomeric compound may be "antisense" to a target nucleic acid, meaning that is is capable of undergoing hybridization to a target nucleic acid through hydrogen bonding.

In certain embodiments, an antisense compound has a nucleobase sequence that, when written in the 5' to 3' direction, comprises the reverse complement of the target segment of a target nucleic acid to which it is targeted. In certain such embodiments, an antisense oligonucleotide has a nucleobase sequence that, when written in the 5' to 3' direction, comprises the reverse complement of the target segment of a target nucleic acid to which it is targeted.

In certain aspects of the disclosure, an antisense compound is 10 to 30 subunits in length. In certain aspects of the disclosure, an antisense compound is 12 to 30 subunits in length. In certain aspects of the disclosure, an antisense compound is 12 to 22 subunits in length. In certain aspects of the disclosure, an antisense compound is 14 to 30 subunits in length. In certain aspects of the disclosure, an antisense compound is 14 to 20 subunits in length. In certain aspects of the disclosure, an antisense compoun is 15 to 30 subunits in length. In certain aspects of the disclosure, an antisense compound is 15 to 20 subunits in length. In certain aspects of the disclosure, an antisense compound is 16 to 30 subunits in length. In certain aspects of the disclosure, an antisense compound is 16 to 20 subunits in length. In certain embodiments, an antisense compound is 17 to 30 subunits in length. In certain embodiments, an antisense compound is 17 to 20 subunits in length. In certain embodiments, an antisense compound is 18 to 30 subunits in length. In certain embodiments, an antisense compound is 18 to 21 subunits in length. In certain embodiments, an antisense compound is 18 to 20 subunits in length. In certain embodiments, an antisense compound is 20 to 30 subunits in length. In other words, such antisense compounds are from 12 to 30 linked subunits, 14 to 30 linked subunits, 14 to 20 subunits, 15 to 30 subunits, 15 to 20 subunits, 16 to 30 subunits, 16 to 20 subunits, 17 to 30 subunits, 17 to 20 subunits, 18 to 30 subunits, 18 to 20 subunits, 18 to 21 subunits, 20 to 30 subunits, or 12 to 22 linked subunits, respectively. In certain aspects of the disclosure, an antisense compound is 14 subunits in length. In certain aspects of the disclosure, an antisense compound is 16 subunits in length. In certain embodiments, an antisense compound is 17 subunits in length. In certain embodiments, an antisense compound is 18 subunits in length. In certain embodiments, an antisense compound is 19 subunits in length. In certain embodiments, an antisense compound is 20 subunits in length. In other aspects of the disclosure, the antisense compound is 8 to 80, 12 to 50, 13 to 30, 13 to 50, 14 to 30, 14 to 50, 15 to 30, 15 to 50, 16 to 30, 16 to 50, 17 to 30, 17 to 50, 18 to 22, 18 to 24, 18 to 30, 18 to 50, 19 to 22, 19 to 30, 19 to 50, or 20 to 30 linked subunits. In certain such aspects of the disclosure, the antisense compounds are 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 linked subunits in length, or a range defined by any two of the above values. In some embodiments the antisense compound is an antisense oligonucleotide, and the linked subunits are nucleotides.

In certain embodiments antisense oligonucleotides may be shortened or truncated. For example, a single subunit may be deleted from the 5' end (5' truncation), or alternatively from the 3' end (3' truncation). A shortened or truncated antisense compound targeted to a GHR nucleic acid may have two subunits deleted from the 5' end, or alternatively may have two subunits deleted from the 3' end, of the antisense compound. Alternatively, the deleted nucleosides may be dispersed throughout the antisense compound, for example, in an antisense compound having one nucleoside deleted from the 5' end and one nucleoside deleted from the 3' end.

When a single additional subunit is present in a lengthened antisense compound, the additional subunit may be located at the 5' or 3' end of the antisense compound. When two or more additional subunits are present, the added subunits may be adjacent to each other, for example, in an antisense compound having two subunits added to the 5' end (5' addition), or alternatively to the 3' end (3' addition), of the antisense compound. Alternatively, the added subunits may be dispersed throughout the antisense compound, for example, in an antisense compound having one subunit added to the 5' end and one subunit added to the 3' end.

It is possible to increase or decrease the length of an antisense compound, such as an antisense oligonucleotide, and/or introduce mismatch bases without eliminating activity. For example, in Woolf et al. (Proc. Natl. Acad. Sci. USA 89:7305-7309, 1992), a series of antisense oligonucleotides 13-25 nucleobases in length were tested for their ability to induce cleavage of a target RNA in an oocyte injection model. Antisense oligonucleotides 25 nucleobases in length with 8 or 11 mismatch bases near the ends of the antisense oligonucleotides were able to direct specific cleavage of the target mRNA, albeit to a lesser extent than the antisense oligonucleotides that contained no mismatches. Similarly, target specific cleavage was achieved using 13 nucleobase antisense oligonucleotides, including those with 1 or 3 mismatches.

Gautschi et al. (J. Natl. Cancer Inst. 93:463-471, March 2001) demonstrated the ability of an oligonucleotide having 100% complementarity to the bcl-2 mRNA and having 3 mismatches to the bcl-xL mRNA to reduce the expression of both bcl-2 and bcl-xL *in vitro* and *in vivo.* Furthermore, this oligonucleotide demonstrated potent anti-tumor activity *in vivo.*

Maher and Dolnick (Nuc. Acid. Res. 16:3341-3358,1988) tested a series of tandem 14 nucleobase antisense oligonucleotides, and a 28 and 42 nucleobase antisense oligonucleotides comprised of the sequence of two or three of the tandem antisense oligonucleotides, respectively, for their ability to arrest translation of human DHFR in a rabbit reticulocyte assay. Each of the three 14 nucleobase antisense oligonucleotides alone was able to inhibit translation, albeit at a more modest level than the 28 or 42 nucleobase antisense oligonucleotides.

### Certain Antisense Compound Motifs and Mechanisms

In certain embodiments, antisense compounds have chemically modified subunits arranged in patterns, or motifs, to confer to the antisense compounds properties such as enhanced inhibitory activity, increased binding affinity for a target nucleic acid, or resistance to degradation by *in vivo* nucleases.

Chimeric antisense compounds typically contain at least one region modified so as to confer increased resistance to nuclease degradation, increased cellular uptake, increased binding affinity for the target nucleic acid, and/or increased inhibitory activity. A second region of a chimeric antisense compound may confer another desired property e.g., serve as a substrate for the cellular endonuclease RNase H, which cleaves the RNA strand of an RNA:DNA duplex.

Antisense activity may result from any mechanism involving the hybridization of the antisense compound (e.g., oligonucleotide) with a target nucleic acid, wherein the hybridization ultimately results in a biological effect. In certain embodiments, the amount and/or activity of the target nucleic acid is modulated. In certain embodiments, the amount and/or activity of the target nucleic acid is reduced. In certain embodiments, hybridization of the antisense compound to the target nucleic acid ultimately results in target nucleic acid degradation. In certain embodiments, hybridization of the antisense compound to the target nucleic acid does not result in target nucleic acid degradation. In certain such embodiments, the presence of the antisense compound hybridized with the target nucleic acid (occupancy) results in a modulation of antisense activity. In certain embodiments, antisense compounds having a particular chemical motif or pattern of chemical modifications are particularly suited to exploit one or more mechanisms. In certain embodiments, antisense compounds function through more than one mechanism and/or through mechanisms that have not been elucidated. Accordingly, the antisense compounds described herein are not limited by particular mechanism.

Antisense mechanisms include, without limitation, RNase H mediated antisense; RNAi mechanisms, which utilize the RISC pathway and include, without limitation, siRNA, ssRNA and microRNA mechanisms; and occupancy based mechanisms. Certain antisense compounds may act through more than one such mechanism and/or through additional mechanisms.

### RNase H-Mediated Antisense

In certain embodiments, antisense activity results at least in part from degradation of target RNA by RNase H. RNase H is a cellular endonuclease that cleaves the RNA strand of an RNA:DNA duplex. It is known in the art that single-stranded antisense compounds which are "DNA-like" elicit RNase H activity in mammalian cells. Accordingly, antisense compounds comprising at least a portion of DNA or DNA-like nucleosides may activate RNase H, resulting in cleavage of the target nucleic acid. In certain embodiments, antisense compounds that utilize RNase H comprise one or more modified nucleosides. In certain embodiments, such antisense compounds comprise at least one block of 1-8 modified nucleosides. In certain such embodiments, the modified nucleosides do not support RNase H activity. In certain embodiments, such antisense compounds are gapmers, as described herein. In certain such embodiments, the gap of the gapmer comprises DNA nucleosides. In certain such embodiments, the gap of the gapmer comprises DNA-like nucleosides. In certain such embodiments, the gap of the gapmer comprises DNA nucleosides and DNA-like nucleosides.

Certain antisense compounds having a gapmer motif are considered chimeric antisense compounds. In a gapmer an internal region having a plurality of nucleotides that supports RNaseH cleavage is positioned between external regions having a plurality of nucleotides that are chemically distinct from the nucleosides of the internal region. In the case of an antisense oligonucleotide having a gapmer motif, the gap segment generally serves as the substrate for endonuclease cleavage, while the wing segments comprise modified nucleosides. In certain embodiments, the regions of a gapmer are differentiated by the types of sugar moieties comprising each distinct region. The types of sugar moieties that are used to differentiate the regions of a gapmer may in some embodiments include β-D-ribonucleosides, β-D-deoxyribonucleosides, 2'-modified nucleosides (such 2'-modified nucleosides may include 2'-MOE and 2'-O-CH₃, among others), and bicyclic sugar modified nucleosides (such bicyclic sugar modified nucleosides may include those having a constrained ethyl). In certain embodiments, nucleosides in the wings may include several modified sugar moieties, including, for example 2'-MOE and bicyclic sugar moieties such as constrained ethyl or LNA. In certain embodiments, wings may include several modified and unmodified sugar moieties. In certain embodiments, wings may include various combinations of 2'-MOE nucleosides, bicyclic sugar moieties such as constrained ethyl nucleosides or LNA nucleosides, and 2'-deoxynucleosides.

Each distinct region may comprise uniform sugar moieties, variant, or alternating sugar moieties. The wing-gap-wing motif is frequently described as "X-Y-Z", where "X" represents the length of the 5'-wing, "Y" represents the length of the gap, and "Z" represents the length of the 3'-wing. "X" and "Z" may comprise uniform, variant, or alternating sugar moieties. In certain embodiments, "X" and "Y" may include one or more 2'-deoxynucleosides."Y" may comprise 2'-deoxynucleosides. As used herein, a gapmer described as "X-Y-Z" has a configuration such that the gap is positioned immediately adjacent to each of the 5'-wing and the 3' wing. Thus, no intervening nucleotides exist between the 5'-wing and gap, or the gap and the 3'-wing. Any of the antisense compounds described herein can have a gapmer motif. In certain embodiments, "X" and "Z" are the same; in other embodiments they are different. In certain embodiments, "Y" is between 8 and 15 nucleosides. X, Y, or Z can be any of 1, 2, 3, 4, 5, 6, 7, 8, 9, 10, 11, 12, 13, 14, 15, 16, 17, 18, 19, 20, 25, 30 or more nucleosides.

In certain embodiments, the antisense compound targeted to a GHR nucleic acid has a gapmer motif in which the gap consists of 6, 7, 8, 9, 10, 11, 12, 13, 14, 15, or 16 linked nucleosides.

In certain embodiments, the antisense oligonucleotide has a sugar motif described by Formula A as follows: (J)ₘ-(B)ₙ-(J)ₚ-(B)ᵣ-(A)ₜ-(D)_{g}-(A)ᵥ-(B)_{w}-(J)ₓ-(B)_{y}-(J)_{z} wherein:
each A is independently a 2'-substituted nucleoside;
each B is independently a bicyclic nucleoside;
each J is independently either a 2'-substituted nucleoside or a 2'-deoxynucleoside;
each D is a 2'-deoxynucleoside;
m is 0-4; n is 0-2; p is 0-2; r is 0-2; t is 0-2; v is 0-2; w is 0-4; x is 0-2; y is 0-2; z is 0-4; g is 6-14;
provided that:
at least one of m, n, and r is other than 0;
at least one of w and y is other than 0;
the sum of m, n, p, r, and t is from 2 to 5; and
the sum of v, w, x, y, and z is from 2 to 5.

### RNAi Compounds

In certain embodiments, antisense compounds are interfering RNA compounds (RNAi), which include double-stranded RNA compounds (also referred to as short-interfering RNA or siRNA) and single-stranded RNAi compounds (or ssRNA). Such compounds work at least in part through the RISC pathway to degrade and/or sequester a target nucleic acid (thus, include microRNA/microRNA-mimic compounds). In certain embodiments, antisense compounds comprise modifications that make them particularly suited for such mechanisms.

### i. ssRNA compounds

In certain embodiments, antisense compounds including those particularly suited for use as single-stranded RNAi compounds (ssRNA) comprise a modified 5'-terminal end. In certain such embodiments, the 5 '-terminal end comprises a modified phosphate moiety. In certain embodiments, such modified phosphate is stabilized (e.g., resistant to degradation/cleavage compared to unmodified 5'-phosphate). In certain embodiments, such 5'-terminal nucleosides stabilize the 5'-phosphorous moiety. Certain modified 5'-terminal nucleosides may be found in the art, for example in WO/2011/139702.

In certain embodiments, the 5'-nucleoside of an ssRNA compound has Formula IIc: 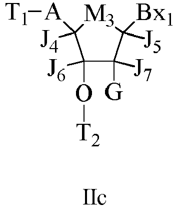 wherein:
T₁ is an optionally protected phosphorus moiety;
T₂ is an internucleoside linking group linking the compound of Formula IIc to the oligomeric compound;
A has one of the formulas: 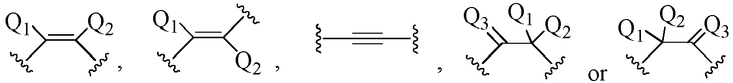
Q₁ and Q₂ are each, independently, H, halogen, C₁-C₆ alkyl, substituted C₁-C₆ alkyl, C₁-C₆alkoxy, substituted C₁-C₆ alkoxy, C₂-C₆ alkenyl, substituted C₂-C₆ alkenyl, C₂-C₆ alkynyl, substituted C₂-C₆ alkynyl or N(R₃)(R₄);
Q₃ is O, S, N(R₅) or C(R₆)(R₇);
each R₃, R₄ R₅, R₆ and R₇ is, independently, H, C₁-C₆ alkyl, substituted C₁-C₆ alkyl or C₁-C₆ alkoxy;
M₃ is O, S, NR₁₄, C(R₁₅)(R₁₆), C(R₁₅)(R₁₆)C(R₁₇)(R₁₈), C(R₁₅)=C(R₁₇), OC(R₁₅)(R₁₆) or OC(R₁₅)(BX₂);
R₁₄ is H, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
R₁₅, R₁₆, R₁₇ and R₁₈ are each, independently, H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
Bx₁ is a heterocyclic base moiety;
or if Bx₂ is present then Bx₂ is a heterocyclic base moiety and Bx₁ is H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
J₄, J₅, J₆ and J₇ are each, independently, H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
or J₄ forms a bridge with one of J₅ or J₇ wherein said bridge comprises from 1 to 3 linked biradical groups selected from O, S, NR₁₉, C(R₂₀)(R₂₁), C(R₂₀)=C(R₂₁), C[=C(R₂₀)(R₂₁)] and C(=O) and the other two of J₅, J₆ and J₇ are each, independently, H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
each R₁₉, R₂₀ and R₂₁ is, independently, H, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
G is H, OH, halogen or O-[C(R₈)(R₉)]ₙ-[(C=O)ₘ-X₁]ⱼ-Z;
each R₈ and R₉ is, independently, H, halogen, C₁-C₆ alkyl or substituted C₁-C₆ alkyl;
X₁ is O, S or N(E₁);
Z is H, halogen, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl or N(E₂)(E₃);
E₁, E₂ and E₃ are each, independently, H, C₁-C₆ alkyl or substituted C₁-C₆ alkyl;
n is from 1 to about 6;
m is 0 or 1;
j is 0 or 1;
each substituted group comprises one or more optionally protected substituent groups independently selected from halogen, OJ₁, N(J₁)(J₂), =NJ₁, SJ₁, N₃, CN, OC(=X₂)J₁, OC(=X₂)N(J₁)(J₂) and C(=X₂)N(J₁)(J₂);
X₂ is O, S or NJ₃;
each J₁, J₂ and J₃ is, independently, H or C₁-C₆ alkyl;
when j is 1 then Z is other than halogen or N(E₂)(E₃); and
wherein said oligomeric compound comprises from 8 to 40 monomeric subunits and is hybridizable to at least a portion of a target nucleic acid.

In certain embodiments, M₃ is O, CH=CH, OCH₂ or OC(H)(Bx₂). In certain embodiments, M₃ is O.

In certain embodiments, J₄, J₅, J₆ and J₇ are each H. In certain embodiments, J₄ forms a bridge with one of J₅ or J₇.

In certain embodiments, A has one of the formulas: 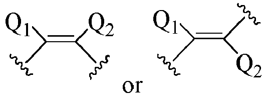 wherein:
Q₁ and Q₂ are each, independently, H, halogen, C₁-C₆ alkyl, substituted C₁-C₆ alkyl, C₁-C₆ alkoxy or substituted C₁-C₆ alkoxy. In certain embodiments, Q₁ and Q₂ are each H. In certain embodiments, Q₁ and Q₂ are each, independently, H or halogen. In certain embodiments, Q₁ and Q₂ is H and the other of Q₁ and Q₂ is F, CH₃ or OCH₃.

In certain embodiments, T₁ has the formula: 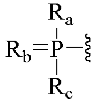 wherein:
Rₐ and R_{c} are each, independently, protected hydroxyl, protected thiol, C₁-C₆ alkyl, substituted C₁-C₆ alkyl, C₁-C₆ alkoxy, substituted C₁-C₆ alkoxy, protected amino or substituted amino; and
R_{b} is O or S. In certain embodiments, R_{b} is O and Rₐ and R_{c} are each, independently, OCH₃, OCH₂CH₃ or CH(CH₃)₂.

In certain embodiments, G is halogen, OCH₃, OCH₂F, OCHF₂, OCF₃, OCH₂CH₃, O(CH₂)₂F, OCH₂CHF₂, OCH₂CF₃, OCH₂-CH=CH₂, O(CH₂)₂-OCH₃, O(CH₂)₂-SCH₃, O(CH₂)₂-OCF₃, O(CH₂)₃-N(R₁₀)(R₁₁), O(CH₂)₂-ON(R₁₀)(R₁₁), O(CH₂)₂-O(CH₂)₂-N(R₁₀)(R₁₁), OCH₂C(=O)-N(R₁₀)(R₁₁), OCH₂C(=O)-N(R₁₂)-(CH₂)₂-N(R₁₀)(R₁₁) or O(CH₂)₂-N(R₁₂)-C(=NR₁₃)[N(R₁₀)(R₁₁)] wherein R₁₀, R₁₁, R₁₂ and R₁₃ are each, independently, H or C₁-C₆ alkyl. In certain embodiments, G is halogen, OCH₃, OCF₃, OCH₂CH₃, OCH₂CF₃, OCH₂-CH=CH₂, O(CH₂)₂-OCH₃, O(CH₂)₂-O(CH₂)₂-N(CH₃)₂, OCH₂C(=O)-N(H)CH₃, OCH₂C(=O)-N(H)-(CH₂)₂-N(CH₃)₂ or OCH₂-N(H)-C(=NH)NH₂. In certain embodiments, G is F, OCH₃ or O(CH₂)₂-OCH₃. In certain embodiments, G is O(CH₂)₂-OCH₃.

In certain embodiments, the 5'-terminal nucleoside has Formula IIe:

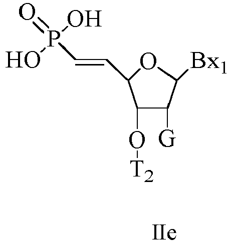

In certain embodiments, antisense compounds, including those particularly suitable for ssRNA comprise one or more type of modified sugar moieties and/or naturally occurring sugar moieties arranged along an oligonucleotide or region thereof in a defined pattern or sugar modification motif. Such motifs may include any of the sugar modifications discussed herein and/or other known sugar modifications.

In certain embodiments, the oligonucleotides comprise or consist of a region having uniform sugar modifications. In certain such embodiments, each nucleoside of the region comprises the same RNA-like sugar modification. In certain embodiments, each nucleoside of the region is a 2'-F nucleoside. In certain embodiments, each nucleoside of the region is a 2'-OMe nucleoside. In certain embodiments, each nucleoside of the region is a 2'-MOE nucleoside. In certain embodiments, each nucleoside of the region is a cEt nucleoside. In certain embodiments, each nucleoside of the region is an LNA nucleoside. In certain embodiments, the uniform region constitutes all or essentially all of the oligonucleotide. In certain embodiments, the region constitutes the entire oligonucleotide except for 1-4 terminal nucleosides.

In certain embodiments, oligonucleotides comprise one or more regions of alternating sugar modifications, wherein the nucleosides alternate between nucleotides having a sugar modification of a first type and nucleotides having a sugar modification of a second type. In certain embodiments, nucleosides of both types are RNA-like nucleosides. In certain embodiments the alternating nucleosides are selected from: 2'-OMe, 2'-F, 2'-MOE, LNA, and cEt. In certain embodiments, the alternating modificatios are 2'-F and 2'-OMe. Such regions may be contiguous or may be interupted by differently modified nucleosides or conjugated nucleosides.

In certain embodiments, the alternating region of alternating modifications each consist of a single nucleoside (i.e., the patern is (AB)ₓA_{y} wheren A is a nucleoside having a sugar modification of a first type and B is a nucleoside having a sugar modification of a second type; x is 1-20 and y is 0 or 1). In certan embodiments, one or more alternating regions in an alternating motif includes more than a single nucleoside of a type. For example, oligonucleotides may include one or more regions of any of the following nucleoside motifs:
AABBAA;
ABBABB;
AABAAB;
ABBABAABB;
ABABAA;
AABABAB;
ABABAA;
ABBAABBABABAA;
BABBAABBABABAA; or
ABABBAABBABABAA;
   wherein A is a nucleoside of a first type and B is a nucleoside of a second type. In certain embodiments, A and B are each selected from 2'-F, 2'-OMe, BNA, and MOE.

In certain embodiments, oligonucleotides having such an alternating motif also comprise a modified 5' terminal nucleoside, such as those of formula IIc or IIe.

In certain embodiments, oligonucleotides comprise a region having a 2-2-3 motif. Such regions comprises the following motif:

-(A)₂-(B)ₓ-(A)₂-(C)_{y}-(A)₃-

wherein: A is a first type of modifed nucleosde;
B and C, are nucleosides that are differently modified than A, however, B and C may have the same or different modifications as one another;
x and y are from 1 to 15.

In certain embodiments, A is a 2'-OMe modified nucleoside. In certain embodiments, B and C are both 2'-F modified nucleosides. In certain embodiments, A is a 2'-OMe modified nucleoside and B and C are both 2'-F modified nucleosides.

In certain embodiments, oligonucleosides have the following sugar motif:

5'-(Q)-(AB)ₓA_{y}-(D)_{z}

wherein:
Q is a nucleoside comprising a stabilized phosphate moiety. In certain embodiments, Q is a nucleoside having Formula IIc or IIe;
A is a first type of modifed nucleoside;
B is a second type of modified nucleoside;
D is a modified nucleoside comprising a modification different from the nucleoside adjacent to it. Thus, if y is 0, then D must be differently modified than B and if y is 1, then D must be differently modified than A. In certain embodiments, D differs from both A and B.
X is 5-15;
Y is 0 or 1;
Z is 0-4.

In certain embodiments, oligonucleosides have the following sugar motif:

5'-(Q)-(A)ₓ-(D)_{z}

wherein:
Q is a nucleoside comprising a stabilized phosphate moiety. In certain embodiments, Q is a nucleoside having Formula IIc or IIe;
A is a first type of modifed nucleoside;
D is a modified nucleoside comprising a modification different from A.
X is 11-30;
Z is 0-4.

In certain embodiments A, B, C, and D in the above motifs are selected from: 2'-OMe, 2'-F, 2'-MOE, LNA, and cEt. In certain embodiments, D represents terminal nucleosides. In certain embodiments, such terminal nucleosides are not designed to hybridize to the target nucleic acid (though one or more might hybridize by chance). In certiain embodiments, the nucleobase of each D nucleoside is adenine, regardless of the identity of the nucleobase at the corresponding position of the target nucleic acid. In certain embodiments the nucleobase of each D nucleoside is thymine.

In certain embodiments, antisense compounds, including those particularly suited for use as ssRNA comprise modified internucleoside linkages arranged along the oligonucleotide or region thereof in a defined pattern or modified internucleoside linkage motif. In certain embodiments, oligonucleotides comprise a region having an alternating internucleoside linkage motif. In certain embodiments, oligonucleotides comprise a region of uniformly modified internucleoside linkages. In certain such embodiments, the oligonucleotide comprises a region that is uniformly linked by phosphorothioate internucleoside linkages. In certain embodiments, the oligonucleotide is uniformly linked by phosphorothioate internucleoside linkages. In certain embodiments, each internucleoside linkage of the oligonucleotide is selected from phosphodiester and phosphorothioate. In certain embodiments, each internucleoside linkage of the oligonucleotide is selected from phosphodiester and phosphorothioate and at least one internucleoside linkage is phosphorothioate.

In certain embodiments, the oligonucleotide comprises at least 6 phosphorothioate internucleoside linkages. In certain embodiments, the oligonucleotide comprises at least 8 phosphorothioate internucleoside linkages. In certain embodiments, the oligonucleotide comprises at least 10 phosphorothioate internucleoside linkages. In certain embodiments, the oligonucleotide comprises at least one block of at least 6 consecutive phosphorothioate internucleoside linkages. In certain embodiments, the oligonucleotide comprises at least one block of at least 8 consecutive phosphorothioate internucleoside linkages. In certain embodiments, the oligonucleotide comprises at least one block of at least 10 consecutive phosphorothioate internucleoside linkages. In certain embodiments, the oligonucleotide comprises at least one block of at least one 12 consecutive phosphorothioate internucleoside linkages. In certain such embodiments, at least one such block is located at the 3' end of the oligonucleotide. In certain such embodiments, at least one such block is located within 3 nucleosides of the 3' end of the oligonucleotide.

Oligonucleotides having any of the various sugar motifs described herein, may have any linkage motif. For example, the oligonucleotides, including but not limited to those described above, may have a linkage motif selected from non-limiting the table below:

| 5' most linkage | Central region | 3'-region |
|---|---|---|
| PS | Alternating PO/PS | 6 PS |
| PS | Alternating PO/PS | 7 PS |
| PS | Alternating PO/PS | 8 PS |

### ii. siRNA compounds

In certain embodiments, antisense compounds are double-stranded RNAi compounds (siRNA). In such embodiments, one or both strands may comprise any modification motif described above for ssRNA. In certain embodiments, ssRNA compounds may be unmodified RNA. In certain embodiments, siRNA compounds may comprise unmodified RNA nucleosides, but modified internucleoside linkages.

Several embodiments relate to double-stranded compositions wherein each strand comprises a motif defined by the location of one or more modified or unmodified nucleosides. In certain embodiments, compositions are provided comprising a first and a second oligomeric compound that are fully or at least partially hybridized to form a duplex region and further comprising a region that is complementary to and hybridizes to a nucleic acid target. It is suitable that such a composition comprise a first oligomeric compound that is an antisense strand having full or partial complementarity to a nucleic acid target and a second oligomeric compound that is a sense strand having one or more regions of complementarity to and forming at least one duplex region with the first oligomeric compound.

The compositions of several embodiments modulate gene expression by hybridizing to a nucleic acid target resulting in loss of its normal function. In some embodiments, the target nucleic acid is GHR. In certain embodiment, the degradation of the targeted GHR is facilitated by an activated RISC complex that is formed with compositions of the invention.

Several embodiments are directed to double-stranded compositions wherein one of the strands is useful in, for example, influencing the preferential loading of the opposite strand into the RISC (or cleavage) complex. The compositions are useful for targeting selected nucleic acid molecules and modulating the expression of one or more genes. In some embodiments, the compositions of the present invention hybridize to a portion of a target RNA resulting in loss of normal function of the target RNA.

Certain embodiments are drawn to double-stranded compositions wherein both the strands comprises a hemimer motif, a fully modified motif, a positionally modified motif or an alternating motif. Each strand of the compositions of the present invention can be modified to fulfil a particular role in for example the siRNA pathway. Using a different motif in each strand or the same motif with different chemical modifications in each strand permits targeting the antisense strand for the RISC complex while inhibiting the incorporation of the sense strand. Within this model, each strand can be independently modified such that it is enhanced for its particular role. The antisense strand can be modified at the 5'-end to enhance its role in one region of the RISC while the 3'-end can be modified differentially to enhance its role in a different region of the RISC.

The double-stranded oligonucleotide molecules can be a double-stranded polynucleotide molecule comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof. The double-stranded oligonucleotide molecules can be assembled from two separate oligonucleotides, where one strand is the sense strand and the other is the antisense strand, wherein the antisense and sense strands are self-complementary (i.e. each strand comprises nucleotide sequence that is complementary to nucleotide sequence in the other strand; such as where the antisense strand and sense strand form a duplex or double-stranded structure, for example wherein the double-stranded region is about 15 to about 30, e.g., about 15, 16, 17, 18, 19, 20, 21, 22, 23, 24, 25, 26, 27, 28, 29 or 30 base pairs; the antisense strand comprises nucleotide sequence that is complementary to nucleotide sequence in a target nucleic acid molecule or a portion thereof and the sense strand comprises nucleotide sequence corresponding to the target nucleic acid sequence or a portion thereof (e.g., about 15 to about 25 or more nucleotides of the double-stranded oligonucleotide molecule are complementary to the target nucleic acid or a portion thereof). Alternatively, the double-stranded oligonucleotide is assembled from a single oligonucleotide, where the self-complementary sense and antisense regions of the siRNA are linked by means of a nucleic acid based or non-nucleic acid-based linker(s).

The double-stranded oligonucleotide can be a polynucleotide with a duplex, asymmetric duplex, hairpin or asymmetric hairpin secondary structure, having self-complementary sense and antisense regions, wherein the antisense region comprises nucleotide sequence that is complementary to nucleotide sequence in a separate target nucleic acid molecule or a portion thereof and the sense region having nucleotide sequence corresponding to the target nucleic acid sequence or a portion thereof. The double-stranded oligonucleotide can be a circular single-stranded polynucleotide having two or more loop structures and a stem comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide can be processed either in vivo or in vitro to generate an active siRNA molecule capable of mediating RNAi.

In certain embodiments, the double-stranded oligonucleotide comprises separate sense and antisense sequences or regions, wherein the sense and antisense regions are covalently linked by nucleotide or non-nucleotide linkers molecules as is known in the art, or are alternately non-covalently linked by ionic interactions, hydrogen bonding, van der waals interactions, hydrophobic interactions, and/or stacking interactions. In certain embodiments, the double-stranded oligonucleotide comprises nucleotide sequence that is complementary to nucleotide sequence of a target gene. In another embodiment, the double-stranded oligonucleotide interacts with nucleotide sequence of a target gene in a manner that causes inhibition of expression of the target gene.

As used herein, double-stranded oligonucleotides need not be limited to those molecules containing only RNA, but further encompasses chemically modified nucleotides and non-nucleotides. In certain embodiments, the short interfering nucleic acid molecules lack 2'-hydroxy (2'-OH) containing nucleotides. In certain embodiments short interfering nucleic acids optionally do not include any ribonucleotides (e.g., nucleotides having a 2'-OH group). Such double-stranded oligonucleotides that do not require the presence of ribonucleotides within the molecule to support RNAi can however have an attached linker or linkers or other attached or associated groups, moieties, or chains containing one or more nucleotides with 2'-OH groups. Optionally, double-stranded oligonucleotides can comprise ribonucleotides at about 5, 10, 20, 30, 40, or 50% of the nucleotide positions. As used herein, the term siRNA is meant to be equivalent to other terms used to describe nucleic acid molecules that are capable of mediating sequence specific RNAi, for example short interfering RNA (siRNA), double-stranded RNA (dsRNA), micro-RNA (miRNA), short hairpin RNA (shRNA), short interfering oligonucleotide, short interfering nucleic acid, short interfering modified oligonucleotide, chemically modified siRNA, post-transcriptional gene silencing RNA (ptgsRNA), and others. In addition, as used herein, the term RNAi is meant to be equivalent to other terms used to describe sequence specific RNA interference, such as post transcriptional gene silencing, translational inhibition, or epigenetics. For example, double-stranded oligonucleotides can be used to epigenetically silence genes at both the post-transcriptional level and the pre-transcriptional level. In a non-limiting example, epigenetic regulation of gene expression by siRNA molecules of the invention can result from siRNA mediated modification of chromatin structure or methylation pattern to alter gene expression (see, for example, Verdel et al., 2004, Science, 303, 672-676; Pal-Bhadra et al., 2004, Science, 303, 669-672; Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237).

It is contemplated that compounds and compositions of several embodiments provided herein can target GHR by a dsRNA-mediated gene silencing or RNAi mechanism, including, e.g., "hairpin" or stem-loop double-stranded RNA effector molecules in which a single RNA strand with self-complementary sequences is capable of assuming a double-stranded conformation, or duplex dsRNA effector molecules comprising two separate strands of RNA. In various embodiments, the dsRNA consists entirely of ribonucleotides or consists of a mixture of ribonucleotides and deoxynucleotides, such as the RNA/DNA hybrids disclosed, for example, by WO 00/63364, filed Apr. 19, 2000, or U.S. Ser. No. 60/130,377, filed Apr. 21, 1999. The dsRNA or dsRNA effector molecule may be a single molecule with a region of self-complementarity such that nucleotides in one segment of the molecule base pair with nucleotides in another segment of the molecule. In various embodiments, a dsRNA that consists of a single molecule consists entirely of ribonucleotides or includes a region of ribonucleotides that is complementary to a region of deoxyribonucleotides. Alternatively, the dsRNA may include two different strands that have a region of complementarity to each other.

In various embodiments, both strands consist entirely of ribonucleotides, one strand consists entirely of ribonucleotides and one strand consists entirely of deoxyribonucleotides, or one or both strands contain a mixture of ribonucleotides and deoxyribonucleotides. In certain embodiments, the regions of complementarity are at least 70, 80, 90, 95, 98, or 100% complementary to each other and to a target nucleic acid sequence. In certain embodiments, the region of the dsRNA that is present in a double-stranded conformation includes at least 19, 20, 21, 22, 23, 24, 25, 26, 27, 28, 29, 30, 50, 75,100, 200, 500, 1000, 2000 or 5000 nucleotides or includes all of the nucleotides in a cDNA or other target nucleic acid sequence being represented in the dsRNA. In some embodiments, the dsRNA does not contain any single stranded regions, such as single stranded ends, or the dsRNA is a hairpin. In other embodiments, the dsRNA has one or more single stranded regions or overhangs. In certain embodiments, RNA/DNA hybrids include a DNA strand or region that is an antisense strand or region (e.g, has at least 70, 80, 90, 95, 98, or 100% complementarity to a target nucleic acid) and an RNA strand or region that is a sense strand or region (e.g, has at least 70, 80, 90, 95, 98, or 100% identity to a target nucleic acid), and vice versa.

In various embodiments, the RNA/DNA hybrid is made in vitro using enzymatic or chemical synthetic methods such as those described herein or those described in WO 00/63364, filed Apr. 19, 2000, or U.S. Ser. No. 60/130,377, filed Apr. 21, 1999. In other embodiments, a DNA strand synthesized in vitro is complexed with an RNA strand made in vivo or in vitro before, after, or concurrent with the transformation of the DNA strand into the cell. In yet other embodiments, the dsRNA is a single circular nucleic acid containing a sense and an antisense region, or the dsRNA includes a circular nucleic acid and either a second circular nucleic acid or a linear nucleic acid (see, for example, WO 00/63364, filed Apr. 19, 2000, or U.S. Ser. No. 60/130,377, filed Apr. 21, 1999.) Exemplary circular nucleic acids include lariat structures in which the free 5' phosphoryl group of a nucleotide becomes linked to the 2' hydroxyl group of another nucleotide in a loop back fashion.

In other embodiments, the dsRNA includes one or more modified nucleotides in which the 2' position in the sugar contains a halogen (such as fluorine group) or contains an alkoxy group (such as a methoxy group) which increases the half-life of the dsRNA in vitro or in vivo compared to the corresponding dsRNA in which the corresponding 2' position contains a hydrogen or an hydroxyl group. In yet other embodiments, the dsRNA includes one or more linkages between adjacent nucleotides other than a naturally-occurring phosphodiester linkage. Examples of such linkages include phosphoramide, phosphorothioate, and phosphorodithioate linkages. The dsRNAs may also be chemically modified nucleic acid molecules as taught in U.S. Pat. No. 6,673,661. In other embodiments, the dsRNA contains one or two capped strands, as disclosed, for example, by WO 00/63364, filed Apr. 19, 2000, or U.S. Ser. No. 60/130,377, filed Apr. 21, 1999.

In other embodiments, the dsRNA can be any of the at least partially dsRNA molecules disclosed in WO 00/63364, as well as any of the dsRNA molecules described in U.S. Provisional Application 60/399,998; and U.S. Provisional Application 60/419,532, and PCT/US2003/033466. Any of the dsRNAs may be expressed in vitro or in vivo using the methods described herein or standard methods, such as those described in WO 00/63364.

### Occupancy

In certain embodiments, antisense compounds are not expected to result in cleavage or the target nucleic acid via RNase H or to result in cleavage or sequestration through the RISC pathway. In certain such embodiments, antisense activity may result from occupancy, wherein the presence of the hybridized antisense compound disrupts the activity of the target nucleic acid. In certain such embodiments, the antisense compound may be uniformly modified or may comprise a mix of modifications and/or modified and unmodified nucleosides.

### Target Nucleic Acids, Target Regions and Nucleotide Sequences

Nucleotide sequences that encode growth hormone receptor (GHR) targetable with the compounds provided herein include, without limitation, the following: GENBANK Accession No. NM_000163.4 (incorporated herein as SEQ ID NO: 1), GENBANK Accession No. NT_006576.16 truncated from nucleotides 42411001 to 42714000 (incorporated herein as SEQ ID NO: 2), GENBANK Accession No X06562.1 (incorporated herein as SEQ ID NO: 3), GENBANK Accession No. DR006395.1 (incorporated herein as SEQ ID NO: 4), GENBANK Accession No. DB052048.1 (incorporated herein as SEQ ID NO: 5), GENBANK Accession No. AF230800.1 (incorporated herein as SEQ ID NO: 6), the complement of GENBANK Accession No. AA398260.1 (incorporated herein as SEQ ID NO: 7), GENBANK Accession No. BC136496.1 (incorporated herein as SEQ ID NO: 8), GENBANK Accession No. NM_001242399.2 (incorporated herein as SEQ ID NO: 9), GENBANK Accession No. NM_001242400.2 (incorporated herein as SEQ ID NO: 10), GENBANK Accession No. NM_001242401.3 (incorporated herein as SEQ ID NO: 11), GENBANK Accession No. NM_001242402.2 (incorporated herein as SEQ ID NO: 12), GENBANK Accession No. NM_001242403.2 (incorporated herein as SEQ ID NO: 13), GENBANK Accession No. NM_001242404.2 (incorporated herein as SEQ ID NO: 14), GENBANK Accession No. NM_001242405.2 (incorporated herein as SEQ ID NO: 15), GENBANK Accession No. NM_001242406.2 (incorporated herein as SEQ ID NO: 16), GENBANK Accession No. NM_001242460.1 (incorporated herein as SEQ ID NO: 17), GENBANK Accession NM_001242461.1 (incorporated herein as SEQ ID NO: 18), GENBANK Accession No. NM_001242462.1 (incorporated herein as SEQ ID NO: 19), or GENBANK Accession No NW 001120958.1 truncated from nucleotides 4410000 to 4720000 (incorporated herein as SEQ ID NO: 2296).

### Hybridization

In some embodiments, hybridization occurs between an antisense compound disclosed herein and a GHR nucleic acid. The most common mechanism of hybridization involves hydrogen bonding (e.g., Watson-Crick, Hoogsteen or reversed Hoogsteen hydrogen bonding) between complementary nucleobases of the nucleic acid molecules.

Hybridization can occur under varying conditions. Stringent conditions are sequence-dependent and are determined by the nature and composition of the nucleic acid molecules to be hybridized.

Methods of determining whether a sequence is specifically hybridizable to a target nucleic acid are well known in the art. In certain embodiments, the antisense compounds provided herein are specifically hybridizable with a GHR nucleic acid.

### Complementarity

An antisense compound and a target nucleic acid are complementary to each other when a sufficient number of nucleobases of the antisense compound can hydrogen bond with the corresponding nucleobases of the target nucleic acid, such that a desired effect will occur (e.g., antisense inhibition of a target nucleic acid, such as a GHR nucleic acid).

Non-complementary nucleobases between an antisense compound and a GHR nucleic acid may be tolerated provided that the antisense compound remains able to specifically hybridize to a target nucleic acid. Moreover, an antisense compound may hybridize over one or more segments of a GHR nucleic acid such that intervening or adjacent segments are not involved in the hybridization event (e.g., a loop structure, mismatch or hairpin structure).

In certain aspects of the disclosure, the antisense compounds provided herein, or a specified portion thereof, are, or are at least, 70%, 80%, 85%, 86%, 87%, 88%, 89%, 90%, 91%, 92%, 93%, 94%, 95%, 96%, 97%, 98%, 99%, or 100% complementary to a GHR nucleic acid, a target region, target segment, or specified portion thereof. Percent complementarity of an antisense compound with a target nucleic acid can be determined using routine methods.

For example, an antisense compound in which 18 of 20 nucleobases of the antisense compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. As such, an antisense compound which is 18 nucleobases in length having four noncomplementary nucleobases which are flanked by two regions of complete complementarity with the target nucleic acid would have 77.8% overall complementarity with the target nucleic acid. Percent complementarity of an antisense compound with a region of a target nucleic acid can be determined routinely using BLAST programs (basic local alignment search tools) and PowerBLAST programs known in the art (Altschul et al., J. Mol. Biol., 1990, 215, 403 410; Zhang and Madden, Genome Res., 1997, 7, 649 656). Percent homology, sequence identity or complementarity, can be determined by, for example, the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482 489).

In certain embodiments, the antisense compounds provided herein, or specified portions thereof, are fully complementary (i.e. 100% complementary) to a target nucleic acid, or specified portion thereof. For example, an antisense compound may be fully complementary to a GHR nucleic acid, or a target region, or a target segment or target sequence thereof. As used herein, "fully complementary" means each nucleobase of an antisense compound is capable of precise base pairing with the corresponding nucleobases of a target nucleic acid. For example, a 20 nucleobase antisense compound is fully complementary to a target sequence that is 400 nucleobases long, so long as there is a corresponding 20 nucleobase portion of the target nucleic acid that is fully complementary to the antisense compound. Fully complementary can also be used in reference to a specified portion of the first and /or the second nucleic acid. For example, a 20 nucleobase portion of a 30 nucleobase antisense compound can be "fully complementary" to a target sequence that is 400 nucleobases long. The 20 nucleobase portion of the 30 nucleobase oligonucleotide is fully complementary to the target sequence if the target sequence has a corresponding 20 nucleobase portion wherein each nucleobase is complementary to the 20 nucleobase portion of the antisense compound. At the same time, the entire 30 nucleobase antisense compound may or may not be fully complementary to the target sequence, depending on whether the remaining 10 nucleobases of the antisense compound are also complementary to the target sequence.

The location of a non-complementary nucleobase may be at the 5' end or 3' end of the antisense compound. Alternatively, the non-complementary nucleobase or nucleobases may be at an internal position of the antisense compound. When two or more non-complementary nucleobases are present, they may be contiguous (i.e. linked) or non-contiguous. In one embodiment, a non-complementary nucleobase is located in the wing segment of a gapmer antisense oligonucleotide.

In certain aspects of the disclosure, antisense compounds that are, or are up to 11, 12, 13, 14, 15, 16, 17, 18, 19, or 20 nucleobases in length comprise no more than 4, no more than 3, no more than 2, or no more than 1 non-complementary nucleobase(s) relative to a target nucleic acid, such as a GHR nucleic acid, or specified portion thereof.

In certain aspects of the disclosure, antisense compounds that are, or are up to 11, 12, 13, 14, 15, 16, 17, 18, 19, 20, 21, 22, 23, 24, 25, 26, 27, 28, 29, or 30 nucleobases in length comprise no more than 6, no more than 5, no more than 4, no more than 3, no more than 2, or no more than 1 non-complementary nucleobase(s) relative to a target nucleic acid, such as a GHR nucleic acid, or specified portion thereof.

The antisense compounds provided also include those which are complementary to a portion of a target nucleic acid. As used herein, "portion" refers to a defined number of contiguous (i.e. linked) nucleobases within a region or segment of a target nucleic acid. A "portion" can also refer to a defined number of contiguous nucleobases of an antisense compound. In certain aspects of the disclosure, the antisense compounds, are complementary to at least an 8 nucleobase portion of a target segment. In certain aspects of the disclosure, the antisense compounds are complementary to at least a 9 nucleobase portion of a target segment. In certain aspects of the disclosure, the antisense compounds are complementary to at least a 10 nucleobase portion of a target segment. In certain aspects of the disclosure, the antisense compounds are complementary to at least an 11 nucleobase portion of a target segment. In certain aspects of the disclosure, the antisense compounds are complementary to at least a 12 nucleobase portion of a target segment. In certain aspects of the disclosure, the antisense compounds are complementary to at least a 13 nucleobase portion of a target segment. In certain aspects of the disclosure, the antisense compounds are complementary to at least a 14 nucleobase portion of a target segment. In certain aspects of the disclosure, the antisense compounds are complementary to at least a 15 nucleobase portion of a target segment. Also contemplated are antisense compounds that are complementary to at least a 9, 10, 11, 12, 13, 14, 15, 16, 17, 18, 19, 20, or more nucleobase portion of a target segment, or a range defined by any two of these values.

### Identity

The antisense compounds provided herein may also have a defined percent identity to a particular nucleotide sequence, SEQ ID NO, or compound represented by a specific Isis number, or portion thereof. As used herein, an antisense compound is identical to the sequence disclosed herein if it has the same nucleobase pairing ability. For example, a RNA which contains uracil in place of thymidine in a disclosed DNA sequence would be considered identical to the DNA sequence since both uracil and thymidine pair with adenine. Shortened and lengthened versions of the antisense compounds described herein as well as compounds having non-identical bases relative to the antisense compounds provided herein also are contemplated. The non-identical bases may be adjacent to each other or dispersed throughout the antisense compound. Percent identity of an antisense compound is calculated according to the number of bases that have identical base pairing relative to the sequence to which it is being compared.

In certain aspects of the disclosure, the antisense compounds, or portions thereof, are, or are at least, 70%, 75%, 80%, 85%, 90%, 95%, 96%, 97%, 98%, 99% or 100% identical to one or more of the antisense compounds or SEQ ID NOs, or a portion thereof, disclosed herein.

In certain embodiments, a portion of the antisense compound is compared to an equal length portion of the target nucleic acid. In certain aspects of the disclosure, an 8, 9, 10, 11, 12, 13, 14, 15, 16, 17, 18, 19, 20, 21, 22, 23, 24, or 25 nucleobase portion is compared to an equal length portion of the target nucleic acid.

In certain embodiments, a portion of the antisense oligonucleotide is compared to an equal length portion of the target nucleic acid. In certain aspects of the disclosure, an 8, 9, 10, 11, 12, 13, 14, 15, 16, 17, 18, 19, 20, 21, 22, 23, 24, or 25 nucleobase portion is compared to an equal length portion of the target nucleic acid.

### Modifications

A nucleoside is a base-sugar combination. The nucleobase (also known as base) portion of the nucleoside is normally a heterocyclic base moiety. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. Oligonucleotides are formed through the covalent linkage of adjacent nucleosides to one another, to form a linear polymeric oligonucleotide. Within the oligonucleotide structure, the phosphate groups are commonly referred to as forming the internucleoside linkages of the oligonucleotide.

Modifications to antisense compounds encompass substitutions or changes to internucleoside linkages, sugar moieties, or nucleobases. Modified antisense compounds are often preferred over native forms because of desirable properties such as, for example, enhanced cellular uptake, enhanced affinity for nucleic acid target, increased stability in the presence of nucleases, or increased inhibitory activity.

Chemically modified nucleosides may also be employed to increase the binding affinity of a shortened or truncated antisense oligonucleotide for its target nucleic acid. Consequently, comparable results can often be obtained with shorter antisense compounds that have such chemically modified nucleosides.

### Modified Internucleoside Linkages

The naturally occuring internucleoside linkage of RNA and DNA is a 3' to 5' phosphodiester linkage. Antisense compounds having one or more modified, i.e. non-naturally occurring, internucleoside linkages are often selected over antisense compounds having naturally occurring internucleoside linkages because of desirable properties such as, for example, enhanced cellular uptake, enhanced affinity for target nucleic acids, and increased stability in the presence of nucleases.

Oligonucleotides having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphosphonates, phosphoramidate, and phosphorothioates. Methods of preparation of phosphorous-containing and non-phosphorous-containing linkages are well known.

In certain embodiments, antisense compounds targeted to a GHR nucleic acid comprise one or more modified internucleoside linkages. In certain embodiments, the modified internucleoside linkages are phosphorothioate linkages. In certain embodiments, each internucleoside linkage of an antisense compound is a phosphorothioate internucleoside linkage.

### Modified Sugar Moieties

Antisense compounds can optionally contain one or more nucleosides wherein the sugar group has been modified. Such sugar modified nucleosides may impart enhanced nuclease stability, increased binding affinity, or some other beneficial biological property to the antisense compounds. In certain embodiments, nucleosides comprise chemically modified ribofuranose ring moieties. Examples of chemically modified ribofuranose rings include without limitation, addition of substitutent groups (including 5' and 2' substituent groups, bridging of non-geminal ring atoms to form bicyclic nucleic acids (BNA), replacement of the ribosyl ring oxygen atom with S, N(R), or C(R₁)(R₂) (R, R₁ and R₂ are each independently H, C₁-C₁₂ alkyl or a protecting group) and combinations thereof. Examples of chemically modified sugars include 2'-F-5'-methyl substituted nucleoside (see PCT International Application WO 2008/101157 Published on 8/21/08 for other disclosed 5',2'-bis substituted nucleosides) or replacement of the ribosyl ring oxygen atom with S with further substitution at the 2'-position (see published U.S. Patent Application US2005-0130923, published on June 16, 2005) or alternatively 5'-substitution of a BNA (see PCT International Application WO 2007/134181 Published on 11/22/07 wherein LNA is substituted with for example a 5'-methyl or a 5'-vinyl group).

Examples of nucleosides having modified sugar moieties include without limitation nucleosides comprising 5'-vinyl, 5'-methyl (*R* or *S*), 4'-S, 2'-F, 2'-OCH₃, 2'-OCH₂CH₃, 2'-OCH₂CH₂F and 2'-O(CH₂)₂OCH₃ substituent groups. The substituent at the 2' position can also be selected from allyl, amino, azido, thio, O-allyl, O-C₁-C₁₀ alkyl, OCF3, OCH2F, O(CH₂)₂SCH₃, O(CH₂)₂-O-N(Rₘ)(Rₙ), O-CH₂-C(=O)-N(Rₘ)(Rₙ), and O-CH₂-C(=O)-N(R₁)-(CH₂)₂-N(Rₘ)(Rₙ), where each R₁, Rₘ and Rₙ is, independently, H or substituted or unsubstituted C₁-C₁₀ alkyl.

As used herein, "bicyclic nucleosides" refer to modified nucleosides comprising a bicyclic sugar moiety. Examples of bicyclic nucleosides include without limitation nucleosides comprising a bridge between the 4' and the 2' ribosyl ring atoms. In certain embodiments, antisense compounds provided herein include one or more bicyclic nucleosides comprising a 4' to 2' bridge. Examples of such 4' to 2' bridged bicyclic nucleosides, include but are not limited to one of the formulae: 4'-(CH₂)-O-2' (LNA); 4'-(CH₂)-S-2'; 4'-(CH₂)₂-O-2' (ENA); 4'-CH(CH3)-O-2' (also referred to as constrained ethyl or cEt) and 4'-CH(CH₂OCH₃)-O-2' (and analogs thereof see U.S. Patent 7,399,845, issued on July 15,2008); 4'-C(CH₃)(CH₃)-O-2' (and analogs thereof see published International Application WO/2009/006478, published January 8, 2009); 4'-CH₂-N(OCH₃)-2' (and analogs thereof see published International Application WO/2008/150729, published December 11, 2008); 4'-CH₂-O-N(CH₃)-2' (see published U.S. Patent Application US2004-0171570, published September 2, 2004); 4'-CH₂-N(R)-O-2', wherein R is H, C₁-C₁₂ alkyl, or a protecting group (see U.S. Patent 7,427,672, issued on September 23, 2008); 4'-CH₂-C(H)(CH₃)-2' (see Chattopadhyaya et al., J. Org. Chem., 2009, 74, 118-134); and 4'-CH₂-C(=CH₂)-2' (and analogs thereof see published International Application WO 2008/154401, published on December 8, 2008).

Further reports related to bicyclic nucleosides can also be found in published literature (see for example: Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 2007,129(26) 8362-8379; Elayadi et al., Curr. Opinion Invest. Drugs, 2001, 2, 558-561; Braasch et al., Chem. Biol., 2001, 8, 1-7; and Orum et al., Curr. Opinion Mol. Ther., 2001, 3, 239-243; U.S. Patent Nos. 6,268,490; 6,525,191; 6,670,461; 6,770,748; 6,794,499; 7,034,133; 7,053,207; 7,399,845; 7,547,684; and 7,696,345; U.S. Patent Publication No. US2008-0039618; US2009-0012281; U.S. Patent Serial Nos. 60/989,574; 61/026,995; 61/026,998; 61/056,564; 61/086,231; 61/097,787; and 61/099,844; Published PCT International applications WO 1994/014226; WO 2004/106356; WO 2005/021570; WO 2007/134181; WO 2008/150729; WO 2008/154401; and WO 2009/006478. Each of the foregoing bicyclic nucleosides can be prepared having one or more stereochemical sugar configurations including for example α-L-ribofuranose and β-D-ribofuranose (see PCT international application PCT/DK98/00393, published on March 25, 1999 as WO 99/14226).

In certain embodiments, bicyclic sugar moieties of BNA nucleosides include, but are not limited to, compounds having at least one bridge between the 4' and the 2' position of the pentofuranosyl sugar moiety wherein such bridges independently comprises 1 or from 2 to 4 linked groups independently selected from - [C(Rₐ)(R_{b})]ₙ-, -C(Rₐ)=C(R_{b})-, -C(Rₐ)=N-, -C(=O)-, -C(=NRₐ)-, -C(=S)-, -O-, -Si(Rₐ)₂-, -S(=O)ₓ-, and -N(Rₐ)-;
wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In certain embodiments, the bridge of a bicyclic sugar moiety is -[C(Rₐ)(R_{b})]ₙ-, -[C(Rₐ)(R_{b})]ₙ-O-, -C(RₐR_{b})-N(R)-O- or -C(RₐR_{b})-O-N(R)-. In certain embodiments, the bridge is 4'-CH₂-2', 4'-(CH₂)₂-2', 4'-(CH₂)₃-2', 4'-CH₂-O-2', 4'-(CH₂)₂-O-2', 4'-CH₂-O-N(R)-2' and 4'-CH₂-N(R)-O-2'- wherein each R is, independently, H, a protecting group or C₁-C₁₂ alkyl.

In certain embodiments, bicyclic nucleosides are further defined by isomeric configuration. For example, a nucleoside comprising a 4'-2' methylene-oxy bridge, may be in the α-L configuration or in the β-D configuration. Previously, α-L-methyleneoxy (4'-CH₂-O-2') BNA's have been incorporated into antisense oligonucleotides that showed antisense activity (Frieden et al., Nucleic Acids Research, 2003, 21, 6365-6372).

In certain embodiments, bicyclic nucleosides include, but are not limited to, (A) α-L-methyleneoxy (4'-CH₂-O-2') BNA, (B) β-D-methyleneoxy (4'-CH₂-O-2') BNA, (C) ethyleneoxy (4'-(CH₂)₂-O-2') BNA , (D) aminooxy (4'-CH₂-O-N(R)-2') BNA, (E) oxyamino (4'-CH₂-N(R)-O-2') BNA, and (F) methyl(methyleneoxy) (4'-CH(CH₃)-O-2') BNA, (G) methylene-thio (4'-CH₂-S-2') BNA, (H) methylene-amino (4'-CH₂-N(R)-2') BNA, (I) methyl carbocyclic (4'-CH₂-CH(CH₃)-2') BNA, (J) propylene carbocyclic (4'-(CH₂)₃-2') BNA and (K) vinyl BNA as depicted below: 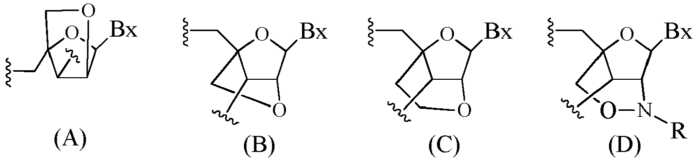 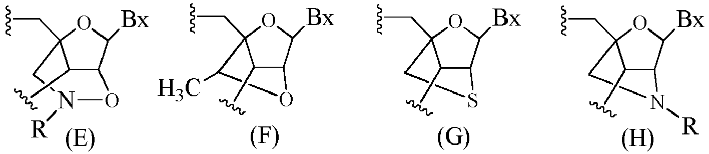 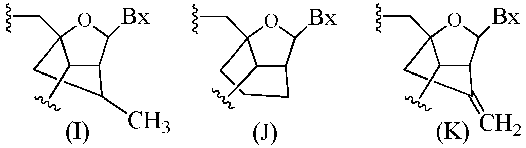 wherein Bx is the base moiety and R is independently H, a protecting group, C₁-C₁₂ alkyl or C₁-C₁₂ alkoxy.

In certain embodiments, bicyclic nucleosides are provided having Formula I: 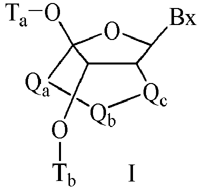 wherein:
Bx is a heterocyclic base moiety;
-Qₐ-Q_{b}-Q_{c}- is -CH₂-N(R_{c})-CH₂-, -C(=O)-N(R_{c})-CH₂-, -CH₂-O-N(R_{c})-, -CH₂-N(R_{c})-O- or -N(R_{c})-O-CH₂;
R_{c} is C₁-C₁₂ alkyl or an amino protecting group; and
Tₐ and T_{b} are each, independently H, a hydroxyl protecting group, a conjugate group, a reactive phosphorus group, a phosphorus moiety or a covalent attachment to a support medium.

In certain embodiments, bicyclic nucleosides are provided having Formula II: 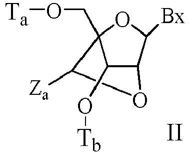 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Zₐ is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, substituted amide, thiol or substituted thio.

In one embodiment, each of the substituted groups is, independently, mono or poly substituted with substituent groups independently selected from halogen, oxo, hydroxyl, OJ_{c}, NJ_{c}J_{d}, SJ_{c}, N₃, OC(=X)J_{c}, and NJ_{c}C(=X)NJ_{c}J_{d}, wherein each J_{c}, J_{d} and Jₑ is, independently, H, C₁-C₆ alkyl, or substituted C₁-C₆ alkyl and X is O or NJ_{c}.

In certain embodiments, bicyclic nucleosides are provided having Formula III: 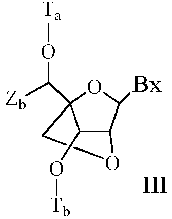 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Z_{b} is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl or substituted acyl (C(=O)-).

In certain embodiments, bicyclic nucleosides are provided having Formula IV: 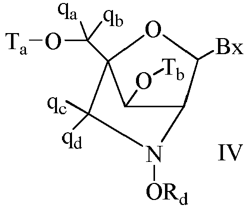 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
R_{d} is C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl;
each qₐ, q_{b}, qₑ and q_{d} is, independently, H, halogen, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl, C₁-C₆ alkoxyl, substituted C₁-C₆ alkoxyl, acyl, substituted acyl, C₁-C₆ aminoalkyl or substituted C₁-C₆ aminoalkyl;

In certain embodiments, bicyclic nucleosides are provided having Formula V: 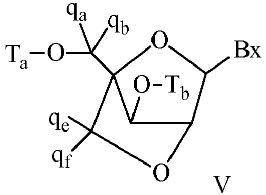 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
qₐ, q_{b}, qₑ and q_{f} are each, independently, hydrogen, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxy, substituted C₁-C₁₂ alkoxy, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ;
or qₑ and q_{f} together are =C(q_{g})(qₕ);
q_{g} and qₕ are each, independently, H, halogen, C₁-C₁₂ alkyl or substituted C₁-C₁₂ alkyl.

The synthesis and preparation of the methyleneoxy (4'-CH₂-O-2') BNA monomers adenine, cytosine, guanine, 5-methyl-cytosine, thymine and uracil, along with their oligomerization, and nucleic acid recognition properties have been described (Koshkin et al., Tetrahedron, 1998, 54, 3607-3630). BNAs and preparation thereof are also described in WO 98/39352 and WO 99/14226.

Analogs of methyleneoxy (4'-CH₂-O-2') BNA and 2'-thio-BNAs, have also been prepared (Kumar et al., Bioorg. Med. Chem. Lett., 1998, 8, 2219-2222). Preparation of locked nucleoside analogs comprising oligodeoxyribonucleotide duplexes as substrates for nucleic acid polymerases has also been described (Wengel et al., WO 99/14226). Furthermore, synthesis of 2'-amino-BNA, a novel comformationally restricted high-affinity oligonucleotide analog has been described in the art (Singh et al., J. Org. Chem., 1998, 63, 10035-10039). In addition, 2'-amino- and 2'-methylamino-BNA's have been prepared and the thermal stability of their duplexes with complementary RNA and DNA strands has been previously reported.

In certain embodiments, bicyclic nucleosides are provided having Formula VI: 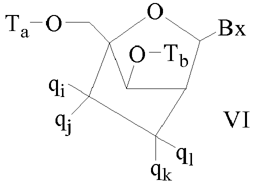 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
each qᵢ, qⱼ, qₖ and qₗ is, independently, H, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxyl, substituted C₁-C₁₂ alkoxyl, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ; and
qᵢ and qⱼ or qₗ and qₖ together are =C(q_{g})(qₕ), wherein q_{g} and qₕ are each, independently, H, halogen, C₁-C₁₂ alkyl or substituted C₁-C₁₂ alkyl.

One carbocyclic bicyclic nucleoside having a 4'-(CH₂)₃-2' bridge and the alkenyl analog bridge 4'-CH=CH-CH₂-2' have been described (Freier et al., Nucleic Acids Research, 1997, 25(22), 4429-4443 and Albaek et al., J. Org. Chem., 2006, 71, 7731-7740). The synthesis and preparation of carbocyclic bicyclic nucleosides along with their oligomerization and biochemical studies have also been described (Srivastava et al., J. Am. Chem. Soc., 2007,129(26), 8362-8379).

As used herein, "4'-2' bicyclic nucleoside" or "4' to 2' bicyclic nucleoside" refers to a bicyclic nucleoside comprising a furanose ring comprising a bridge connecting two carbon atoms of the furanose ring connects the 2' carbon atom and the 4' carbon atom of the sugar ring.

As used herein, "monocylic nucleosides" refer to nucleosides comprising modified sugar moieties that are not bicyclic sugar moieties. In certain embodiments, the sugar moiety, or sugar moiety analogue, of a nucleoside may be modified or substituted at any position.

As used herein, "2'-modified sugar" means a furanosyl sugar modified at the 2' position. In certain embodiments, such modifications include substituents selected from: a halide, including, but not limited to substituted and unsubstituted alkoxy, substituted and unsubstituted thioalkyl, substituted and unsubstituted amino alkyl, substituted and unsubstituted alkyl, substituted and unsubstituted allyl, and substituted and unsubstituted alkynyl. In certain embodiments, 2' modifications are selected from substituents including, but not limited to: O[(CH₂)ₙO]ₘCH₃, O(CH₂)ₙNH₂, O(CH₂)ₙCH₃, O(CH₂)ₙF, O(CH₂)ₙONH₂, OCH₂C(=O)N(H)CH₃, and O(CH₂)ₙON[(CH₂)ₙCH₃]₂, where n and m are from 1 to about 10. Other 2'-substituent groups can also be selected from: C₁-C₁₂ alkyl, substituted alkyl, alkenyl, alkynyl, alkaryl, aralkyl, O-alkaryl or O-aralkyl, SH, SCH₃, OCN, Cl, Br, CN, F, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving pharmacokinetic properties, or a group for improving the pharmacodynamic properties of an antisense compound, and other substituents having similar properties. In certain embodiments, modifed nucleosides comprise a 2'-MOE side chain (Baker et al., J. Biol. Chem., 1997, 272, 11944-12000). Such 2'-MOE substitution have been described as having improved binding affinity compared to unmodified nucleosides and to other modified nucleosides, such as 2'-*O*-methyl, *O-*propyl, and *O*-aminopropyl. Oligonucleotides having the 2'-MOE substituent also have been shown to be antisense inhibitors of gene expression with promising features for *in vivo* use (Martin, Helv. Chim. Acta, 1995, 78, 486-504; Altmann et al., Chimia, 1996, 50, 168-176; Altmann et al., Biochem. Soc. Trans., 1996, 24, 630-637; and Altmann et al., Nucleosides Nucleotides, 1997, 16, 917-926).

As used herein, a "modified tetrahydropyran nucleoside" or "modified THP nucleoside" means a nucleoside having a six-membered tetrahydropyran "sugar" substituted in for the pentofuranosyl residue in normal nucleosides (a sugar surrogate). Modified THP nucleosides include, but are not limited to, what is referred to in the art as hexitol nucleic acid (HNA), anitol nucleic acid (ANA), manitol nucleic acid (MNA) (see Leumann, Bioorg. Med. Chem., 2002, 10, 841-854) or fluoro HNA (F-HNA) having a tetrahydropyran ring system as illustrated below:

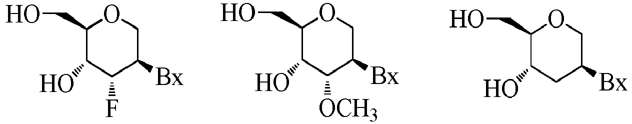

In certain embodiments, sugar surrogates are selected having Formula VII: 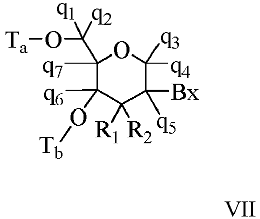 wherein independently for each of said at least one tetrahydropyran nucleoside analog of Formula VII:
Bx is a heterocyclic base moiety;
Tₐ and T_{b} are each, independently, an internucleoside linking group linking the tetrahydropyran nucleoside analog to the antisense compound or one of Tₐ and T_{b} is an internucleoside linking group linking the tetrahydropyran nucleoside analog to the antisense compound and the other of Tₐ and T_{b} is H, a hydroxyl protecting group, a linked conjugate group or a 5' or 3'-terminal group;
q₁, q₂, q₃, q₄, q₅, q₆ and q₇ are each independently, H, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl; and each of R₁ and R₂ is selected from hydrogen, hydroxyl, halogen, substituted or unsubstituted alkoxy, NJ₁J₂, SJ₁, N₃, OC(=X)J₁, OC(=X)NJ₁J₂, NJ₃C(=X)NJ₁J₂ and CN, wherein X is O, S or NJ₁ and each J₁, J₂ and J₃ is, independently, H or C₁-C₆ alkyl.

In certain embodiments, the modified THP nucleosides of Formula VII are provided wherein q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, THP nucleosides of Formula VII are provided wherein one of R₁ and R₂ is fluoro. In certain embodiments, R₁ is fluoro and R₂ is H; R₁ is methoxy and R₂ is H, and R₁ is methoxyethoxy and R₂ is H.

In certain embodiments, sugar surrogates comprise rings having more than 5 atoms and more than one heteroatom. For example nucleosides comprising morpholino sugar moieties and their use in oligomeric compounds has been reported (see for example: Braasch et al., Biochemistry, 2002, 41, 4503-4510; and U.S. Patents 5,698,685; 5,166,315; 5,185,444; and 5,034,506). As used here, the term "morpholino" means a sugar surrogate having the following formula:

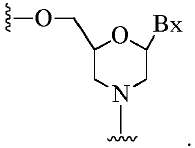

In certain embodiments, morpholinos may be modified, for example by adding or altering various substituent groups from the above morpholino structure. Such sugar surrogates are referred to herein as "modifed morpholinos."

Combinations of modifications are also provided without limitation, such as 2'-F-5'-methyl substituted nucleosides (see PCT International Application WO 2008/101157 published on 8/21/08 for other disclosed 5', 2'-bis substituted nucleosides) and replacement of the ribosyl ring oxygen atom with S and further substitution at the 2'-position (see published U.S. Patent Application US2005-0130923, published on June 16, 2005) or alternatively 5'-substitution of a bicyclic nucleic acid (see PCT International Application WO 2007/134181, published on 11/22/07 wherein a 4'-CH2-O-2' bicyclic nucleoside is further substituted at the 5' position with a 5'-methyl or a 5'-vinyl group). The synthesis and preparation of carbocyclic bicyclic nucleosides along with their oligomerization and biochemical studies have also been described (*see, e.g.,* Srivastava et al., J. Am. Chem. Soc. 2007, 129(26), 8362-8379).

In certain embodiments, antisense compounds comprise one or more modified cyclohexenyl nucleosides, which is a nucleoside having a six-membered cyclohexenyl in place of the pentofuranosyl residue in naturally occurring nucleosides. Modified cyclohexenyl nucleosides include, but are not limited to those described in the art (see for example commonly owned, published PCT Application WO 2010/036696, published on April 10, 2010, Robeyns et al., J. Am. Chem. Soc., 2008, 130(6), 1979-1984; Horváth et al., Tetrahedron Letters, 2007, 48, 3621-3623; Nauwelaerts et al., J. Am. Chem. Soc., 2007, 129(30), 9340-9348; Gu et al.,, Nucleosides, Nucleotides & Nucleic Acids, 2005, 24(5-7), 993-998; Nauwelaerts et al., Nucleic Acids Research, 2005, 33(8), 2452-2463; Robeyns et al., Acta Crystallographica, Section F: Structural Biology and Crystallization Communications, 2005, F61(6), 585-586; Gu et al., Tetrahedron, 2004, 60(9), 2111-2123; Gu et al., Oligonucleotides, 2003, 13(6), 479-489; Wang et al., J. Org. Chem., 2003, 68, 4499-4505; Verbeure et al., Nucleic Acids Research, 2001, 29(24), 4941-4947; Wang et al., J. Org. Chem., 2001, 66, 8478-82; Wang et al., Nucleosides, Nucleotides & Nucleic Acids, 2001, 20(4-7), 785-788; Wang et al., J. Am. Chem., 2000, 122, 8595-8602; Published PCT application, WO 06/047842; and Published PCT Application WO 01/049687). Certain modified cyclohexenyl nucleosides have Formula X. 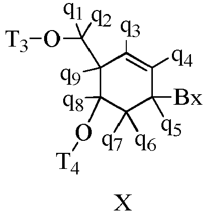 wherein independently for each of said at least one cyclohexenyl nucleoside analog of Formula X:
Bx is a heterocyclic base moiety;
T₃ and T₄ are each, independently, an internucleoside linking group linking the cyclohexenyl nucleoside analog to an antisense compound or one of T₃ and T₄ is an internucleoside linking group linking the tetrahydropyran nucleoside analog to an antisense compound and the other of T₃ and T₄ is H, a hydroxyl protecting group, a linked conjugate group, or a 5'-or 3'-terminal group; and
q₁, q₂, q₃, q₄, q₅, q₆, q₇, q₈ and q₉ are each, independently, H, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl or other sugar substituent group.

As used herein, "2'-modified" or "2'-substituted" refers to a nucleoside comprising a sugar comprising a substituent at the 2' position other than H or OH. 2'-modified nucleosides, include, but are not limited to, bicyclic nucleosides wherein the bridge connecting two carbon atoms of the sugar ring connects the 2' carbon and another carbon of the sugar ring; and nucleosides with non-bridging 2'substituents, such as allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O-N(Rₘ)(Rₙ), or O-CH₂-C(=O)-N(Rₘ)(Rₙ), where each Rₘ and Rₙ is, independently, H or substituted or unsubstituted C₁-C₁₀ alkyl. 2'-modifed nucleosides may further comprise other modifications, for example at other positions of the sugar and/or at the nucleobase.

As used herein, "2'-F" refers to a nucleoside comprising a sugar comprising a fluoro group at the 2' position of the sugar ring.

As used herein, "2'-OMe" or "2'-OCH₃" or "2'-O-methyl" each refers to a nucleoside comprising a sugar comprising an -OCH₃ group at the 2' position of the sugar ring.

As used herein, "MOE" or "2'-MOE" or "2'-OCH₂CH₂OCH₃" or "2'-O-methoxyethyl" each refers to a nucleoside comprising a sugar comprising a -OCH₂CH₂OCH₃ group at the 2' position of the sugar ring.

As used herein, "oligonucleotide" refers to a compound comprising a plurality of linked nucleosides. In certain embodiments, one or more of the plurality of nucleosides is modified. In certain embodiments, an oligonucleotide comprises one or more ribonucleosides (RNA) and/or deoxyribonucleosides (DNA).

Many other bicyclo and tricyclo sugar surrogate ring systems are also known in the art that can be used to modify nucleosides for incorporation into antisense compounds (see for example review article: Leumann, Bioorg. Med. Chem., 2002, 10, 841-854). Such ring systems can undergo various additional substitutions to enhance activity.

Methods for the preparations of modified sugars are well known to those skilled in the art. Some representative U.S. patents that teach the preparation of such modified sugars include without limitation, U.S.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,670,633; 5,700,920; 5,792,847 and 6,600,032 and International Application PCT/US2005/019219, filed June 2, 2005 and published as WO 2005/121371 on December 22, 2005.

In nucleotides having modified sugar moieties, the nucleobase moieties (natural, modified or a combination thereof) are maintained for hybridization with an appropriate nucleic acid target.

In certain embodiments, antisense compounds comprise one or more nucleosides having modified sugar moieties. In certain embodiments, the modified sugar moiety is 2'-MOE. In certain embodiments, the 2'-MOE modified nucleosides are arranged in a gapmer motif. In certain embodiments, the modified sugar moiety is a bicyclic nucleoside having a (4'-CH(CH₃)-O-2') bridging group. In certain embodiments, the (4'-CH(CH₃)-O-2') modified nucleosides are arranged throughout the wings of a gapmer motif.

### Modified Nucleobases

Nucleobase (or base) modifications or substitutions are structurally distinguishable from, yet functionally interchangeable with, naturally occurring or synthetic unmodified nucleobases. Both natural and modified nucleobases are capable of participating in hydrogen bonding. Such nucleobase modifications can impart nuclease stability, binding affinity or some other beneficial biological property to antisense compounds. Modified nucleobases include synthetic and natural nucleobases such as, for example, 5 -methylcytosine (5-me-C). Certain nucleobase substitutions, including 5-methylcytosine substitutions, are particularly useful for increasing the binding affinity of an antisense compound for a target nucleic acid. For example, 5-methylcytosine substitutions have been shown to increase nucleic acid duplex stability by 0.6-1.2°C (Sanghvi, Y.S., Crooke, S.T. and Lebleu, B., eds., Antisense Research and Applications, CRC Press, Boca Raton, 1993, pp. 276-278).

Additional modified nucleobases include 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine.

Heterocyclic base moieties can also include those in which the purine or pyrimidine base is replaced with other heterocycles, for example 7-deaza-adenine, 7-deazaguanosine, 2-aminopyridine and 2-pyridone. Nucleobases that are particularly useful for increasing the binding affinity of antisense compounds include 5-substituted pyrimidines, 6-azapyrimidines and N-2, N-6 and O-6 substituted purines, including 2 aminopropyladenine, 5-propynyluracil and 5-propynylcytosine.

In certain embodiments, antisense compounds targeted to a GHR nucleic acid comprise one or more modified nucleobases. In certain embodiments, shortened or gap-widened antisense oligonucleotides targeted to a GHR nucleic acid comprise one or more modified nucleobases. In certain embodiments, the modified nucleobase is 5-methylcytosine. In certain embodiments, each cytosine is a 5-methylcytosine.

### Conjugated Antisense compounds

Antisense compounds may be covalently linked to one or more moieties or conjugates which enhance the activity, cellular distribution or cellular uptake of the resulting antisense oligonucleotides. Typical conjugate groups include cholesterol moieties and lipid moieties. Additional conjugate groups include carbohydrates, phospholipids, biotin, phenazine, folate, phenanthridine, anthraquinone, acridine, fluoresceins, rhodamines, coumarins, and dyes.

Antisense compounds can also be modified to have one or more stabilizing groups that are generally attached to one or both termini of antisense compounds to enhance properties such as, for example, nuclease stability. Included in stabilizing groups are cap structures. These terminal modifications protect the antisense compound having terminal nucleic acid from exonuclease degradation, and can help in delivery and/or localization within a cell. The cap can be present at the 5'-terminus (5'-cap), or at the 3'-terminus (3'-cap), or can be present on both termini. Cap structures are well known in the art and include, for example, inverted deoxy abasic caps. Further 3' and 5'-stabilizing groups that can be used to cap one or both ends of an antisense compound to impart nuclease stability include those disclosed in WO 03/004602 published on January 16, 2003.

In certain embodiments, antisense compounds, including, but not limited to those particularly suited for use as ssRNA, are modified by attachment of one or more conjugate groups. In general, conjugate groups modify one or more properties of the attached oligonucleotide, including but not limited to pharmacodynamics, pharmacokinetics, stability, binding, absorption, cellular distribution, cellular uptake, charge and clearance. Conjugate groups are routinely used in the chemical arts and are linked directly or via an optional conjugate linking moiety or conjugate linking group to a parent compound such as an oligonucleotide. Conjugate groups includes without limitation, intercalators, reporter molecules, polyamines, polyamides, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins and dyes. Certain conjugate groups have been described previously, for example: cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, e.g., hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, e.g., do-decan-diol or undecyl residues (Saison-Behmoaras et al., EMBO J., 1991,10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-oxycholesterol moiety (Crooke et al., J. Pharmacol. Exp. Ther., 1996, 277, 923-937).

For additional conjugates including those useful for ssRNA and their placement within antisense compounds, see e.g., US Application No.; 61/583,963.

### In vitro testing of antisense oligonucleotides

Described herein are methods for treatment of cells with antisense oligonucleotides, which can be modified appropriately for treatment with other antisense compounds.

Cells may be treated with antisense oligonucleotides when the cells reach approximately 60-80% confluency in culture.

One reagent commonly used to introduce antisense oligonucleotides into cultured cells includes the cationic lipid transfection reagent LIPOFECTIN (Invitrogen, Carlsbad, CA). Antisense oligonucleotides may be mixed with LIPOFECTIN in OPTI-MEM 1 (Invitrogen, Carlsbad, CA) to achieve the desired final concentration of antisense oligonucleotide and a LIPOFECTIN concentration that may range from 2 to 12 ug/mL per 100 nM antisense oligonucleotide.

Another reagent used to introduce antisense oligonucleotides into cultured cells includes LIPOFECTAMINE (Invitrogen, Carlsbad, CA). Antisense oligonucleotide is mixed with LIPOFECTAMINE in OPTI-MEM 1 reduced serum medium (Invitrogen, Carlsbad, CA) to achieve the desired concentration of antisense oligonucleotide and a LIPOFECTAMINE concentration that may range from 2 to 12 ug/mL per 100 nM antisense oligonucleotide.

Another technique used to introduce antisense oligonucleotides into cultured cells includes electroporation.

Yet another technique used to introduce antisense oligonucleotides into cultured cells includes free uptake of the oligonucleotides by the cells.

Cells are treated with antisense oligonucleotides by routine methods. Cells may be harvested 16-24 hours after antisense oligonucleotide treatment, at which time RNA or protein levels of target nucleic acids are measured by methods known in the art and described herein. In general, when treatments are performed in multiple replicates, the data are presented as the average of the replicate treatments.

The concentration of antisense oligonucleotide used varies from cell line to cell line. Methods to determine the optimal antisense oligonucleotide concentration for a particular cell line are well known in the art. Antisense oligonucleotides are typically used at concentrations ranging from 1 nM to 300 nM when transfected with LIPOFECTAMINE. Antisense oligonucleotides are used at higher concentrations ranging from 625 to 20,000 nM when transfected using electroporation.

### RNA Isolation

RNA analysis can be performed on total cellular RNA or poly(A)+ mRNA. Methods of RNA isolation are well known in the art. RNA is prepared using methods well known in the art, for example, using the TRIZOL Reagent (Invitrogen, Carlsbad, CA) according to the manufacturer's recommended protocols.

### Certain Indications

Certain embodiments provided herein relate to methods of treating, preventing, or ameliorating a disease associated with excess growth hormone in a subject by administering a GHR specific inhibitor, such as an antisense compound or oligonucleotide targeted to GHR. In certain aspects, the disease associated with excess growth hormone is acromegaly. In certain aspects, the disease associated with excess growth hormone is gigantism.

Certain embodiments provide a method of treating, preventing, or ameliorating acromegaly in a subject by administering a GHR specific inhibitor, such as an antisense compound or oligonucleotide targeted to GHR. Acromegaly is a disease associated with excess growth hormone (GH). In over 90 percent of acromegaly patients, the overproduction of growth hormones is caused by a benign tumor of the pituitary gland, called an adenoma, which produces excess growth hormone and compresses surrounding brain tissues. Expansion of the adenoma can cause headaches and visual impairment that often accompany acromegaly. In some instances, acromegaly is caused by tumors of the pancreas, lungs, or adrenal glands that lead to an excess of GH, either by producing GH or by producing Growth Hormone Releasing Hormone (GHRH), the hormone that stimulates the pituitary to make GH.

Acromegaly most commonly affects adults in middle age and can result in severe disfigurement, complicating conditions, and premature death. Because of its pathogenesis and slow progression, acromegaly often goes undiagnosed until changes in external features become noticeable, such as changes in the face. Acromegaly is often associated with gigantism.

Features of acromegaly include soft tissue swelling resulting in enlargement of the hands, feet, nose, lips and ears, and a general thickening of the skin; soft tissue swelling of internal organs, such as the heart and kidney; vocal cord swelling resulting in a low voice and slow speech; expansion of the skull; pronounced eyebrow protrusion, often with ocular distension; pronounced lower jaw protrusion and enlargement of the tongue; teeth gapping; and carpal tunnel syndrome. In certain embodiments, any one or combination of these features of acromegaly can be treated, prevented, or ameliorated by administering a compound or composition targeted to GHR provided herein.

### EXAMPLES

### Non-limiting disclosure

While certain compounds, compositions and methods described herein have been described with specificity in accordance with certain embodiments, the following examples serve only to illustrate the compounds described herein and are not intended to limit the same.

### Example 1: Antisense inhibition of human growth hormone receptor in Hep3B cells by MOE gapmers

Antisense oligonucleotides were designed targeting a growth hormone receptor (GHR) nucleic acid and were tested for their effects on GHR mRNA in vitro. The antisense oligonucleotides were tested in a series of experiments that had similar culture conditions. The results for each experiment are presented in separate tables shown below. Cultured Hep3B cells at a density of 20,000 cells per well were transfected using electroporation with 4,500 nM antisense oligonucleotide. After a treatment period of approximately 24 hours, RNA was isolated from the cells and GHR mRNA levels were measured by quantitative real-time PCR. Human primer probe set RTS3437_MGB (forward sequence CGAGTTCAGTGAGGTGCTCTATGT, designated herein as SEQ ID NO: 2297; reverse sequence AAGAGCCATGGAAAGTAGAAATCTTC, designated herein as SEQ ID NO: 2298; probe sequence TTCCTCAGATGAGCCAATT, designated herein as SEQ ID NO: 2299) was used to measure mRNA levels. GHR mRNA levels were adjusted according to total RNA content, as measured by RIBOGREEN®. Results are presented as percent inhibition of GHR, relative to untreated control cells.

The newly designed chimeric antisense oligonucleotides in the Tables below were designed as 5-10-5 MOE or 3-10-4 MOE gapmers. The 5-10-5 MOE gapmers are 20 nucleosides in length, wherein the central gap segment comprises of ten 2'-deoxynucleosides and is flanked by wing segments on the 5' direction and the 3' direction comprising five nucleosides each. The 3-10-4 MOE gapmers are 17 nucleosides in length, wherein the central gap segment comprises of ten 2'-deoxynucleosides and is flanked by wing segments on the 5' direction and the 3' direction comprising three and four nucleosides respectively. Each nucleoside in the 5' wing segment and each nucleoside in the 3' wing segment has a 2'-MOE modification. The internucleoside linkages throughout each gapmer are phosphorothioate (P=S) linkages. All cytosine residues throughout each gapmer are 5-methylcytosines. "Start site" indicates the 5'-most nucleoside to which the gapmer is targeted in the human gene sequence. "Stop site" indicates the 3'-most nucleoside to which the gapmer is targeted human gene sequence. Each gapmer listed in the Tables below is targeted to either the human GHR mRNA, designated herein as SEQ ID NO: 1 (GENBANK Accession No. NM_000163.4) or the human GHR genomic sequence, designated herein as SEQ ID NO: 2 (GENBANK Accession No. NT_006576.16 truncated from nucleotides 42411001 to 42714000). 'n/a' indicates that the antisense oligonucleotide does not target that particular gene sequence with 100% complementarity. In case the sequence alignment for a target gene in a particular table is not shown, it is understood that none of the oligonucleotides presented in that table align with 100% complementarity with that target gene.

**Table 1**

| Inhibition of GHR mRNA by 5-10-5 MOE gapmers targeting exonic regions of SEQ ID NO: 1 and 2 | | | | | | | | |
|---|---|---|---|---|---|---|---|---|
| ISIS NO | SEQ ID NO: 1 Start Site | SEQ ID NO: 1 Stop Site | Target Region | Sequence | % inhibition | SEQ ID NO: 2 Start Site | SEQ ID NO: 2 Stop Site | SEQ ID NO |
| 523266 | 164 | 183 | Exon 1 | ACCTCCGAGCTTCGCCTCTG | 64 | 3040 | 3059 | 20 |
| 523267 | 171 | 190 | Exon-exon junction | CTGTAGGACCTCCGAGCTTC | 31 | n/a | n/a | 21 |
| 523268 | 178 | 197 | Exon-exon junction | TCCATACCTGTAGGACCTCC | 37 | n/a | n/a | 22 |
| 523271 | 206 | 225 | Exon 2 | TGCCAAGGTCAACAGCAGCT | 80 | 144990 | 145009 | 23 |
| 523272 | 213 | 232 | Exon 2 | CTGCCAGTGCCAAGGTCAAC | 53 | 144997 | 145016 | 24 |
| 523273 | 220 | 239 | Exon 2 | CTTGATCCTGCCAGTGCCAA | 49 | 145004 | 145023 | 25 |
| 523274 | 227 | 246 | Exon 2 | AGCATCACTTGATCCTGCCA | 67 | 145011 | 145030 | 26 |
| 523275 | 234 | 253 | Exon 2 | CAGAAAAAGCATCACTTGAT | 0 | 145018 | 145037 | 27 |
| 523276 | 241 | 260 | Exon 2 | TCACTTCCAGAAAAAGCATC | 1 | 145025 | 145044 | 28 |
| 523284 | 361 | 380 | Exon 4 | GTCTCTCGCTCAGGTGAACG | 48 | 268024 | 268043 | 29 |
| 523285 | 368 | 387 | Exon 4 | TGAAAAAGTCTCTCGCTCAG | 15 | 268031 | 268050 | 30 |
| 523286 | 375 | 394 | Exon 4 | AGTGGCATGAAAAAGTCTCT | 14 | 268038 | 268057 | 31 |
| 523287 | 382 | 401 | Exon 4 | TCTGTCCAGTGGCATGAAAA | 4 | 268045 | 268064 | 32 |
| 523301 | 625 | 644 | Exon 6 | GGATCTGGTTGCACTATTTC | 36 | n/a | n/a | 33 |
| 523302 | 632 | 651 | Exon 6 | AATGGGTGGATCTGGTTGCA | 28 | 278926 | 278945 | 34 |
| 523303 | 647 | 666 | Exon 6 | AGTCCAGTTGAGGGCAATGG | 26 | 278941 | 278960 | 35 |
| 523304 | 654 | 673 | Exon 6 | TCAGTAAAGTCCAGTTGAGG | 0 | 278948 | 278967 | 36 |
| 523305 | 675 | 694 | Exon 6 | GAATCCCAGTTAAACTGACG | 19 | 278969 | 278988 | 37 |
| 523306 | 682 | 701 | Exon 6 | TCTGCATGAATCCCAGTTAA | 39 | 278976 | 278995 | 38 |
| 523309 | 736 | 755 | Exon 6 | ATCCATCCTTTCTGAATATC | 34 | 279030 | 279049 | 39 |
| 523310 | 743 | 762 | Exon 6 | CAGAACCATCCATCCTTTCT | 31 | 279037 | 279056 | 40 |
| 523311 | 750 | 769 | Exon 6 | CATACTCCAGAACCATCCAT | 44 | 279044 | 279063 | 41 |
| 523312 | 757 | 776 | Exon 6 | TGAAGTTCATACTCCAGAAC | 23 | 279051 | 279070 | 42 |
| 523313 | 764 | 783 | Exon 6 | TTTGTATTGAAGTTCATACT | 6 | 279058 | 279077 | 43 |
| 523314 | 771 | 790 | Exon 6 | TTACTTCTTTGTATTGAAGT | 0 | 279065 | 279084 | 44 |
| 523315 | 778 | 797 | Exon 6 | GTTTCATTTACTTCTTTGTA | 3 | 279072 | 279091 | 45 |
| 523316 | 785 | 804 | Exon 6 | CCATTTAGTTTCATTTACTT | 0 | 279079 | 279098 | 46 |
| 523317 | 792 | 811 | Exon 4-exon 5 junction | TCATTTTCCATTTAGTTTCA | 19 | n/a | n/a | 47 |
| 523323 | 862 | 881 | Exon 7 | ACACGCACTTCATATTCCTT | 63 | 290360 | 290379 | 48 |
| 523324 | 869 | 888 | Exon 7 | GGATCTCACACGCACTTCAT | 80 | 290367 | 290386 | 49 |
| 523328 | 926 | 945 | Exon 7 | AAGTGTTACATAGAGCACCT | 56 | 290424 | 290443 | 50 |
| 523329 | 933 | 952 | Exon 7 | TCTGAGGAAGTGTTACATAG | 53 | 290431 | 290450 | 51 |
| 523330 | 957 | 976 | Exon 7 | CTTCTTCACATGTAAATTGG | 32 | 290455 | 290474 | 52 |
| 523331 | 964 | 983 | Exon 5-exon 6 junction | TAGAAATCTTCTTCACATGT | 4 | n/a | n/a | 53 |
| 523332 | 971 | 990 | Exon 5-exon 6 junction | TGGAAAGTAGAAATCTTCTT | 9 | n/a | n/a | 54 |
| 523333 | 978 | 997 | Exon 8 | AGAGCCATGGAAAGTAGAAA | 46 | 292532 | 292551 | 55 |
| 523334 | 985 | 1004 | Exon 8 | ATAATTAAGAGCCATGGAAA | 0 | 292539 | 292558 | 56 |

**Table 2**

| Inhibition of GHR mRNA by 5-10-5 MOE gapmers targeting exonic regions of SEQ ID NO: 1 and 2 | | | | | | | | |
|---|---|---|---|---|---|---|---|---|
| ISIS NO | SEQ ID NO: 1 Start Site | SEQ ID NO: 1 Stop Site | Target Region | Sequence | % inhibition | SEQ ID NO: 2 Start Site | SEQ ID NO: 2 Stop Site | SEQ ID NO |
| 523421 | 2072 | 2091 | exon 10 | CAGTTGGTCTGTGCTCACAT | 76 | 298489 | 298508 | 57 |
| 533002 | 207 | 226 | exon 2 | GTGCCAAGGTCAACAGCAGC | 63 | 144991 | 145010 | 58 |
| 533003 | 208 | 227 | exon 2 | AGTGCCAAGGTCAACAGCAG | 62 | 144992 | 145011 | 59 |
| 533004 | 225 | 244 | exon 2 | CATCACTTGATCCTGCCAGT | 53 | 145009 | 145028 | 60 |
| 533005 | 226 | 245 | exon 2 | GCATCACTTGATCCTGCCAG | 80 | 145010 | 145029 | 61 |
| 533006 | 228 | 247 | exon 2 | AAGCATCACTTGATCCTGCC | 75 | 145012 | 145031 | 62 |
| 533007 | 229 | 248 | exon 2 | AAAGCATCACTTGATCCTGC | 61 | 145013 | 145032 | 63 |
| 533019 | 867 | 886 | exon 7 | ATCTCACACGCACTTCATAT | 35 | 290365 | 290384 | 64 |
| 533020 | 868 | 887 | exon 7 | GATCTCACACGCACTTCATA | 47 | 290366 | 290385 | 65 |
| 533021 | 870 | 889 | exon 7 | TGGATCTCACACGCACTTCA | 86 | 290368 | 290387 | 66 |
| 533022 | 871 | 890 | exon 7 | TTGGATCTCACACGCACTTC | 70 | 290369 | 290388 | 67 |
| 533037 | 1360 | 1379 | exon 10 | TCCAGAATGTCAGGTTCACA | 59 | 297777 | 297796 | 68 |
| 533038 | 1361 | 1380 | exon 10 | CTCCAGAATGTCAGGTTCAC | 74 | 297778 | 297797 | 69 |
| 533039 | 1363 | 1382 | exon 10 | GTCTCCAGAATGTCAGGTTC | 45 | 297780 | 297799 | 70 |
| 533040 | 1364 | 1383 | exon 10 | AGTCTCCAGAATGTCAGGTT | 51 | 297781 | 297800 | 71 |
| 533042 | 1525 | 1544 | exon 10 | GCTTGGATAACACTGGGCTG | 41 | 297942 | 297961 | 72 |
| 533043 | 1526 | 1545 | exon 10 | TGCTTGGATAACACTGGGCT | 46 | 297943 | 297962 | 73 |
| 533044 | 1528 | 1547 | exon 10 | TCTGCTTGGATAACACTGGG | 55 | 297945 | 297964 | 74 |
| 533045 | 1529 | 1548 | exon 10 | CTCTGCTTGGATAACACTGG | 47 | 297946 | 297965 | 75 |
| 533046 | 1530 | 1549 | exon 10 | TCTCTGCTTGGATAACACTG | 54 | 297947 | 297966 | 76 |
| 533047 | 1744 | 1763 | exon 10 | CAGAGTGAGACCATTTCCGG | 47 | 298161 | 298180 | 77 |
| 533048 | 1745 | 1764 | exon 10 | GCAGAGTGAGACCATTTCCG | 60 | 298162 | 298181 | 78 |
| 533049 | 1747 | 1766 | exon 10 | TGGCAGAGTGAGACCATTTC | 65 | 298164 | 298183 | 79 |
| 533050 | 1748 | 1767 | exon 10 | TTGGCAGAGTGAGACCATTT | 47 | 298165 | 298184 | 80 |
| 533051 | 1749 | 1768 | exon 10 | CTTGGCAGAGTGAGACCATT | 30 | 298166 | 298185 | 81 |
| 533066 | 2685 | 2704 | exon 10 | CAGTGTGTAGTGTAATATAA | 53 | 299102 | 299121 | 82 |
| 533067 | 2686 | 2705 | exon 10 | ACAGTGTGTAGTGTAATATA | 68 | 299103 | 299122 | 83 |
| 533068 | 2688 | 2707 | exon 10 | ACACAGTGTGTAGTGTAATA | 62 | 299105 | 299124 | 84 |
| 533069 | 2689 | 2708 | exon 10 | TACACAGTGTGTAGTGTAAT | 55 | 299106 | 299125 | 85 |
| 533070 | 2690 | 2709 | exon 10 | GTACACAGTGTGTAGTGTAA | 50 | 299107 | 299126 | 86 |
| 533071 | 3205 | 3224 | exon 10 | TGTACCTTATTCCCTTCCTG | 68 | 299622 | 299641 | 87 |
| 533072 | 3206 | 3225 | exon 10 | TTGTACCTTATTCCCTTCCT | 61 | 299623 | 299642 | 88 |
| 533073 | 3208 | 3227 | exon 10 | TCTTGTACCTTATTCCCTTC | 60 | 299625 | 299644 | 89 |
| 533074 | 3209 | 3228 | exon 10 | TTCTTGTACCTTATTCCCTT | 46 | 299626 | 299645 | 90 |

**Table 3**

| Inhibition of GHR mRNA by 5-10-5 MOE gapmers targeting intronic and exonic regions of SEQ ID NO: 1 and 2 | | | | | | | | |
|---|---|---|---|---|---|---|---|---|
| ISIS NO | SEQ ID NO: 1 Start Site | SEQ ID NO: 1 Stop Site | Target Region | Sequence | % inhibition | SEQ NO: 2 Start Site | SEQ ID NO: 2 Stop Site | SEQ ID NO |
| 532174 | n/a | n/a | Intron 1 | ACATGTACCCAAACCAACAC | 37 | 18731 | 18750 | 91 |
| 533086 | 3210 | 3229 | Exon 10 | CTTCTTGTACCTTATTCCCT | 72 | 299627 | 299646 | 92 |
| 533087 | 3212 | 3231 | Exon 10 | TGCTTCTTGTACCTTATTCC | 77 | 299629 | 299648 | 93 |
| 533088 | 3213 | 3232 | Exon 10 | ATGCTTCTTGTACCTTATTC | 63 | 299630 | 299649 | 94 |
| 533089 | 3215 | 3234 | Exon 10 | AAATGCTTCTTGTACCTTAT | 67 | 299632 | 299651 | 95 |
| 533090 | 3216 | 3235 | Exon 10 | AAAATGCTTCTTGTACCTTA | 50 | 299633 | 299652 | 96 |
| 533091 | 3217 | 3236 | Exon 10 | CAAAATGCTTCTTGTACCTT | 44 | 299634 | 299653 | 97 |
| 533092 | 3518 | 3537 | Exon 10 | CTTCTGAATGCTTGCTTTGA | 29 | 299935 | 299954 | 98 |
| 533093 | 3519 | 3538 | Exon 10 | TCTTCTGAATGCTTGCTTTG | 47 | 299936 | 299955 | 99 |
| 533094 | 3521 | 3540 | Exon 10 | TTTCTTCTGAATGCTTGCTT | 63 | 299938 | 299957 | 100 |
| 533095 | 3522 | 3541 | Exon 10 | TTTTCTTCTGAATGCTTGCT | 51 | 299939 | 299958 | 101 |
| 533096 | 3523 | 3542 | Exon 10 | TTTTTCTTCTGAATGCTTGC | 34 | 299940 | 299959 | 102 |
| 533097 | 4041 | 4060 | Exon 10 | TGCGATAAATGGGAAATACT | 36 | 300458 | 300477 | 103 |
| 533098 | 4042 | 4061 | Exon 10 | CTGCGATAAATGGGAAATAC | 52 | 300459 | 300478 | 104 |
| 533099 | 4043 | 4062 | Exon 10 | TCTGCGATAAATGGGAAATA | 41 | 300460 | 300479 | 105 |
| 533100 | 4045 | 4064 | Exon 10 | GGTCTGCGATAAATGGGAAA | 40 | 300462 | 300481 | 106 |
| 533101 | 4046 | 4065 | Exon 10 | AGGTCTGCGATAAATGGGAA | 39 | 300463 | 300482 | 107 |
| 533102 | 4048 | 4067 | Exon 10 | AAAGGTCTGCGATAAATGGG | 34 | 300465 | 300484 | 108 |
| 533103 | 4049 | 4068 | Exon 10 | AAAAGGTCTGCGATAAATGG | 35 | 300466 | 300485 | 109 |
| 533104 | 4050 | 4069 | Exon 10 | AAAAAGGTCTGCGATAAATG | 15 | 300467 | 300486 | 110 |
| 533115 | n/a | n/a | Intron 1 | CATGAAGGCCACTCTTCCAA | 63 | 12777 | 12796 | 111 |
| 533116 | n/a | n/a | Intron 1 | CCATGAAGGCCACTCTTCCA | 78 | 12778 | 12797 | 112 |
| 533117 | n/a | n/a | Intron 1 | CCCATGAAGGCCACTCTTCC | 71 | 12779 | 12798 | 113 |
| 533118 | n/a | n/a | Intron 1 | TGCCCATGAAGGCCACTCTT | 66 | 12781 | 12800 | 114 |
| 533119 | n/a | n/a | Intron 1 | TTGCCCATGAAGGCCACTCT | 60 | 12782 | 12801 | 115 |
| 533120 | n/a | n/a | Intron 1 | GTTGCCCATGAAGGCCACTC | 74 | 12783 | 12802 | 116 |
| 533121 | n/a | n/a | Intron 1 | GGTCTTTCATGAATCAAGCT | 79 | 17927 | 17946 | 117 |
| 533122 | n/a | n/a | Intron 1 | TGGTCTTTCATGAATCAAGC | 83 | 17928 | 17947 | 118 |
| 533123 | n/a | n/a | Intron 1 | ATGGTCTTTCATGAATCAAG | 83 | 17929 | 17948 | 119 |
| 533124 | n/a | n/a | Intron 1 | TGATGGTCTTTCATGAATCA | 78 | 17931 | 17950 | 120 |
| 533125 | n/a | n/a | Intron 1 | CTGATGGTCTTTCATGAATC | 82 | 17932 | 17951 | 121 |
| 533126 | n/a | n/a | Intron 1 | GCTGATGGTCTTTCATGAAT | 74 | 17933 | 17952 | 122 |
| 533127 | n/a | n/a | Intron 1 | GTACCCAAACCAACACTAAT | 57 | 18727 | 18746 | 123 |
| 533128 | n/a | n/a | Intron 1 | TGTACCCAAACCAACACTAA | 65 | 18728 | 18747 | 124 |
| 533129 | n/a | n/a | Intron 1 | ATGTACCCAAACCAACACTA | 64 | 18729 | 18748 | 125 |
| 533130 | n/a | n/a | Intron 1 | GACATGTACCCAAACCAACA | 63 | 18732 | 18751 | 126 |
| 533131 | n/a | n/a | Intron 1 | AGACATGTACCCAAACCAAC | 81 | 18733 | 18752 | 127 |
| 533132 | n/a | n/a | Intron 1 | AGGAATGGAAAACCAAATAT | 49 | 26494 | 26513 | 128 |
| 533133 | n/a | n/a | Intron 1 | CAGGAATGGAAAACCAAATA | 74 | 26495 | 26514 | 129 |
| | | | | | | 121986 | 122005 | |
| 533134 | n/a | n/a | Intron 1 | TCAGGAATGGAAAACCAAAT | 73 | 26496 | 26515 | 130 |
| | | | | | | 121987 | 122006 | |
| 533135 | n/a | n/a | Intron 1 | ACTCAGGAATGGAAAACCAA | 77 | 26498 | 26517 | 131 |
| | | | | | | 113032 | 113051 | |
| | | | | | | 121989 | 122008 | |
| 533136 | n/a | n/a | Intron 1 | AACTCAGGAATGGAAAACCA | 79 | 26499 | 26518 | 132 |
| | | | | | | 113033 | 113052 | |
| | | | | | | 121990 | 122009 | |
| 533137 | n/a | n/a | Intron 1 | TAACTCAGGAATGGAAAACC | 67 | 26500 | 26519 | 133 |
| | | | | | | 113034 | 113053 | |
| | | | | | | 121991 | 122010 | |
| 533138 | n/a | n/a | Intron 1 | CAAAATTACTGCAGTCACAG | 67 | 39716 | 39735 | 134 |
| 533139 | n/a | n/a | Intron 1 | ACAAAATTACTGCAGTCACA | 81 | 39717 | 39736 | 135 |
| 533140 | n/a | n/a | Intron 1 | TACAAAATTACTGCAGTCAC | 81 | 39718 | 39737 | 136 |
| 533141 | n/a | n/a | Intron 1 | CATACAAAATTACTGCAGTC | 67 | 39720 | 39739 | 137 |
| 533142 | n/a | n/a | Intron 1 | ACATACAAAATTACTGCAGT | 48 | 39721 | 39740 | 138 |
| 533143 | n/a | n/a | Intron 1 | AACATACAAAATTACTGCAG | 53 | 39722 | 39741 | 139 |
| 533144 | n/a | n/a | Intron 1 | TTTTAGTATGAACCTTAAAA | 0 | 42139 | 42158 | 140 |
| 533145 | n/a | n/a | Intron 1 | CTTTTAGTATGAACCTTAAA | 38 | 42140 | 42159 | 141 |
| 533146 | n/a | n/a | Intron 1 | TCTTTTAGTATGAACCTTAA | 57 | 42141 | 42160 | 142 |
| 533147 | n/a | n/a | Intron 1 | AATCTTTTAGTATGAACCTT | 60 | 42143 | 42162 | 143 |
| 533148 | n/a | n/a | Intron 1 | CAATCTTTTAGTATGAACCT | 70 | 42144 | 42163 | 144 |
| 533149 | n/a | n/a | Intron 1 | ACAATCTTTTAGTATGAACC | 60 | 42145 | 42164 | 145 |
| 533150 | n/a | n/a | Intron 1 | AAGTTATGTGACTCTGAGCA | 67 | 43174 | 43193 | 146 |
| 533151 | n/a | n/a | Intron 1 | CAAGTTATGTGACTCTGAGC | 67 | 43175 | 43194 | 147 |
| 533152 | n/a | n/a | Intron 1 | TCAAGTTATGTGACTCTGAG | 63 | 43176 | 43195 | 148 |
| 533153 | n/a | n/a | Intron 1 | AGTTCTCCATTAGGGTTCTG | 83 | 50948 | 50967 | 149 |
| 533154 | n/a | n/a | Intron 1 | TAGTTCTCCATTAGGGTTCT | 76 | 50949 | 50968 | 150 |
| 533155 | n/a | n/a | Intron 1 | ATAGTTCTCCATTAGGGTTC | 51 | 50950 | 50969 | 151 |
| 533156 | n/a | n/a | Intron 1 | AAGCAGGTTGGCAGACAGAC | 79 | 53467 | 53486 | 152 |
| 533157 | n/a | n/a | Intron 1 | GAAGCAGGTTGGCAGACAGA | 60 | 53468 | 53487 | 153 |
| 533158 | n/a | n/a | Intron 1 | GGAAGCAGGTTGGCAGACAG | 67 | 53469 | 53488 | 154 |
| 533159 | n/a | n/a | Intron 1 | TCTTCTTGTGAGCTGGCTTC | 61 | 64882 | 64901 | 155 |
| 533160 | n/a | n/a | Intron 1 | GTCTTCTTGTGAGCTGGCTT | 83 | 64883 | 64902 | 156 |
| 533161 | n/a | n/a | Intron 1 | AGTCTTCTTGTGAGCTGGCT | 81 | 64884 | 64903 | 157 |

**Table 4**

| Inhibition of GHR mRNA by 5-10-5 MOE gapmers targeting intronic and exonic regions of SEQ ID NO: 1 and 2 | | | | | | | | |
|---|---|---|---|---|---|---|---|---|
| ISIS NO | SEQ ID NO: 1 Start Site | SEQ ID NO: 1 Stop Site | Target Region | Sequence | % inhibition | SEQ ID NO: 2 Start Site | SEQ ID NO: 2 Stop Site | SEQ ID NO |
| 533133 | n/a | n/a | Intron 1 | CAGGAATGGAAAACCAAATA | 76 | 26495 | 26514 | 129 |
| | | | | | | 121986 | 122005 | |
| 533134 | n/a | n/a | Intron 1 | TCAGGAATGGAAAACCAAAT | 83 | 26496 | 26515 | 130 |
| | | | | | | 121987 | 122006 | |
| 533174 | n/a | n/a | Intron 1 | TAAGTCTTCTTGTGAGCTGG | 73 | 64886 | 64905 | 158 |
| 533175 | n/a | n/a | Intron 1 | TTAAGTCTTCTTGTGAGCTG | 58 | 64887 | 64906 | 159 |
| 533176 | n/a | n/a | Intron 1 | ATTAAGTCTTCTTGTGAGCT | 51 | 64888 | 64907 | 160 |
| 533177 | n/a | n/a | Intron 1 | TCTCTTCCACTCACATCCAT | 72 | 65989 | 66008 | 161 |
| 533178 | n/a | n/a | Intron 1 | GTCTCTTCCACTCACATCCA | 86 | 65990 | 66009 | 162 |
| 533179 | n/a | n/a | Intron 1 | AGTCTCTTCCACTCACATCC | 80 | 65991 | 66010 | 163 |
| 533180 | n/a | n/a | Intron 1 | TAAGTATTTGTAGCAGTTGC | 31 | 78195 | 78214 | 164 |
| 533181 | n/a | n/a | Intron 1 | CTAAGTATTTGTAGCAGTTG | 14 | 78196 | 78215 | 165 |
| 533182 | n/a | n/a | Intron 1 | GCTAAGTATTTGTAGCAGTT | 59 | 78197 | 78216 | 166 |
| 533183 | n/a | n/a | Intron 1 | TGGCTAAGTATTTGTAGCAG | 34 | 78199 | 78218 | 167 |
| 533184 | n/a | n/a | Intron 1 | TTGGCTAAGTATTTGTAGCA | 18 | 78200 | 78219 | 168 |
| 533185 | n/a | n/a | Intron 1 | TTTGGCTAAGTATTTGTAGC | 21 | 78201 | 78220 | 169 |
| 533186 | n/a | n/a | Intron 1 | AAAATGTCAACAGTGCATAG | 61 | 80636 | 80655 | 170 |
| 533187 | n/a | n/a | Intron 1 | CAAAATGTCAACAGTGCATA | 78 | 80637 | 80656 | 171 |
| 533188 | n/a | n/a | Intron 1 | CCAAAATGTCAACAGTGCAT | 85 | 80638 | 80657 | 172 |
| 533189 | n/a | n/a | Intron 1 | GCCCAAAATGTCAACAGTGC | 82 | 80640 | 80659 | 173 |
| 533190 | n/a | n/a | Intron 1 | GGCCCAAAATGTCAACAGTG | 60 | 80641 | 80660 | 174 |
| 533191 | n/a | n/a | Intron 1 | TGGCCCAAAATGTCAACAGT | 31 | 80642 | 80661 | 175 |
| 533192 | n/a | n/a | Intron 1 | CAGAATCTTCTCTTTGGCCA | 66 | 98624 | 98643 | 176 |
| 533193 | n/a | n/a | Intron 1 | GCAGAATCTTCTCTTTGGCC | 81 | 98625 | 98644 | 177 |
| 533194 | n/a | n/a | Intron 1 | TGCAGAATCTTCTCTTTGGC | 72 | 98626 | 98645 | 178 |
| 533195 | n/a | n/a | Intron 1 | TTTGCAGAATCTTCTCTTTG | 33 | 98628 | 98647 | 179 |
| 533196 | n/a | n/a | Intron 1 | ATTTGCAGAATCTTCTCTTT | 27 | 98629 | 98648 | 180 |
| 533197 | n/a | n/a | Intron 1 | AATTTGCAGAATCTTCTCTT | 38 | 98630 | 98649 | 181 |
| 533198 | n/a | n/a | Intron 1 | ATAAAGCTATGCCATAAAGC | 37 | 99478 | 99497 | 182 |
| 533199 | n/a | n/a | Intron 1 | CATAAAGCTATGCCATAAAG | 14 | 99479 | 99498 | 183 |
| 533200 | n/a | n/a | Intron 1 | CCATAAAGCTATGCCATAAA | 30 | 99480 | 99499 | 184 |
| 533201 | n/a | n/a | Intron 1 | GACCATAAAGCTATGCCATA | 54 | 99482 | 99501 | 185 |
| 533202 | n/a | n/a | Intron 1 | TGACCATAAAGCTATGCCAT | 64 | 99483 | 99502 | 186 |
| 533203 | n/a | n/a | Intron 1 | CTGACCATAAAGCTATGCCA | 61 | 99484 | 99503 | 187 |
| 533204 | n/a | n/a | Intron 1 | CAAAAAGTTGAGCTGAGAAA | 0 | 101078 | 101097 | 188 |
| 533205 | n/a | n/a | Intron 1 | CCAAAAAGTTGAGCTGAGAA | 28 | 101079 | 101098 | 189 |
| 533206 | n/a | n/a | Intron 1 | CCCAAAAAGTTGAGCTGAGA | 52 | 101080 | 101099 | 190 |
| 533207 | n/a | n/a | Intron 1 | CACCCAAAAAGTTGAGCTGA | 60 | 101082 | 101101 | 191 |
| 533208 | n/a | n/a | Intron 1 | ACACCCAAAAAGTTGAGCTG | 34 | 101083 | 101102 | 192 |
| 533209 | n/a | n/a | Intron 1 | TACACCCAAAAAGTTGAGCT | 36 | 101084 | 101103 | 193 |
| 533210 | n/a | n/a | Intron 1 | CTTTTAATGGCACCCAAGCA | 41 | 103566 | 103585 | 194 |
| 533211 | n/a | n/a | Intron 1 | GCTTTTAATGGCACCCAAGC | 54 | 103567 | 103586 | 195 |
| 533212 | n/a | n/a | Intron 1 | TGCTTTTAATGGCACCCAAG | 67 | 103568 | 103587 | 196 |
| 533213 | n/a | n/a | Intron 1 | AATGCTTTTAATGGCACCCA | 73 | 103570 | 103589 | 197 |
| 533214 | n/a | n/a | Intron 1 | AAATGCTTTTAATGGCACCC | 73 | 103571 | 103590 | 198 |
| 533215 | n/a | n/a | Intron 1 | GAAATGCTTTTAATGGCACC | 41 | 103572 | 103591 | 199 |
| 533216 | n/a | n/a | Intron 1 | TAATTCTTAAGGGCCCTCTG | 36 | 106963 | 106982 | 200 |
| 533217 | n/a | n/a | Intron 1 | ATAATTCTTAAGGGCCCTCT | 45 | 106964 | 106983 | 201 |
| 533218 | n/a | n/a | Intron 1 | CATAATTCTTAAGGGCCCTC | 50 | 106965 | 106984 | 202 |
| 533219 | n/a | n/a | Intron 1 | AGCATAATTCTTAAGGGCCC | 48 | 106967 | 106986 | 203 |
| 533220 | n/a | n/a | Intron 1 | TAGCATAATTCTTAAGGGCC | 52 | 106968 | 106987 | 204 |
| 533221 | n/a | n/a | Intron 1 | TTAGCATAATTCTTAAGGGC | 28 | 106969 | 106988 | 205 |
| 533222 | n/a | n/a | Intron 1 | AGGAATGGAAAACCAAACAT | 13 | 113028 | 113047 | 206 |
| 533223 | n/a | n/a | Intron 1 | CAGGAATGGAAAACCAAACA | 64 | 113029 | 113048 | 207 |
| 533224 | n/a | n/a | Intron 1 | TCAGGAATGGAAAACCAAAC | 61 | 113030 | 113049 | 208 |
| 533225 | n/a | n/a | Intron 1 | AGGAATGGAAAACCAAATAC | 18 | 121985 | 122004 | 209 |
| 533226 | n/a | n/a | Intron 1 | CATGACTATGTTCTGGCAAG | 37 | 125591 | 125610 | 210 |
| 533227 | n/a | n/a | Intron 1 | ACATGACTATGTTCTGGCAA | 44 | 125592 | 125611 | 211 |
| 533228 | n/a | n/a | Intron 1 | CACATGACTATGTTCTGGCA | 63 | 125593 | 125612 | 212 |
| 533229 | n/a | n/a | Intron 1 | GTCACATGACTATGTTCTGG | 47 | 125595 | 125614 | 213 |
| 533230 | n/a | n/a | Intron 1 | GGTCACATGACTATGTTCTG | 49 | 125596 | 125615 | 214 |
| 533231 | n/a | n/a | Intron 1 | TGGTCACATGACTATGTTCT | 30 | 125597 | 125616 | 215 |
| 533232 | n/a | n/a | Intron 2 | CTGAATTCTGAGCTCTGGAA | 73 | 145428 | 145447 | 216 |
| 533233 | n/a | n/a | Intron 2 | CCTGAATTCTGAGCTCTGGA | 88 | 145429 | 145448 | 217 |
| 533234 | n/a | n/a | Intron 2 | GCCTGAATTCTGAGCTCTGG | 92 | 145430 | 145449 | 218 |
| 533235 | n/a | n/a | Intron 2 | AAGCCTGAATTCTGAGCTCT | 83 | 145432 | 145451 | 219 |
| 533236 | n/a | n/a | Intron 2 | CAAGCCTGAATTCTGAGCTC | 68 | 145433 | 145452 | 220 |
| 533237 | n/a | n/a | Intron 2 | ACAAGCCTGAATTCTGAGCT | 81 | 145434 | 145453 | 221 |
| 533238 | n/a | n/a | Intron 2 | GGATCTCAGCTGCAATTCTT | 72 | 146235 | 146254 | 222 |
| 533239 | n/a | n/a | Intron 2 | AGGATCTCAGCTGCAATTCT | 53 | 146236 | 146255 | 223 |
| 533240 | n/a | n/a | Intron 2 | GAGGATCTCAGCTGCAATTC | 69 | 146237 | 146256 | 224 |
| 533241 | n/a | n/a | Intron 2 | CAGAGGATCTCAGCTGCAAT | 69 | 146239 | 146258 | 225 |
| 533242 | n/a | n/a | Intron 2 | GCAGAGGATCTCAGCTGCAA | 76 | 146240 | 146259 | 226 |
| 533243 | 230 | 249 | Exon 2 | AAAAGCATCACTTGATCCTG | 23 | 145014 | 145033 | 227 |

**Table 5**

| Inhibition of GHR mRNA by 3-10-4 MOE gapmers targeting intronic and exonic regions of SEQ ID NO: 1 and 2 | | | | | | | | |
|---|---|---|---|---|---|---|---|---|
| ISIS NO | SEQ ID NO: 1 Start Site | SEQ ID NO: 1 Stop Site | Target Region | Sequence | % inhibition | SEQ ID NO: 2 Start Site | SEQ ID NO: 2 Stop Site | SEQ ID NO |
| 539284 | 206 | 222 | Exon 2 | CAAGGTCAACAGCAGCT | 62 | 144990 | 145006 | 228 |
| 539285 | 207 | 223 | Exon 2 | CCAAGGTCAACAGCAGC | 74 | 144991 | 145007 | 229 |
| 539286 | 208 | 224 | Exon 2 | GCCAAGGTCAACAGCAG | 73 | 144992 | 145008 | 230 |
| 539290 | 869 | 885 | Exon 7 | TCTCACACGCACTTCAT | 29 | 290367 | 290383 | 231 |
| 539291 | 870 | 886 | Exon 7 | ATCTCACACGCACTTCA | 51 | 290368 | 290384 | 232 |
| 539292 | 871 | 887 | Exon 7 | GATCTCACACGCACTTC | 56 | 290369 | 290385 | 233 |
| 539299 | n/a | n/a | Intron 1 | CTTTCATGAATCAAGCT | 63 | 17927 | 17943 | 234 |
| 539300 | n/a | n/a | Intron 1 | TCTTTCATGAATCAAGC | 49 | 17928 | 17944 | 235 |
| 539301 | n/a | n/a | Intron 1 | GTCTTTCATGAATCAAG | 61 | 17929 | 17945 | 236 |
| 539302 | n/a | n/a | Intron 1 | GGTCTTTCATGAATCAA | 93 | 17930 | 17946 | 237 |
| 539303 | n/a | n/a | Intron 1 | ATGGTCTTTCATGAATC | 74 | 17932 | 17948 | 238 |
| 539304 | n/a | n/a | Intron 1 | GATGGTCTTTCATGAAT | 56 | 17933 | 17949 | 239 |
| 539305 | n/a | n/a | Intron 1 | TATATCAATATTCTCCC | 42 | 21820 | 21836 | 240 |
| 539306 | n/a | n/a | Intron 1 | TTATATCAATATTCTCC | 33 | 21821 | 21837 | 241 |
| 539307 | n/a | n/a | Intron 1 | GTTATATCAATATTCTC | 12 | 21822 | 21838 | 242 |
| 539308 | n/a | n/a | Intron 1 | TTTCTTTAGCAATAGTT | 21 | 22518 | 22534 | 243 |
| 539309 | n/a | n/a | Intron 1 | CTTTCTTTAGCAATAGT | 38 | 22519 | 22535 | 244 |
| 539310 | n/a | n/a | Intron 1 | GCTTTCTTTAGCAATAG | 39 | 22520 | 22536 | 245 |
| 539311 | n/a | n/a | Intron 1 | AGGAATGGAAAACCAAA | 18 | 26497 | 26513 | 246 |
| | | | | | | 113031 | 113047 | |
| | | | | | | 121988 | 122004 | |
| 539312 | n/a | n/a | Intron 1 | CAGGAATGGAAAACCAA | 40 | 26498 | 26514 | 247 |
| | | | | | | 113032 | 113048 | |
| | | | | | | 121989 | 122005 | |
| 539313 | n/a | n/a | Intron 1 | TCAGGAATGGAAAACCA | 49 | 26499 | 26515 | 248 |
| | | | | | | 113033 | 113049 | |
| | | | | | | 121990 | 122006 | |
| 539314 | n/a | n/a | Intron 1 | TCTCCATTAGGGTTCTG | 87 | 50948 | 50964 | 249 |
| 539315 | n/a | n/a | Intron 1 | TTCTCCATTAGGGTTCT | 57 | 50949 | 50965 | 250 |
| 539316 | n/a | n/a | Intron 1 | GTTCTCCATTAGGGTTC | 73 | 50950 | 50966 | 251 |
| 539317 | n/a | n/a | Intron 1 | AGGTTGGCAGACAGACA | 73 | 53466 | 53482 | 252 |
| 539318 | n/a | n/a | Intron 1 | CAGGTTGGCAGACAGAC | 84 | 53467 | 53483 | 253 |
| 539319 | n/a | n/a | Intron 1 | GCAGGTTGGCAGACAGA | 85 | 53468 | 53484 | 254 |
| 539320 | n/a | n/a | Intron 1 | CTTCTTGTGAGCTGGCT | 87 | 64884 | 64900 | 255 |
| 539321 | n/a | n/a | Intron 1 | TCTTCTTGTGAGCTGGC | 89 | 64885 | 64901 | 256 |
| 539322 | n/a | n/a | Intron 1 | GTCTTCTTGTGAGCTGG | 87 | 64886 | 64902 | 257 |
| 539323 | n/a | n/a | Intron 1 | AGTCTTCTTGTGAGCTG | 70 | 64887 | 64903 | 258 |
| 539324 | n/a | n/a | Intron 1 | TCTTCCACTCACATCCA | 65 | 65990 | 66006 | 259 |
| 539325 | n/a | n/a | Intron 1 | CTCTTCCACTCACATCC | 78 | 65991 | 66007 | 260 |
| 539326 | n/a | n/a | Intron 1 | TCTCTTCCACTCACATC | 68 | 65992 | 66008 | 261 |
| 539327 | n/a | n/a | Intron 1 | GTCTCTTCCACTCACAT | 74 | 65993 | 66009 | 262 |
| 539328 | n/a | n/a | Intron 1 | ATAGATTTTGACTTCCC | 57 | 72107 | 72123 | 263 |
| 539329 | n/a | n/a | Intron 1 | CATAGATTTTGACTTCC | 35 | 72108 | 72124 | 264 |
| 539330 | n/a | n/a | Intron 1 | GCATAGATTTTGACTTC | 53 | 72109 | 72125 | 265 |
| 539331 | n/a | n/a | Intron 1 | AAAATGTCAACAGTGCA | 86 | 80639 | 80655 | 266 |
| 539332 | n/a | n/a | Intron 1 | CAAAATGTCAACAGTGC | 73 | 80640 | 80656 | 267 |
| 539333 | n/a | n/a | Intron 1 | CCAAAATGTCAACAGTG | 34 | 80641 | 80657 | 268 |
| 539334 | n/a | n/a | Intron 1 | CCCAAAATGTCAACAGT | 66 | 80642 | 80658 | 269 |
| 539335 | n/a | n/a | Intron 1 | CATGACTATGTTCTGGC | 67 | 125594 | 125610 | 270 |
| 539336 | n/a | n/a | Intron 1 | ACATGACTATGTTCTGG | 42 | 125595 | 125611 | 271 |
| 539337 | n/a | n/a | Intron 1 | CACATGACTATGTTCTG | 29 | 125596 | 125612 | 272 |
| 539338 | n/a | n/a | Intron 2 | GAATTCTGAGCTCTGGA | 77 | 145429 | 145445 | 273 |
| 539339 | n/a | n/a | Intron 2 | TGAATTCTGAGCTCTGG | 84 | 145430 | 145446 | 274 |
| 539340 | n/a | n/a | Intron 2 | CTGAATTCTGAGCTCTG | 80 | 145431 | 145447 | 275 |
| 539341 | n/a | n/a | Intron 2 | CCTGAATTCTGAGCTCT | 84 | 145432 | 145448 | 276 |
| 539342 | n/a | n/a | Intron 2 | GCCTGAATTCTGAGCTC | 84 | 145433 | 145449 | 277 |
| 539343 | n/a | n/a | Intron 2 | AGCCTGAATTCTGAGCT | 80 | 145434 | 145450 | 278 |
| 539344 | n/a | n/a | Intron 2 | ATATTGTAATTCTTGGT | 0 | 148059 | 148075 | 279 |
| 539345 | n/a | n/a | Intron 2 | GATATTGTAATTCTTGG | 20 | 148060 | 148076 | 280 |
| 539346 | n/a | n/a | Intron 2 | TGATATTGTAATTCTTG | 13 | 148061 | 148077 | 281 |
| 539347 | n/a | n/a | Intron 2 | CTGATATTGTAATTCTT | 8 | 148062 | 148078 | 282 |
| 539348 | n/a | n/a | Intron 2 | CCTGATATTGTAATTCT | 67 | 148063 | 148079 | 283 |
| 539349 | n/a | n/a | Intron 2 | GCCTGATATTGTAATTC | 73 | 148064 | 148080 | 284 |
| 539350 | n/a | n/a | Intron 2 | TGCCTGATATTGTAATT | 32 | 148065 | 148081 | 285 |
| 539351 | n/a | n/a | Intron 2 | AATTATGTGCTTTGCCT | 58 | 148907 | 148923 | 286 |
| 539352 | n/a | n/a | Intron 2 | CAATTATGTGCTTTGCC | 82 | 148908 | 148924 | 287 |
| 539353 | n/a | n/a | Intron 2 | TCAATTATGTGCTTTGC | 68 | 148909 | 148925 | 288 |
| 539354 | n/a | n/a | Intron 2 | GTCAATTATGTGCTTTG | 80 | 148910 | 148926 | 289 |
| 539355 | n/a | n/a | Intron 2 | GCCATCACCAAACACCA | 94 | 150972 | 150988 | 290 |
| 539356 | n/a | n/a | Intron 2 | TGCCATCACCAAACACC | 84 | 150973 | 150989 | 291 |
| 539357 | n/a | n/a | Intron 2 | TTGCCATCACCAAACAC | 74 | 150974 | 150990 | 292 |
| 539358 | n/a | n/a | Intron 2 | TGGTGACTCTGCCTGAT | 85 | 151387 | 151403 | 293 |
| 539359 | n/a | n/a | Intron 2 | CTGGTGACTCTGCCTGA | 86 | 151388 | 151404 | 294 |

**Table 6**

| Inhibition of GHR mRNA by 5-10-5 MOE gapmers targeting intron 1 of SEQ ID NO: 2 | | | | | |
|---|---|---|---|---|---|
| ISIS NO | Sequence | % inhibition | SEQ ID NO: 2 Start Site | SEQ ID NO: 2 Stop Site | SEQ ID NO |
| 523561 | TATTTCAGAAAGACTTTCTG | 11 | 10373 | 10392 | 295 |
| 523562 | AGGAAAAAATCAAGGAGTTA | 8 | 11173 | 11192 | 296 |
| 523563 | TATTTACTGAACACCTATTC | 12 | 11973 | 11992 | 297 |
| 523564 | GCCCATGAAGGCCACTCTTC | 70 | 12780 | 12799 | 298 |
| 523565 | ACCTATAAATAAAGTGAGGA | 0 | 13581 | 13600 | 299 |
| 523566 | GTTTCATAACCTGCTAATAA | 40 | 14451 | 14470 | 300 |
| 523567 | ATGTGCCTTACAGTTATCAG | 36 | 15251 | 15270 | 301 |
| 523568 | TTCTGAATTTAGAATTATAG | 0 | 16051 | 16070 | 302 |
| 523569 | GTTTATAATCTAGCAGTTAC | 26 | 17130 | 17149 | 303 |
| 523570 | GATGGTCTTTCATGAATCAA | 62 | 17930 | 17949 | 304 |
| 523571 | CATGTACCCAAACCAACACT | 65 | 18730 | 18749 | 305 |
| 523572 | TAAAATACAGCCTACATCAT | 0 | 19637 | 19656 | 306 |
| 523573 | CCATCACTACAACAAACTCA | 39 | 20451 | 20470 | 307 |
| 523574 | ATCTGAAATGATCCCCTTTC | 33 | 21283 | 21302 | 308 |
| 523575 | TGTTGCCCCTCCAAAAAGAC | 12 | 22144 | 22163 | 309 |
| 523576 | ATTAAAATTTTAAATGATGT | 0 | 22944 | 22963 | 310 |
| 523577 | CTCAGGAATGGAAAACCAAA | 71 | 26497 | 26516 | 311 |
| | | | 113031 | 113050 | |
| | | | 121988 | 122007 | |
| 523578 | AAAATTCTAGAAGATAACAT | 0 | 27838 | 27857 | 312 |
| 523579 | CTAGAAGTCCTAGCCAGAGT | 2 | 28748 | 28767 | 313 |
| 523580 | AACCGATATCACAGAAATAC | 0 | 29548 | 29567 | 314 |
| 523581 | AAGATAGACAGTAACATAAT | 0 | 30348 | 30367 | 315 |
| 523582 | GCACTACAAGAACTGCTTAA | 40 | 31172 | 31191 | 316 |
| 523583 | TTTCCAGACAAAGAATTCAG | 6 | 31978 | 31997 | 317 |
| 523584 | GTAGACAGCCTTTCTGGAAC | 20 | 32827 | 32846 | 318 |
| 523585 | CATCCTACATAGTGGCTGTG | 47 | 33635 | 33654 | 319 |
| 523586 | CAGAACAGTGTGTGGAGACT | 8 | 34452 | 34471 | 320 |
| 523587 | AGCTTTAAAAATACCTCTGC | 52 | 35466 | 35485 | 321 |
| 523588 | CCCAGGTACTTGCTCTCAGA | 22 | 36266 | 36285 | 322 |
| 523589 | TTACACCTGATTCTAGAAAT | 30 | 37066 | 37085 | 323 |
| 523590 | CTTTTCTCTACAACCTCACA | 34 | 38094 | 38113 | 324 |
| 523591 | TAGTAGTTTGAATTTCAAAG | 1 | 38909 | 38928 | 325 |
| 523592 | ATACAAAATTACTGCAGTCA | 60 | 39719 | 39738 | 326 |
| 523593 | GCCACTGCCAAAAAGGAGGA | 30 | 40519 | 40538 | 327 |
| 523594 | TGACAGAAACAGAGCTATGA | 33 | 41342 | 41361 | 328 |
| 523595 | ATCTTTTAGTATGAACCTTA | 65 | 42142 | 42161 | 329 |
| 523596 | AGTTATGTGACTCTGAGCAC | 63 | 43173 | 43192 | 330 |
| 523597 | ACTATGCCCTAGTTACTTCT | 29 | 43973 | 43992 | 331 |
| 523598 | TATAGTGGAAGTGATAGATC | 0 | 44812 | 44831 | 332 |
| 523599 | TGTTTTCTGAAATGGAATGT | 0 | 45733 | 45752 | 333 |
| 523600 | GCTGTAAATGTAATGAGTGT | 34 | 46553 | 46572 | 334 |
| 523601 | GAGAGAAGCCATGGCCCTAG | 20 | 47392 | 47411 | 335 |
| 523602 | CTCTCTTTCCCAGAACAAGA | 32 | 48210 | 48229 | 336 |
| 523603 | TCCAAAATGTCCAGTATAAT | 33 | 50072 | 50091 | 337 |
| 523604 | GTTCTCCATTAGGGTTCTGG | 74 | 50947 | 50966 | 338 |
| 523605 | TTAGTCACCCATCCACCACT | 41 | 51747 | 51766 | 339 |
| 523606 | CATGAATTCACCGAGTTAGG | 51 | 52573 | 52592 | 340 |
| 523607 | AGCAGGTTGGCAGACAGACA | 62 | 53466 | 53485 | 341 |
| 523608 | GAAAGACTTAAATTTTCACA | 0 | 54306 | 54325 | 342 |
| 523609 | TAGTAGAGGAAAAGGAGAAT | 0 | 55730 | 55749 | 343 |
| 523610 | AAACAGGGTCTGGAGTGGAC | 3 | 61243 | 61262 | 344 |
| 523611 | CAAGCTGATAATTAAAAAGA | 0 | 62462 | 62481 | 345 |
| 523612 | ATAAAGATACATTTTCTGGG | 8 | 63277 | 63296 | 346 |
| 523613 | CAGGATTCTTCCTGCCTGGC | 47 | 64085 | 64104 | 347 |
| 523614 | AAGTCTTCTTGTGAGCTGGC | 71 | 64885 | 64904 | 348 |
| 523615 | CTCTTCCACTCACATCCATT | 63 | 65988 | 66007 | 349 |
| 523616 | CCTATATCAGAAGACAAATG | 5 | 66806 | 66825 | 350 |
| 523617 | TCAAAACCCTGCCAAGGTAC | 44 | 67662 | 67681 | 351 |
| 523618 | TCATATTCTACTTCTGTTTA | 11 | 68462 | 68481 | 352 |
| 523619 | CATTCCAGTGTTTCAGTAAG | 13 | 69262 | 69281 | 353 |
| 523620 | GGCCTGGAATTAATCCTCAG | 49 | 70114 | 70133 | 354 |
| 523621 | AATGCCCTCTCCCTGTGCCT | 48 | 70925 | 70944 | 355 |
| 523622 | TTTATAATCAACCTTTGCTA | 9 | 71741 | 71760 | 356 |
| 523623 | ATATAACTACTTAAAATAAT | 0 | 72541 | 72560 | 357 |
| 523624 | TTAGCCAGGATATGGTTGCC | 50 | 73350 | 73369 | 358 |
| 523625 | CTACCTCCATCAAAGAAAAT | 0 | 74190 | 74209 | 359 |
| 523626 | GCATGCATAGATAAGTTTGA | 20 | 74990 | 75009 | 360 |
| 523627 | ATGAGAGTAAATGGATTTTC | 10 | 75790 | 75809 | 361 |
| 523628 | TTGGCAATCCTTGCTTAAAA | 34 | 76598 | 76617 | 362 |
| 523629 | GAATTAAGCCAGACTTATTT | 3 | 77398 | 77417 | 363 |
| 523630 | GGCTAAGTATTTGTAGCAGT | 55 | 78198 | 78217 | 364 |
| 523631 | TTGCCTGTGTGCAACTGGCG | 0 | 79005 | 79024 | 365 |
| 523632 | GTGGCCTTAGTAGGCCAGCT | 0 | 79827 | 79846 | 366 |
| 523633 | CCCAAAATGTCAACAGTGCA | 70 | 80639 | 80658 | 367 |
| 523634 | TTAAGCCTTCAATTTGAAAA | 0 | 81455 | 81474 | 368 |
| 523635 | TGCTCAGAAGGTTGAGCATA | 0 | 82261 | 82280 | 369 |
| 523636 | TTAATGCTTTCCCAAAGCTC | 35 | 83061 | 83080 | 370 |
| 523637 | AAAAGACTTCATACCTTTAC | 52 | 83884 | 83903 | 371 |

**Table 7**

| Inhibition of GHR mRNA by 5-10-5 MOE gapmers targeting intron 1 of SEQ ID NO: 2 | | | | | |
|---|---|---|---|---|---|
| ISIS NO | Sequence | % inhibition | SEQ ID NO: 2 Start Site | SEQ ID NO: 2 Stop Site | SEQ ID NO |
| 532146 | GGCCCCCTGGCACAACAGGA | 60 | 3097 | 3116 | 372 |
| 532147 | TCTAGGGTGATTCAGGTGGA | 62 | 4537 | 4556 | 373 |
| 532148 | CTTAGATTAATGCAAAACAA | 25 | 4875 | 4894 | 374 |
| 532149 | AGGCAGAGGAGGGTGGAACC | 34 | 6246 | 6265 | 375 |
| 532150 | AGTCTAATGAGATCTGATGG | 76 | 6499 | 6518 | 376 |
| 532151 | GCTGAAATGAGTTAAGACTT | 89 | 6737 | 6756 | 377 |
| 532152 | ACTTTGGACTGTGGATTTTT | 78 | 6765 | 6784 | 378 |
| 532153 | GCATATTTACACAATGCCTG | 84 | 6871 | 6890 | 379 |
| 532154 | GGAAATGCCTGGATGTCCAG | 27 | 7241 | 7260 | 380 |
| 532155 | CTGCTGATTTTGGAATGGAG | 68 | 10660 | 10679 | 381 |
| 532156 | ACTGAACACCTATTCTATGG | 51 | 11968 | 11987 | 382 |
| 532157 | TTTACTGAACACCTATTCTA | 23 | 11971 | 11990 | 383 |
| 532158 | CCCTCAAATTATCCACAAAC | 89 | 12053 | 12072 | 384 |
| 532159 | CTTCTAAATGTTTCCAAGGC | 63 | 12186 | 12205 | 385 |
| 532160 | TTACATCCTGTAGGCTAATT | 82 | 12469 | 12488 | 386 |
| 532161 | CCACTAGCCTGGCCAGACTT | 73 | 12487 | 12506 | 387 |
| 532162 | CTGGTAGATGATCTCAAGTT | 84 | 13351 | 13370 | 388 |
| 532163 | AAAGAATTGAGTTATAAATC | 23 | 13670 | 13689 | 389 |
| 532164 | AACTCATCTCTGGCCAGCAG | 89 | 14361 | 14380 | 390 |
| 532165 | CAACATCATTGTATTTTCTG | 33 | 14965 | 14984 | 391 |
| 532166 | TCTTAGCTTACCAATGAGGA | 81 | 15085 | 15104 | 392 |
| 532167 | TTCCCAGAGCCAAAGCTCAA | 77 | 15982 | 16001 | 393 |
| 532168 | TTTGGCCAATCCCAGCTTAT | 59 | 16253 | 16272 | 394 |
| 532169 | GTTTGCAAATCTTCATTCAC | 71 | 16447 | 16466 | 395 |
| 532170 | CAATAGTCCCTGAGGCTTGG | 74 | 16476 | 16495 | 396 |
| 532171 | TTTCCCCAGATTAAATGCCC | 85 | 17650 | 17669 | 397 |
| 532172 | TTCAATAATGCAGTTATTAT | 0 | 18308 | 18327 | 398 |
| 532173 | AAATTCTTGGGCTTAAGCAC | 69 | 18638 | 18657 | 399 |
| 532174 | ACATGTACCCAAACCAACAC | 71 | 18731 | 18750 | 91 |
| 532175 | TGATCCAAATTCAGTACCTA | 82 | 18752 | 18771 | 400 |
| 532176 | GATGATCCAAATTCAGTACC | 54 | 18754 | 18773 | 401 |
| 532177 | CAATATTCATCTTTATATTC | 25 | 19106 | 19125 | 402 |
| 532178 | ATTGCTCTTAAGATAAGTAA | 41 | 19661 | 19680 | 403 |
| 532179 | CAGCTCCCTGAATATCTCTT | 74 | 19783 | 19802 | 404 |
| 532180 | ACTTCACAAATATATTATAA | 0 | 19885 | 19904 | 405 |
| 532181 | GTACAGTCAACTTTACTTCA | 89 | 19899 | 19918 | 406 |
| 532182 | CAATTCCCACTCTTGTCAAC | 55 | 20288 | 20307 | 407 |
| 532183 | TCAACTGCTTTCTGGAGCAG | 66 | 21215 | 21234 | 408 |
| 532184 | ACTGCTGAGCACCTCCAAAA | 73 | 21454 | 21473 | 409 |
| 532185 | CTTAGATTCCTGGTTTATCA | 78 | 21587 | 21606 | 410 |
| 532186 | AGTTATATCAATATTCTCCC | 88 | 21820 | 21839 | 411 |
| 532187 | TATACCATCTTCCCCATAAA | 32 | 22038 | 22057 | 412 |
| 532188 | GGCTTTCTTTAGCAATAGTT | 86 | 22518 | 22537 | 413 |
| 532189 | TACCAGGGATGTAGGTTTAC | 82 | 29050 | 29069 | 414 |
| 532190 | TCACAGCTGAATTCTATCTG | 80 | 29323 | 29342 | 415 |
| 532191 | GGAGATGGACAAATTCCTGC | 77 | 29470 | 29489 | 416 |
| 532192 | CTAGACATGTCATCAAGACA | 19 | 30294 | 30313 | 417 |
| 532193 | CAAATTAATAAAACAATTAC | 10 | 30385 | 30404 | 418 |
| 532194 | TATTCTTATATCAGACAAAA | 30 | 30532 | 30551 | 419 |
| 532195 | TCAAGGGATCCCTGCCATTC | 32 | 32361 | 32380 | 420 |
| 532196 | CGTCAAGGGATCCCTGCCAT | 47 | 32363 | 32382 | 421 |
| 532197 | GGCACTCCCAGTCTCCAGCT | 83 | 34138 | 34157 | 422 |
| 532198 | TTTCTCCAGCAGAAGTGTCA | 60 | 34845 | 34864 | 423 |
| 532199 | AAGTCCTCTTCCGCCTCCCT | 82 | 36023 | 36042 | 424 |
| 532200 | GGAATTTACCAAAAACAGTT | 63 | 36721 | 36740 | 425 |
| 532201 | AGTTAGGTATTGTCCATTTT | 74 | 37032 | 37051 | 426 |
| 532202 | ACATGGGTATCTTCTAGGAA | 77 | 37111 | 37130 | 427 |
| 532203 | TCAGTTTCAGAGAGACAAAA | 41 | 37276 | 37295 | 428 |
| 532204 | TTTGCCAGGTCCTATGTCGA | 69 | 37656 | 37675 | 429 |
| 532205 | ATTCCCTTTTCTCTACAACC | 70 | 38099 | 38118 | 430 |
| 532206 | ATGATAAGAGCCAAGATTTG | 13 | 38994 | 39013 | 431 |
| 532207 | GAAAAAAGGTCCACTGTGGT | 49 | 40356 | 40375 | 432 |
| 532208 | CCTGTCCTGGAATAGTTTCA | 49 | 41164 | 41183 | 433 |
| 532209 | TAGAAAAGTAAATAAGGAAT | 15 | 41501 | 41520 | 434 |
| 532210 | TTATAAAACTATGCAATAGG | 0 | 41889 | 41908 | 435 |
| 532211 | TTATTTCATATTTCCAGAAA | 0 | 42675 | 42694 | 436 |
| 532212 | CATGAATTACAGCTAAAGAT | 20 | 42741 | 42760 | 437 |
| 532213 | TTGCATGTATGTGTTTCTGA | 62 | 43518 | 43537 | 438 |
| 532214 | TCAATCTCTTTATACCCTTA | 75 | 43765 | 43784 | 439 |
| 532215 | TCTTCAATCTCTTTATACCC | 58 | 43768 | 43787 | 440 |
| 532216 | CTATGCCCTAGTTACTTCTA | 47 | 43972 | 43991 | 441 |
| 532217 | AAAGAGAATCTCTTCCTTTT | 27 | 44070 | 44089 | 442 |
| 532218 | TCATTAAAGATTATTATAAC | 0 | 44222 | 44241 | 443 |
| 532219 | TTTGGATGAGTGGAAGGCTA | 0 | 44528 | 44547 | 444 |
| 532220 | GGAAATGGCCTTTTTCCTTA | 72 | 45400 | 45419 | 445 |
| 532221 | GGAGAAGCCCTCTGCCTGTA | 60 | 46477 | 46496 | 446 |
| 532222 | AAACCATATTGTCCACCAGA | 84 | 46510 | 46529 | 447 |

**Table 8**

| Inhibition of GHR mRNA by 5-10-5 MOE gapmers targeting intron 1 of SEQ ID NO: 2 | | | | | |
|---|---|---|---|---|---|
| ISIS NO | Sequence | % inhibition | SEQ ID NO: 2 Start Site | SEQ ID NO: 2 Stop Site | SEQ ID NO |
| 532223 | CTCAAACCATATTGTCCACC | 90 | 46513 | 46532 | 448 |
| 532224 | GTGTAAATAGTGACTTGTAC | 76 | 50123 | 50142 | 449 |
| 532225 | TGAGGCACAGGAAAGTTAAC | 52 | 50719 | 50738 | 450 |
| 532226 | AGCTATAGTTCTCCATTAGG | 74 | 50954 | 50973 | 451 |
| 532227 | TTACTTGCTGACTAAGCCAT | 69 | 51071 | 51090 | 452 |
| 532228 | GTTTGTCAACTCAACATCAA | 73 | 51215 | 51234 | 453 |
| 532229 | GACTATTTGTATATATATAC | 33 | 51491 | 51510 | 454 |
| 532230 | ATGACTATTTGTATATATAT | 11 | 51493 | 51512 | 455 |
| 532231 | ACTCTTCCTTATATTTGCTC | 76 | 51778 | 51797 | 456 |
| 532232 | ATACACTGACTTTTAACATT | 67 | 52039 | 52058 | 457 |
| 532233 | CTTAGAAACAGTAGTTTCAT | 42 | 52124 | 52143 | 458 |
| 532234 | CTGAGCTTTGCCTTAAGAAT | 79 | 52633 | 52652 | 459 |
| 532235 | CACCAGACAGCAGGTAGAGC | 81 | 53540 | 53559 | 460 |
| 532236 | GAGATGGAGTAGAAGGCAAA | 43 | 55926 | 55945 | 461 |
| 532237 | TAGGAAAGGAAGAATACACT | 33 | 63881 | 63900 | 462 |
| 532238 | TAGACCAGGAAGGGTGAGAG | 27 | 64376 | 64395 | 463 |
| 532239 | AAGTTGGATCTGGCATGCAT | 64 | 64574 | 64593 | 464 |
| 532240 | AAAGTTGGATCTGGCATGCA | 70 | 64575 | 64594 | 465 |
| 532241 | CCATAACTCTTCTAACTGGG | 84 | 64643 | 64662 | 466 |
| 532242 | ATATTAAAGTTTGAGAACTA | 37 | 65080 | 65099 | 467 |
| 532243 | CTTAACTACAAAATGCTGGA | 71 | 66164 | 66183 | 468 |
| 532244 | TGAGCAGCTGTCCTCAGTTC | 43 | 67061 | 67080 | 469 |
| 532245 | GAGTTCATAAAAGTTTTACT | 26 | 67251 | 67270 | 470 |
| 532246 | CTATCCACACCATTCCATAA | 73 | 69203 | 69222 | 471 |
| 532247 | AACATCTAAGTAATGCAAAC | 58 | 69223 | 69242 | 472 |
| 532248 | TTTGCATTCAAAGCCCTGGG | 91 | 69565 | 69584 | 473 |
| 532249 | TCCATATTATAGGCTATGAT | 73 | 69889 | 69908 | 474 |
| 532250 | ATTTTATGATAATGTAAAAC | 27 | 69942 | 69961 | 475 |
| 532251 | GAGATCACATTTTCTGAGTA | 50 | 70352 | 70371 | 476 |
| 532252 | ACCTCCCTAGGATTACCTCA | 56 | 71617 | 71636 | 477 |
| 532253 | AAAATCTGATTTATAATCAA | 40 | 71750 | 71769 | 478 |
| 532254 | AGCATAGATTTTGACTTCCC | 92 | 72107 | 72126 | 479 |
| 532255 | AAAGTCATATACACAGGTCT | 53 | 72584 | 72603 | 480 |
| 532256 | CTCATAGCAAATTCCCAGAA | 66 | 73689 | 73708 | 481 |
| 532257 | CAACATGGAGGCTAGCATGT | 55 | 74112 | 74131 | 482 |
| 532258 | AGACTAAGTGGCCTGAATGT | 52 | 74317 | 74336 | 483 |
| 532259 | ACCTACCATGTCACTCTCAA | 61 | 74418 | 74437 | 484 |
| 532260 | AACTTTCTTGTGTTTTATCA | 9 | 75511 | 75530 | 485 |
| 532261 | TTTGCAAGACAAAGAAATGA | 31 | 75915 | 75934 | 486 |
| 532262 | CATGCAAAGTGTTCCTCTTC | 63 | 76024 | 76043 | 487 |
| 532263 | AGTGCTTTGCTTTCTCTTAT | 79 | 76047 | 76066 | 488 |
| 532264 | GAACAAGAAACACTTGGTAA | 44 | 76555 | 76574 | 489 |
| 532265 | AGTGTTCCAATTAAATGGCA | 34 | 76643 | 76662 | 490 |
| 532266 | AAACAATGCCCTTGTAGTGA | 57 | 76703 | 76722 | 491 |
| 532267 | TATTCTAGGTTTTGAGGTGA | 60 | 76752 | 76771 | 492 |
| 532268 | ATATTCTAGGTTTTGAGGTG | 24 | 76753 | 76772 | 493 |
| 532269 | GTTTTCCATTCTTTAAGAAA | 41 | 76896 | 76915 | 494 |
| 532270 | AGCAATCCATTGATTGTATG | 59 | 77044 | 77063 | 495 |
| 532271 | AATTATGGCAAAATGGAAAA | 37 | 77076 | 77095 | 496 |
| 532272 | ACATTTGCTTATGAGACTAT | 62 | 77638 | 77657 | 497 |
| 532273 | GCAGAGATAATCCTATGATG | 42 | 77841 | 77860 | 498 |
| 532274 | TCCATCTGTTACCTCTCTGT | 77 | 78122 | 78141 | 499 |
| 532275 | TTTGCCTGAAGGGCAGAACC | 40 | 79478 | 79497 | 500 |
| 532276 | GAAAAAATCAGATTTTCACA | 0 | 79664 | 79683 | 501 |
| 532277 | AACTTAATTTAATCATTTCT | 0 | 79959 | 79978 | 502 |
| 532278 | TTTGGTTGTCATGAGTTGAG | 67 | 80756 | 80775 | 503 |
| 532279 | TTCCATCTCTAGGGCACTTT | 74 | 80900 | 80919 | 504 |
| 532280 | AGAGCTTATTTTCAAAATTC | 36 | 80920 | 80939 | 505 |
| 532281 | ATAAAGAGCAAACAAACATA | 42 | 81524 | 81543 | 506 |
| 532282 | TATAAATTCCTTGGTCTGAT | 33 | 82835 | 82854 | 507 |
| 532283 | AAAATATAAATTCCTTGGTC | 13 | 82839 | 82858 | 508 |
| 532284 | TTTTATAACAGCCTCTGACA | 38 | 82959 | 82978 | 509 |
| 532285 | AAAAGACCATGTTGCTTATT | 72 | 83179 | 83198 | 510 |
| 532286 | ATAGTCAGTCAGAATGTGGT | 72 | 83330 | 83349 | 511 |
| 532287 | TGCCTTAGCTTGGAAAAGAC | 78 | 83897 | 83916 | 512 |
| 532288 | AGGGCTAGCTGATGCCTCTC | 69 | 84026 | 84045 | 513 |
| 532289 | TTGGACTGGGCTCAAACAGA | 72 | 84381 | 84400 | 514 |
| 532290 | AAAGTCAGGCTAGAGGGACT | 49 | 85713 | 85732 | 515 |
| 532291 | TCCTTGTTTTCTTGTAATGA | 50 | 85945 | 85964 | 516 |
| 532292 | ACACCAGAGGAAGGAAATCA | 44 | 86554 | 86573 | 517 |
| 532293 | GATGTACACCATTTTGAATT | 15 | 86629 | 86648 | 518 |
| 532294 | TGCTCTGGCCTAGCCTATGT | 62 | 86901 | 86920 | 519 |
| 532295 | CAGAGGTGTCTCCCAAGAAA | 60 | 89940 | 89959 | 520 |
| 532296 | AAAGAGAATGGATCAAAGCT | 36 | 91930 | 91949 | 521 |
| 532297 | GATTTGCAGAACAAATCTTG | 37 | 93332 | 93351 | 522 |
| 532298 | TGGTTATGAAGGTTGGACCA | 52 | 94839 | 94858 | 523 |
| 532299 | TGGCTAATTAATGGGCAATT | 63 | 95292 | 95311 | 524 |

**Table 9**

| Inhibition of GHR mRNA by 5-10-5 MOE gapmers targeting intron 1 of SEQ ID NO: 2 | | | | | |
|---|---|---|---|---|---|
| ISIS NO | Sequence | % inhibition | SEQ ID NO: 2 Start Site | SEQ ID NO: 2 Stop Site | SEQ ID NO |
| 532300 | CTGTGCCATATTGCCTCTAA | 87 | 95471 | 95490 | 525 |
| 532301 | GATTTCAACCAGCTCACCTG | 48 | 95510 | 95529 | 526 |
| 532302 | GCAAAAGGGAACCCTGAAGC | 71 | 95564 | 95583 | 527 |
| 532303 | CTAAGTGTTATAACAAACAC | 43 | 96137 | 96156 | 528 |
| 532304 | GTCCATTGGTATAAAACTCA | 84 | 96282 | 96301 | 529 |
| 532305 | TTTCAATACAATAAGATTTA | 34 | 96793 | 96812 | 530 |
| 532306 | GTCCTTAGACCCCTCAATGG | 62 | 96987 | 97006 | 531 |
| 532307 | GAGGATTTATTCATCTAGGC | 68 | 97806 | 97825 | 532 |
| 532308 | CAGTGGGAGGATCAGATATC | 46 | 97870 | 97889 | 533 |
| 532309 | ATCCCATCCAGCAGCTGGAC | 67 | 98132 | 98151 | 534 |
| 532310 | AACTTGGGATGAGTTACTGA | 56 | 98653 | 98672 | 535 |
| 532311 | GAAGGCTACCTAAAAGAAAT | 43 | 98810 | 98829 | 536 |
| 532312 | AAAGAAATATTCACAACATT | 39 | 99096 | 99115 | 537 |
| 532313 | ATGCTTATACTGCTGCTGTA | 69 | 99791 | 99810 | 538 |
| 532314 | TCCTCACTTCAATCACCTTT | 70 | 99819 | 99838 | 539 |
| 532315 | CTCTTTCTTCATAAATAAGT | 33 | 100809 | 100828 | 540 |
| 532316 | TGGTAATCTGTGTCCCTTTA | 96 | 101242 | 101261 | 541 |
| 532317 | TAATAAAAAAGTTTGAAACA | 41 | 102549 | 102568 | 542 |
| 532318 | GGTGGTGGCAAGAGAAAAAT | 56 | 103015 | 103034 | 543 |
| 532319 | CAAAAGGCCCTTTTTACATG | 28 | 103034 | 103053 | 544 |
| 532320 | ACTCTACTGGTACCAATTTA | 31 | 103173 | 103192 | 545 |
| 532321 | TCTGAACTTTTATGCTCTGT | 76 | 103606 | 103625 | 546 |
| 532322 | AACTTTTGCCTGGGCATCCA | 16 | 104067 | 104086 | 547 |
| 532323 | TGACTCCATGTCTCACATCC | 66 | 104392 | 104411 | 548 |
| 532324 | TTACTTCCTAGATACAACAG | 53 | 104541 | 104560 | 549 |
| 532325 | CTGGCCCCCATGATTCAATT | 44 | 104835 | 104854 | 550 |
| 532326 | AAGACTGGCCCCCATGATTC | 49 | 104839 | 104858 | 551 |
| 532327 | TGTCACTGGTCTGTGTATTT | 60 | 106233 | 106252 | 552 |
| 532328 | ACAGAGTAGATTTAGCATAA | 23 | 106980 | 106999 | 553 |
| 532329 | TAAACAGGTGTACTATTACA | 27 | 107030 | 107049 | 554 |
| 532330 | GCTTTATCAACTAAGTTTAT | 22 | 107716 | 107735 | 555 |
| 532331 | CAGAACTTCTTTTAAAATTG | 8 | 107763 | 107782 | 556 |
| 532332 | GAATACAGACATACCTTGAA | 25 | 108514 | 108533 | 557 |
| 532333 | CCATGACAACAATTTCAGAG | 58 | 109486 | 109505 | 558 |
| 532334 | ACAAATAGCAATGAATGGGT | 45 | 110878 | 110897 | 559 |
| 532335 | CAACAAATAGCAATGAATGG | 47 | 110880 | 110899 | 560 |
| 532336 | GTACACAAATCAGTAGCTCT | 72 | 115087 | 115106 | 561 |
| 532337 | CTATGTCAAAAAGACTGAAA | 4 | 116370 | 116389 | 562 |
| 532338 | ATATACAGAACATTTCATCC | 13 | 116743 | 116762 | 563 |
| 532339 | AGAATAGATAAGAACTCACC | 32 | 117195 | 117214 | 564 |
| 532340 | AGGAAAGATACAGTCATTTT | 5 | 117507 | 117526 | 565 |
| 532341 | GCACAAAGAACACCTGGGAA | 43 | 117781 | 117800 | 566 |
| 532342 | CAAGAAGTCTGGGATTATGT | 0 | 117938 | 117957 | 567 |
| 532343 | GTTAGTTATTAAGCTAATCA | 48 | 118245 | 118264 | 568 |
| 532344 | AACCATTATTTATAGGCTAA | 14 | 119127 | 119146 | 569 |
| 532345 | CCAGAATGCGATCACTTCTT | 76 | 120826 | 120845 | 570 |
| 532346 | CCAGAAATTATCCTCCTCTC | 70 | 121209 | 121228 | 571 |
| 532347 | AGGGAAATGCAAATTAAAAC | 20 | 122479 | 122498 | 572 |
| 532348 | GCATCAAGATACAGAAAAAT | 24 | 122751 | 122770 | 573 |
| 532349 | GAATGTTTATGAGATTTTTC | 0 | 123571 | 123590 | 574 |
| 532350 | GCCAATTATATTGCCACATT | 23 | 124413 | 124432 | 575 |
| 532351 | ATACTTGCTTATGTAGAAAT | 45 | 124589 | 124608 | 576 |
| 532352 | TAATACTTGCTTATGTAGAA | 3 | 124591 | 124610 | 577 |
| 532353 | GAACACATGGCATTCTGATA | 36 | 125178 | 125197 | 578 |
| 532354 | CAGAATTTGCAGTATAAATC | 0 | 126051 | 126070 | 579 |
| 532355 | TATGTTTTGAAATCTTATTT | 0 | 126157 | 126176 | 580 |
| 532356 | ACTCACTGCTACCTCATTAA | 11 | 126998 | 127017 | 581 |
| 532357 | AAGCAGTGATAGGGTATCTG | 59 | 127080 | 127099 | 582 |
| 532358 | ATGAGGCCTATTACAATGGA | 14 | 127170 | 127189 | 583 |
| 532359 | CTGGAGTCTCATGAGGCCTA | 53 | 127180 | 127199 | 584 |
| 532360 | TGACTATCAGCCTTTTAATC | 45 | 127663 | 127682 | 585 |
| 532361 | TTCAGAGAACAACCTTTGAA | 0 | 127959 | 127978 | 586 |
| 532362 | AGCCATGTGTGATCTGATGT | 53 | 128813 | 128832 | 587 |
| 532363 | GAAATTTACTCCAAACTAGC | 17 | 128992 | 129011 | 588 |
| 532364 | AACATCCAGACCACCATCTA | 35 | 130094 | 130113 | 589 |
| 532365 | GTACCAAACCATTCATGCTC | 56 | 131036 | 131055 | 590 |
| 532366 | AGTACCAAACCATTCATGCT | 24 | 131037 | 131056 | 591 |
| 532367 | TTATAGAGCTTGAGATTGAC | 7 | 132165 | 132184 | 592 |
| 532368 | AGTCCATTATAGAGCTTGAG | 58 | 132171 | 132190 | 593 |
| 532369 | AACCATGAGATGCAATGCAG | 40 | 132498 | 132517 | 594 |
| 532370 | AGGATTGAGAATCGCTGATT | 42 | 133168 | 133187 | 595 |
| 532371 | TCTAAAGCATGGCCAGGATT | 48 | 133182 | 133201 | 596 |
| 532372 | GGGACTGAGTATTGATACTT | 44 | 133222 | 133241 | 597 |
| 532373 | AGAAGTAGGGTGTTCCAGAT | 29 | 133523 | 133542 | 598 |
| 532374 | AGAAATAGTCTTCCTACTAA | 0 | 133547 | 133566 | 599 |
| 532375 | GCCTCCTTTAAGCTTCTATG | 22 | 134240 | 134259 | 600 |
| 532376 | GGCCTGCCTTTACTTTCCCA | 36 | 134598 | 134617 | 601 |

**Table 10**

| Inhibition of GHR mRNA by 5-10-5 MOE gapmers targeting introns 1 and 2 of SEQ ID NO: 2 | | | | | | | | |
|---|---|---|---|---|---|---|---|---|
| ISIS NO | SEQ ID NO: 1 Start Site | SEQ ID NO: 1 Stop Site | Sequence | Target region | % inhibition | SEQ ID NO: 2 Start Site | SEQ ID NO: 2 Stop Site | SEQ ID NO |
| 523638 | n/a | n/a | ACCTCAGTGGACTCTTTCCA | Intron 1 | 4 | 84684 | 84703 | 602 |
| 523639 | n/a | n/a | CAAACCTAAGTTCAAGTCCT | Intron 1 | 62 | 85523 | 85542 | 603 |
| 523640 | n/a | n/a | AGTTTCACTTCTTGAATCAA | Intron 1 | 38 | 86373 | 86392 | 604 |
| 523641 | n/a | n/a | AAGATCAAATGAGGTCAAGG | Intron 1 | 30 | 87181 | 87200 | 605 |
| 523642 | n/a | n/a | TAGATACAAATTTCATCACA | Intron 1 | 23 | 88063 | 88082 | 606 |
| 523643 | n/a | n/a | ATTCCTAAAATAGGAGCAGG | Intron 1 | 45 | 88870 | 88889 | 607 |
| 523644 | n/a | n/a | TTTTTATGTTGTATAAGATA | Intron 1 | 0 | 89670 | 89689 | 608 |
| 523645 | n/a | n/a | GTTCAGCCAATACATGAGTA | Intron 1 | 48 | 90473 | 90492 | 609 |
| 523646 | n/a | n/a | CCAGAGGGAGTTCATTACCA | Intron 1 | 62 | 91273 | 91292 | 610 |
| 523647 | n/a | n/a | TCTCTCTAATTCAACCTTAT | Intron 1 | 44 | 92107 | 92126 | 611 |
| 523648 | n/a | n/a | ATAATCCTCAGACCTCTTTA | Intron 1 | 29 | 92925 | 92944 | 612 |
| 523649 | n/a | n/a | CACTGTGGCAGAATTCCAAG | Intron 1 | 28 | 93762 | 93781 | 613 |
| 523650 | n/a | n/a | ACACCTTGGTGCCTAGAAGC | Intron 1 | 54 | 94581 | 94600 | 614 |
| 523651 | n/a | n/a | GTAGCAATGACACCTAAGAA | Intron 1 | 58 | 95394 | 95413 | 615 |
| 523652 | n/a | n/a | TTTAAAATAATAAATGCTTA | Intron 1 | 0 | 96194 | 96213 | 616 |
| 523653 | n/a | n/a | TCATTTGGTCCTTAGACCCC | Intron 1 | 27 | 96994 | 97013 | 617 |
| 523654 | n/a | n/a | TTATTCATCTAGGCCGAGTG | Intron 1 | 57 | 97800 | 97819 | 618 |
| 523655 | n/a | n/a | TTGCAGAATCTTCTCTTTGG | Intron 1 | 65 | 98627 | 98646 | 619 |
| 523656 | n/a | n/a | ACCATAAAGCTATGCCATAA | Intron 1 | 63 | 99481 | 99500 | 620 |
| 523657 | n/a | n/a | GGCAAGGAGCACAATAGGAC | Intron 1 | 20 | 100281 | 100300 | 621 |
| 523658 | n/a | n/a | ACCCAAAAAGTTGAGCTGAG | Intron 1 | 66 | 101081 | 101100 | 622 |
| 523659 | n/a | n/a | TAGATTTTCAGACTCTTTCT | Intron 1 | 46 | 101887 | 101906 | 623 |
| 523660 | n/a | n/a | AATTTCAATATTGTTGTGTT | Intron 1 | 0 | 102760 | 102779 | 624 |
| 523661 | n/a | n/a | ATGCTTTTAATGGCACCCAA | Intron 1 | 69 | 103569 | 103588 | 625 |
| 523662 | n/a | n/a | CATGTCTCACATCCAGGTCA | Intron 1 | 37 | 104386 | 104405 | 626 |
| 523663 | n/a | n/a | TTCACTGGAGTAGACTTTTA | Intron 1 | 45 | 105255 | 105274 | 627 |
| 523664 | n/a | n/a | CTTATAAGGGAGGTCTGGTA | Intron 1 | 41 | 106147 | 106166 | 628 |
| 523665 | n/a | n/a | GCATAATTCTTAAGGGCCCT | Intron 1 | 71 | 106966 | 106985 | 629 |
| 523666 | n/a | n/a | CCACAGAACTTCTTTTAAAA | Intron 1 | 27 | 107766 | 107785 | 630 |
| 523667 | n/a | n/a | GGTGACCATGATTTTAACAA | Intron 1 | 25 | 108566 | 108585 | 631 |
| 523668 | n/a | n/a | AACAGCTGCATGACAATTTT | Intron 1 | 50 | 109382 | 109401 | 632 |
| 523669 | n/a | n/a | AGAAACAGAATCAGTGACTT | Intron 1 | 44 | 110403 | 110422 | 633 |
| 523670 | n/a | n/a | CAGATTCCAGAGAAAAGCCA | Intron 1 | 14 | 111203 | 111222 | 634 |
| 523671 | n/a | n/a | TGTGAGAAGAACTCTATCAC | Intron 1 | 12 | 112030 | 112049 | 635 |
| 523672 | n/a | n/a | CTCACAAATCACCACTAAAG | Intron 1 | 31 | 112842 | 112861 | 636 |
| 523673 | n/a | n/a | CAACGAGTGGATAAAGAAAC | Intron 1 | 28 | 113646 | 113665 | 637 |
| 523674 | n/a | n/a | ATAAAACTGGATCCTCATCT | Intron 1 | 13 | 114446 | 114465 | 638 |
| 523675 | n/a | n/a | ATTAAAACTCTCAGCAAAAT | Intron 1 | 0 | 115450 | 115469 | 639 |
| 523676 | n/a | n/a | AAAGACTGAAAGAACACAAA | Intron 1 | 0 | 116361 | 116380 | 640 |
| 523677 | n/a | n/a | TATCTGCTGCCTTCAGGAGA | Intron 1 | 0 | 117168 | 117187 | 641 |
| 523678 | n/a | n/a | TTTGAATTAACCCAATTCAA | Intron 1 | 0 | 117999 | 118018 | 642 |
| 523679 | n/a | n/a | TCTTAATTTACAACAGAGGA | Intron 1 | 25 | 118821 | 118840 | 643 |
| 523680 | n/a | n/a | AGAAAAGTGACAGGCTTCCC | Intron 1 | 31 | 119659 | 119678 | 644 |
| 523681 | n/a | n/a | ATGTTCCTTGAAGATCCCAA | Intron 1 | 37 | 120478 | 120497 | 645 |
| 523682 | n/a | n/a | ATGAATAACACTTGCCACAA | Intron 1 | 0 | 121379 | 121398 | 646 |
| 523683 | n/a | n/a | GTATGTTTATCACAGCACAG | Intron 1 | 56 | 122180 | 122199 | 647 |
| 523684 | n/a | n/a | AAACACTGCAATATTAGGTT | Intron 1 | 34 | 123031 | 123050 | 648 |
| 523685 | n/a | n/a | GATTGGTGCTTTTCAAACTG | Intron 1 | 39 | 123936 | 123955 | 649 |
| 523686 | n/a | n/a | ATTTGTAAGACAAACATGAA | Intron 1 | 9 | 124764 | 124783 | 650 |
| 523687 | n/a | n/a | TCACATGACTATGTTCTGGC | Intron 1 | 72 | 125594 | 125613 | 651 |
| 523688 | n/a | n/a | AGTCCTGTCCACACTATTAA | Intron 1 | 6 | 126415 | 126434 | 652 |
| 523689 | n/a | n/a | CTGGGCTCTGCCTGCTGAAC | Intron 1 | 17 | 127217 | 127236 | 653 |
| 523690 | n/a | n/a | AAAACCCTTAAGTATTTCCT | Intron 1 | 12 | 128054 | 128073 | 654 |
| 523691 | n/a | n/a | CTCTGTTTCAAACCCCCCAG | Intron 1 | 21 | 128854 | 128873 | 655 |
| 523692 | n/a | n/a | GGACAGAACACCAATCACAA | Intron 1 | 18 | 129654 | 129673 | 656 |
| 523693 | n/a | n/a | ACCTACCCTTCAAAGTCACG | Intron 1 | 0 | 130486 | 130505 | 657 |
| 523694 | n/a | n/a | TTCAGTTCCCAGGAGGCTTA | Intron 1 | 5 | 131286 | 131305 | 658 |
| 523695 | n/a | n/a | TTTTGCAATGTCTAGCAATT | Intron 1 | 0 | 132086 | 132105 | 659 |
| 523696 | n/a | n/a | ATTAAGATCAGAAAATATTA | Intron 1 | 0 | 132953 | 132972 | 660 |
| 523697 | n/a | n/a | TTAATGAGATATTTTGCACC | Intron 1 | 34 | 133858 | 133877 | 661 |
| 523698 | n/a | n/a | GAGAGGTTAAGTAAATCTCC | Intron 1 | 0 | 134678 | 134697 | 662 |
| 523699 | n/a | n/a | CAGACTCAAATTTGAAAATT | Intron 1 | 14 | 135500 | 135519 | 663 |
| 523700 | n/a | n/a | GATAAGGCAATAATACAGCC | Intron 1 | 1 | 136306 | 136325 | 664 |
| 523701 | n/a | n/a | ATCATTTGCCAATTTCTGTG | Intron 1 | 28 | 137133 | 137152 | 665 |
| 523702 | n/a | n/a | CAAGAAGAAAAGATGCAAAA | Intron 1 | 0 | 138035 | 138054 | 666 |
| 523703 | n/a | n/a | AATTTATTTCCTTCCTATGA | Intron 1 | 0 | 138857 | 138876 | 667 |
| 523704 | n/a | n/a | TTTTGGAAATGTGAGAAACG | Intron 1 | 0 | 139771 | 139790 | 668 |
| 523705 | n/a | n/a | AAACACATGAGAAAAGATGA | Intron 1 | 0 | 140593 | 140612 | 669 |
| 523706 | n/a | n/a | TGTTGGCTCAGTGGGAATGA | Intron 1 | 0 | 141412 | 141431 | 670 |
| 523707 | n/a | n/a | TGAACAGGTTTGCATTTCTC | Intron 1 | 42 | 142229 | 142248 | 671 |
| 523708 | n/a | n/a | TCCTAGGTGAACAGGCTATG | Intron 1 | 38 | 143029 | 143048 | 672 |
| 523709 | n/a | n/a | CCCTAATCAGGCTGAAATAA | Intron 1 | 0 | 143829 | 143848 | 673 |
| 523710 | n/a | n/a | AGGGCCAGTAAGGTTTGCTT | Intron 1 | 12 | 144631 | 144650 | 674 |
| 523711 | n/a | n/a | AGCCTGAATTCTGAGCTCTG | Intron 2 | 88 | 145431 | 145450 | 675 |
| 523712 | n/a | n/a | AGAGGATCTCAGCTGCAATT | Intron 2 | 71 | 146238 | 146257 | 676 |
| 523713 | n/a | n/a | GAAAATCCCTGCTCAAGTGC | Intron 2 | 67 | 147262 | 147281 | 677 |
| 523714 | n/a | n/a | TGCCTGATATTGTAATTCTT | Intron 2 | 90 | 148062 | 148081 | 678 |

**Table 11**

| Inhibition of GHR mRNA by 5-10-5 MOE gapmers targeting introns 1 and 2 of SEQ ID NO: 2 | | | | | | |
|---|---|---|---|---|---|---|
| ISIS NO | Sequence | Target Region | % inhibition | SEQ ID NO: 2 Start Site | SEQ ID NO: 2 Stop Site | SEQ ID NO |
| 532377 | CTCATACAGTGAAGTCTTCA | Intron 1 | 73 | 135431 | 135450 | 679 |
| 532378 | CTCACTAAGCTTGATTCACT | Intron 1 | 67 | 135818 | 135837 | 680 |
| 532379 | GATACAGAAATCCCAGTGAC | Intron 1 | 46 | 136111 | 136130 | 681 |
| 532380 | TGTGCTTGGGTGTACAGGCA | Intron 1 | 71 | 136282 | 136301 | 682 |
| 532381 | TCAAGCACTTACATCATATG | Intron 1 | 42 | 136377 | 136396 | 683 |
| 532382 | AGGGTTAGTTATTACACTTA | Intron 1 | 60 | 136576 | 136595 | 684 |
| 532383 | AGGCTTCATGTGAGGTAACA | Intron 1 | 58 | 136996 | 137015 | 685 |
| 532384 | TGAAAGCTTAGTACAAGAAG | Intron 1 | 51 | 138048 | 138067 | 686 |
| 532385 | CTCTCCTCTTGGAGATCCAG | Intron 1 | 58 | 138782 | 138801 | 687 |
| 532386 | GCTGAGATTTCTCTCCTCTT | Intron 1 | 78 | 138792 | 138811 | 688 |
| 532387 | CTTTTGCTGAGATTTCTCTC | Intron 1 | 58 | 138797 | 138816 | 689 |
| 532388 | GAACATATGTCCATAGAATG | Intron 1 | 57 | 141700 | 141719 | 690 |
| 532389 | GAACAGGCTATGTAATCAAA | Intron 1 | 68 | 143021 | 143040 | 691 |
| 532390 | TTTTTATTACTGTGCAAACC | Intron 1 | 41 | 143878 | 143897 | 692 |
| 532391 | ACTGAGGGTGGAAATGGAAA | Intron 2 | 23 | 145059 | 145078 | 693 |
| 532392 | ATGCCATACTTTTCATTTCA | Intron 2 | 87 | 146351 | 146370 | 694 |
| 532393 | TCTTTAAAGATTTCCTATGC | Intron 2 | 66 | 146367 | 146386 | 695 |
| 532394 | TCACAATTAAATTATGTTTA | Intron 2 | 47 | 149858 | 149877 | 696 |
| 532395 | TTTGCCATCACCAAACACCA | Intron 2 | 94 | 150972 | 150991 | 697 |
| 532396 | TCAGAATGCTGAAGGATGGG | Intron 2 | 70 | 152208 | 152227 | 698 |
| 532397 | ACAATTGCAGGAGAGAACTG | Intron 2 | 57 | 152296 | 152315 | 699 |
| 532398 | GTTCAGTCACCTGGAAAGAG | Intron 2 | 62 | 152549 | 152568 | 700 |
| 532399 | CGGAGTTCAGTCACCTGGAA | Intron 2 | 77 | 152553 | 152572 | 701 |
| 532400 | AATCTAAAGTTCAATGTCCA | Intron 2 | 77 | 152752 | 152771 | 702 |
| 532401 | CCACCTTTGGGTGAATAGCA | Intron 2 | 95 | 153921 | 153940 | 703 |
| 532402 | CAACATCAAAAGTTTCCACC | Intron 2 | 81 | 153936 | 153955 | 704 |
| 532403 | AAGCTTCTATCAACCAACTG | Intron 2 | 87 | 154093 | 154112 | 705 |
| 532404 | ACCATTTTCTAATAATTCAC | Intron 2 | 46 | 154502 | 154521 | 706 |
| 532405 | ACCTGCACTTGGACAACTGA | Intron 2 | 60 | 154727 | 154746 | 707 |
| 532406 | GTCAGTGCTTTGGTGATGTA | Intron 2 | 11 | 155283 | 155302 | 708 |
| 532407 | TAGAAGCACAGGAACTAGAG | Intron 2 | 68 | 155889 | 155908 | 709 |
| 532408 | TTTAATTTTATTAGAAGCAC | Intron 2 | 14 | 155900 | 155919 | 710 |
| 532409 | GAGCAAGAATTAAGAAAATC | Intron 2 | 29 | 155973 | 155992 | 711 |
| 532410 | CTCTGCAGTCATGTACACAA | Intron 2 | 93 | 156594 | 156613 | 712 |
| 532411 | GCTTGGTTTGTCAATCCTTT | Intron 2 | 95 | 156889 | 156908 | 713 |
| 532412 | GTTCTCAAGCAGGAGCCATT | Intron 2 | 70 | 157330 | 157349 | 714 |
| 532413 | AGGGTGATCTTCCAAAACAA | Intron 2 | 87 | 158612 | 158631 | 715 |
| 532414 | TCTCCTATGCTTCCTTTAAT | Intron 2 | 25 | 158813 | 158832 | 716 |
| 532415 | GACATAAATATGTTCACTGA | Intron 2 | 81 | 159216 | 159235 | 717 |
| 532416 | TTACTGAGTGACAGTACAGT | Intron 2 | 65 | 161588 | 161607 | 718 |
| 532417 | CCAGGCACCAGCACAGGCAC | Intron 2 | 47 | 161950 | 161969 | 719 |
| 532418 | TTAATGTCAGTAGAAAGCTG | Intron 2 | 0 | 162349 | 162368 | 720 |
| 532419 | GCAGGTGGAAAGAAGATGTC | Intron 2 | 50 | 162531 | 162550 | 721 |
| 532420 | GCCAGGGTCTTTACAAAGTT | Intron 2 | 93 | 162751 | 162770 | 722 |
| 532421 | CATTACCTTTGTACATGTAC | Intron 2 | 83 | 164839 | 164858 | 723 |
| 532422 | GAAGCAACTTCTCTGAGGTC | Intron 2 | 68 | 165040 | 165059 | 724 |
| 532423 | GCCTGGCAAGAAGGGCCCTT | Intron 2 | 56 | 165856 | 165875 | 725 |
| 532424 | ACACATGTTTTTAAATTTAT | Intron 2 | 21 | 166241 | 166260 | 726 |
| 532425 | TCACAATGCACTAAAAGAAA | Intron 2 | 53 | 168760 | 168779 | 727 |
| 532426 | TCCCAATGACTTACTGTAGA | Intron 2 | 78 | 169073 | 169092 | 728 |
| 532427 | TAAGCATTTATGGAGGAATG | Intron 2 | 46 | 169134 | 169153 | 729 |
| 532428 | TGAGGTGGGTGGCCAACAGG | Intron 2 | 66 | 170081 | 170100 | 730 |
| 532429 | GTTTTTCATTTTGATTGCAG | Intron 2 | 88 | 170158 | 170177 | 731 |
| 532430 | AGCTCAAGTGTTTTTCATTT | Intron 2 | 64 | 170167 | 170186 | 732 |
| 532431 | CAATGTCACAGCTGTTTCCT | Intron 2 | 62 | 170272 | 170291 | 733 |
| 532432 | GAACTTTGGAGGCTTTTAGA | Intron 2 | 55 | 170703 | 170722 | 734 |
| 532433 | TGTATGCCCCAAACTCCCAT | Intron 2 | 83 | 171431 | 171450 | 735 |
| 532434 | ACACAAATAAGGGAATAATA | Intron 2 | 24 | 171549 | 171568 | 736 |
| 532435 | TAGTTCAGCCACTATGGAAA | Intron 2 | 47 | 171926 | 171945 | 737 |
| 532436 | CTCCAAATTCCAGTCCTAGG | Intron 2 | 93 | 172746 | 172765 | 738 |
| 532437 | AGTTGGCACTGCTATATCAG | Intron 2 | 66 | 173668 | 173687 | 739 |
| 532438 | GGCCTTAGATTGTAAGTTTT | Intron 2 | 69 | 174122 | 174141 | 740 |
| 532439 | TTTTAGTATTATTGTAGGAA | Intron 2 | 16 | 174188 | 174207 | 741 |
| 532440 | TTTCATTAATGAAACCTGAT | Intron 2 | 39 | 174812 | 174831 | 742 |
| 532441 | CCCTCAGCTGCCTCTTCAAT | Intron 2 | 51 | 175014 | 175033 | 743 |
| 532442 | TATTGTATCCTGGCCCCTAA | Intron 2 | 68 | 175689 | 175708 | 744 |
| 532443 | AGAACAAGAGCCTAGAAGTA | Intron 2 | 35 | 176592 | 176611 | 745 |
| 532444 | GTGACTATGTCACTGAATTT | Intron 2 | 14 | 176918 | 176937 | 746 |
| 532445 | GCCCTACCCAGCAGCCTGTG | Intron 2 | 79 | 177540 | 177559 | 747 |
| 532446 | CAAACATAAAGAGAGTTCCA | Intron 2 | 79 | 177811 | 177830 | 748 |
| 532447 | CTTTAAATGAAGTAGAGCTC | Intron 2 | 0 | 178090 | 178109 | 749 |
| 532448 | CTGTTCAAAGAATGCAGGCC | Intron 2 | 70 | 178905 | 178924 | 750 |
| 532449 | GTCTAGCCTAACAGAGATAT | Intron 2 | 47 | 179137 | 179156 | 751 |
| 532450 | AAAGAGTGATGTCTAGCCTA | Intron 2 | 55 | 179147 | 179166 | 752 |
| 532451 | CACTTCTTACTCCTTTGAGG | Intron 2 | 50 | 179631 | 179650 | 753 |
| 532452 | TTCCACAAGAAACTCAGTTT | Intron 2 | 56 | 181514 | 181533 | 754 |
| 532453 | AGAAATGCCAAAGATAGCTC | Intron 2 | 56 | 182105 | 182124 | 755 |

**Table 12**

| Inhibition of GHR mRNA by 5-10-5 MOE gapmers targeting intron 2 of SEQ ID NO: 2 | | | | | |
|---|---|---|---|---|---|
| ISIS NO | Sequence | % inhibition | SEQ ID NO: 2 Start Site | SEQ ID NO: 2 Stop Site | SEQ ID NO |
| 533249 | AGCAGAGGATCTCAGCTGCA | 84 | 146241 | 146260 | 756 |
| 533250 | AATCCCTGCTCAAGTGCTAC | 75 | 147259 | 147278 | 757 |
| 533251 | AAATCCCTGCTCAAGTGCTA | 71 | 147260 | 147279 | 758 |
| 533252 | AAAATCCCTGCTCAAGTGCT | 73 | 147261 | 147280 | 759 |
| 533253 | AGAAAATCCCTGCTCAAGTG | 56 | 147263 | 147282 | 760 |
| 533254 | AAGAAAATCCCTGCTCAAGT | 58 | 147264 | 147283 | 761 |
| 533255 | CAAGAAAATCCCTGCTCAAG | 46 | 147265 | 147284 | 762 |
| 533256 | CTGATATTGTAATTCTTGGT | 91 | 148059 | 148078 | 763 |
| 533257 | CCTGATATTGTAATTCTTGG | 90 | 148060 | 148079 | 764 |
| 533258 | GCCTGATATTGTAATTCTTG | 94 | 148061 | 148080 | 765 |
| 533259 | ATGCCTGATATTGTAATTCT | 91 | 148063 | 148082 | 766 |
| 533260 | AATGCCTGATATTGTAATTC | 74 | 148064 | 148083 | 767 |
| 533261 | CAATGCCTGATATTGTAATT | 76 | 148065 | 148084 | 768 |
| 533262 | AATTATGTGCTTTGCCTGCA | 92 | 148904 | 148923 | 769 |
| 533263 | CAATTATGTGCTTTGCCTGC | 83 | 148905 | 148924 | 770 |
| 533264 | TCAATTATGTGCTTTGCCTG | 83 | 148906 | 148925 | 771 |
| 533265 | TGTCAATTATGTGCTTTGCC | 91 | 148908 | 148927 | 772 |
| 533266 | ATGTCAATTATGTGCTTTGC | 83 | 148909 | 148928 | 773 |
| 533267 | GATGTCAATTATGTGCTTTG | 74 | 148910 | 148929 | 774 |
| 533268 | CTGGTGACTCTGCCTGATGA | 77 | 151385 | 151404 | 775 |
| 533269 | GCTGGTGACTCTGCCTGATG | 87 | 151386 | 151405 | 776 |
| 533270 | TGCTGGTGACTCTGCCTGAT | 89 | 151387 | 151406 | 777 |
| 533271 | GCTGCTGGTGACTCTGCCTG | 94 | 151389 | 151408 | 778 |
| 533272 | GGCTGCTGGTGACTCTGCCT | 77 | 151390 | 151409 | 779 |
| 533273 | TGGCTGCTGGTGACTCTGCC | 82 | 151391 | 151410 | 780 |
| 533274 | GCTGAAGGATGGGCATCCAG | 85 | 152201 | 152220 | 781 |
| 533275 | TGCTGAAGGATGGGCATCCA | 85 | 152202 | 152221 | 782 |
| 533276 | ATGCTGAAGGATGGGCATCC | 78 | 152203 | 152222 | 783 |
| 533277 | GAATGCTGAAGGATGGGCAT | 66 | 152205 | 152224 | 784 |
| 533278 | AGAATGCTGAAGGATGGGCA | 81 | 152206 | 152225 | 785 |
| 533279 | CAGAATGCTGAAGGATGGGC | 85 | 152207 | 152226 | 786 |
| 533280 | TCCAGTAGTCAATATTATTT | 87 | 153001 | 153020 | 787 |
| 533281 | ATCCAGTAGTCAATATTATT | 85 | 153002 | 153021 | 788 |
| 533282 | TATCCAGTAGTCAATATTAT | 69 | 153003 | 153022 | 789 |
| 533283 | GTTATCCAGTAGTCAATATT | 77 | 153005 | 153024 | 790 |
| 533284 | GGTTATCCAGTAGTCAATAT | 85 | 153006 | 153025 | 791 |
| 533285 | TGGTTATCCAGTAGTCAATA | 86 | 153007 | 153026 | 792 |
| 533286 | CAACTTGAGGACAATAAGAG | 35 | 155591 | 155610 | 793 |
| 533287 | TCAACTTGAGGACAATAAGA | 62 | 155592 | 155611 | 794 |
| 533288 | CTCAACTTGAGGACAATAAG | 86 | 155593 | 155612 | 795 |
| 533289 | AACTCAACTTGAGGACAATA | 82 | 155595 | 155614 | 796 |
| 533290 | TAACTCAACTTGAGGACAAT | 66 | 155596 | 155615 | 797 |
| 533291 | ATAACTCAACTTGAGGACAA | 87 | 155597 | 155616 | 798 |
| 533292 | CAGGAAGAAAGGAACCTTAG | 77 | 156391 | 156410 | 799 |
| 533293 | CCAGGAAGAAAGGAACCTTA | 84 | 156392 | 156411 | 800 |
| 533294 | ACCAGGAAGAAAGGAACCTT | 86 | 156393 | 156412 | 801 |
| 533295 | AGACCAGGAAGAAAGGAACC | 74 | 156395 | 156414 | 802 |
| 533296 | TAGACCAGGAAGAAAGGAAC | 59 | 156396 | 156415 | 803 |
| 533297 | ATAGACCAGGAAGAAAGGAA | 65 | 156397 | 156416 | 804 |
| 533298 | TACAATGCACAGGACACGCC | 73 | 157198 | 157217 | 805 |
| 533299 | CTACAATGCACAGGACACGC | 85 | 157199 | 157218 | 806 |
| 533300 | GCTACAATGCACAGGACACG | 83 | 157200 | 157219 | 807 |
| 533301 | ATGCTACAATGCACAGGACA | 89 | 157202 | 157221 | 808 |
| 533302 | TATGCTACAATGCACAGGAC | 82 | 157203 | 157222 | 809 |
| 533303 | ATATGCTACAATGCACAGGA | 84 | 157204 | 157223 | 810 |
| 533304 | CTGATATTTATTGCTGTACG | 76 | 158006 | 158025 | 811 |
| 533305 | CTCTGATATTTATTGCTGTA | 80 | 158008 | 158027 | 812 |
| 533306 | TCTCTGATATTTATTGCTGT | 86 | 158009 | 158028 | 813 |
| 533307 | GTCTCTGATATTTATTGCTG | 80 | 158010 | 158029 | 814 |
| 533308 | CCAGAAGAATTACCCATGCA | 85 | 165550 | 165569 | 815 |
| 533309 | TCCAGAAGAATTACCCATGC | 84 | 165551 | 165570 | 816 |
| 533310 | TTCCAGAAGAATTACCCATG | 81 | 165552 | 165571 | 817 |
| 533311 | TCTTCCAGAAGAATTACCCA | 58 | 165554 | 165573 | 818 |
| 533312 | ATCTTCCAGAAGAATTACCC | 64 | 165555 | 165574 | 819 |
| 533313 | CATCTTCCAGAAGAATTACC | 58 | 165556 | 165575 | 820 |
| 533314 | TTTCTGCAGTATCCTAGCCT | 78 | 166350 | 166369 | 821 |
| 533315 | GTTTCTGCAGTATCCTAGCC | 88 | 166351 | 166370 | 822 |
| 533316 | AGTTTCTGCAGTATCCTAGC | 86 | 166352 | 166371 | 823 |
| 533317 | TCAGTTTCTGCAGTATCCTA | 88 | 166354 | 166373 | 824 |
| 533318 | TTCAGTTTCTGCAGTATCCT | 87 | 166355 | 166374 | 825 |
| 533319 | TTTCAGTTTCTGCAGTATCC | 80 | 166356 | 166375 | 826 |
| 533320 | GTTTCCATTTTCTTGATTCC | 70 | 169601 | 169620 | 827 |
| 533321 | TGTTTCCATTTTCTTGATTC | 54 | 169602 | 169621 | 828 |
| 533322 | GTGTTTCCATTTTCTTGATT | 55 | 169603 | 169622 | 829 |
| 533323 | TGGTGTTTCCATTTTCTTGA | 73 | 169605 | 169624 | 830 |
| 533324 | ATGGTGTTTCCATTTTCTTG | 76 | 169606 | 169625 | 831 |
| 533325 | AATGGTGTTTCCATTTTCTT | 78 | 169607 | 169626 | 832 |

**Table 13**

| Inhibition of GHR mRNA by 5-10-5 MOE gapmers targeting introns 2 and 3 of SEQ ID NO: 2 | | | | | | |
|---|---|---|---|---|---|---|
| ISIS NO | Sequence | Target region | % inhibition | SEQ ID NO: 2 Start Site | SEQ ID NO: 2 Stop Site | SEQ ID NO |
| 533326 | AACCCATTTCATCCATTTAA | Intron 2 | 93 | 175369 | 175388 | 833 |
| 533327 | GAACCCATTTCATCCATTTA | Intron 2 | 83 | 175370 | 175389 | 834 |
| 533328 | GGAACCCATTTCATCCATTT | Intron 2 | 92 | 175371 | 175390 | 835 |
| 533329 | TAGGAACCCATTTCATCCAT | Intron 2 | 91 | 175373 | 175392 | 836 |
| 533330 | GTAGGAACCCATTTCATCCA | Intron 2 | 95 | 175374 | 175393 | 837 |
| 533331 | GGTAGGAACCCATTTCATCC | Intron 2 | 92 | 175375 | 175394 | 838 |
| 533332 | TGAGGGATTGCCTCAGTAGC | Intron 2 | 66 | 179616 | 179635 | 839 |
| 533333 | TTGAGGGATTGCCTCAGTAG | Intron 2 | 72 | 179617 | 179636 | 840 |
| 533334 | TTTGAGGGATTGCCTCAGTA | Intron 2 | 67 | 179618 | 179637 | 841 |
| 533335 | CCTTTGAGGGATTGCCTCAG | Intron 2 | 74 | 179620 | 179639 | 842 |
| 533336 | TCCTTTGAGGGATTGCCTCA | Intron 2 | 66 | 179621 | 179640 | 843 |
| 533337 | CTCCTTTGAGGGATTGCCTC | Intron 2 | 76 | 179622 | 179641 | 844 |
| 533338 | AACTTAGGACTTGGGACATT | Intron 2 | 64 | 184575 | 184594 | 845 |
| 533339 | TAACTTAGGACTTGGGACAT | Intron 2 | 54 | 184576 | 184595 | 846 |
| 533340 | CTAACTTAGGACTTGGGACA | Intron 2 | 63 | 184577 | 184596 | 847 |
| 533341 | CACTAACTTAGGACTTGGGA | Intron 2 | 82 | 184579 | 184598 | 848 |
| 533342 | TCACTAACTTAGGACTTGGG | Intron 2 | 77 | 184580 | 184599 | 849 |
| 533343 | GTCACTAACTTAGGACTTGG | Intron 2 | 83 | 184581 | 184600 | 850 |
| 533344 | TGGGCTAGATCAGGATTGGT | Intron 2 | 81 | 188617 | 188636 | 851 |
| 533345 | ATGGGCTAGATCAGGATTGG | Intron 2 | 70 | 188618 | 188637 | 852 |
| 533346 | CATGGGCTAGATCAGGATTG | Intron 2 | 64 | 188619 | 188638 | 853 |
| 533347 | ACCATGGGCTAGATCAGGAT | Intron 2 | 82 | 188621 | 188640 | 854 |
| 533348 | TACCATGGGCTAGATCAGGA | Intron 2 | 88 | 188622 | 188641 | 855 |
| 533349 | CTACCATGGGCTAGATCAGG | Intron 2 | 87 | 188623 | 188642 | 856 |
| 533350 | ATGAGCTTAGCAGTCACTTA | Intron 2 | 83 | 189482 | 189501 | 857 |
| 533351 | CATGAGCTTAGCAGTCACTT | Intron 2 | 87 | 189483 | 189502 | 858 |
| 533352 | CCATGAGCTTAGCAGTCACT | Intron 2 | 92 | 189484 | 189503 | 859 |
| 533353 | GTCTCAGCAAACCTGGGATA | Intron 2 | 84 | 190283 | 190302 | 860 |
| 533354 | TGTCTCAGCAAACCTGGGAT | Intron 2 | 82 | 190284 | 190303 | 861 |
| 533355 | ATGTCTCAGCAAACCTGGGA | Intron 2 | 81 | 190285 | 190304 | 862 |
| 533356 | GAATGTCTCAGCAAACCTGG | Intron 2 | 76 | 190287 | 190306 | 863 |
| 533357 | GGAATGTCTCAGCAAACCTG | Intron 2 | 82 | 190288 | 190307 | 864 |
| 533358 | AGGAATGTCTCAGCAAACCT | Intron 2 | 85 | 190289 | 190308 | 865 |
| 533359 | TACAGACATAGCTCTAACCT | Intron 2 | 79 | 191139 | 191158 | 866 |
| 533360 | ATACAGACATAGCTCTAACC | Intron 2 | 79 | 191140 | 191159 | 867 |
| 533361 | GATACAGACATAGCTCTAAC | Intron 2 | 71 | 191141 | 191160 | 868 |
| 533362 | TGGATACAGACATAGCTCTA | Intron 2 | 79 | 191143 | 191162 | 869 |
| 533363 | CTGGATACAGACATAGCTCT | Intron 2 | 82 | 191144 | 191163 | 870 |
| 533364 | GCTGGATACAGACATAGCTC | Intron 2 | 95 | 191145 | 191164 | 871 |
| 533365 | ACACTGTTTGTGAGGGTCAA | Intron 2 | 87 | 191939 | 191958 | 872 |
| 533366 | AACACTGTTTGTGAGGGTCA | Intron 2 | 81 | 191940 | 191959 | 873 |
| 533367 | CAACACTGTTTGTGAGGGTC | Intron 2 | 85 | 191941 | 191960 | 874 |
| 533368 | AACAACACTGTTTGTGAGGG | Intron 2 | 65 | 191943 | 191962 | 875 |
| 533369 | AAACAACACTGTTTGTGAGG | Intron 2 | 76 | 191944 | 191963 | 876 |
| 533370 | CAAACAACACTGTTTGTGAG | Intron 2 | 67 | 191945 | 191964 | 877 |
| 533371 | TTCAAGTTTAGGATCTGCAG | Intron 2 | 73 | 196536 | 196555 | 878 |
| 533372 | CTTCAAGTTTAGGATCTGCA | Intron 2 | 88 | 196537 | 196556 | 879 |
| 533373 | GCTTCAAGTTTAGGATCTGC | Intron 2 | 86 | 196538 | 196557 | 880 |
| 533374 | GGGCTTCAAGTTTAGGATCT | Intron 2 | 67 | 196540 | 196559 | 881 |
| 533375 | AGGGCTTCAAGTTTAGGATC | Intron 2 | 66 | 196541 | 196560 | 882 |
| 533376 | CAGGGCTTCAAGTTTAGGAT | Intron 2 | 74 | 196542 | 196561 | 883 |
| 533377 | TGTGGCTTTAATTCACTAAT | Intron 2 | 84 | 198145 | 198164 | 884 |
| 533378 | ATGTGGCTTTAATTCACTAA | Intron 2 | 86 | 198146 | 198165 | 885 |
| 533379 | TATGTGGCTTTAATTCACTA | Intron 2 | 79 | 198147 | 198166 | 886 |
| 533380 | GGTATGTGGCTTTAATTCAC | Intron 2 | 83 | 198149 | 198168 | 887 |
| 533381 | TGGTATGTGGCTTTAATTCA | Intron 2 | 81 | 198150 | 198169 | 888 |
| 533382 | GTGGTATGTGGCTTTAATTC | Intron 2 | 86 | 198151 | 198170 | 889 |
| 533383 | TCTGTGTTCAGTTGCATCAC | Intron 2 | 75 | 199817 | 199836 | 890 |
| 533384 | TTCTGTGTTCAGTTGCATCA | Intron 2 | 82 | 199818 | 199837 | 891 |
| 533385 | GTTCTGTGTTCAGTTGCATC | Intron 2 | 86 | 199819 | 199838 | 892 |
| 533386 | GTACTCATGAGGAGGCACTT | Intron 2 | 81 | 201413 | 201432 | 893 |
| 533387 | GGTACTCATGAGGAGGCACT | Intron 2 | 82 | 201414 | 201433 | 894 |
| 533388 | TGGTACTCATGAGGAGGCAC | Intron 2 | 78 | 201415 | 201434 | 895 |
| 533389 | ATTGGTACTCATGAGGAGGC | Intron 2 | 64 | 201417 | 201436 | 896 |
| 533390 | AATTGGTACTCATGAGGAGG | Intron 2 | 47 | 201418 | 201437 | 897 |
| 533391 | CAATTGGTACTCATGAGGAG | Intron 2 | 54 | 201419 | 201438 | 898 |
| 533392 | AAACTCTGCAACTCCAACCC | Intron 2 | 69 | 205549 | 205568 | 899 |
| 533393 | GAAACTCTGCAACTCCAACC | Intron 2 | 64 | 205550 | 205569 | 900 |
| 533394 | GGAAACTCTGCAACTCCAAC | Intron 2 | 83 | 205551 | 205570 | 901 |
| 533395 | ATGGAAACTCTGCAACTCCA | Intron 2 | 88 | 205553 | 205572 | 902 |
| 533396 | CATGGAAACTCTGCAACTCC | Intron 2 | 70 | 205554 | 205573 | 903 |
| 533397 | TCATGGAAACTCTGCAACTC | Intron 2 | 69 | 205555 | 205574 | 904 |
| 533398 | ACATCTGGATGTGAGGCTCG | Intron 3 | 64 | 210559 | 210578 | 905 |
| 533399 | CACATCTGGATGTGAGGCTC | Intron 3 | 84 | 210560 | 210579 | 906 |
| 533400 | GTCACATCTGGATGTGAGGC | Intron 3 | 75 | 210562 | 210581 | 907 |
| 533401 | TGTCACATCTGGATGTGAGG | Intron 3 | 51 | 210563 | 210582 | 908 |
| 533402 | CTGTCACATCTGGATGTGAG | Intron 3 | 30 | 210564 | 210583 | 909 |

**Table 14**

| Inhibition of GHR mRNA by 5-10-5 MOE gapmers targeting introns 2 and 3 of SEQ ID NO: 2 | | | | | | |
|---|---|---|---|---|---|---|
| ISIS NO | Sequence | Target region | % inhibition | SEQ ID NO: 2 Start Site | SEQ ID NO: 2 Stop Site | SEQ ID NO |
| 523715 | GTCAATTATGTGCTTTGCCT | Intron 2 | 91 | 148907 | 148926 | 910 |
| 523716 | ACATTCAAAATTCTTCCTTG | Intron 2 | 50 | 149787 | 149806 | 911 |
| 523717 | ATCCTGCATATATTTTATTG | Intron 2 | 20 | 150588 | 150607 | 912 |
| 523718 | CTGCTGGTGACTCTGCCTGA | Intron 2 | 77 | 151388 | 151407 | 913 |
| 523719 | AATGCTGAAGGATGGGCATC | Intron 2 | 66 | 152204 | 152223 | 914 |
| 523720 | TTATCCAGTAGTCAATATTA | Intron 2 | 71 | 153004 | 153023 | 915 |
| 523721 | TCTCATGTTAAAGTTCTTAA | Intron 2 | 48 | 153831 | 153850 | 916 |
| 523722 | TGCACTTGGACAACTGATAG | Intron 2 | 29 | 154724 | 154743 | 917 |
| 523723 | ACTCAACTTGAGGACAATAA | Intron 2 | 88 | 155594 | 155613 | 918 |
| 523724 | GACCAGGAAGAAAGGAACCT | Intron 2 | 72 | 156394 | 156413 | 919 |
| 523725 | TGCTACAATGCACAGGACAC | Intron 2 | 80 | 157201 | 157220 | 920 |
| 523726 | TCTGATATTTATTGCTGTAC | Intron 2 | 73 | 158007 | 158026 | 921 |
| 523727 | ATGCTTCCTTTAATAAATGT | Intron 2 | 0 | 158807 | 158826 | 922 |
| 523728 | AACATTTAGAACCTAGGAGA | Intron 2 | 20 | 159610 | 159629 | 923 |
| 523729 | CAAGCTTGCAAGTAGGAAAA | Intron 2 | 51 | 160410 | 160429 | 924 |
| 523730 | CCAGGCTGTTCATGCCAAGG | Intron 2 | 26 | 161248 | 161267 | 925 |
| 523731 | CCTGCCAAGGGCAAGCCAGG | Intron 2 | 17 | 162064 | 162083 | 926 |
| 523732 | TTTCACCTGGTGACTGGAAG | Intron 2 | 51 | 163019 | 163038 | 927 |
| 523733 | ATTTTCTACCATCAAAGAGA | Intron 2 | 4 | 163943 | 163962 | 928 |
| 523734 | GATTAAGTTTTCTTTAAAAA | Intron 2 | 0 | 164746 | 164765 | 929 |
| 523735 | CTTCCAGAAGAATTACCCAT | Intron 2 | 56 | 165553 | 165572 | 930 |
| 523736 | CAGTTTCTGCAGTATCCTAG | Intron 2 | 77 | 166353 | 166372 | 931 |
| 523737 | TATTTTGAAAATGAGATTCA | Intron 2 | 0 | 167195 | 167214 | 932 |
| 523738 | GTGGCCCGAGTAAAGATAAA | Intron 2 | 21 | 167995 | 168014 | 933 |
| 523739 | CCTGTCAATCCTCTTATATG | Intron 2 | 37 | 168804 | 168823 | 934 |
| 523740 | GGTGTTTCCATTTTCTTGAT | Intron 2 | 65 | 169604 | 169623 | 935 |
| 523741 | ACAGGGTCAAAAGTTCACTT | Intron 2 | 44 | 170407 | 170426 | 936 |
| 523742 | TAGGAAAGCTGAGAGAATCC | Intron 2 | 35 | 171207 | 171226 | 937 |
| 523743 | AGCATATGAAAAAATACTCA | Intron 2 | 0 | 172101 | 172120 | 938 |
| 523744 | CTTCAGAAATCAGCATCTGA | Intron 2 | 45 | 172937 | 172956 | 939 |
| 523745 | TTACAAGTGACAGTGTTTGT | Intron 2 | 28 | 173737 | 173756 | 940 |
| 523746 | ATCAGACCCTGAAGAATTTA | Intron 2 | 29 | 174560 | 174579 | 941 |
| 523747 | AGGAACCCATTTCATCCATT | Intron 2 | 83 | 175372 | 175391 | 942 |
| 523748 | CACATTGGTAACTTAAAGTT | Intron 2 | 18 | 176263 | 176282 | 943 |
| 523749 | TATTATCTGACTCATTTCTG | Intron 2 | 16 | 177072 | 177091 | 944 |
| 523750 | AAATAAGACAAAGAAAATTC | Intron 2 | 0 | 177872 | 177891 | 945 |
| 523751 | TTTTAAAAATAACCAATTCA | Intron 2 | 0 | 178788 | 178807 | 946 |
| 523752 | CTTTGAGGGATTGCCTCAGT | Intron 2 | 66 | 179619 | 179638 | 947 |
| 523753 | ACAGTCCTCATGAACAGATT | Intron 2 | 37 | 180513 | 180532 | 948 |
| 523754 | ACTATCATTAATAATATTGT | Intron 2 | 0 | 181323 | 181342 | 949 |
| 523755 | ATCTAGATTTGCCTTATAAG | Intron 2 | 27 | 182123 | 182142 | 950 |
| 523756 | TGGTTGAGGAAGACAGTCTC | Intron 2 | 16 | 182962 | 182981 | 951 |
| 523757 | TGGCTCATAACTTCCTTAGC | Intron 2 | 43 | 183762 | 183781 | 952 |
| 523758 | ACTAACTTAGGACTTGGGAC | Intron 2 | 72 | 184578 | 184597 | 953 |
| 523759 | CTTATAGCATTACTAAGTGG | Intron 2 | 49 | 185403 | 185422 | 954 |
| 523760 | TGGTGGCAGGAGAGAGGGAA | Intron 2 | 48 | 186203 | 186222 | 955 |
| 523761 | TTTGCCAGGAAATCTTGAAA | Intron 2 | 35 | 187003 | 187022 | 956 |
| 523762 | ATAACTTTTCTCTGAAATTT | Intron 2 | 8 | 187803 | 187822 | 957 |
| 523763 | CCATGGGCTAGATCAGGATT | Intron 2 | 59 | 188620 | 188639 | 958 |
| 523764 | TGAGCTTAGCAGTCACTTAG | Intron 2 | 62 | 189481 | 189500 | 959 |
| 523765 | AATGTCTCAGCAAACCTGGG | Intron 2 | 62 | 190286 | 190305 | 960 |
| 523766 | GGATACAGACATAGCTCTAA | Intron 2 | 75 | 191142 | 191161 | 961 |
| 523767 | ACAACACTGTTTGTGAGGGT | Intron 2 | 66 | 191942 | 191961 | 962 |
| 523768 | TCTATTTTCTAATAGCTGTT | Intron 2 | 49 | 192742 | 192761 | 963 |
| 523769 | GGCCCCACCTCTGACCTTCA | Intron 2 | 7 | 193542 | 193561 | 964 |
| 523770 | TGGTAAAGCTAGAAAAAAAA | Intron 2 | 0 | 194346 | 194365 | 965 |
| 523771 | AAGTGGTAAATATGATCACA | Intron 2 | 23 | 195159 | 195178 | 966 |
| 523772 | GGCTTCAAGTTTAGGATCTG | Intron 2 | 52 | 196539 | 196558 | 967 |
| 523773 | TTGTTGACACTCTCTTTTGG | Intron 2 | 18 | 197348 | 197367 | 968 |
| 523774 | GTATGTGGCTTTAATTCACT | Intron 2 | 71 | 198148 | 198167 | 969 |
| 523775 | AATTAGTTGTTTTGGCAAAT | Intron 2 | 14 | 198988 | 199007 | 970 |
| 523776 | CTGTGTTCAGTTGCATCACG | Intron 2 | 75 | 199816 | 199835 | 971 |
| 523777 | AATGTGGAAGTTTCCTAACA | Intron 2 | 15 | 200616 | 200635 | 972 |
| 523778 | TTGGTACTCATGAGGAGGCA | Intron 2 | 58 | 201416 | 201435 | 973 |
| 523779 | TTTCTCTGTGTTTAAAATTG | Intron 2 | 13 | 202308 | 202327 | 974 |
| 523780 | GTAAAGCACAATGAACAAAA | Intron 2 | 21 | 203115 | 203134 | 975 |
| 523781 | ATCACAGATCTTTGCTACAA | Intron 2 | 51 | 203915 | 203934 | 976 |
| 523782 | TCCTGCCTTTCTGAACCAAA | Intron 2 | 50 | 204721 | 204740 | 977 |
| 523783 | TGGAAACTCTGCAACTCCAA | Intron 2 | 58 | 205552 | 205571 | 978 |
| 523784 | ACACAGTAGGGAACAATTTT | Intron 2 | 8 | 206412 | 206431 | 979 |
| 523785 | AGACAGATGGTGAAATGATG | Intron 2 | 0 | 207219 | 207238 | 980 |
| 523786 | AAACAGAAAGAGAAGAAAAC | Intron 2 | 0 | 208117 | 208136 | 981 |
| 523787 | CTTAGATAAATACTTCAAGA | Intron 3 | 0 | 208938 | 208957 | 982 |
| 523788 | AGCCACTTCTTTTACAACCT | Intron 3 | 0 | 209742 | 209761 | 983 |
| 523789 | TCACATCTGGATGTGAGGCT | Intron 3 | 80 | 210561 | 210580 | 984 |
| 523790 | GACTGAAACTTAAAGGTGGG | Intron 3 | 7 | 211399 | 211418 | 985 |
| 523791 | AAAGATGTGCAATCATCTAA | Intron 3 | 44 | 212204 | 212223 | 986 |

**Table 15**

| Inhibition of GHR mRNA by 3-10-4 MOE gapmers targeting introns 2 and 3 of SEQ ID NO: 2 | | | | | | |
|---|---|---|---|---|---|---|
| ISIS NO | Sequence | Target region | % inhibition | SEQ ID NO: 2 Start Site | SEQ ID NO: 2 Stop Site | SEQ ID NO |
| 539360 | GCTGGTGACTCTGCCTG | Intron 2 | 95 | 151389 | 151405 | 987 |
| 539361 | TGCTGGTGACTCTGCCT | Intron 2 | 95 | 151390 | 151406 | 988 |
| 539362 | CTGCTGGTGACTCTGCC | Intron 2 | 93 | 151391 | 151407 | 989 |
| 539363 | AGTAGTCAATATTATTT | Intron 2 | 31 | 153001 | 153017 | 990 |
| 539364 | CAGTAGTCAATATTATT | Intron 2 | 13 | 153002 | 153018 | 991 |
| 539365 | CCAGTAGTCAATATTAT | Intron 2 | 34 | 153003 | 153019 | 992 |
| 539366 | CCTTTGGGTGAATAGCA | Intron 2 | 64 | 153921 | 153937 | 993 |
| 539367 | ACCTTTGGGTGAATAGC | Intron 2 | 78 | 153922 | 153938 | 994 |
| 539368 | CACCTTTGGGTGAATAG | Intron 2 | 40 | 153923 | 153939 | 995 |
| 539369 | CAACTTGAGGACAATAA | Intron 2 | 38 | 155594 | 155610 | 996 |
| 539370 | TCAACTTGAGGACAATA | Intron 2 | 63 | 155595 | 155611 | 997 |
| 539371 | CTCAACTTGAGGACAAT | Intron 2 | 81 | 155596 | 155612 | 998 |
| 539372 | CAGGAAGAAAGGAACCT | Intron 2 | 70 | 156394 | 156410 | 999 |
| 539373 | CCAGGAAGAAAGGAACC | Intron 2 | 59 | 156395 | 156411 | 1000 |
| 539374 | ACCAGGAAGAAAGGAAC | Intron 2 | 43 | 156396 | 156412 | 1001 |
| 539375 | TGCAGTCATGTACACAA | Intron 2 | 93 | 156594 | 156610 | 1002 |
| 539376 | CTGCAGTCATGTACACA | Intron 2 | 91 | 156595 | 156611 | 1003 |
| 539377 | TCTGCAGTCATGTACAC | Intron 2 | 87 | 156596 | 156612 | 1004 |
| 539378 | TGGTTTGTCAATCCTTT | Intron 2 | 95 | 156889 | 156905 | 1005 |
| 539379 | TTGGTTTGTCAATCCTT | Intron 2 | 97 | 156890 | 156906 | 1006 |
| 539380 | CTTGGTTTGTCAATCCT | Intron 2 | 97 | 156891 | 156907 | 1007 |
| 539381 | TACAATGCACAGGACAC | Intron 2 | 65 | 157201 | 157217 | 1008 |
| 539382 | CTACAATGCACAGGACA | Intron 2 | 85 | 157202 | 157218 | 1009 |
| 539383 | GCTACAATGCACAGGAC | Intron 2 | 96 | 157203 | 157219 | 1010 |
| 539384 | GATATTTATTGCTGTAC | Intron 2 | 43 | 158007 | 158023 | 1011 |
| 539385 | TGATATTTATTGCTGTA | Intron 2 | 35 | 158008 | 158024 | 1012 |
| 539386 | CTGATATTTATTGCTGT | Intron 2 | 38 | 158009 | 158025 | 1013 |
| 539387 | AGGGTCTTTACAAAGTT | Intron 2 | 61 | 162751 | 162767 | 1014 |
| 539388 | CAGGGTCTTTACAAAGT | Intron 2 | 65 | 162752 | 162768 | 1015 |
| 539389 | CCAGGGTCTTTACAAAG | Intron 2 | 88 | 162753 | 162769 | 1016 |
| 539390 | TTCTGCAGTATCCTAGC | Intron 2 | 72 | 166352 | 166368 | 1017 |
| 539391 | TTTCTGCAGTATCCTAG | Intron 2 | 53 | 166353 | 166369 | 1018 |
| 539392 | GTTTCTGCAGTATCCTA | Intron 2 | 84 | 166354 | 166370 | 1019 |
| 539393 | AGTTTCTGCAGTATCCT | Intron 2 | 78 | 166355 | 166371 | 1020 |
| 539394 | CAGTTTCTGCAGTATCC | Intron 2 | 77 | 166356 | 166372 | 1021 |
| 539395 | CAAATTCCAGTCCTAGG | Intron 2 | 60 | 172746 | 172762 | 1022 |
| 539396 | CCAAATTCCAGTCCTAG | Intron 2 | 75 | 172747 | 172763 | 1023 |
| 539397 | TCCAAATTCCAGTCCTA | Intron 2 | 62 | 172748 | 172764 | 1024 |
| 539398 | AACCCATTTCATCCATT | Intron 2 | 82 | 175372 | 175388 | 1025 |
| 539399 | GAACCCATTTCATCCAT | Intron 2 | 86 | 175373 | 175389 | 1026 |
| 539400 | GGAACCCATTTCATCCA | Intron 2 | 84 | 175374 | 175390 | 1027 |
| 539401 | GCTTCATGTCTTTCTAG | Intron 2 | 88 | 189119 | 189135 | 1028 |
| 539402 | TGCTTCATGTCTTTCTA | Intron 2 | 77 | 189120 | 189136 | 1029 |
| 539403 | GTGCTTCATGTCTTTCT | Intron 2 | 95 | 189121 | 189137 | 1030 |
| 539404 | TGAGCTTAGCAGTCACT | Intron 2 | 92 | 189484 | 189500 | 1031 |
| 539405 | CATGAGCTTAGCAGTCA | Intron 2 | 82 | 189486 | 189502 | 1032 |
| 539406 | TACAGACATAGCTCTAA | Intron 2 | 45 | 191142 | 191158 | 1033 |
| 539407 | ATACAGACATAGCTCTA | Intron 2 | 53 | 191143 | 191159 | 1034 |
| 539408 | GATACAGACATAGCTCT | Intron 2 | 67 | 191144 | 191160 | 1035 |
| 539409 | TGTGGCTTTAATTCACT | Intron 2 | 70 | 198148 | 198164 | 1036 |
| 539410 | ATGTGGCTTTAATTCAC | Intron 2 | 40 | 198149 | 198165 | 1037 |
| 539411 | TATGTGGCTTTAATTCA | Intron 2 | 35 | 198150 | 198166 | 1038 |
| 539412 | TGTTCAGTTGCATCACG | Intron 2 | 84 | 199816 | 199832 | 1039 |
| 539413 | GTGTTCAGTTGCATCAC | Intron 2 | 80 | 199817 | 199833 | 1040 |
| 539414 | TGTGTTCAGTTGCATCA | Intron 2 | 74 | 199818 | 199834 | 1041 |
| 539415 | CATCTGGATGTGAGGCT | Intron 3 | 82 | 210561 | 210577 | 1042 |
| 539416 | ACATCTGGATGTGAGGC | Intron 3 | 86 | 210562 | 210578 | 1043 |
| 539417 | CACATCTGGATGTGAGG | Intron 3 | 55 | 210563 | 210579 | 1044 |
| 539418 | TCAGGTAATTTCTGGAA | Intron 3 | 35 | 219019 | 219035 | 1045 |
| 539419 | CTCAGGTAATTTCTGGA | Intron 3 | 44 | 219020 | 219036 | 1046 |
| 539420 | TCTCAGGTAATTTCTGG | Intron 3 | 31 | 219021 | 219037 | 1047 |
| 539421 | TTGCTTATTTACCTGGG | Intron 3 | 0 | 225568 | 225584 | 1048 |
| 539422 | TTTGCTTATTTACCTGG | Intron 3 | 38 | 225569 | 225585 | 1049 |
| 539423 | TTTTGCTTATTTACCTG | Intron 3 | 33 | 225570 | 225586 | 1050 |
| 539424 | ATGATGTTACTACTACT | Intron 3 | 29 | 229618 | 229634 | 1051 |
| 539425 | AATGATGTTACTACTAC | Intron 3 | 10 | 229619 | 229635 | 1052 |
| 539426 | CAATGATGTTACTACTA | Intron 3 | 0 | 229620 | 229636 | 1053 |
| 539427 | CCCCTAGAGCAATGGTC | Intron 3 | 67 | 232826 | 232842 | 1054 |
| 539428 | CCCCCTAGAGCAATGGT | Intron 3 | 65 | 232827 | 232843 | 1055 |
| 539429 | TCCCCCTAGAGCAATGG | Intron 3 | 45 | 232828 | 232844 | 1056 |
| 539430 | TCAATTGCAGATGCTCT | Intron 3 | 78 | 237675 | 237691 | 1057 |
| 539431 | CTCAATTGCAGATGCTC | Intron 3 | 82 | 237676 | 237692 | 1058 |
| 539432 | GCTCAATTGCAGATGCT | Intron 3 | 92 | 237677 | 237693 | 1059 |
| 539433 | AGCTCAATTGCAGATGC | Intron 3 | 85 | 237678 | 237694 | 1060 |
| 539434 | GTATATTCAGTCCAAGG | Intron 3 | 73 | 248231 | 248247 | 1061 |
| 539435 | AGTATATTCAGTCCAAG | Intron 3 | 70 | 248232 | 248248 | 1062 |
| 539436 | CAGTATATTCAGTCCAA | Intron 3 | 40 | 248233 | 248249 | 1063 |

**Table 16**

| Inhibition of GHR mRNA by 5-10-5 MOE gapmers targeting introns 1 and 3 of SEQ ID NO: 2 | | | | | | |
|---|---|---|---|---|---|---|
| ISIS NO | Sequence | Target region | % inhibition | SEQ ID NO: 2 Start Site | SEQ ID NO: 2 Stop Site | SEQ ID NO |
| 532502 | GAGTATTTCAGGCTGGAAAA | Intron 3 | 43 | 214623 | 214642 | 1064 |
| 533404 | GTAACTCAGGAATGGAAAAC | Intron 1 | 56 | 26501 | 26520 | 1065 |
| | | | | 113035 | 113054 | |
| | | | | 121992 | 122011 | |
| 533405 | AGTAACTCAGGAATGGAAAA | Intron 1 | 41 | 26502 | 26521 | 1066 |
| | | | | 113036 | 113055 | |
| | | | | 121993 | 122012 | |
| 533406 | AAGTAACTCAGGAATGGAAA | Intron 1 | 43 | 26503 | 26522 | 1067 |
| | | | | 113037 | 113056 | |
| | | | | 121994 | 122013 | |
| 533407 | GAGATTTCAAATAAATCTCA | Intron 1 | 0 | 143207 | 143226 | 1068 |
| | | | | 143235 | 143254 | |
| | | | | 143263 | 143282 | |
| | | | | 143291 | 143310 | |
| | | | | 143319 | 143338 | |
| | | | | 143347 | 143366 | |
| | | | | 143375 | 143394 | |
| | | | | 143403 | 143422 | |
| | | | | 143431 | 143450 | |
| | | | | 143459 | 143478 | |
| 533408 | TGAGATTTCAAATAAATCTC | Intron 1 | 11 | 143208 | 143227 | 1069 |
| | | | | 143236 | 143255 | |
| | | | | 143264 | 143283 | |
| | | | | 143292 | 143311 | |
| | | | | 143320 | 143339 | |
| | | | | 143348 | 143367 | |
| | | | | 143376 | 143395 | |
| | | | | 143404 | 143423 | |
| | | | | 143432 | 143451 | |
| | | | | 143460 | 143479 | |
| 533409 | GTGAGATTTCAAATAAATCT | Intron 1 | 0 | 143209 | 143228 | 1070 |
| | | | | 143237 | 143256 | |
| | | | | 143265 | 143284 | |
| | | | | 143293 | 143312 | |
| | | | | 143321 | 143340 | |
| | | | | 143349 | 143368 | |
| | | | | 143377 | 143396 | |
| | | | | 143405 | 143424 | |
| | | | | 143433 | 143452 | |
| | | | | 143461 | 143480 | |
| 533410 | TGTGAGATTTCAAATAAATC | Intron 1 | 0 | 143210 | 143229 | 1071 |
| | | | | 143238 | 143257 | |
| | | | | 143266 | 143285 | |
| | | | | 143294 | 143313 | |
| | | | | 143322 | 143341 | |
| | | | | 143350 | 143369 | |
| | | | | 143378 | 143397 | |
| | | | | 143406 | 143425 | |
| | | | | 143434 | 143453 | |
| | | | | 143462 | 143481 | |
| 533411 | TTGTGAGATTTCAAATAAAT | Intron 1 | 10 | 143183 | 143202 | 1072 |
| | | | | 143211 | 143230 | |
| | | | | 143239 | 143258 | |
| | | | | 143267 | 143286 | |
| | | | | 143295 | 143314 | |
| | | | | 143323 | 143342 | |
| | | | | 143351 | 143370 | |
| | | | | 143379 | 143398 | |
| | | | | 143407 | 143426 | |
| | | | | 143435 | 143454 | |
| | | | | 143463 | 143482 | |
| 533412 | TTTGTGAGATTTCAAATAAA | Intron 1 | 0 | 143184 | 143203 | 1073 |
| | | | | 143212 | 143231 | |
| | | | | 143240 | 143259 | |
| | | | | 143296 | 143315 | |
| | | | | 143324 | 143343 | |
| | | | | 143352 | 143371 | |
| | | | | 143380 | 143399 | |
| | | | | 143464 | 143483 | |
| 533413 | CTTTGTGAGATTTCAAATAA | Intron 1 | 20 | 143185 | 143204 | 1074 |
| | | | | 143213 | 143232 | |
| | | | | 143241 | 143260 | |
| | | | | 143297 | 143316 | |
| | | | | 143325 | 143344 | |
| | | | | 143353 | 143372 | |
| | | | | 143381 | 143400 | |
| | | | | 143465 | 143484 | |
| 533414 | ACTTTGTGAGATTTCAAATA | Intron 1 | 57 | 143186 | 143205 | 1075 |
| | | | | 143214 | 143233 | |
| | | | | 143242 | 143261 | |
| | | | | 143298 | 143317 | |
| | | | | 143326 | 143345 | |
| | | | | 143354 | 143373 | |
| | | | | 143382 | 143401 | |
| | | | | 143466 | 143485 | |
| 533415 | CACTTTGTGAGATTTCAAAT | Intron 1 | 69 | 143187 | 143206 | 1076 |
| | | | | 143215 | 143234 | |
| | | | | 143243 | 143262 | |
| | | | | 143299 | 143318 | |
| | | | | 143327 | 143346 | |
| | | | | 143355 | 143374 | |
| | | | | 143383 | 143402 | |
| | | | | 143467 | 143486 | |
| 533895 | AGTATTTCAGGCTGGAAAAA | Intron 3 | 35 | 214622 | 214641 | 1077 |
| 533896 | TGAGTATTTCAGGCTGGAAA | Intron 3 | 55 | 214624 | 214643 | 1078 |
| 533897 | TCTGAGTATTTCAGGCTGGA | Intron 3 | 71 | 214626 | 214645 | 1079 |
| 533898 | ATCTGAGTATTTCAGGCTGG | Intron 3 | 77 | 214627 | 214646 | 1080 |
| 533899 | TATCTGAGTATTTCAGGCTG | Intron 3 | 58 | 214628 | 214647 | 1081 |
| 533900 | TTTTGTGTTATGCCTTGAGG | Intron 3 | 51 | 221483 | 221502 | 1082 |
| 533901 | TTTTTGTGTTATGCCTTGAG | Intron 3 | 55 | 221484 | 221503 | 1083 |
| 533902 | ATTTTTGTGTTATGCCTTGA | Intron 3 | 57 | 221485 | 221504 | 1084 |
| 533903 | ATATTTTTGTGTTATGCCTT | Intron 3 | 56 | 221487 | 221506 | 1085 |
| 533904 | AATATTTTTGTGTTATGCCT | Intron 3 | 61 | 221488 | 221507 | 1086 |
| 533905 | AAATATTTTTGTGTTATGCC | Intron 3 | 18 | 221489 | 221508 | 1087 |
| 533906 | TTGCTTATTTACCTGGGTAA | Intron 3 | 58 | 225565 | 225584 | 1088 |
| 533907 | TTTGCTTATTTACCTGGGTA | Intron 3 | 64 | 225566 | 225585 | 1089 |
| 533908 | TTTTGCTTATTTACCTGGGT | Intron 3 | 77 | 225567 | 225586 | 1090 |
| 533909 | CCTTTTGCTTATTTACCTGG | Intron 3 | 69 | 225569 | 225588 | 1091 |
| 533910 | GCCTTTTGCTTATTTACCTG | Intron 3 | 69 | 225570 | 225589 | 1092 |
| 533911 | TGCCTTTTGCTTATTTACCT | Intron 3 | 55 | 225571 | 225590 | 1093 |
| 533912 | ATGATGTTACTACTACTCAA | Intron 3 | 60 | 229615 | 229634 | 1094 |
| 533913 | AATGATGTTACTACTACTCA | Intron 3 | 48 | 229616 | 229635 | 1095 |
| 533914 | CAATGATGTTACTACTACTC | Intron 3 | 57 | 229617 | 229636 | 1096 |
| 533915 | TCCAATGATGTTACTACTAC | Intron 3 | 69 | 229619 | 229638 | 1097 |
| 533916 | TTCCAATGATGTTACTACTA | Intron 3 | 74 | 229620 | 229639 | 1098 |
| 533917 | ATTCCAATGATGTTACTACT | Intron 3 | 74 | 229621 | 229640 | 1099 |
| 533918 | CCCCTAGAGCAATGGTCTAG | Intron 3 | 71 | 232823 | 232842 | 1100 |
| 533919 | CCCCCTAGAGCAATGGTCTA | Intron 3 | 44 | 232824 | 232843 | 1101 |
| 533920 | TCCCCCTAGAGCAATGGTCT | Intron 3 | 54 | 232825 | 232844 | 1102 |
| 533921 | TATCCCCCTAGAGCAATGGT | Intron 3 | 62 | 232827 | 232846 | 1103 |
| 533922 | ATATCCCCCTAGAGCAATGG | Intron 3 | 50 | 232828 | 232847 | 1104 |
| 533923 | AATATCCCCCTAGAGCAATG | Intron 3 | 61 | 232829 | 232848 | 1105 |
| 533924 | GCTCACATTTGGAAGACAGT | Intron 3 | 68 | 233623 | 233642 | 1106 |
| 533925 | GGCTCACATTTGGAAGACAG | Intron 3 | 74 | 233624 | 233643 | 1107 |
| 533926 | AGGCTCACATTTGGAAGACA | Intron 3 | 56 | 233625 | 233644 | 1108 |
| 533927 | AGAGGCTCACATTTGGAAGA | Intron 3 | 34 | 233627 | 233646 | 1109 |
| 533928 | TAGAGGCTCACATTTGGAAG | Intron 3 | 18 | 233628 | 233647 | 1110 |
| 533929 | TTAGAGGCTCACATTTGGAA | Intron 3 | 19 | 233629 | 233648 | 1111 |
| 533930 | CTCAATTGCAGATGCTCTGA | Intron 3 | 66 | 237673 | 237692 | 1112 |
| 533931 | GCTCAATTGCAGATGCTCTG | Intron 3 | 72 | 237674 | 237693 | 1113 |
| 533932 | AGCTCAATTGCAGATGCTCT | Intron 3 | 74 | 237675 | 237694 | 1114 |
| 533933 | AAAGCTCAATTGCAGATGCT | Intron 3 | 66 | 237677 | 237696 | 1115 |
| 533934 | TAAAGCTCAATTGCAGATGC | Intron 3 | 59 | 237678 | 237697 | 1116 |
| 533935 | ATAAAGCTCAATTGCAGATG | Intron 3 | 23 | 237679 | 237698 | 1117 |
| 533936 | GTGAGTCCATTAAACCTCTT | Intron 3 | 73 | 244873 | 244892 | 1118 |
| 533937 | TGTGAGTCCATTAAACCTCT | Intron 3 | 73 | 244874 | 244893 | 1119 |
| 533938 | ACTGTGAGTCCATTAAACCT | Intron 3 | 17 | 244876 | 244895 | 1120 |
| 533939 | AACTGTGAGTCCATTAAACC | Intron 3 | 19 | 244877 | 244896 | 1121 |
| 533940 | GAACTGTGAGTCCATTAAAC | Intron 3 | 28 | 244878 | 244897 | 1122 |
| 533941 | ATATTGAAAGGCCCATCAAA | Intron 3 | 13 | 246498 | 246517 | 1123 |
| 533942 | AATATTGAAAGGCCCATCAA | Intron 3 | 31 | 246499 | 246518 | 1124 |
| 533943 | AAATATTGAAAGGCCCATCA | Intron 3 | 51 | 246500 | 246519 | 1125 |
| 533944 | GAAAATATTGAAAGGCCCAT | Intron 3 | 22 | 246502 | 246521 | 1126 |
| 533945 | GGAAAATATTGAAAGGCCCA | Intron 3 | 42 | 246503 | 246522 | 1127 |
| 533946 | AGGAAAATATTGAAAGGCCC | Intron 3 | 28 | 246504 | 246523 | 1128 |
| 533947 | GTATATTCAGTCCAAGGATC | Intron 3 | 65 | 248228 | 248247 | 1129 |
| 533948 | AGTATATTCAGTCCAAGGAT | Intron 3 | 63 | 248229 | 248248 | 1130 |
| 533949 | CAGTATATTCAGTCCAAGGA | Intron 3 | 67 | 248230 | 248249 | 1131 |
| 533950 | AACAGTATATTCAGTCCAAG | Intron 3 | 56 | 248232 | 248251 | 1132 |
| 533951 | AAACAGTATATTCAGTCCAA | Intron 3 | 60 | 248233 | 248252 | 1133 |
| 533952 | AAAACAGTATATTCAGTCCA | Intron 3 | 59 | 248234 | 248253 | 1134 |
| 533953 | TCTATTGTTGCCACCTTTAT | Intron 3 | 45 | 252838 | 252857 | 1135 |
| 533954 | TTCTATTGTTGCCACCTTTA | Intron 3 | 52 | 252839 | 252858 | 1136 |
| 533955 | TTTCTATTGTTGCCACCTTT | Intron 3 | 46 | 252840 | 252859 | 1137 |
| 533956 | AGTTTCTATTGTTGCCACCT | Intron 3 | 59 | 252842 | 252861 | 1138 |
| 533957 | CAGTTTCTATTGTTGCCACC | Intron 3 | 41 | 252843 | 252862 | 1139 |
| 533958 | CCAGTTTCTATTGTTGCCAC | Intron 3 | 48 | 252844 | 252863 | 1140 |

**Table 17**

| Inhibition of GHR mRNA by 5-10-5 MOE gapmers targeting intron 3 of SEQ ID NO: 2 | | | | | |
|---|---|---|---|---|---|
| ISIS NO | Sequence | % inhibition | SEQ ID NO: 2 Start Site | SEQ ID NO: 2 Stop Site | SEQ ID NO |
| 532454 | GCAGAACTGATTGCTTACTT | 78 | 182862 | 182881 | 1141 |
| 532455 | AGGTCATAAGATTTTCATTT | 48 | 183533 | 183552 | 1142 |
| 532456 | GCCTCTGGCCATAAAGAAAT | 54 | 183578 | 183597 | 1143 |
| 532457 | AAAGTTTAAGAGGCACCCCA | 31 | 184508 | 184527 | 1144 |
| 532458 | GAATAAGCACAAAAGTTTAA | 28 | 184519 | 184538 | 1145 |
| 532459 | GAACCAAATAAACCTCTCTT | 52 | 185452 | 185471 | 1146 |
| 532460 | ATGTTGAAATTTGATCCCCA | 79 | 185763 | 185782 | 1147 |
| 532461 | TGTGAGAGCTCACTCACTAT | 42 | 186134 | 186153 | 1148 |
| 532462 | CTTGTGAGAGCTCACTCACT | 72 | 186136 | 186155 | 1149 |
| 532463 | ACATGGTGGCAGGAGAGAGG | 42 | 186206 | 186225 | 1150 |
| 532464 | CTAGAAAGAAACTACCTGAG | 12 | 186341 | 186360 | 1151 |
| 532465 | AACTTCAGTTGTAAAATAAT | 27 | 187044 | 187063 | 1152 |
| 532466 | GAAAAGGATTTTGAGATTTC | 43 | 188897 | 188916 | 1153 |
| 532467 | CTTAGCTGTCAAGGCCCTTT | 80 | 189084 | 189103 | 1154 |
| 532468 | TGTGCTTCATGTCTTTCTAG | 88 | 189119 | 189138 | 1155 |
| 532469 | CCCTTGAACATGCTATCCTT | 85 | 189256 | 189275 | 1156 |
| 532470 | CTTGCAGGGATGCATCTCAG | 87 | 189625 | 189644 | 1157 |
| 532471 | TCTCTTGCACATCTAATTTC | 82 | 189656 | 189675 | 1158 |
| 532472 | CTTCCAGCACAACCCATCAC | 77 | 190109 | 190128 | 1159 |
| 532473 | GTAACTACATTCCCTTTATC | 52 | 190860 | 190879 | 1160 |
| 532474 | AGTAACTACATTCCCTTTAT | 58 | 190861 | 190880 | 1161 |
| 532475 | CAGATAGCACAGGGCTAAAA | 84 | 190979 | 190998 | 1162 |
| 532476 | AGAATCAGGAATGTTTGCCT | 86 | 192904 | 192923 | 1163 |
| 532477 | TGACTCAATCATTTAGACTT | 45 | 192990 | 193009 | 1164 |
| 532478 | TCAACAGTCAATGGACTTGT | 71 | 193042 | 193061 | 1165 |
| 532479 | AATTTCTACTGCTATGATGC | 75 | 194806 | 194825 | 1166 |
| 532480 | ATGGTTCCAAATTTCTATCT | 86 | 195704 | 195723 | 1167 |
| 532481 | CTGTATGGCTTTAAGTATTC | 63 | 196756 | 196775 | 1168 |
| 532482 | AACTTATGAACTGTTCACCA | 86 | 198307 | 198326 | 1169 |
| 532483 | AATAAGCTTGAAGTCTGAAG | 63 | 199520 | 199539 | 1170 |
| 532484 | TAGTTATCTAACTGCCCAAT | 77 | 199544 | 199563 | 1171 |
| 532485 | TTCTGCAAAGCTTCCCAGTA | 72 | 200314 | 200333 | 1172 |
| 532486 | ACAACTTCAAGCTTCACATA | 65 | 200599 | 200618 | 1173 |
| 532487 | GAATCAATGTTCTGGCAAGA | 52 | 201842 | 201861 | 1174 |
| 532488 | CAGCCTTTCAGCTGTGAAAG | 52 | 204181 | 204200 | 1175 |
| 532489 | AACAATGCCAAGAAATCTAT | 74 | 204369 | 204388 | 1176 |
| 532490 | CCCACAGTAACAATGCCAAG | 90 | 204377 | 204396 | 1177 |
| 532491 | TTTTACCTCCCAGTGAAACT | 34 | 205896 | 205915 | 1178 |
| 532492 | TAATTGTTGATCCATGATGT | 5 | 208856 | 208875 | 1179 |
| 532493 | GTTGGAGAGACAAGTTTAAC | 29 | 208975 | 208994 | 1180 |
| 532494 | AGTCATAAAATTCAAATTAT | 39 | 209537 | 209556 | 1181 |
| 532495 | GGCCTTGGGCACACTTTCTC | 82 | 207510 | 207529 | 1182 |
| | | | 210189 | 210208 | |
| 532496 | AAGTTTTTATTGAAGTTAAT | 0 | 212551 | 212570 | 1183 |
| 532497 | AAGAAAAATTAGGAAGCTAG | 31 | 212649 | 212668 | 1184 |
| 532498 | CAGGGAGATAAGTTTATTCA | 61 | 212797 | 212816 | 1185 |
| 532499 | ATTTAATACACATTGGAATA | 15 | 213390 | 213409 | 1186 |
| 532500 | GTAGGACTATTTATGATTCC | 86 | 213914 | 213933 | 1187 |
| 532501 | CACTCTCTTGGGCTGTTAAG | 82 | 214479 | 214498 | 1188 |
| 532502 | GAGTATTTCAGGCTGGAAAA | 66 | 214623 | 214642 | 1064 |
| 532503 | TTGTTTGAGTTCCAAAAGAA | 39 | 214932 | 214951 | 1189 |
| 532504 | TTTGCCATGAGACACACAAT | 77 | 215932 | 215951 | 1190 |
| 532505 | CACCAAACCTCAGAGACATG | 80 | 216468 | 216487 | 1191 |
| 532506 | CCACTGTTAAGTGATGCATG | 83 | 217480 | 217499 | 1192 |
| 532507 | CTCTCAGGTAATTTCTGGAA | 86 | 219019 | 219038 | 1193 |
| 532508 | GCTCCTCACAATGACCCTTT | 84 | 219452 | 219471 | 1194 |
| 532509 | GGGACTGGCACTGGTAATTT | 56 | 220062 | 220081 | 1195 |
| 532510 | CTAACCATTAGTTACTGTAT | 69 | 220558 | 220577 | 1196 |
| 532511 | GGATTTTAGGTTCTTGCTGT | 51 | 221588 | 221607 | 1197 |
| 532512 | TGAATCATATACTGATATCA | 63 | 222914 | 222933 | 1198 |
| 532513 | TTGAGGTATTAAATTTTAAA | 0 | 223001 | 223020 | 1199 |
| 532514 | AGTTTGTAATGTAGTGATTT | 19 | 223156 | 223175 | 1200 |
| 532515 | AAATATTTGATAGCTCACAT | 18 | 224409 | 224428 | 1201 |
| 532516 | AGAAATATTTGATAGCTCAC | 57 | 224411 | 224430 | 1202 |
| 532517 | CCACATTTCAAATGTTCTCT | 80 | 224717 | 224736 | 1203 |
| 532518 | GCAGGAAGAGTGGCATGGAC | 59 | 224750 | 224769 | 1204 |
| 532519 | CACTTATCCAAATGCAGAGA | 82 | 225742 | 225761 | 1205 |
| 532520 | CAAGGTAATGGGAGGCTAGC | 47 | 225903 | 225922 | 1206 |
| 532521 | ATAGTCAAAGCTAAGGATAT | 4 | 226177 | 226196 | 1207 |
| 532522 | GTAATTTCATTCATGCTTCC | 67 | 226804 | 226823 | 1208 |
| 532523 | GTCCACATTCAGCTGTGTGT | 72 | 231912 | 231931 | 1209 |
| 532524 | TCATTCAGGAAATTCTGCTA | 62 | 232286 | 232305 | 1210 |
| 532525 | AACATGTCTCATTCAGGAAA | 71 | 232294 | 232313 | 1211 |
| 532526 | TAACATGTCTCATTCAGGAA | 85 | 232295 | 232314 | 1212 |
| 532527 | AGATTCCTCAAATTCAGTGA | 66 | 232389 | 232408 | 1213 |
| 532528 | TAAGCGGAAAAGGAGAAAAG | 0 | 233684 | 233703 | 1214 |
| 532529 | AAAGCAAGAGAATTCCTAAA | 32 | 234203 | 234222 | 1215 |
| 532530 | AATGAACCTTTAACTTAGTA | 40 | 234876 | 234895 | 1216 |

**Table 18**

| Inhibition of GHR mRNA by 5-10-5 MOE gapmers targeting introns 3-8 and intron-exonic regions of SEQ ID NO: 2 | | | | | | |
|---|---|---|---|---|---|---|
| ISIS NO | Sequence | Target region | % inhibition | SEQ ID NO: 2 Start Site | SEQ ID NO: 2 Stop Site | SEQ ID NO |
| 523792 | AAAGCTTTGTGGATAAAGTT | Intron 3 | 44 | 213025 | 213044 | 1217 |
| 523793 | GAAGGAAAGGTTCTGTGGAA | Intron 3 | 38 | 213825 | 213844 | 1218 |
| 523794 | CTGAGTATTTCAGGCTGGAA | Intron 3 | 84 | 214625 | 214644 | 1219 |
| 523795 | TTGAATTATCCCTTTAAAAA | Intron 3 | 38 | 215446 | 215465 | 1220 |
| 523796 | TTTAGAATGGTTTGGCATAC | Intron 3 | 66 | 216365 | 216384 | 1221 |
| 523797 | GATATGTCCACATTGATTAG | Intron 3 | 65 | 218132 | 218151 | 1222 |
| 523798 | ATTATTTAAGCTTCTACTTT | Intron 3 | 44 | 218973 | 218992 | 1223 |
| 523799 | ATACATGGCAATTAAAAGAT | Intron 3 | 26 | 219886 | 219905 | 1224 |
| 523800 | TGAGATAGTGTGGGAAATAT | Intron 3 | 18 | 220686 | 220705 | 1225 |
| 523801 | TATTTTTGTGTTATGCCTTG | Intron 3 | 73 | 221486 | 221505 | 1226 |
| 523802 | TTATTAACTAGAATATGCCT | Intron 3 | 16 | 223110 | 223129 | 1227 |
| 523803 | GATTATTCTATTTTTATTTT | Intron 3 | 33 | 223948 | 223967 | 1228 |
| 523804 | AGGAAGAGTGGCATGGACAT | Intron 3 | 43 | 224748 | 224767 | 1229 |
| 523805 | CTTTTGCTTATTTACCTGGG | Intron 3 | 84 | 225568 | 225587 | 1230 |
| 523806 | TTTATATTATTAATATCATT | Intron 3 | 31 | 226371 | 226390 | 1231 |
| 523807 | GGTACATGGCTTTTAAGTGG | Intron 3 | 53 | 227218 | 227237 | 1232 |
| 523808 | AATATTGGTCAGGTTTAAGA | Intron 3 | 28 | 228018 | 228037 | 1233 |
| 523809 | ATTTCATCTCTTTCTTAGTT | Intron 3 | 45 | 228818 | 228837 | 1234 |
| 523810 | CCAATGATGTTACTACTACT | Intron 3 | 89 | 229618 | 229637 | 1235 |
| 523811 | GTTCCCCCAACCCCTTGGAA | Intron 3 | 28 | 230418 | 230437 | 1236 |
| 523812 | TATAGGAAGTGAGATGTATG | Intron 3 | 46 | 231218 | 231237 | 1237 |
| 523813 | ATTATTCTAGAAGAAGATTT | Intron 3 | 12 | 232018 | 232037 | 1238 |
| 523814 | ATCCCCCTAGAGCAATGGTC | Intron 3 | 79 | 232826 | 232845 | 1239 |
| 523815 | GAGGCTCACATTTGGAAGAC | Intron 3 | 69 | 233626 | 233645 | 1240 |
| 523816 | TACACAAATCCAAGGCAGAG | Intron 3 | 57 | 234447 | 234466 | 1241 |
| 523817 | AGGAAGAGTGGGAGTGTTAC | Intron 3 | 35 | 235258 | 235277 | 1242 |
| 523818 | GTCCCTGACTAGGCATTTTG | Intron 3 | 43 | 236071 | 236090 | 1243 |
| 523819 | AAGCTCAATTGCAGATGCTC | Intron 3 | 80 | 237676 | 237695 | 1244 |
| 523820 | CTGTGAGTCCATTAAACCTC | Intron 3 | 81 | 244875 | 244894 | 1245 |
| 523821 | TGAAATGTGGCTAGTGTGAC | Intron 3 | 51 | 245701 | 245720 | 1246 |
| 523822 | AAAATATTGAAAGGCCCATC | Intron 3 | 68 | 246501 | 246520 | 1247 |
| 523823 | AATGTCAATAGTGCCCTATT | Intron 3 | 48 | 247431 | 247450 | 1248 |
| 523824 | ACAGTATATTCAGTCCAAGG | Intron 3 | 82 | 248231 | 248250 | 1249 |
| 523825 | TGTCTATTTAAGTTTGTTGC | Intron 3 | 45 | 250001 | 250020 | 1250 |
| 523826 | TTCAAGTACTGTCATGAATA | Intron 3 | 47 | 251214 | 251233 | 1251 |
| 523827 | TTTCTTTTTCTTAAACTAAG | Intron 3 | 11 | 252041 | 252060 | 1252 |
| 523828 | GTTTCTATTGTTGCCACCTT | Intron 3 | 70 | 252841 | 252860 | 1253 |
| 523829 | AAGGCCACATATTATAGTAT | Intron 3 | 29 | 253698 | 253717 | 1254 |
| 523830 | ACCTGAACTATTAATTTCTT | Intron 3 | 19 | 255397 | 255416 | 1255 |
| 523831 | GAATGGGCTGAGTAGTTGAA | Intron 3 | 47 | 256197 | 256216 | 1256 |
| 523832 | TGATGAACATTGCTAATTTG | Intron 3 | 26 | 257018 | 257037 | 1257 |
| 523833 | ATCTTGCCTCGATGAAAGTT | Intron 3 | 17 | 257818 | 257837 | 1258 |
| 523834 | TTAAGTGGCACAGCCATGAT | Intron 3 | 9 | 258774 | 258793 | 1259 |
| 523835 | AATGAGTTAAGTTGGAACAC | Intron 3 | 25 | 261294 | 261313 | 1260 |
| 523836 | TCCTTAGTAGAATGCCTGGA | Intron 3 | 57 | 263338 | 263357 | 1261 |
| 523837 | TATGTAGAAAAATAAGCTGG | Intron 3 | 0 | 266514 | 266533 | 1262 |
| 523838 | GCCGAGGCAGGCACCTGAGT | Intron 3 | 43 | 267375 | 267394 | 1263 |
| 523839 | TGGTACCTATATTGAGAGGT | Intron 4 | 46 | 269052 | 269071 | 1264 |
| 523840 | TTAAGGAAAAATATAGTATA | Intron 4 | 7 | 269854 | 269873 | 1265 |
| 523841 | TTATTTATGTGTCAGGGATG | Intron 4 | 28 | 270668 | 270687 | 1266 |
| 523842 | CAAAAGTTAAGTGCTTTAGG | Intron 4 | 10 | 271468 | 271487 | 1267 |
| 523843 | TTCATAGATGTCTAAGGAAT | Intron 4 | 32 | 273341 | 273360 | 1268 |
| 523844 | ACCTGTGATTTACCTATTTC | Exon 5-intron 5 junction | 18 | 274185 | 274204 | 1269 |
| 523845 | TGCCTAGAAAACCACATAAA | Intron 5 | 38 | 274985 | 275004 | 1270 |
| 523846 | AAACATCCTCAAAGGTACCT | Intron 5 | 64 | 275808 | 275827 | 1271 |
| 523847 | CTTCCCTGAGACACACACAT | Intron 5 | 35 | 276617 | 276636 | 1272 |
| 523848 | CTTCTTCAATCTTCTCATAC | Intron 5 | 33 | 278288 | 278307 | 1273 |
| 523849 | TACCATTTTCCATTTAGTTT | Exon 6-intron 6 junction | 7 | 279088 | 279107 | 1274 |
| 523850 | ATTGGCATCTTTTTCAGTGG | Intron 6 | 34 | 279902 | 279921 | 1275 |
| 523851 | TCAAGCTCACGGTTGGAGAC | Intron 6 | 36 | 280799 | 280818 | 1276 |
| 523852 | AAATGAAATCAGTATGTTGA | Intron 6 | 0 | 281622 | 281641 | 1277 |
| 523853 | TGATTTATCACAAAGGTGCT | Intron 6 | 29 | 282437 | 282456 | 1278 |
| 523854 | AAAACAGTAGAAAAGATTAA | Intron 6 | 14 | 284073 | 284092 | 1279 |
| 523855 | CTACATCACAGCAGTCAGAA | Intron 6 | 23 | 285187 | 285206 | 1280 |
| 523856 | AAAAGATGTAAGTGTGACAT | Intron 6 | 28 | 286349 | 286368 | 1281 |
| | | | | 286919 | 286938 | |
| 523857 | TTACAAGAACTGCTAAAGGG | Intron 6 | 15 | 287151 | 287170 | 1282 |
| 523858 | ATAAAGAAAAAGTTAACTGA | Intron 6 | 9 | 287982 | 288001 | 1283 |
| 523859 | AGATAATATACTTCTTCTAT | Intron 6 | 4 | 288809 | 288828 | 1284 |
| 523860 | CCTTCTTCACATGTAAATTG | Exon 7-intron 7 junction | 19 | 290456 | 290475 | 1285 |
| 523861 | TTTCTATGTAGCTTGTGGTT | Intron 7 | 30 | 291258 | 291277 | 1286 |
| 523862 | AGGCAGAGTTTTTATTGATA | Intron 7 | 19 | 292058 | 292077 | 1287 |
| 523863 | ATAGTCACCAGCCTAAGCCT | Intron 8 | 28 | 292858 | 292877 | 1288 |
| 523864 | AGACTTTTAGCATGCTTGAC | Intron 8 | 56 | 293658 | 293677 | 1289 |
| 523865 | TTTACAGCCCTACAGTTCTA | Intron 8 | 7 | 294464 | 294483 | 1290 |
| 523866 | CCAGAGAACCTGACTCCAAA | Intron 8 | 6 | 295330 | 295349 | 1291 |
| 523867 | CAGAAGAAAATATTAGACAG | Intron 8 | 10 | 296993 | 297012 | 1292 |

**Table 19**

| Inhibition of GHR mRNA by 5-10-5 MOE gapmers targeting introns 3-8 of SEQ ID NO: 2 | | | | | | |
|---|---|---|---|---|---|---|
| ISIS NO | Sequence | Target Region | % inhibition | SEQ ID NO: 2 Start Site | SEQ ID NO: 2 Stop Site | SEQ ID NO |
| 532531 | TATTATACTTCTAAATTCCC | Intron 3 | 70 | 236716 | 236735 | 1293 |
| 532532 | TAAAAGCAAGAAAAAGGAAC | Intron 3 | 52 | 236889 | 236908 | 1294 |
| 532533 | CCTAATTTATATGAACAAAC | Intron 3 | 56 | 237177 | 237196 | 1295 |
| 532534 | TGCAATGCCTTAGCCTAAAA | Intron 3 | 86 | 238087 | 238106 | 1296 |
| 532535 | CACCACCATTATTACACTAC | Intron 3 | 75 | 238186 | 238205 | 1297 |
| 532536 | AAATAAATCAGATTATTATA | Intron 3 | 52 | 238242 | 238261 | 1298 |
| 532537 | CTTAGATCTGTGCTGTCCAA | Intron 3 | 81 | 245758 | 245777 | 1299 |
| 532538 | GTTAGTGTTAGATTCTTTGA | Intron 3 | 67 | 246152 | 246171 | 1300 |
| 532539 | CATGCTCACGGCTGTGTTAC | Intron 3 | 66 | 246248 | 246267 | 1301 |
| 532540 | CCCATCAAATACTGAGTTCT | Intron 3 | 86 | 246487 | 246506 | 1302 |
| 532541 | GAAAGTAGTGATTAATGAGA | Intron 3 | 38 | 247012 | 247031 | 1303 |
| 532542 | ATTAATCAACAAGTGGCATT | Intron 3 | 72 | 247203 | 247222 | 1304 |
| 532543 | TTTAATTTTAGGGTTTAGAG | Intron 3 | 48 | 248344 | 248363 | 1305 |
| 532544 | CTTGCTACCACTAGAGCCTT | Intron 3 | 69 | 248694 | 248713 | 1306 |
| 532545 | ACCACTGACTTATATCATTT | Intron 3 | 58 | 248743 | 248762 | 1307 |
| 532546 | TTCCCCATTGCTAATTTTGT | Intron 3 | 48 | 251601 | 251620 | 1308 |
| 532547 | TCCTGAAACTTAGTAGCTGG | Intron 3 | 83 | 253147 | 253166 | 1309 |
| 532548 | TGTCTTAAAAAGGAATAAAA | Intron 3 | 52 | 253785 | 253804 | 1310 |
| 532549 | CCTATAATAAAGTATTGTCT | Intron 3 | 70 | 253800 | 253819 | 1311 |
| 532550 | ATGTAAAATGGTATAGCTAC | Intron 3 | 50 | 254040 | 254059 | 1312 |
| 532551 | AACCCTCACACACTTCTGTT | Intron 3 | 71 | 254064 | 254083 | 1313 |
| 532552 | ATTCTGCATAAGCAGTGTTT | Intron 3 | 53 | 254246 | 254265 | 1314 |
| 532553 | TTACTACCCTGAAGAAGAAC | Intron 3 | 35 | 254314 | 254333 | 1315 |
| 532554 | AAGACCTATAACTTACTACC | Intron 3 | 49 | 254326 | 254345 | 1316 |
| 532555 | TTTCACAAGATTTACTTGGT | Intron 3 | 77 | 254641 | 254660 | 1317 |
| 532556 | CAGTTGTGATTGTCAACCTA | Intron 3 | 77 | 257073 | 257092 | 1318 |
| 532557 | AATCTTGCCTCGATGAAAGT | Intron 3 | 57 | 257819 | 257838 | 1319 |
| 532558 | TGGCCTAAATGTATCAGTTA | Intron 3 | 66 | 259157 | 259176 | 1320 |
| 532559 | AGGCTTTGGGTAAAATCTTT | Intron 3 | 67 | 259184 | 259203 | 1321 |
| 532560 | TATGATTTTTAAAGATTAAA | Intron 3 | 20 | 261419 | 261438 | 1322 |
| 532561 | GTACAGTGAAAAAGATGTGT | Intron 3 | 56 | 263666 | 263685 | 1323 |
| 532562 | GACAGGTATGAAGCAAAACA | Intron 3 | 64 | 267033 | 267052 | 1324 |
| 532563 | TGAGCTGAGGGTCTTTGCCG | Intron 3 | 61 | 267391 | 267410 | 1325 |
| 532564 | AGGCTGAGTTGTACACAAAC | Intron 4 | 52 | 269422 | 269441 | 1326 |
| 532565 | ATGAGGAGGCTGAGTTGTAC | Intron 4 | 43 | 269428 | 269447 | 1327 |
| 532566 | TCATAAAGTGGGCCCAGCTT | Intron 4 | 70 | 270044 | 270063 | 1328 |
| 532567 | ACTCCTAATCCCTCAGTTTT | Intron 4 | 62 | 270492 | 270511 | 1329 |
| 532568 | TTTACATGCAAGGAGCTGAG | Intron 4 | 61 | 271047 | 271066 | 1330 |
| 532569 | TAATGCCCTTTCTCCCTACT | Intron 4 | 60 | 271215 | 271234 | 1331 |
| 532570 | CCTGTTTAGATTATCCCAAA | Intron 4 | 62 | 271763 | 271782 | 1332 |
| 532571 | CATGATTCACAGAATTTCTC | Intron 4 | 56 | 271831 | 271850 | 1333 |
| 532572 | AGTTAGAAAACTCAAAGTAT | Intron 4 | 2 | 271915 | 271934 | 1334 |
| 532573 | TCAAATGTACTTAGCATAAG | Intron 4 | 9 | 271947 | 271966 | 1335 |
| 532574 | ATATCAAATGTACTTAGCAT | Intron 4 | 59 | 271950 | 271969 | 1336 |
| 532575 | AAAGTTCAGAAGAGGGAATG | Intron 4 | 51 | 273233 | 273252 | 1337 |
| 532576 | AATTCCCATCTGAGTAGTTT | Intron 4 | 56 | 273440 | 273459 | 1338 |
| 532577 | GTCCCCTAATTTCAGGCTAA | Intron 4 | 31 | 273471 | 273490 | 1339 |
| 532578 | CTATGTCAAATGAAACAAAA | Intron 5 | 38 | 274205 | 274224 | 1340 |
| 532579 | TGATTATGCTTTGTGATAAA | Intron 5 | 42 | 274624 | 274643 | 1341 |
| 532580 | TCCAGCTGACTAGGAGGGCT | Intron 5 | 7 | 275732 | 275751 | 1342 |
| 532581 | CATACCAGTCTCCTCGCTCA | Intron 5 | 0 | 276738 | 276757 | 1343 |
| 532582 | ATATAACAGAATCCAACCAT | Intron 5 | 47 | 277045 | 277064 | 1344 |
| | | | | 278361 | 278380 | |
| 532583 | TGCAAAATGGCCAAACTACA | Intron 5 | 56 | 277577 | 277596 | 1345 |
| 532584 | TCTTCCTAGCCACATGTGAT | Intron 5 | 32 | 278227 | 278246 | 1346 |
| 532585 | TACCATGCTCTCTAATTGCC | Intron 6 | 47 | 279624 | 279643 | 1347 |
| 532586 | AGTGATCTGTGCCAGGCTGC | Intron 6 | 65 | 279848 | 279867 | 1348 |
| 532587 | AAGTTACAGAACAGATATCT | Intron 6 | 61 | 280012 | 280031 | 1349 |
| 532588 | GTATTGTGAAAATAGTACTG | Intron 6 | 45 | 280226 | 280245 | 1350 |
| 532589 | AAACACTATCAAGCTCACGG | Intron 6 | 54 | 280807 | 280826 | 1351 |
| 532590 | TTCAAGAAAAGTCTTCAAAT | Intron 6 | 24 | 280831 | 280850 | 1352 |
| 532591 | GGATCATTTCCCCATGCATG | Intron 6 | 52 | 280982 | 281001 | 1353 |
| 532592 | ATATTATATTAAGAAAAATG | Intron 6 | 4 | 281422 | 281441 | 1354 |
| 532593 | CTCCCATGTTCATTACTTAT | Intron 6 | 49 | 281587 | 281606 | 1355 |
| 532594 | CATGACATTGGTTTGGGCAA | Intron 6 | 43 | 282229 | 282248 | 1356 |
| 532595 | AATGTTGTTGGGAAAATTGG | Intron 6 | 42 | 282383 | 282402 | 1357 |
| 532596 | AGCTGCAGGATACAAAGTCA | Intron 6 | 49 | 282986 | 283005 | 1358 |
| 532597 | ATATCCTTTCATGATAAAAA | Intron 6 | 31 | 283354 | 283373 | 1359 |
| 532598 | ATGGGCTAATATCTCTGATA | Intron 6 | 50 | 283590 | 283609 | 1360 |
| 532599 | ACATTACTAATAATTAGAGA | Intron 6 | 0 | 285236 | 285255 | 1361 |
| 532600 | ATAAAAACATATGAAAGTAT | Intron 6 | 12 | 287093 | 287112 | 1362 |
| 532601 | TTCTGAATTAAATCTATTAG | Intron 6 | 16 | 287408 | 287427 | 1363 |
| 532602 | TTACATTTTTGCAAATTTAT | Intron 6 | 31 | 287472 | 287491 | 1364 |
| 532603 | TGAACAGTTGATTAACAAAG | Intron 6 | 15 | 287887 | 287906 | 1365 |
| 532604 | AAGTTATTGGTTTACTAGAT | Intron 6 | 0 | 288598 | 288617 | 1366 |
| 532605 | TTGGAAAAGGTCCTAGAAAA | Intron 6 | 24 | 289808 | 289827 | 1367 |
| 532606 | CATGACAGAAACTTCTTAGA | Intron 7 | 25 | 292035 | 292054 | 1368 |
| 532607 | CCATACTTGCTGACAAATAT | Intron 8 | 39 | 294389 | 294408 | 1369 |

### Example 2: Dose-dependent antisense inhibition of human GHR in Hep3B cells by MOE gapmers

Gapmers from Example 1 exhibiting significant *in vitro* inhibition of GHR mRNA were selected and tested at various doses in Hep3B cells. The antisense oligonucleotides were tested in a series of experiments that had similar culture conditions. The results for each experiment are presented in separate tables shown below. Cells were plated at a density of 20,000 cells per well and transfected using electroporation with 0.625 µM, 1.25 µM, 2.50 µM, 5.00 µM and 10.00 µM concentrations of antisense oligonucleotide, as specified in the Tables below. After a treatment period of approximately 16 hours, RNA was isolated from the cells and GHR mRNA levels were measured by quantitative real-time PCR. Human primer probe set RTS3437_MGB was used to measure mRNA levels. GHR mRNA levels were adjusted according to total RNA content, as measured by RIBOGREEN®. Results are presented as percent inhibition of GHR, relative to untreated control cells.

The half maximal inhibitory concentration (IC₅₀) of each oligonucleotide is also presented. GHR mRNA levels were significantly reduced in a dose-dependent manner in antisense oligonucleotide treated cells.

**Table 20**

| ISIS No | 0.625 µM | 1.250 µM | 2.50 µM | 5.00 µM | 10.00 µM | IC₅₀ (µM) |
|---|---|---|---|---|---|---|
| 523271 | 41 | 61 | 73 | 86 | 92 | 0.8 |
| 523274 | 20 | 36 | 64 | 80 | 92 | 1.8 |
| 523324 | 35 | 45 | 68 | 91 | 90 | 1.2 |

**Table 21**

| ISIS No | 0.625 µM | 1.250 µM | 2.50 µM | 5.00 µM | 10.00 µM | IC₅₀ (µM) |
|---|---|---|---|---|---|---|
| 523604 | 21 | 42 | 68 | 58 | 86 | 2.0 |
| 523577 | 6 | 22 | 56 | 66 | 91 | 2.7 |
| 523614 | 14 | 44 | 61 | 84 | 87 | 1.9 |
| 523564 | 4 | 26 | 48 | 67 | 86 | 2.8 |
| 523633 | 30 | 43 | 71 | 82 | 84 | 1.4 |
| 523571 | 2 | 9 | 38 | 55 | 82 | 3.9 |

**Table 22**

| ISIS No | 0.625 µM | 1.250 µM | 2.50 µM | 5.00 µM | 10.00 µM | IC₅₀ (µM) |
|---|---|---|---|---|---|---|
| 523570 | 25 | 50 | 64 | 77 | 88 | 1.5 |
| 523592 | 27 | 42 | 59 | 79 | 88 | 1.7 |
| 523595 | 21 | 50 | 62 | 76 | 90 | 1.6 |
| 523596 | 36 | 47 | 62 | 75 | 77 | 1.4 |
| 523607 | 49 | 62 | 71 | 82 | 84 | 0.5 |
| 523615 | 20 | 49 | 63 | 83 | 91 | 1.6 |
| 523630 | 4 | 28 | 54 | 79 | 78 | 2.6 |
| 523661 | 4 | 34 | 48 | 73 | 79 | 2.7 |
| 523665 | 4 | 28 | 54 | 73 | 79 | 2.7 |
| 523687 | 30 | 56 | 61 | 78 | 81 | 1.4 |
| 523711 | 42 | 66 | 78 | 94 | 95 | 0.7 |
| 523712 | 6 | 37 | 60 | 72 | 89 | 2.3 |
| 523713 | 4 | 32 | 55 | 72 | 85 | 2.5 |
| 523714 | 59 | 75 | 88 | 95 | 97 | 0.2 |

**Table 23**

| ISIS No | 0.625 µM | 1.250 µM | 2.50 µM | 5.00 µM | 10.00 µM | IC₅₀ (µM) |
|---|---|---|---|---|---|---|
| 523655 | 26 | 33 | 60 | 67 | 78 | 2.1 |
| 523656 | 19 | 33 | 45 | 69 | 87 | 2.4 |
| 523658 | 0 | 42 | 62 | 67 | 79 | 3.1 |
| 523715 | 78 | 90 | 92 | 93 | 95 | <0.6 |
| 523718 | 30 | 46 | 67 | 84 | 92 | 1.4 |
| 523723 | 56 | 69 | 83 | 92 | 94 | 0.3 |
| 523725 | 45 | 64 | 79 | 89 | 95 | 0.6 |
| 523726 | 32 | 48 | 77 | 88 | 89 | 1.2 |
| 523736 | 0 | 64 | 75 | 90 | 96 | 1.5 |
| 523747 | 48 | 64 | 80 | 91 | 92 | 0.6 |
| 523758 | 25 | 39 | 61 | 74 | 84 | 1.9 |
| 523766 | 7 | 37 | 66 | 81 | 93 | 2.0 |
| 523776 | 26 | 54 | 72 | 78 | 83 | 1.3 |
| 523789 | 62 | 68 | 81 | 85 | 90 | 0.2 |

**Table 24**

| ISIS No | 0.625 µM | 1.250 µM | 2.50 µM | 5.00 µM | 10.00 µM | IC₅₀ (µM) |
|---|---|---|---|---|---|---|
| 523719 | 24 | 46 | 65 | 84 | 93 | 1.5 |
| 523720 | 18 | 49 | 72 | 85 | 93 | 1.5 |
| 523724 | 43 | 61 | 77 | 91 | 91 | 0.7 |
| 523735 | 8 | 42 | 63 | 81 | 93 | 2.0 |
| 523740 | 37 | 58 | 72 | 83 | 88 | 1.0 |
| 523752 | 9 | 29 | 52 | 72 | 86 | 2.5 |
| 523763 | 8 | 32 | 57 | 70 | 80 | 2.6 |
| 523764 | 43 | 52 | 67 | 77 | 79 | 0.9 |
| 523765 | 24 | 48 | 62 | 88 | 4 | 1.5 |
| 523767 | 49 | 62 | 67 | 72 | 82 | 0.6 |
| 523772 | 29 | 39 | 54 | 62 | 61 | 2.7 |
| 523774 | 28 | 59 | 63 | 88 | 91 | 1.2 |
| 523778 | 25 | 32 | 63 | 78 | 84 | 1.9 |
| 523783 | 0 | 22 | 53 | 72 | 88 | 2.8 |

**Table 25**

| ISIS No | 0.625 µM | 1.250 µM | 2.50 µM | 5.00 µM | 10.00 µM | IC₅₀ (µM) |
|---|---|---|---|---|---|---|
| 532151 | 57 | 69 | 76 | 85 | 88 | <0.6 |
| 532153 | 23 | 43 | 54 | 80 | 86 | 1.8 |
| 532158 | 46 | 58 | 81 | 87 | 87 | 0.6 |
| 532160 | 17 | 26 | 55 | 76 | 92 | 2.2 |
| 532162 | 14 | 46 | 71 | 83 | 93 | 1.7 |
| 532164 | 37 | 76 | 82 | 90 | 93 | 0.6 |
| 532171 | 41 | 81 | 67 | 81 | 83 | <0.6 |
| 532181 | 56 | 81 | 84 | 89 | 93 | 0.2 |
| 532186 | 26 | 65 | 75 | 83 | 91 | 1.1 |
| 532188 | 51 | 68 | 80 | 89 | 93 | <0.6 |
| 532189 | 24 | 31 | 52 | 75 | 86 | 2.1 |
| 532197 | 0 | 40 | 66 | 85 | 93 | 2.1 |
| 532199 | 24 | 37 | 50 | 73 | 87 | 2.1 |
| 532222 | 12 | 41 | 67 | 84 | 94 | 1.8 |

**Table 26**

| ISIS No | 0.625 µM | 1.250 µM | 2.50 µM | 5.00 µM | 10.00 µM | IC₅₀ (µM) |
|---|---|---|---|---|---|---|
| 532175 | 41 | 54 | 76 | 84 | 89 | 0.9 |
| 532223 | 53 | 69 | 75 | 88 | 94 | <0.6 |
| 532235 | 43 | 58 | 67 | 77 | 82 | 0.8 |
| 532241 | 39 | 53 | 62 | 73 | 87 | 1.2 |
| 532248 | 49 | 65 | 72 | 85 | 93 | 0.6 |
| 532254 | 52 | 62 | 85 | 87 | 92 | <0.6 |
| 532300 | 20 | 29 | 49 | 66 | 78 | 2.7 |
| 532304 | 26 | 39 | 66 | 78 | 90 | 1.7 |
| 532316 | 41 | 66 | 76 | 86 | 94 | 0.7 |
| 532395 | 32 | 56 | 84 | 93 | 97 | 1.0 |
| 532401 | 47 | 80 | 92 | 96 | 98 | <0.6 |
| 532411 | 73 | 90 | 94 | 97 | 98 | <0.6 |
| 532420 | 38 | 49 | 82 | 85 | 97 | 1.0 |
| 532436 | 37 | 58 | 75 | 90 | 96 | 0.9 |

**Table 27**

| ISIS No | 0.625 µM | 1.250 µM | 2.50 µM | 5.00 µM | 10.00 µM | IC₅₀ (µM) |
|---|---|---|---|---|---|---|
| 532410 | 66 | 83 | 92 | 94 | 97 | <0.6 |
| 532468 | 45 | 68 | 78 | 93 | 94 | 0.6 |
| 532469 | 0 | 17 | 56 | 76 | 92 | 2.8 |
| 532470 | 10 | 34 | 62 | 84 | 94 | 2.0 |
| 532475 | 13 | 36 | 52 | 64 | 87 | 2.5 |
| 532476 | 34 | 64 | 73 | 79 | 93 | 0.9 |
| 532480 | 28 | 54 | 67 | 78 | 87 | 1.4 |
| 532482 | 21 | 39 | 69 | 83 | 92 | 1.7 |
| 532490 | 42 | 60 | 68 | 84 | 93 | 0.9 |
| 532500 | 37 | 50 | 63 | 81 | 87 | 1.2 |
| 532506 | 13 | 41 | 66 | 75 | 89 | 1.9 |
| 532507 | 47 | 59 | 71 | 86 | 89 | 0.7 |
| 532508 | 0 | 31 | 73 | 83 | 89 | 2.2 |
| 532526 | 31 | 56 | 78 | 79 | 88 | 1.1 |

**Table 28**

| ISIS No | 0.625 µM | 1.250 µM | 2.50 µM | 5.00 µM | 10.00 µM | IC₅₀ (µM) |
|---|---|---|---|---|---|---|
| 532495 | 59 | 74 | 81 | 87 | 95 | <0.6 |
| 532501 | 49 | 53 | 71 | 83 | 84 | 0.7 |
| 532534 | 53 | 75 | 85 | 91 | 97 | <0.6 |
| 532535 | 0 | 34 | 61 | 84 | 92 | 2.6 |
| 532537 | 49 | 67 | 80 | 90 | 94 | <0.6 |
| 532540 | 59 | 70 | 87 | 93 | 95 | <0.6 |
| 532547 | 57 | 71 | 81 | 91 | 92 | <0.6 |
| 532555 | 48 | 36 | 61 | 72 | 85 | 1.3 |
| 532556 | 33 | 57 | 67 | 86 | 90 | 1.1 |

**Table 29**

| ISIS No | 0.625 µM | 1.250 µM | 2.50 µM | 5.00 µM | 10.00 µM | IC₅₀ (µM) |
|---|---|---|---|---|---|---|
| 523421 | 32 | 57 | 81 | 82 | 88 | 1.0 |
| 533006 | 46 | 43 | 69 | 83 | 91 | 1.0 |
| 533121 | 53 | 75 | 75 | 88 | 93 | <0.6 |
| 533122 | 65 | 77 | 82 | 90 | 93 | <0.6 |
| 533123 | 39 | 71 | 84 | 91 | 95 | 0.6 |
| 533125 | 49 | 61 | 81 | 85 | 91 | 0.6 |
| 533131 | 3 | 57 | 59 | 82 | 90 | 1.9 |
| 533136 | 32 | 65 | 62 | 81 | 88 | 1.1 |
| 533139 | 13 | 51 | 72 | 90 | 94 | 1.5 |
| 533140 | 36 | 66 | 39 | 87 | 92 | 1.2 |
| 533153 | 50 | 65 | 83 | 89 | 90 | <0.6 |
| 533156 | 43 | 64 | 74 | 85 | 90 | 0.7 |
| 533160 | 57 | 80 | 87 | 91 | 95 | <0.6 |
| 533161 | 54 | 62 | 81 | 89 | 92 | <0.6 |

**Table 30**

| ISIS No | 0.625 µM | 1.250 µM | 2.50 µM | 5.00 µM | 10.00 µM | IC₅₀ (µM) |
|---|---|---|---|---|---|---|
| 533234 | 50 | 70 | 86 | 93 | 95 | <0.6 |
| 533237 | 5 | 45 | 63 | 84 | 93 | 1.9 |
| 533233 | 43 | 55 | 76 | 90 | 95 | 0.8 |
| 533179 | 31 | 63 | 75 | 87 | 87 | 1.0 |
| 533178 | 53 | 67 | 76 | 89 | 94 | <0.6 |
| 533187 | 5 | 15 | 53 | 79 | 86 | 2.7 |
| 533188 | 49 | 68 | 83 | 89 | 94 | <0.6 |
| 533271 | 45 | 66 | 85 | 92 | 94 | 0.6 |
| 533134 | 22 | 45 | 64 | 81 | 89 | 1.6 |
| 533258 | 52 | 72 | 88 | 93 | 95 | <0.6 |
| 533235 | 50 | 54 | 75 | 82 | 90 | 0.7 |
| 533262 | 23 | 54 | 78 | 91 | 96 | 1.2 |
| 533189 | 48 | 66 | 78 | 82 | 88 | <0.6 |
| 533193 | 38 | 53 | 72 | 77 | 91 | 1.0 |

**Table 31**

| ISIS No | 0.625 µM | 1.250 µM | 2.50 µM | 5.00 µM | 10.00 µM | IC₅₀ (µM) |
|---|---|---|---|---|---|---|
| 533259 | 63 | 78 | 84 | 90 | 92 | <0.6 |
| 533291 | 25 | 57 | 75 | 86 | 96 | 1.2 |
| 533256 | 67 | 76 | 90 | 95 | 95 | <0.6 |
| 533269 | 42 | 75 | 82 | 94 | 97 | 0.6 |
| 533265 | 67 | 78 | 91 | 95 | 97 | <0.6 |
| 533318 | 16 | 45 | 77 | 87 | 95 | 1.5 |
| 533257 | 55 | 84 | 91 | 96 | 96 | <0.6 |
| 533280 | 34 | 62 | 80 | 91 | 91 | 0.9 |
| 533301 | 52 | 77 | 84 | 93 | 96 | <0.6 |
| 533316 | 41 | 50 | 79 | 93 | 94 | 0.9 |
| 533270 | 62 | 71 | 88 | 94 | 97 | <0.6 |
| 533330 | 46 | 76 | 93 | 97 | 98 | <0.6 |
| 533317 | 55 | 60 | 82 | 87 | 96 | <0.6 |
| 533315 | 39 | 56 | 82 | 87 | 93 | 0.9 |

**Table 32**

| ISIS No | 0.625 µM | 1.250 µM | 2.50 µM | 5.00 µM | 10.00 µM | IC₅₀ (µM) |
|---|---|---|---|---|---|---|
| 533364 | 71 | 77 | 92 | 90 | 94 | <0.6 |
| 533925 | 26 | 55 | 61 | 85 | 91 | 1.4 |
| 533326 | 54 | 77 | 80 | 93 | 95 | <0.6 |
| 533916 | 18 | 62 | 69 | 83 | 93 | 1.4 |
| 533328 | 52 | 68 | 89 | 94 | 98 | <0.6 |
| 533932 | 42 | 49 | 80 | 86 | 92 | 0.9 |
| 533352 | 42 | 82 | 88 | 93 | 94 | <0.6 |
| 533917 | 20 | 37 | 57 | 78 | 84 | 2.0 |
| 533331 | 54 | 83 | 89 | 93 | 96 | <0.6 |
| 533936 | 21 | 46 | 73 | 84 | 88 | 1.5 |
| 533329 | 56 | 73 | 84 | 92 | 98 | <0.6 |
| 533937 | 26 | 32 | 79 | 86 | 94 | 1.5 |
| 533908 | 58 | 66 | 81 | 88 | 94 | <0.6 |
| 533898 | 61 | 64 | 84 | 90 | 92 | <0.6 |

**Table 33**

| ISIS No | 0.625 µM | 1.250 µM | 2.50 µM | 5.00 µM | 10.00 µM | IC₅₀ (µM) |
|---|---|---|---|---|---|---|
| 539371 | 32 | 41 | 82 | 92 | 98 | 1.2 |
| 539382 | 18 | 58 | 74 | 91 | 97 | 1.3 |
| 539392 | 34 | 59 | 79 | 94 | 96 | 0.9 |
| 539398 | 31 | 53 | 89 | 94 | 98 | 1.0 |
| 539399 | 31 | 72 | 87 | 95 | 97 | 0.8 |
| 539400 | 36 | 60 | 79 | 93 | 97 | 0.9 |
| 539405 | 33 | 58 | 74 | 91 | 94 | 1.0 |
| 539412 | 23 | 61 | 80 | 93 | 95 | 1.1 |
| 539413 | 53 | 75 | 86 | 92 | 96 | <0.6 |
| 539415 | 47 | 62 | 84 | 91 | 96 | 0.6 |
| 539416 | 61 | 85 | 94 | 97 | 96 | <0.6 |
| 539430 | 24 | 48 | 68 | 80 | 93 | 1.5 |
| 539431 | 14 | 40 | 71 | 89 | 95 | 1.7 |
| 539433 | 46 | 67 | 74 | 92 | 95 | 0.6 |

### Example 3: Dose-dependent antisense inhibition of human GHR in Hep3B cells by MOE gapmers

Gapmers from the studies described above exhibiting significant *in vitro* inhibition of GHR mRNA were selected and tested at various doses in Hep3B cells. The antisense oligonucleotides were tested in a series of experiments that had similar culture conditions. The results for each experiment are presented in separate tables shown below. Cells were plated at a density of 20,000 cells per well and transfected using electroporation with 0.3125 µM, 0.625 µM, 1.25 µM, 2.50 µM, 5.00 µM and 10.00 µM concentrations of antisense oligonucleotide, as specified in the Tables below. After a treatment period of approximately 16 hours, RNA was isolated from the cells and GHR mRNA levels were measured by quantitative real-time PCR. Human primer probe set RTS3437_MGB was used to measure mRNA levels. GHR mRNA levels were adjusted according to total RNA content, as measured by RIBOGREEN®. Results are presented as percent inhibition of GHR, relative to untreated control cells.

The half maximal inhibitory concentration (IC₅₀) of each oligonucleotide is also presented. GHR mRNA levels were significantly reduced in a dose-dependent manner in antisense oligonucleotide treated cells.

**Table 34**

| ISIS No | 0.3125 µM | 0.625 µM | 1.250 µM | 2.50 µM | 5.00 µM | 10.00 µM | IC₅₀ (µM) |
|---|---|---|---|---|---|---|---|
| 523814 | 0 | 24 | 48 | 52 | 68 | 82 | 2.2 |
| 523805 | 13 | 29 | 55 | 0 | 79 | 85 | 1.5 |
| 523822 | 0 | 19 | 26 | 41 | 65 | 85 | 2.8 |
| 523820 | 0 | 19 | 29 | 58 | 74 | 86 | 2.3 |
| 523815 | 3 | 6 | 19 | 37 | 45 | 71 | 4.8 |
| 523828 | 12 | 19 | 32 | 51 | 64 | 74 | 2.7 |
| 523801 | 3 | 9 | 31 | 43 | 59 | 76 | 3.3 |
| 523824 | 12 | 28 | 44 | 63 | 77 | 85 | 1.7 |
| 523794 | 13 | 21 | 30 | 51 | 66 | 78 | 2.5 |
| 523810 | 15 | 34 | 55 | 72 | 78 | 86 | 1.3 |
| 523819 | 0 | 24 | 40 | 60 | 66 | 75 | 2.4 |

**Table 35**

| ISIS No | 0.3125 µM | 0.625 µM | 1.250 µM | 2.50 µM | 5.00 µM | 10.00 µM | IC₅₀ (µM) |
|---|---|---|---|---|---|---|---|
| 539302 | 31 | 56 | 80 | 92 | 97 | 98 | 0.5 |
| 539314 | 16 | 28 | 49 | 69 | 85 | 95 | 1.3 |
| 539319 | 8 | 30 | 45 | 71 | 90 | 94 | 1.4 |
| 539320 | 11 | 42 | 64 | 83 | 92 | 95 | 1.0 |
| 539321 | 25 | 48 | 64 | 82 | 95 | 97 | 0.8 |
| 539322 | 19 | 34 | 58 | 72 | 90 | 96 | 1.1 |
| 539331 | 7 | 14 | 46 | 69 | 88 | 96 | 1.6 |
| 539355 | 28 | 35 | 67 | 89 | 96 | 98 | 0.8 |
| 539358 | 12 | 39 | 56 | 80 | 93 | 98 | 1.1 |
| 539359 | 15 | 23 | 58 | 77 | 93 | 98 | 1.2 |

**Table 36**

| ISIS No | 0.3125 µM | 0.625 µM | 1.250 µM | 2.50 µM | 5.00 µM | 10.00 µM | IC₅₀ (µM) |
|---|---|---|---|---|---|---|---|
| 539318 | 23 | 21 | 56 | 73 | 88 | 94 | 1.2 |
| 539325 | 14 | 26 | 38 | 74 | 92 | 98 | 1.4 |
| 539339 | 18 | 23 | 58 | 83 | 92 | 98 | 1.1 |
| 539341 | 17 | 29 | 62 | 84 | 94 | 95 | 1.0 |
| 539342 | 20 | 31 | 43 | 71 | 90 | 95 | 1.2 |
| 539352 | 15 | 23 | 41 | 61 | 89 | 95 | 1.5 |
| 539356 | 24 | 46 | 62 | 83 | 90 | 97 | 0.8 |
| 539361 | 37 | 42 | 73 | 88 | 96 | 98 | 0.6 |
| 539379 | 53 | 66 | 83 | 96 | 96 | 98 | 0.2 |
| 539380 | 52 | 77 | 91 | 97 | 97 | 99 | 0.1 |
| 539383 | 34 | 61 | 71 | 89 | 98 | 98 | 0.5 |

**Table 37**

| ISIS No | 0.3125 µM | 0.625 µM | 1.250 µM | 2.50 µM | 5.00 µM | 10.00 µM | IC₅₀ (µM) |
|---|---|---|---|---|---|---|---|
| 539360 | 45 | 60 | 81 | 94 | 97 | 98 | 0.3 |
| 539362 | 21 | 36 | 72 | 90 | 98 | 99 | 0.8 |
| 539375 | 23 | 36 | 66 | 85 | 95 | 99 | 0.9 |
| 539376 | 26 | 35 | 58 | 82 | 95 | 99 | 0.9 |
| 539377 | 29 | 31 | 43 | 64 | 85 | 89 | 1.3 |
| 539378 | 37 | 59 | 81 | 93 | 97 | 98 | 0.4 |
| 539389 | 34 | 61 | 61 | 87 | 95 | 97 | 0.5 |
| 539401 | 34 | 52 | 63 | 84 | 92 | 95 | 0.6 |
| 539403 | 52 | 73 | 83 | 94 | 97 | 98 | 0.1 |
| 539404 | 22 | 55 | 74 | 88 | 94 | 96 | 0.6 |
| 539432 | 32 | 50 | 75 | 86 | 94 | 96 | 0.6 |

### Example 4: Dose-dependent antisense inhibition of human GHR in Hep3B cells by MOE gapmers

Gapmers from studies described above exhibiting significant *in vitro* inhibition of GHR mRNA were selected and tested at various doses in Hep3B cells. The antisense oligonucleotides were tested in a series of experiments that had similar culture conditions. The results for each experiment are presented in separate tables shown below. Cells were plated at a density of 20,000 cells per well and transfected using electroporation with 0.625 µM, 1.25 µM, 2.50 µM, 5.00 µM and 10.00 µM concentrations of antisense oligonucleotide, as specified in the Tables below. After a treatment period of approximately 16 hours, RNA was isolated from the cells and GHR mRNA levels were measured by quantitative real-time PCR. Human primer probe set RTS3437_MGB was used to measure mRNA levels. GHR mRNA levels were adjusted according to total RNA content, as measured by RIBOGREEN®. Results are presented as percent inhibition of GHR, relative to untreated control cells.

The half maximal inhibitory concentration (IC₅₀) of each oligonucleotide is also presented. GHR mRNA levels were significantly reduced in a dose-dependent manner in antisense oligonucleotide treated cells.

**Table 38**

| ISIS No | 0.625 µM | 1.250 µM | 2.50 µM | 5.00 µM | 10.00 µM | IC₅₀ (µM) |
|---|---|---|---|---|---|---|
| 523271 | 26 | 41 | 80 | 89 | 94 | 1.4 |
| 523274 | 13 | 35 | 63 | 85 | 95 | 1.9 |
| 523324 | 26 | 40 | 64 | 88 | 95 | 1.6 |
| 523577 | 27 | 50 | 72 | 87 | 95 | 1.3 |
| 523604 | 49 | 66 | 74 | 81 | 87 | 0.5 |
| 523614 | 43 | 54 | 82 | 92 | 89 | 0.8 |

**Table 39**

| ISIS No | 0.625 µM | 1.250 µM | 2.50 µM | 5.00 µM | 10.00 µM | IC₅₀ (µM) |
|---|---|---|---|---|---|---|
| 523564 | 16 | 48 | 69 | 75 | 91 | 1.7 |
| 523570 | 24 | 52 | 65 | 71 | 88 | 1.6 |
| 523592 | 6 | 31 | 52 | 65 | 81 | 2.8 |
| 523595 | 13 | 49 | 60 | 79 | 92 | 1.8 |
| 523596 | 20 | 49 | 62 | 71 | 75 | 1.9 |
| 523607 | 38 | 63 | 66 | 74 | 76 | 0.8 |
| 523615 | 17 | 48 | 60 | 80 | 92 | 1.8 |
| 523630 | 19 | 42 | 42 | 67 | 80 | 2.5 |
| 523633 | 41 | 69 | 78 | 79 | 80 | 0.6 |
| 523665 | 16 | 45 | 56 | 71 | 80 | 2.1 |
| 523687 | 37 | 59 | 73 | 75 | 78 | 0.9 |
| 523711 | 33 | 63 | 78 | 91 | 93 | 0.9 |
| 523712 | 13 | 36 | 61 | 78 | 87 | 2.1 |
| 523714 | 63 | 85 | 91 | 96 | 96 | <0.6 |

**Table 40**

| ISIS No | 0.625 µM | 1.250 µM | 2.50 µM | 5.00 µM | 10.00 µM | IC₅₀ (µM) |
|---|---|---|---|---|---|---|
| 523655 | 28 | 42 | 57 | 74 | 76 | 1.9 |
| 523656 | 33 | 43 | 53 | 74 | 88 | 1.7 |
| 523661 | 29 | 29 | 66 | 79 | 82 | 1.9 |
| 523713 | 35 | 45 | 64 | 83 | 87 | 1.3 |
| 523715 | 83 | 86 | 92 | 93 | 94 | <0.6 |
| 523718 | 27 | 52 | 69 | 84 | 95 | 1.3 |
| 523723 | 65 | 74 | 86 | 85 | 94 | <0.6 |
| 523725 | 37 | 63 | 78 | 78 | 92 | 0.8 |
| 523726 | 43 | 57 | 72 | 86 | 89 | 0.8 |
| 523736 | 39 | 65 | 80 | 88 | 95 | 0.8 |
| 523747 | 51 | 71 | 83 | 86 | 93 | <0.6 |
| 523766 | 30 | 50 | 70 | 82 | 89 | 1.3 |
| 523776 | 45 | 59 | 67 | 79 | 84 | 0.7 |
| 523789 | 63 | 75 | 76 | 83 | 83 | <0.6 |

**Table 41**

| ISIS No | 0.625 µM | 1.250 µM | 2.50 µM | 5.00 µM | 10.00 µM | IC₅₀ (µM) |
|---|---|---|---|---|---|---|
| 523719 | 18 | 40 | 56 | 73 | 83 | 2.1 |
| 523720 | 36 | 46 | 59 | 64 | 89 | 1.5 |
| 523724 | 44 | 60 | 75 | 81 | 87 | 0.7 |
| 523735 | 11 | 40 | 60 | 78 | 84 | 2.1 |
| 523740 | 17 | 47 | 61 | 80 | 81 | 1.8 |
| 523752 | 25 | 31 | 38 | 70 | 84 | 2.5 |
| 523758 | 23 | 48 | 58 | 72 | 80 | 1.8 |
| 523763 | 2 | 24 | 48 | 64 | 75 | 3.3 |
| 523764 | 22 | 49 | 45 | 73 | 75 | 2.1 |
| 523765 | 42 | 40 | 57 | 79 | 87 | 1.4 |
| 523767 | 43 | 53 | 56 | 69 | 79 | 1.2 |
| 523774 | 36 | 52 | 71 | 81 | 89 | 1.1 |
| 523778 | 15 | 45 | 59 | 75 | 79 | 2.0 |
| 523783 | 5 | 30 | 48 | 66 | 83 | 2.9 |

**Table 42**

| ISIS No | 0.625 µM | 1.250 µM | 2.50 µM | 5.00 µM | 10.00 µM | IC₅₀ (µM) |
|---|---|---|---|---|---|---|
| 532151 | 40 | 45 | 64 | 71 | 82 | 1.3 |
| 532158 | 28 | 47 | 63 | 70 | 87 | 1.6 |
| 532164 | 36 | 47 | 64 | 75 | 89 | 1.3 |
| 532171 | 35 | 47 | 50 | 69 | 89 | 1.6 |
| 532175 | 27 | 38 | 43 | 75 | 87 | 2.1 |
| 532181 | 21 | 56 | 63 | 69 | 80 | 1.7 |
| 532186 | 28 | 49 | 62 | 73 | 91 | 1.5 |
| 532188 | 40 | 52 | 73 | 75 | 90 | 1.0 |
| 532223 | 22 | 34 | 53 | 71 | 90 | 2.2 |
| 532235 | 35 | 31 | 48 | 68 | 73 | 2.3 |
| 532241 | 6 | 24 | 29 | 51 | 72 | 4.5 |
| 532248 | 19 | 37 | 47 | 73 | 84 | 2.3 |
| 532254 | 56 | 56 | 72 | 85 | 90 | 0.5 |
| 532316 | 32 | 55 | 50 | 78 | 90 | 1.5 |

**Table 43**

| ISIS No | 0.625 µM | 1.250 µM | 2.50 µM | 5.00 µM | 10.00 µM | IC₅₀ (µM) |
|---|---|---|---|---|---|---|
| 532304 | 44 | 57 | 68 | 78 | 73 | 0.7 |
| 532395 | 47 | 62 | 82 | 91 | 96 | 0.6 |
| 532401 | 70 | 83 | 91 | 94 | 96 | <0.6 |
| 532410 | 56 | 71 | 85 | 90 | 96 | <0.6 |
| 532411 | 88 | 93 | 96 | 97 | 98 | <0.6 |
| 532420 | 61 | 67 | 82 | 85 | 96 | <0.6 |
| 532436 | 48 | 49 | 77 | 90 | 97 | 0.8 |
| 532468 | 42 | 67 | 82 | 89 | 94 | 0.6 |
| 532476 | 32 | 58 | 75 | 84 | 90 | 1.1 |
| 532482 | 5 | 26 | 56 | 71 | 87 | 2.6 |
| 532490 | 18 | 47 | 55 | 69 | 86 | 2.0 |
| 532501 | 4 | 22 | 43 | 59 | 77 | 3.5 |
| 532507 | 39 | 63 | 66 | 83 | 89 | 0.9 |
| 532526 | 30 | 48 | 67 | 82 | 88 | 1.4 |

**Table 44**

| ISIS No | 0.625 µM | 1.250 µM | 2.50 µM | 5.00 µM | 10.00 µM | IC₅₀ (µM) |
|---|---|---|---|---|---|---|
| 533121 | 59 | 67 | 78 | 83 | 87 | 0.2 |
| 533122 | 48 | 73 | 78 | 84 | 90 | 0.4 |
| 533125 | 47 | 61 | 74 | 89 | 89 | 0.6 |
| 533136 | 5 | 25 | 58 | 79 | 90 | 2.4 |
| 533156 | 37 | 48 | 69 | 77 | 87 | 1.2 |
| 533161 | 28 | 67 | 77 | 89 | 90 | 1.0 |
| 533178 | 30 | 60 | 72 | 90 | 92 | 1.1 |
| 533179 | 37 | 66 | 76 | 76 | 87 | 0.8 |
| 533188 | 32 | 64 | 74 | 80 | 90 | 1.0 |
| 533189 | 49 | 66 | 77 | 81 | 81 | 0.4 |
| 533193 | 26 | 48 | 69 | 75 | 85 | 1.5 |
| 533233 | 39 | 60 | 59 | 84 | 93 | 1.0 |
| 533234 | 45 | 69 | 84 | 91 | 94 | 0.5 |
| 533235 | 28 | 49 | 69 | 82 | 90 | 1.4 |

**Table 45**

| ISIS No | 0.625 µM | 1.250 µM | 2.50 µM | 5.00 µM | 10.00 µM | IC₅₀ (µM) |
|---|---|---|---|---|---|---|
| 533256 | 47 | 72 | 86 | 90 | 94 | <0.6 |
| 533257 | 63 | 77 | 88 | 91 | 96 | <0.6 |
| 533258 | 66 | 81 | 88 | 95 | 95 | <0.6 |
| 533259 | 48 | 70 | 84 | 90 | 93 | <0.6 |
| 533262 | 44 | 66 | 79 | 90 | 96 | 0.7 |
| 533265 | 59 | 74 | 85 | 93 | 96 | <0.6 |
| 533269 | 25 | 55 | 74 | 86 | 87 | 1.2 |
| 533270 | 34 | 59 | 73 | 86 | 95 | 1.0 |
| 533271 | 63 | 82 | 88 | 92 | 92 | <0.6 |
| 533291 | 14 | 46 | 64 | 84 | 89 | 1.8 |
| 533301 | 49 | 61 | 75 | 83 | 91 | 0.6 |
| 533315 | 22 | 39 | 73 | 76 | 91 | 1.7 |
| 533317 | 26 | 53 | 68 | 85 | 94 | 1.3 |
| 533318 | 29 | 40 | 46 | 77 | 91 | 1.9 |

**Table 46**

| ISIS No | 0.625 µM | 1.250 µM | 2.50 µM | 5.00 µM | 10.00 µM | IC₅₀ (µM) |
|---|---|---|---|---|---|---|
| 533280 | 58 | 64 | 77 | 82 | 87 | 0.3 |
| 533316 | 35 | 55 | 68 | 87 | 91 | 1.1 |
| 533326 | 34 | 68 | 76 | 89 | 96 | 0.8 |
| 533328 | 54 | 55 | 79 | 83 | 92 | 0.5 |
| 533329 | 46 | 62 | 72 | 83 | 95 | 0.7 |
| 533330 | 56 | 75 | 83 | 91 | 94 | 0.3 |
| 533331 | 54 | 61 | 80 | 86 | 89 | 0.4 |
| 533352 | 54 | 62 | 79 | 83 | 89 | 0.4 |
| 533364 | 52 | 73 | 83 | 91 | 94 | 0.4 |
| 533898 | 17 | 47 | 63 | 78 | 87 | 1.8 |
| 533908 | 35 | 58 | 74 | 82 | 87 | 1 |
| 533916 | 22 | 46 | 72 | 78 | 88 | 1.6 |
| 533932 | 51 | 62 | 70 | 79 | 80 | 0.5 |
| 533937 | 20 | 40 | 61 | 79 | 85 | 1.9 |

### Example 5: Dose-dependent antisense inhibition of human GHR in Hep3B cells by MOE gapmers

Gapmers from studies described above exhibiting significant *in vitro* inhibition of GHR mRNA were selected and tested at various doses in Hep3B cells. The antisense oligonucleotides were tested in a series of experiments that had similar culture conditions. The results for each experiment are presented in separate tables shown below. Cells were plated at a density of 20,000 cells per well and transfected using electroporation with 0.3125 µM, 0.625 µM, 1.25 µM, 2.50 µM, 5.00 µM and 10.00 µM concentrations of antisense oligonucleotide, as specified in the Tables below. After a treatment period of approximately 16 hours, RNA was isolated from the cells and GHR mRNA levels were measured by quantitative real-time PCR. Human primer probe set RTS3437_MGB was used to measure mRNA levels. GHR mRNA levels were adjusted according to total RNA content, as measured by RIBOGREEN®. Results are presented as percent inhibition of GHR, relative to untreated control cells.

The half maximal inhibitory concentration (IC₅₀) of each oligonucleotide is also presented. GHR mRNA levels were significantly reduced in a dose-dependent manner in antisense oligonucleotide treated cells.

**Table 47**

| ISIS No | 0.3125 µM | 0.625 µM | 1.250 µM | 2.50 µM | 5.00 µM | 10.00 µM | IC₅₀ (µM) |
|---|---|---|---|---|---|---|---|
| 523577 | 0 | 16 | 33 | 59 | 72 | 94 | 2.2 |
| 523633 | 15 | 33 | 66 | 73 | 82 | 86 | 1.1 |
| 523764 | 11 | 33 | 50 | 68 | 78 | 83 | 1.5 |
| 523794 | 12 | 30 | 33 | 56 | 76 | 82 | 1.9 |
| 523805 | 21 | 48 | 66 | 78 | 85 | 92 | 0.8 |
| 523810 | 18 | 36 | 61 | 80 | 89 | 90 | 1.0 |
| 523814 | 13 | 35 | 52 | 67 | 81 | 88 | 1.3 |
| 523819 | 11 | 30 | 57 | 72 | 81 | 89 | 1.3 |
| 523820 | 0 | 15 | 43 | 61 | 84 | 92 | 1.8 |
| 523824 | 21 | 27 | 59 | 72 | 84 | 90 | 1.2 |

**Table 48**

| ISIS No | 0.3125 µM | 0.625 µM | 1.250 µM | 2.50 µM | 5.00 µM | 10.00 µM | IC₅₀ (µM) |
|---|---|---|---|---|---|---|---|
| 539302 | 34 | 41 | 56 | 83 | 83 | 96 | 0.8 |
| 539321 | 30 | 32 | 76 | 73 | 80 | 94 | 0.8 |
| 539322 | 22 | 36 | 57 | 72 | 78 | 94 | 1.1 |
| 539355 | 23 | 42 | 48 | 72 | 71 | 88 | 1.2 |
| 539359 | 21 | 38 | 48 | 73 | 78 | 92 | 1.2 |
| 539320 | 14 | 32 | 53 | 72 | 82 | 91 | 1.3 |
| 539341 | 3 | 19 | 35 | 56 | 78 | 89 | 2.0 |
| 539342 | 6 | 18 | 33 | 51 | 70 | 83 | 2.3 |
| 539356 | 0 | 0 | 21 | 45 | 73 | 94 | 2.7 |
| 539358 | 0 | 15 | 23 | 50 | 52 | 91 | 2.9 |

**Table 49**

| ISIS No | 0.3125 µM | 0.625 µM | 1.250 µM | 2.50 µM | 5.00 µM | 10.00 µM | IC₅₀ (µM) |
|---|---|---|---|---|---|---|---|
| 539339 | 22 | 37 | 52 | 77 | 90 | 92 | 1.0 |
| 539360 | 28 | 49 | 72 | 82 | 95 | 97 | 0.7 |
| 539361 | 36 | 56 | 75 | 86 | 95 | 98 | 0.5 |
| 539362 | 24 | 26 | 63 | 77 | 91 | 97 | 1.0 |
| 539375 | 21 | 29 | 39 | 63 | 77 | 91 | 1.5 |
| 539378 | 8 | 42 | 64 | 85 | 92 | 97 | 1.0 |
| 539379 | 43 | 59 | 80 | 89 | 96 | 98 | 0.3 |
| 539380 | 61 | 73 | 90 | 95 | 98 | 98 | 0.1 |
| 539383 | 30 | 49 | 75 | 87 | 97 | 98 | 0.6 |
| 539403 | 48 | 55 | 75 | 85 | 94 | 96 | 0.3 |
| 539432 | 36 | 42 | 69 | 79 | 88 | 95 | 0.7 |

**Table 50**

| ISIS No | 0.3125 µM | 0.625 µM | 1.250 µM | 2.50 µM | 5.00 µM | 10.00 µM | IC₅₀ (µM) |
|---|---|---|---|---|---|---|---|
| 539376 | 34 | 46 | 62 | 82 | 94 | 98 | 0.7 |
| 539389 | 53 | 58 | 78 | 86 | 94 | 97 | 0.2 |
| 539392 | 1 | 19 | 26 | 68 | 81 | 94 | 1.9 |
| 539399 | 27 | 52 | 65 | 78 | 92 | 98 | 0.7 |
| 539400 | 7 | 26 | 43 | 59 | 88 | 95 | 1.6 |
| 539401 | 32 | 39 | 77 | 90 | 92 | 95 | 0.6 |
| 539404 | 22 | 59 | 77 | 87 | 93 | 95 | 0.6 |
| 539413 | 16 | 33 | 53 | 82 | 86 | 96 | 1.1 |
| 539415 | 4 | 44 | 56 | 74 | 81 | 94 | 1.2 |
| 539416 | 37 | 61 | 70 | 85 | 92 | 95 | 0.4 |
| 539433 | 31 | 52 | 70 | 85 | 87 | 94 | 0.6 |

### Example 6: Antisense inhibition of human growth hormone receptor in Hep3B cells by deoxy, MOE and cEt gapmers

Additional antisense oligonucleotides were designed targeting a growth hormone receptor (GHR) nucleic acid and were tested for their effects on GHR mRNA in vitro. The antisense oligonucleotides were tested in a series of experiments that had similar culture conditions. The results for each experiment are presented in separate tables shown below. Cultured Hep3B cells at a density of 20,000 cells per well were transfected using electroporation with 5,000 nM antisense oligonucleotide. After a treatment period of approximately 24 hours, RNA was isolated from the cells and GHR mRNA levels were measured by quantitative real-time PCR. Human primer probe set RTS3437_MGB was used to measure mRNA levels. GHR mRNA levels were adjusted according to total RNA content, as measured by RIBOGREEN®. Results are presented as percent inhibition of GHR, relative to untreated control cells.

The newly designed chimeric antisense oligonucleotides in the Tables below were designed as deoxy, MOE, and cEt gapmers. The deoxy, MOE and cEt oligonucleotides are 16 nucleosides in length wherein the nucleoside have either a MOE sugar modification, an cEt sugar modification, or a deoxy modification. The 'Chemistry' column describes the sugar modifications of each oligonucleotide. 'k' indicates a cEt sugar modification; 'd' indicates deoxyribose; and 'e' indicates a MOE modification. The internucleoside linkages throughout each gapmer are phosphorothioate (P=S) linkages. All cytosine residues throughout each gapmer are 5-methylcytosines. "Start site" indicates the 5'-most nucleoside to which the gapmer is targeted in the human gene sequence. "Stop site" indicates the 3'-most nucleoside to which the gapmer is targeted human gene sequence. Each gapmer listed in the Tables below is targeted to either the human GHR mRNA, designated herein as SEQ ID NO: 1 (GENBANK Accession No. NM 000163.4) or the human GHR genomic sequence, designated herein as SEQ ID NO: 2 (GENBANK Accession No. NT_006576.16 truncated from nucleotides 42411001 to 42714000). 'n/a' indicates that the antisense oligonucleotide does not target that particular gene sequence with 100% complementarity. In case the sequence alignment for a target gene in a particular table is not shown, it is understood that none of the oligonucleotides presented in that table align with 100% complementarity with that target gene.

**Table 51**

| Inhibition of GHR mRNA by deoxy, MOE and cEt gapmers targeting intronic and exonic regions of SEQ ID NO: 1 and 2 | | | | | | | |
|---|---|---|---|---|---|---|---|
| ISIS NO | SEQ ID NO: 1 Start Site | Target Region | Sequence | Chemistry | % inhibition | SEQ ID NO: 2 Start Site | SEQ ID NO |
| 541262 | n/a | Intron 2 | TTGGTTTGTCAATCCT | eekddddddddddkke | 84 | 156891 | 1370 |
| 541263 | 164 | Intron 1 | CCGAGCTTCGCCTCTG | eekddddddddddkke | 89 | 3040 | 1371 |
| 541264 | 167 | Intron 1 | CCTCCGAGCTTCGCCT | eekddddddddddkke | 90 | 3043 | 1372 |
| 541265 | 170 | Junction spanning two exons | GGACCTCCGAGCTTCG | eekddddddddddkke | 89 | n/a | 1373 |
| 541266 | 176 | Junction spanning two exons | CCTGTAGGACCTCCGA | eekddddddddddkke | 83 | n/a | 1374 |
| 541268 | 214 | Exon 2 | CCAGTGCCAAGGTCAA | eekddddddddddkke | 87 | 144998 | 1375 |
| 541269 | 226 | Exon 2 | CACTTGATCCTGCCAG | eekddddddddddkke | 67 | 145010 | 1376 |
| 541270 | 244 | Exon 2 | CACTTCCAGAAAAAGC | eekddddddddddkke | 34 | 145028 | 1377 |
| 541278 | 365 | Exon 4/Intron 3 | GTCTCTCGCTCAGGTG | eekddddddddddkke | 77 | 268028 | 1378 |
| 541279 | 368 | Exon 4/Intron 3 | AAAGTCTCTCGCTCAG | eekddddddddddkke | 76 | 268031 | 1379 |
| 541280 | 373 | Exon 4/Intron 3 | ATGAAAAAGTCTCTCG | eekddddddddddkke | 66 | 268036 | 1380 |
| 541283 | 445 | exon 2-exon 3 junction | TCCTTCTGGTATAGAA | eekddddddddddkke | 37 | n/a | 1381 |
| 541288 | 554 | Exon 5 | CAATAAGGTATCCAGA | eekddddddddddkke | 49 | 274114 | 1382 |
| 541289 | 561 | Exon 5 | CTTGATACAATAAGGT | eekddddddddddkke | 66 | 274121 | 1383 |
| 541290 | 569 | Exon 5 | CTAGTTAGCTTGATAC | eekddddddddddkke | 61 | 274129 | 1384 |
| 541293 | 628 | exon 3-exon 4 junction | GATCTGGTTGCACTAT | eekddddddddddkke | 57 | n/a | 1385 |
| 541294 | 639 | Exon 6 | GGCAATGGGTGGATCT | eekddddddddddkke | 38 | 278933 | 1386 |
| 541295 | 648 | Exon 6 | CCAGTTGAGGGCAATG | eekddddddddddkke | 67 | 278942 | 1387 |
| 541296 | 654 | Exon 6 | TAAAGTCCAGTTGAGG | eekddddddddddkke | 43 | 278948 | 1388 |
| 541301 | 924 | Exon 7 | TACATAGAGCACCTCA | eekddddddddddkke | 86 | 290422 | 1389 |
| 541302 | 927 | Exon 7 | TGTTACATAGAGCACC | eekddddddddddkke | 78 | 290425 | 1390 |
| 541303 | 930 | Exon 7 | AAGTGTTACATAGAGC | eekddddddddddkke | 59 | 290428 | 1391 |
| 541304 | 958 | Exon 7 | CTTCACATGTAAATTG | eekddddddddddkke | 26 | 290456 | 1392 |
| 541305 | 981 | Exon 8 | GAGCCATGGAAAGTAG | eekddddddddddkke | 66 | 292535 | 1393 |
| 541310 | 1127 | Exon 7-exon 8 junction | CCTTCCTTGAGGAGAT | eekddddddddddkke | 26 | n/a | 1394 |
| 541320 | 1317 | Exon 10 | CTTCACCCCTAGGTTA | eekddddddddddkke | 38 | 297734 | 1395 |
| 541321 | 1322 | Exon 10 | CCATCCTTCACCCCTA | eekddddddddddkke | 81 | 297739 | 1396 |
| 541322 | 1326 | Exon 10 | GTCGCCATCCTTCACC | eekddddddddddkke | 79 | 297743 | 1397 |
| 541323 | 1331 | Exon 10 | CCAGAGTCGCCATCCT | eekddddddddddkke | 64 | 297748 | 1398 |
| 541325 | 1420 | Exon 10 | GTGGCTGAGCAACCTC | eekddddddddddkke | 79 | 297837 | 1399 |
| 541326 | 1434 | Exon 10 | CCCTTTTAACCTCTGT | eekddddddddddkke | 67 | 297851 | 1400 |
| 541331 | 1492 | Exon 10 | CATCATGATAAGGTGA | eekddddddddddkke | 16 | 297909 | 1401 |
| 541332 | 1526 | Exon 10 | TGGATAACACTGGGCT | eekddddddddddkke | 30 | 297943 | 1402 |
| 541333 | 1532 | Exon 10 | TCTGCTTGGATAACAC | eekddddddddddkke | 63 | 297949 | 1403 |
| 541335 | 1597 | Exon 10 | GAATATGGGCAGCTTG | eekddddddddddkke | 33 | 298014 | 1404 |
| 541336 | 1601 | Exon 10 | AGCTGAATATGGGCAG | eekddddddddddkke | 34 | 298018 | 1405 |
| 541337 | 1607 | Exon 10 | TTGCTTAGCTGAATAT | eekddddddddddkke | 39 | 298024 | 1406 |
| 541338 | 1611 | Exon 10 | TGGATTGCTTAGCTGA | eekddddddddddkke | 79 | 298028 | 1407 |
| 541339 | 1614 | Exon 10 | ACTTGGATTGCTTAGC | eekddddddddddkke | 73 | 298031 | 1408 |

### Example 7: Antisense inhibition of human growth hormone receptor in Hep3B cells by deoxy, MOE and cEt gapmers

Additional antisense oligonucleotides were designed targeting a growth hormone receptor (GHR) nucleic acid and were tested for their effects on GHR mRNA in vitro. The antisense oligonucleotides were tested in a series of experiments that had similar culture conditions. The results for each experiment are presented in separate tables shown below. Cultured Hep3B cells at a density of 20,000 cells per well were transfected using electroporation with 4,500 nM antisense oligonucleotide. After a treatment period of approximately 24 hours, RNA was isolated from the cells and GHR mRNA levels were measured by quantitative real-time PCR. Human primer probe set RTS3437_MGB was used to measure mRNA levels. GHR mRNA levels were adjusted according to total RNA content, as measured by RIBOGREEN®. Results are presented as percent inhibition of GHR, relative to untreated control cells.

The newly designed chimeric antisense oligonucleotides in the Tables below were designed as deoxy, MOE, and cEt gapmers. The deoxy, MOE and cEt oligonucleotides are 16 nucleosides in length wherein the nucleoside have either a MOE sugar modification, a cEt sugar modification, or a deoxy modification. The 'Chemistry' column describes the sugar modifications of each oligonucleotide. 'k' indicates a cEt sugar modification; 'd' indicates deoxyribose; and 'e' indicates a MOE modification. The internucleoside linkages throughout each gapmer are phosphorothioate (P=S) linkages. All cytosine residues throughout each gapmer are 5-methylcytosines. "Start site" indicates the 5'-most nucleoside to which the gapmer is targeted in the human gene sequence. "Stop site" indicates the 3'-most nucleoside to which the gapmer is targeted human gene sequence. Each gapmer listed in the Tables below is targeted to either the human GHR mRNA, designated herein as SEQ ID NO: 1 (GENBANK Accession No. NM 000163.4) or the human GHR genomic sequence, designated herein as SEQ ID NO: 2 (GENBANK Accession No. NT_006576.16 truncated from nucleotides 42411001 to 42714000). 'n/a' indicates that the antisense oligonucleotide does not target that particular gene sequence with 100% complementarity. In case the sequence alignment for a target gene in a particular table is not shown, it is understood that none of the oligonucleotides presented in that table align with 100% complementarity with that target gene. The oligonucleotides of Table 54 do not target SEQ ID NOs: 1 or 2, but instead target variant gene sequences SEQ ID NO: 4 (GENBANK Accession No. DR006395.1) or SEQ ID NO: 7 (the complement of GENBANK Accession No. AA398260.1).

**Table 52**

| Inhibition of GHR mRNA by deoxy, MOE and cEt gapmers targeting intronic and exonic regions of SEQ ID NO: 1 and 2 | | | | | | | |
|---|---|---|---|---|---|---|---|
| ISIS NO | SEQ ID NO: 1 Start Site | Target Region | Sequence | Chemistry | % inhibition | SEQ ID NO: 2 Start Site | SEQ ID NO |
| 541262 | n/a | Intron 2 | TTGGTTTGTCAATCCT | eekddddddddddkke | 86 | 156891 | 1370 |
| 541340 | 1619 | Exon 10 | AGTGAACTTGGATTGC | eekddddddddddkke | 73 | 298036 | 1409 |
| 541341 | 1641 | Exon 10 | GGCATAAAAGTCGATG | eekddddddddddkke | 41 | 298058 | 1410 |
| 541342 | 1644 | Exon 10 | CTGGGCATAAAAGTCG | eekddddddddddkke | 33 | 298061 | 1411 |
| 541343 | 1683 | Exon 10 | GGAAAGGACCACACTA | eekddddddddddkke | 34 | 298100 | 1412 |
| 541344 | 1746 | Exon 10 | GAGTGAGACCATTTCC | eekddddddddddkke | 65 | 298163 | 1413 |
| 541345 | 1827 | Exon 10 | GATGTGAGGAGCCACA | eekddddddddddkke | 54 | 298244 | 1414 |
| 541346 | 1830 | Exon 10 | CTTGATGTGAGGAGCC | eekddddddddddkke | 70 | 298247 | 1415 |
| 541347 | 1835 | Exon 10 | TCAACCTTGATGTGAG | eekddddddddddkke | 38 | 298252 | 1416 |
| 541348 | 1839 | Exon 10 | TGATTCAACCTTGATG | eekddddddddddkke | 39 | 298256 | 1417 |
| 541349 | 1842 | Exon 10 | GTGTGATTCAACCTTG | eekddddddddddkke | 74 | 298259 | 1418 |
| 541350 | 1845 | Exon 10 | TATGTGTGATTCAACC | eekddddddddddkke | 58 | 298262 | 1419 |
| 541351 | 1949 | Exon 10 | GGCATCTCAGAACCTG | eekddddddddddkke | 41 | 298366 | 1420 |
| 541352 | 1965 | Exon 10 | GGTATAGTCTGGGACA | eekddddddddddkke | 18 | 298382 | 1421 |
| 541353 | 1969 | Exon 10 | TGGAGGTATAGTCTGG | eekddddddddddkke | 17 | 298386 | 1422 |
| 541354 | 1972 | Exon 10 | GAATGGAGGTATAGTC | eekddddddddddkke | 0 | 298389 | 1423 |
| 541355 | 1975 | Exon 10 | TATGAATGGAGGTATA | eekddddddddddkke | 0 | 298392 | 1424 |
| 541356 | 1978 | Exon 10 | CTATATGAATGGAGGT | eekddddddddddkke | 30 | 298395 | 1425 |
| 541357 | 1981 | Exon 10 | GTACTATATGAATGGA | eekddddddddddkke | 43 | 298398 | 1426 |
| 541358 | 1987 | Exon 10 | GGGACTGTACTATATG | eekddddddddddkke | 12 | 298404 | 1427 |
| 541369 | 2306 | Exon 10 | TTACATTGCACAATAG | eekddddddddddkke | 21 | 298723 | 1428 |
| 541373 | 2667 | Exon 10 | TAGCCATGCTTGAAGT | eekddddddddddkke | 34 | 299084 | 1429 |
| 541374 | 2686 | Exon 10 | TGTGTAGTGTAATATA | eekddddddddddkke | 10 | 299103 | 1430 |
| 541375 | 2690 | Exon 10 | ACAGTGTGTAGTGTAA | eekddddddddddkke | 82 | 299107 | 1431 |
| 541376 | 2697 | Exon 10 | GCAGTACACAGTGTGT | eekddddddddddkke | 46 | 299114 | 1432 |
| 541377 | 2700 | Exon 10 | ACTGCAGTACACAGTG | eekddddddddddkke | 32 | 299117 | 1433 |
| 541378 | 2740 | Exon 10 | TTAGACTGTAGTTGCT | eekddddddddddkke | 25 | 299157 | 1434 |
| 541379 | 2746 | Exon 10 | CCAGCTTTAGACTGTA | eekddddddddddkke | 69 | 299163 | 1435 |
| 541380 | 2750 | Exon 10 | TAAACCAGCTTTAGAC | eekddddddddddkke | 20 | 299167 | 1436 |
| 541381 | 2755 | Exon 10 | AACATTAAACCAGCTT | eekddddddddddkke | 64 | 299172 | 1437 |
| 541382 | 2849 | Exon 10 | ACTACAATCATTTTAG | eekddddddddddkke | 0 | 299266 | 1438 |
| 541383 | 2853 | Exon 10 | GATTACTACAATCATT | eekddddddddddkke | 0 | 299270 | 1439 |
| 541384 | 2859 | Exon 10 | AATGCAGATTACTACA | eekddddddddddkke | 46 | 299276 | 1440 |
| 541385 | 2865 | Exon 10 | TCCAATAATGCAGATT | eekddddddddddkke | 52 | 299282 | 1441 |
| 541386 | 2941 | Exon 10 | GTTGATCTGTGCAAAC | eekddddddddddkke | 74 | 299358 | 1442 |
| 541389 | 3037 | Exon 10 | TCTACTTCTCTTAGCA | eekddddddddddkke | 50 | 299454 | 1443 |
| 541393 | 3215 | Exon 10 | GCTTCTTGTACCTTAT | eekddddddddddkke | 84 | 299632 | 1444 |
| 541394 | 3237 | Exon 10 | GATTTGCTTCAACTTA | eekddddddddddkke | 47 | 299654 | 1445 |
| 541395 | 3305 | Exon 10 | GGTTATAGGCTGTGAA | eekddddddddddkke | 0 | 299722 | 1446 |
| 541396 | 3308 | Exon 10 | TCTGGTTATAGGCTGT | eekddddddddddkke | 88 | 299725 | 1447 |
| 541397 | 3311 | Exon 10 | GTGTCTGGTTATAGGC | eekddddddddddkke | 56 | 299728 | 1448 |
| 541398 | 3316 | Exon 10 | AGTATGTGTCTGGTTA | eekddddddddddkke | 76 | 299733 | 1449 |
| 541399 | 3371 | Exon 10 | GGGACTGAAAACCTTG | eekddddddddddkke | 50 | 299788 | 1450 |
| 541400 | 3975 | Exon 10 | AGTATTCTTCACTGAG | eekddddddddddkke | 36 | 300392 | 1451 |
| 541401 | 4044 | Exon 10 | GCGATAAATGGGAAAT | eekddddddddddkke | 36 | 300461 | 1452 |
| 541402 | 4048 | Exon 10 | GTCTGCGATAAATGGG | eekddddddddddkke | 52 | 300465 | 1453 |
| 541403 | 4058 | Exon 10 | CCTAAAAAAGGTCTGC | eekddddddddddkke | 51 | 300475 | 1454 |
| 541404 | 4072 | Exon 10 | CATTAAGCTTGCTTCC | eekddddddddddkke | 53 | 300489 | 1455 |

**Table 53**

| Inhibition of GHR mRNA by deoxy, MOE and cEt gapmers targeting intronic and exonic regions of SEQ ID NO: 1 and 2 | | | | | | | |
|---|---|---|---|---|---|---|---|
| ISIS NO | SEQ ID NO: 1 Start Site | Target Region | Sequence | Chemistry | % inhibition | SEQ ID NO: 2 Start Site | SEQ ID NO |
| 541262 | n/a | Intron 2 | TTGGTTTGTCAATCCT | eekddddddddddkke | 85 | 156891 | 1370 |
| 541421 | 4418 | Exon 10 | CACAACTAGTCATACT | eekddddddddddkke | 42 | 300835 | 1456 |
| 541422 | 4428 | Exon 10 | AACTGCCAGACACAAC | eekddddddddddkke | 68 | 300845 | 1457 |
| 541423 | 4431 | Exon 10 | ATAAACTGCCAGACAC | eekddddddddddkke | 86 | 300848 | 1458 |
| 541424 | 4503 | Exon 10 | TATCAGGAATCCAAGA | eekddddddddddkke | 11 | 300920 | 1459 |
| 541425 | 4521 | Exon 10 | TTGATAACAGAAGCAC | eekddddddddddkke | 16 | 300938 | 1460 |
| 541426 | 4528 | Exon 10 | TTGGTGTTTGATAACA | eekddddddddddkke | 31 | 300945 | 1461 |
| 541427 | 4531 | Exon 10 | ATGTTGGTGTTTGATA | eekddddddddddkke | 32 | 300948 | 1462 |
| 541429 | 30 | Exon 1 | CCGCCACTGTAGCAGC | eekddddddddddkke | 77 | 2906 | 1463 |
| 541430 | 35 | Exon 1 | CGCCACCGCCACTGTA | eekddddddddddkke | 88 | 2911 | 1464 |
| 541431 | 63 | Exon 1 | GCCGCCCGGGCTCAGC | eekddddddddddkke | 86 | 2939 | 1465 |
| 541432 | 67 | Exon 1 | CGCCGCCGCCCGGGCT | eekddddddddddkke | 61 | 2943 | 1466 |
| 541433 | 144 | Exon 1 | GAGAGCGCGGGTTCGC | eekddddddddddkke | 57 | 3020 | 1467 |
| 541434 | n/a | Exon 1/Intron 1 | CTACTGACCCCAGTTC | eekddddddddddkke | 80 | 3655 | 1468 |
| 541435 | n/a | Exon 1/Intron 1 | TCACTCTACTGACCCC | eekddddddddddkke | 90 | 3660 | 1469 |
| 541436 | n/a | Exon 1/Intron 1 | TCATGCGGACTGGTGG | eekddddddddddkke | 56 | 3679 | 1470 |
| 541437 | n/a | Exon 3/Intron 3 | ATGTGAGCATGGACCC | eekddddddddddkke | 82 | 225438 | 1471 |
| 541438 | n/a | Exon 3/Intron 3 | TCTTGATATGTGAGCA | eekddddddddddkke | 93 | 225445 | 1472 |
| 541439 | n/a | Exon 3/Intron 3 | TTCAAGTTGGTGAGCT | eekddddddddddkke | 72 | 226788 | 1473 |
| 541440 | n/a | Exon 3/Intron 3 | TGCTTCCTTCAAGTTG | eekddddddddddkke | 68 | 226795 | 1474 |
| 541441 | n/a | Exon 3/Intron 3 | TGTAATTTCATTCATG | eekddddddddddkke | 62 | 226809 | 1475 |
| 541442 | n/a | Exon 3/Intron 3 | CCTTTTGCCAAGAGCA | eekddddddddddkke | 85 | 226876 | 1476 |
| 541443 | n/a | Exon 3/Intron 3 | GATCCTTTTGCCAAGA | eekddddddddddkke | 77 | 226879 | 1477 |
| 541444 | n/a | Exon 3/Intron 3 | GCTAGTAATGTTACAT | eekddddddddddkke | 68 | 238331 | 1478 |
| 541445 | n/a | Exon 3/Intron 3 | GCAACTTGCTAGTAAT | eekddddddddddkke | 65 | 238338 | 1479 |
| 541446 | n/a | Exon 3/Intron 3 | TGTGCAACTTGCTAGT | eekddddddddddkke | 44 | 238341 | 1480 |
| 541447 | n/a | Exon 3/Intron 3 | GGATTTCAGTTTGAAT | eekddddddddddkke | 0 | 238363 | 1481 |
| 541448 | n/a | Exon 3/Intron 3 | CTCAGAGCCTTGGTAG | eekddddddddddkke | 65 | 238428 | 1482 |
| 541449 | n/a | Exon 1/Intron 1 | CAAACGCGCAAAAGAC | eekddddddddddkke | 1 | 3608 | 1483 |
| 541450 | n/a | Exon 1/Intron 1 | GCCCGCACAAACGCGC | eekddddddddddkke | 11 | 3615 | 1484 |
| 541451 | n/a | Exon 1/Intron 1 | GGTTAAAGAAGTTGCT | eekddddddddddkke | 60 | 93190 | 1485 |
| 541452 | n/a | Exon 1/Intron 1 | CCCAGTGAATTCAGCA | eekddddddddddkke | 85 | 93245 | 1486 |
| 541453 | n/a | Exon 1/Intron 1 | GCGCCCAGTGAATTCA | eekddddddddddkke | 74 | 93248 | 1487 |
| 541454 | n/a | Exon 1/Intron 1 | AAGATGCGCCCAGTGA | eekddddddddddkke | 71 | 93253 | 1488 |
| 541455 | n/a | Exon 1/Intron 1 | TGTAAGATGCGCCCAG | eekddddddddddkke | 75 | 93256 | 1489 |
| 541456 | n/a | Exon 1/Intron 1 | AATTACTTGTAAGATG | eekddddddddddkke | 15 | 93263 | 1490 |
| 541457 | n/a | Exon 1/Intron 1 | CCCAGAAGGCACTTGT | eekddddddddddkke | 61 | 93302 | 1491 |
| 541458 | n/a | Exon 1/Intron 1 | TTGCAGAACAAATCTT | eekddddddddddkke | 3 | 93333 | 1492 |
| 541459 | n/a | Exon 1/Intron 1 | CATGGAAGATTTGCAG | eekddddddddddkke | 17 | 93343 | 1493 |
| 541460 | n/a | Exon 1/Intron 1 | GGTCATGGAAGATTTG | eekddddddddddkke | 57 | 93346 | 1494 |
| 541461 | n/a | Exon 1/Intron 1 | GACCTTGGTCATGGAA | eekddddddddddkke | 51 | 93352 | 1495 |
| 541462 | n/a | Exon 1/Intron 1 | TGCCAATCCAAAGAGG | eekddddddddddkke | 34 | 93369 | 1496 |
| 541463 | n/a | Exon 1/Intron 1 | GGGTCTGCCAATCCAA | eekddddddddddkke | 67 | 93374 | 1497 |
| 541464 | n/a | Exon 1/Intron 1 | TCCCTGGGTCTGCCAA | eekddddddddddkke | 82 | 93379 | 1498 |
| 541465 | n/a | Exon 1/Intron 1 | AAGTGTGAATTTATCT | eekddddddddddkke | 16 | 93408 | 1499 |
| 541466 | n/a | Exon 1/Intron 1 | GGAGATCTCAACAAGG | eekddddddddddkke | 38 | 93428 | 1500 |
| 541468 | n/a | Exon 1/Intron 1 | TCGCCCATCACTCTTC | eekddddddddddkke | 43 | 93989 | 1501 |
| 541469 | n/a | Exon 1/Intron 1 | CCTGTCGCCCATCACT | eekddddddddddkke | 61 | 93993 | 1502 |
| 541470 | n/a | Exon 1/Intron 1 | TCACCTGTCGCCCATC | eekddddddddddkke | 70 | 93996 | 1503 |
| 541471 | n/a | Exon 1/Intron 1 | CCATCACCTGTCGCCC | eekddddddddddkke | 89 | 93999 | 1504 |
| 541472 | n/a | Exon 1/Intron 1 | TCACCATCACCTGTCG | eekddddddddddkke | 72 | 94002 | 1505 |
| 541473 | n/a | Exon 1/Intron 1 | TAATAGTTGTCACCAT | eekddddddddddkke | 42 | 94011 | 1506 |
| 541474 | n/a | Exon 1/Intron 1 | TTCAGATCTTATTAAT | eekddddddddddkke | 0 | 94023 | 1507 |
| 541475 | n/a | Exon 1/Intron 1 | TTGCAAATTCAGTCTG | eekddddddddddkke | 32 | 94096 | 1508 |
| 541477 | n/a | Exon 2/Intron 2 | CGTTCTCTTGGAAGTA | eekddddddddddkke | 78 | 198766 | 1509 |
| 541478 | n/a | Exon 2/Intron 2 | TCTTGAATAAATTTCG | eekddddddddddkke | 25 | 198780 | 1510 |
| 541479 | n/a | Exon 2/Intron 2 | AAGCTCACTCTTCAAT | eekddddddddddkke | 60 | 198810 | 1511 |
| 541480 | n/a | Exon 2/Intron 2 | TCCAAGCTCACTCTTC | eekddddddddddkke | 49 | 198813 | 1512 |
| 541481 | n/a | Exon 2/Intron 2 | GCTCCTGCCACTCTGT | eekddddddddddkke | 75 | 198837 | 1513 |
| 541482 | n/a | Exon 2/Intron 2 | ATGGGCAAAGGCATCT | eekddddddddddkke | 60 | 198874 | 1514 |
| 541483 | n/a | 5'UTR | AGTCTTCCCGGCGAGG | eekddddddddddkke | 32 | 2571 | 1515 |
| 541484 | n/a | 5' and overlappig with exon 1 | CCGCCGCTCCCTAGCC | eekddddddddddkke | 73 | 2867 | 1516 |
| 541485 | n/a | Intron 1 | GCCCGCAACTCCCTGC | eekddddddddddkke | 37 | 3341 | 1517 |
| 541486 | n/a | Intron 1 | CGCCTCCCCAGGCGCA | eekddddddddddkke | 34 | 4024 | 1518 |
| 541487 | n/a | Intron 1 | GAGTGTCTTCCCAGGC | eekddddddddddkke | 86 | 4446 | 1519 |
| 541488 | n/a | Intron 1 | CTGAAGACTCCTTGAA | eekddddddddddkke | 39 | 4721 | 1520 |
| 541489 | n/a | Intron 1 | GGCTAGCCAAGTTGGA | eekddddddddddkke | 54 | 5392 | 1521 |
| 541490 | n/a | Intron 1 | TGACTCCAGTCTTACC | eekddddddddddkke | 76 | 5802 | 1522 |
| 541491 | n/a | Intron 1 | ATTCATTGTGGTCAGC | eekddddddddddkke | 91 | 6128 | 1523 |
| 541492 | n/a | Intron 1 | GAAGTGGGTTTTTCCC | eekddddddddddkke | 86 | 6543 | 1524 |
| 541493 | n/a | Intron 1 | GCCTTGGTTCAGGTGA | eekddddddddddkke | 79 | 6786 | 1525 |

**Table 54**

| Inhibition of GHR mRNA by deoxy, MOE and cEt gapmers targeting SEQ ID NO: 3 and 4 | | | | | | |
|---|---|---|---|---|---|---|
| ISIS NO | Target Start Site | Target SEQ ID NO | Sequence | Chemistry | % inhibition | SEQ ID NO |
| 541428 | 66 | 3 | CCACTGTAGCAGCCGC | eekddddddddddkke | 92 | 1526 |
| 541476 | 263 | 4 | TAGGTATTTCAGAGCC | eekddddddddddkke | 80 | 1527 |

**Table 55**

| Inhibition of GHR mRNA by deoxy, MOE and cEt gapmers targeting intronic regions of SEQ ID NO: 2 | | | | | | | |
|---|---|---|---|---|---|---|---|
| ISIS NO | SEQ NO: 1 Start Site | SEQ ID NO: 2 Start Site | Target Region | Sequence | Chemistry | % inhibition | SEQ ID NO |
| 541262 | 156891 | 541277 | Intron 2 | TTGGTTTGTCAATCCT | eekddddddddddkke | 80 | 1370 |
| 541494 | 7231 | 541509 | Intron 1 | GTCCAGGCAGAGTTGT | eekddddddddddkke | 30 | 1528 |
| 541495 | 7570 | 541510 | Intron 1 | AGCCAAATGTTGGTCA | eekddddddddddkke | 19 | 1529 |
| 541496 | 8395 | 541511 | Intron 1 | GAGGGCGAGTTTTTCC | eekddddddddddkke | 71 | 1530 |
| 541497 | 9153 | 541512 | Intron 1 | GTGGCATTGGCAAGCC | eekddddddddddkke | 81 | 1531 |
| 541498 | 9554 | 541513 | Intron 1 | ACCCCACTGCACCAAG | eekddddddddddkke | 67 | 1532 |
| 541499 | 9931 | 541514 | Intron 1 | TCCAAGTACTTGCCAA | eekddddddddddkke | 83 | 1533 |
| 541500 | 10549 | 541515 | Intron 1 | AGTGCCTGGCCTAAGG | eekddddddddddkke | 75 | 1534 |
| 541501 | 11020 | 541516 | Intron 1 | GCGCTTCTTCCCTAGG | eekddddddddddkke | 71 | 1535 |
| 541502 | 11793 | 541517 | Intron 1 | CATCTTGCCCAGGGAT | eekddddddddddkke | 84 | 1536 |
| 541503 | 12214 | 541518 | Intron 1 | CCATCTTGCTCCAAGT | eekddddddddddkke | 93 | 1537 |
| 541504 | 12474 | 541519 | Intron 1 | CTTACATCCTGTAGGC | eekddddddddddkke | 71 | 1538 |
| 541505 | 12905 | 541520 | Intron 1 | CGCCTCCTGGTCCTCA | eekddddddddddkke | 97 | 1539 |
| 541506 | 13400 | 541521 | Intron 1 | CCCTATGCACTACCTA | eekddddddddddkke | 49 | 1540 |
| 541507 | 13717 | 541522 | Intron 1 | GAGGGACTGTGGTGCT | eekddddddddddkke | 65 | 1541 |
| 541508 | 14149 | 541523 | Intron 1 | GCCCAATATGTGCCAG | eekddddddddddkke | 60 | 1542 |
| 541509 | 14540 | 541524 | Intron 1 | GCTCTCTCATCGCTGG | eekddddddddddkke | 90 | 1543 |
| 541510 | 15264 | 541525 | Intron 1 | CTCAAGGCTATGTGCC | eekddddddddddkke | 67 | 1544 |
| 541511 | 15849 | 541526 | Intron 1 | TCCACATCCCTCATGT | eekddddddddddkke | 68 | 1545 |
| 541512 | 16530 | 541527 | Intron 1 | AGGACTGAAGGCCCAT | eekddddddddddkke | 49 | 1546 |
| 541513 | 17377 | 541528 | Intron 1 | GTGCGACTTACCAGCT | eekddddddddddkke | 85 | 1547 |
| 541514 | 17581 | 541529 | Intron 1 | TCGCTAAAGCCACACA | eekddddddddddkke | 89 | 1548 |
| 541515 | 17943 | 541530 | Intron 1 | GCTCTGGCTGATGGTC | eekddddddddddkke | 92 | 1549 |
| 541516 | 18353 | 541531 | Intron 1 | TTCCCATGAGGATTTC | eekddddddddddkke | 70 | 1550 |
| 541517 | 18636 | 541532 | Intron 1 | TTGGGCTTAAGCACTA | eekddddddddddkke | 71 | 1551 |
| 541518 | 19256 | 541533 | Intron 1 | GCTAGCACCTAGTCCA | eekddddddddddkke | 71 | 1552 |
| 541519 | 19814 | 541534 | Intron 1 | CCTCTGGCCTACAACA | eekddddddddddkke | 64 | 1553 |
| 541520 | 20365 | 541535 | Intron 1 | ACCCCTCATCAGCACC | eekddddddddddkke | 93 | 1554 |
| 541521 | 20979 | 541536 | Intron 1 | GGCCACCCCTGATCCT | eekddddddddddkke | 66 | 1555 |
| 541522 | 21566 | 541537 | Intron 1 | GAAGCTCCCTTGCCCA | eekddddddddddkke | 96 | 1556 |
| 541523 | 22150 | 541538 | Intron 1 | AGTGTTGCCCCTCCAA | eekddddddddddkke | 83 | 1557 |
| 541524 | 22803 | 541539 | Intron 1 | GGGTCTCCAACCTACT | eekddddddddddkke | 70 | 1558 |
| 541525 | 29049 | 541540 | Intron 1 | GGGATGTAGGTTTACC | eekddddddddddkke | 74 | 1559 |
| 541526 | 29554 | 541541 | Intron 1 | GCAACCGATATCACAG | eekddddddddddkke | 60 | 1560 |
| 541527 | 30245 | 541542 | Intron 1 | TGCCCTGGAACAAATT | eekddddddddddkke | 13 | 1561 |
| 541528 | 30550 | 541543 | Intron 1 | AGTCTAGGAGTAGCTA | eekddddddddddkke | 50 | 1562 |
| 541529 | 30915 | 541544 | Intron 1 | GCTGTTGTCAAGAGAC | eekddddddddddkke | 55 | 1563 |
| 541530 | 31468 | 541545 | Intron 1 | CACCTAGACACTCAGT | eekddddddddddkke | 47 | 1564 |
| 541531 | 32366 | 541546 | Intron 1 | GTCAAGGGATCCCTGC | eekddddddddddkke | 34 | 1565 |
| 541532 | 32897 | 541547 | Intron 1 | TCCCCCTGGCACTCCA | eekddddddddddkke | 79 | 1566 |
| 541533 | 33187 | 541548 | Intron 1 | GCCTGGTAACTCCATT | eekddddddddddkke | 56 | 1567 |
| 541534 | 33780 | 541549 | Intron 1 | GGGCTCACCAACTGTG | eekddddddddddkke | 39 | 1568 |
| 541535 | 34407 | 541550 | Intron 1 | CCACAGGATCATATCA | eekddddddddddkke | 37 | 1569 |
| 541536 | 34846 | 541551 | Intron 1 | CTCCAGCAGAAGTGTC | eekddddddddddkke | 10 | 1570 |
| 541537 | 35669 | 541552 | Intron 1 | AGCCCAACTGTTGCCT | eekddddddddddkke | 79 | 1571 |
| 541538 | 36312 | 541553 | Intron 1 | TGCCAGGCAGTTGCCA | eekddddddddddkke | 75 | 1572 |
| 541539 | 36812 | 541554 | Intron 1 | GCCAGTAAGCACCTTG | eekddddddddddkke | 93 | 1573 |
| 541540 | 37504 | 541555 | Intron 1 | CTAGCTTCCCAGCCCC | eekddddddddddkke | 46 | 1574 |
| 541541 | 38841 | 541556 | Intron 1 | TCAAGCCCAGCTAGCA | eekddddddddddkke | 39 | 1575 |
| 541542 | 39108 | 541557 | Intron 1 | CCTCACAGGCCCTAAT | eekddddddddddkke | 4 | 1576 |
| 541543 | 39408 | 541558 | Intron 1 | ACCTGCTTACATGGTA | eekddddddddddkke | 21 | 1577 |
| 541544 | 40250 | 541559 | Intron 1 | CCTTTGCTAGGACCCA | eekddddddddddkke | 52 | 1578 |
| 541545 | 40706 | 541560 | Intron 1 | GGGACTGCCACCAAGG | eekddddddddddkke | 27 | 1579 |
| 541546 | 40922 | 541561 | Intron 1 | GCTAGATGTTCAGGCC | eekddddddddddkke | 34 | 1580 |
| 541547 | 41424 | 541562 | Intron 1 | CCTATGGCCATGCTGA | eekddddddddddkke | 32 | 1581 |
| 541548 | 41999 | 541563 | Intron 1 | GTATGCTAGTTCCCAT | eekddddddddddkke | 83 | 1582 |
| 541549 | 42481 | 541564 | Intron 1 | CCCTCATAATCTTGGG | eekddddddddddkke | 13 | 1583 |
| 541550 | 42700 | 541565 | Intron 1 | GTCCAACCACTACCAC | eekddddddddddkke | 74 | 1584 |
| 541551 | 43291 | 541566 | Intron 1 | ACTTGCAGATAGCTGA | eekddddddddddkke | 73 | 1585 |
| 541552 | 43500 | 541567 | Intron 1 | GCATGACCCCACTGCC | eekddddddddddkke | 72 | 1586 |
| 541553 | 43947 | 541568 | Intron 1 | GAGGGTCACATTCCCT | eekddddddddddkke | 23 | 1587 |
| 541554 | 44448 | 541569 | Intron 1 | TCTCTTACTGGTGGGT | eekddddddddddkke | 90 | 1588 |
| 541555 | 45162 | 541570 | Intron 1 | GCCCCCTTCCTGGATA | eekddddddddddkke | 28 | 1589 |
| 541556 | 46010 | 541571 | Intron 1 | CCTCATGCGACACCAC | eekddddddddddkke | 71 | 1590 |
| 541557 | 46476 | 541572 | Intron 1 | AGCCCTCTGCCTGTAA | eekddddddddddkke | 67 | 1591 |
| 541558 | 47447 | 541573 | Intron 1 | CTCCCAGCTATAGGCG | eekddddddddddkke | 38 | 1592 |
| 541559 | 47752 | 541574 | Intron 1 | GCTAGCTGCGCAAGGA | eekddddddddddkke | 5 | 1593 |
| 541560 | 48001 | 541575 | Intron 1 | GCGCAGCCCGCTGCAA | eekddddddddddkke | 18 | 1594 |
| 541561 | 48423 | 541576 | Intron 1 | TGCATGATCCACCCCA | eekddddddddddkke | 65 | 1595 |
| 541562 | 50195 | 541577 | Intron 1 | GCTTAGTGCTGGCCCA | eekddddddddddkke | 72 | 1596 |
| 541563 | 50470 | 541578 | Intron 1 | CCTTCCAGTCCTCATA | eekddddddddddkke | 81 | 1597 |
| 541564 | 51104 | 541579 | Intron 1 | ATAGTGTCAAGGCCCA | eekddddddddddkke | 91 | 1598 |
| 541565 | 51756 | 541580 | Intron 1 | AGGCCTTAGTCACCCA | eekddddddddddkke | 88 | 1599 |
| 541566 | 52015 | 541581 | Intron 1 | TAACCAACCTAAGGGA | eekddddddddddkke | 11 | 1600 |
| 541567 | 52230 | 541582 | Intron 1 | ATTCTGGTGATGCCCT | eekddddddddddkke | 66 | 1601 |
| 541568 | 52588 | 541583 | Intron 1 | GTGTTCACTGCCATGA | eekddddddddddkke | 67 | 1602 |
| 541569 | 53532 | 541584 | Intron 1 | GGTAGAGCACACTGCC | eekddddddddddkke | 47 | 1603 |
| 541570 | 54645 | 541585 | Intron 1 | CCACTTTAATGCCACC | eekddddddddddkke | 76 | 1604 |

**Table 56**

| Inhibition of GHR mRNA by deoxy, MOE and cEt gapmers targeting intronic regions of SEQ ID NO: 2 | | | | | | | |
|---|---|---|---|---|---|---|---|
| ISIS NO | SEQ ID NO: 2 Start Site | SEQ ID NO: 2 Stop Site | Target Region | Sequence | Chemistry | % inhibition | SEQ ID NO |
| 541262 | 156891 | 156906 | Intron 2 | TTGGTTTGTCAATCCT | eekddddddddddkke | 88 | 1370 |
| 541571 | 54886 | 54901 | Intron 1 | GTCAAATGCTGTTGGG | eekddddddddddkke | 91 | 1605 |
| 541572 | 55900 | 55915 | Intron 1 | CATCCCCTATCAGGGT | eekddddddddddkke | 53 | 1606 |
| 541573 | 62266 | 62281 | Intron 1 | CTCGAATCCCTTGAGC | eekddddddddddkke | 73 | 1607 |
| 541574 | 62733 | 62748 | Intron 1 | GATTCCCTCCCCTAAC | eekddddddddddkke | 27 | 1608 |
| 541575 | 63173 | 63188 | Intron 1 | ATCCATCCATGTGCTG | eekddddddddddkke | 92 | 1609 |
| 541576 | 63751 | 63766 | Intron 1 | GAGCATGCCTCAGTGG | eekddddddddddkke | 81 | 1610 |
| 541577 | 63964 | 63979 | Intron 1 | CAGAAGGACTGCCTCT | eekddddddddddkke | 50 | 1611 |
| 541578 | 64213 | 64228 | Intron 1 | ACAATGCTCAACAGCC | eekddddddddddkke | 75 | 1612 |
| 541579 | 64576 | 64591 | Intron 1 | GTTGGATCTGGCATGC | eekddddddddddkke | 80 | 1613 |
| 541580 | 65027 | 65042 | Intron 1 | CGGCTGAGAGCAAGGG | eekddddddddddkke | 88 | 1614 |
| 541581 | 65363 | 65378 | Intron 1 | GAGAGGGTTCAGCCTG | eekddddddddddkke | 62 | 1615 |
| 541582 | 65600 | 65615 | Intron 1 | ACTTAGTTCCTAGCCA | eekddddddddddkke | 91 | 1616 |
| 541583 | 66087 | 66102 | Intron 1 | GTGAACCAGATGTGCT | eekddddddddddkke | 86 | 1617 |
| 541584 | 66566 | 66581 | Intron 1 | GGAGTGACAGCTAAGT | eekddddddddddkke | 98 | 1618 |
| 541585 | 66978 | 66993 | Intron 1 | AAGTGTTCAGAGCCAC | eekddddddddddkke | 97 | 1619 |
| 541586 | 67662 | 67677 | Intron 1 | AACCCTGCCAAGGTAC | eekddddddddddkke | 45 | 1620 |
| 541587 | 67914 | 67929 | Intron 1 | GATGGTGAGCACTACC | eekddddddddddkke | 78 | 1621 |
| 541588 | 68278 | 68293 | Intron 1 | GGCAGGATAGGACAGA | eekddddddddddkke | 11 | 1622 |
| 541589 | 68727 | 68742 | Intron 1 | GCAAAGTGATGAGCCT | eekddddddddddkke | 81 | 1623 |
| 541590 | 69207 | 69222 | Intron 1 | CTATCCACACCATTCC | eekddddddddddkke | 93 | 1624 |
| 541591 | 69605 | 69620 | Intron 1 | GGATCATGGGCCCCTA | eekddddddddddkke | 70 | 1625 |
| 541592 | 70130 | 70145 | Intron 1 | GTGAATTTGCTGGGCC | eekddddddddddkke | 94 | 1626 |
| 541593 | 70569 | 70584 | Intron 1 | GTGATGGGCCCAAGGC | eekddddddddddkke | 67 | 1627 |
| 541594 | 71056 | 71071 | Intron 1 | TCCTCAGTCGGCTTGC | eekddddddddddkke | 69 | 1628 |
| 541595 | 71314 | 71329 | Intron 1 | CAGCCTTTTGCCAGAT | eekddddddddddkke | 93 | 1629 |
| 541596 | 71620 | 71635 | Intron 1 | CCTCCCTAGGATTACC | eekddddddddddkke | 42 | 1630 |
| 541597 | 72226 | 72241 | Intron 1 | ACGCCCCAATCACTCA | eekddddddddddkke | 79 | 1631 |
| 541598 | 72655 | 72670 | Intron 1 | GCATGACCCATTATGT | eekddddddddddkke | 94 | 1632 |
| 541599 | 73061 | 73076 | Intron 1 | TCCCTCCAAGAGCTCA | eekddddddddddkke | 83 | 1633 |
| 541600 | 73708 | 73723 | Intron 1 | GATGCCTGTGGCTGAC | eekddddddddddkke | 84 | 1634 |
| 541601 | 74107 | 74122 | Intron 1 | GGCTAGCATGTTGCCT | eekddddddddddkke | 19 | 1635 |
| 541602 | 74542 | 74557 | Intron 1 | TAACCCACTAGGCTGG | eekddddddddddkke | 84 | 1636 |
| 541603 | 74947 | 74962 | Intron 1 | TGGCCCAAAACTAATC | eekddddddddddkke | 34 | 1637 |
| 541604 | 75192 | 75207 | Intron 1 | GGAGCAGTCTGGCACC | eekddddddddddkke | 85 | 1638 |
| 541605 | 75699 | 75714 | Intron 1 | TATTCTGTGGGACAAG | eekddddddddddkke | 51 | 1639 |
| 541606 | 75979 | 75994 | Intron 1 | GTGTCTAGTTCCAGCC | eekddddddddddkke | 86 | 1640 |
| 541607 | 76410 | 76425 | Intron 1 | TACTATCATGTAGCGC | eekddddddddddkke | 87 | 1641 |
| 541608 | 76701 | 76716 | Intron 1 | TGCCCTTGTAGTGAGA | eekddddddddddkke | 31 | 1642 |
| 541609 | 76980 | 76995 | Intron 1 | TCCCCAACCTACAAGC | eekddddddddddkke | 41 | 1643 |
| 541610 | 77292 | 77307 | Intron 1 | GCTCTAGGCATATGAA | eekddddddddddkke | 63 | 1644 |
| 541611 | 77555 | 77570 | Intron 1 | TACCTCCCTTGTAGGG | eekddddddddddkke | 27 | 1645 |
| 541612 | 77854 | 77869 | Intron 1 | GGTTCCCTTGCAGAGA | eekddddddddddkke | 62 | 1646 |
| 541613 | 78311 | 78326 | Intron 1 | GTGCCCTCTTCATGCC | eekddddddddddkke | 68 | 1647 |
| 541614 | 79006 | 79021 | Intron 1 | CCTGTGTGCAACTGGC | eekddddddddddkke | 85 | 1648 |
| 541615 | 79490 | 79505 | Intron 1 | CTGAGTCATTTGCCTG | eekddddddddddkke | 93 | 1649 |
| 541616 | 79829 | 79844 | Intron 1 | GGCCTTAGTAGGCCAG | eekddddddddddkke | 0 | 1650 |
| 541617 | 80277 | 80292 | Intron 1 | GTCCTTGCAGTCAACC | eekddddddddddkke | 77 | 1651 |
| 541618 | 80575 | 80590 | Intron 1 | GCTGGGCCAAGTCCAT | eekddddddddddkke | 77 | 1652 |
| 541619 | 80895 | 80910 | Intron 1 | TAGGGCACTTTTTGCC | eekddddddddddkke | 31 | 1653 |
| 541620 | 81207 | 81222 | Intron 1 | GCTGAGGTCCCTCTCT | eekddddddddddkke | 34 | 1654 |
| 541621 | 81761 | 81776 | Intron 1 | CTTTGGTCCCATTGCC | eekddddddddddkke | 83 | 1655 |
| 541622 | 82233 | 82248 | Intron 1 | GGAACATGCCAAGGGC | eekddddddddddkke | 91 | 1656 |
| 541623 | 82738 | 82753 | Intron 1 | AGGTGGTCTCCCTTCA | eekddddddddddkke | 74 | 1657 |
| 541624 | 83056 | 83071 | Intron 1 | TCCCAAAGCTCCCCTC | eekddddddddddkke | 53 | 1658 |
| 541625 | 83401 | 83416 | Intron 1 | CCTGGCCTAGCAAGCT | eekddddddddddkke | 47 | 1659 |
| 541626 | 84048 | 84063 | Intron 1 | TCTTAGCCCTGGGCTA | eekddddddddddkke | 12 | 1660 |
| 541627 | 84388 | 84403 | Intron 1 | GACTTGGACTGGGCTC | eekddddddddddkke | 81 | 1661 |
| 541628 | 85261 | 85276 | Intron 1 | GGCCTAGGATCTAGGA | eekddddddddddkke | 0 | 1662 |
| 541629 | 85714 | 85729 | Intron 1 | GTCAGGCTAGAGGGAC | eekddddddddddkke | 41 | 1663 |
| 541630 | 86220 | 86235 | Intron 1 | GGAAGTTCTCCCAGCC | eekddddddddddkke | 47 | 1664 |
| 541631 | 86640 | 86655 | Intron 1 | CCTGACTGATGTACAC | eekddddddddddkke | 35 | 1665 |
| 541632 | 86903 | 86918 | Intron 1 | CTCTGGCCTAGCCTAT | eekddddddddddkke | 54 | 1666 |
| 541633 | 87247 | 87262 | Intron 1 | GGCTGCTGTCAGATGC | eekddddddddddkke | 79 | 1667 |
| 541634 | 88293 | 88308 | Intron 1 | TCTCAGGTGTAGGCAG | eekddddddddddkke | 59 | 1668 |
| 541635 | 88605 | 88620 | Intron 1 | GGTCACTGAGACTGGG | eekddddddddddkke | 88 | 1669 |
| 541636 | 88952 | 88967 | Intron 1 | ACCCACTAGCAGCTAG | eekddddddddddkke | 61 | 1670 |
| 541637 | 89160 | 89175 | Intron 1 | CGGATGAGGCAGTTAG | eekddddddddddkke | 42 | 1671 |
| 541638 | 89855 | 89870 | Intron 1 | TGGTAGGCCCTCTGGC | eekddddddddddkke | 28 | 1672 |
| 541639 | 90240 | 90255 | Intron 1 | GTCACAAGGTGGGTGC | eekddddddddddkke | 28 | 1673 |
| 541640 | 90513 | 90528 | Intron 1 | GTCTTGCCCTCACGGA | eekddddddddddkke | 73 | 1674 |
| 541641 | 91073 | 91088 | Intron 1 | GCAGTCTGTGGACTTA | eekddddddddddkke | 93 | 1675 |
| 541642 | 91647 | 91662 | Intron 1 | TGCTCTCTGGTCACAC | eekddddddddddkke | 75 | 1676 |
| 541643 | 92069 | 92084 | Intron 1 | TATCCCCCAGAGCCAT | eekddddddddddkke | 68 | 1677 |
| 541644 | 92356 | 92371 | Intron 1 | AAGGTGAGAGGGCACT | eekddddddddddkke | 75 | 1678 |
| 541645 | 92904 | 92919 | Intron 1 | GTTTTAACCTCACCCT | eekddddddddddkke | 0 | 1679 |
| 541646 | 93846 | 93861 | Intron 1 | CCTTCCACTGACCTTC | eekddddddddddkke | 56 | 1680 |
| 541647 | 94374 | 94389 | Intron 1 | GACACTAGCCTAAGCC | eekddddddddddkke | 37 | 1681 |

**Table 57**

| Inhibition of GHR mRNA by deoxy, MOE and cEt gapmers targeting intronic regions of SEQ ID NO: 2 | | | | | | | |
|---|---|---|---|---|---|---|---|
| ISIS NO | SEQ ID NO: 2 Start Site | SEQ ID NO: 2 Stop Site | Target Region | Sequence | Chemistry | % inhibition | SEQ ID NO |
| 541262 | 156891 | 156906 | Intron 2 | TTGGTTTGTCAATCCT | eekddddddddddkke | 94 | 1370 |
| 541648 | 94638 | 94653 | Intron 1 | GGTTAGCCCTCAGCCT | eekddddddddddkke | 61 | 1682 |
| 541649 | 94839 | 94854 | Intron 1 | TATGAAGGTTGGACCA | eekddddddddddkke | 69 | 1683 |
| 541650 | 95509 | 95524 | Intron 1 | CAACCAGCTCACCTGA | eekddddddddddkke | 37 | 1684 |
| 541651 | 95829 | 95844 | Intron 1 | GGGCTCCAAGGCTCTC | eekddddddddddkke | 75 | 1685 |
| 541652 | 96158 | 96173 | Intron 1 | AGCTGTTACATGCCAA | eekddddddddddkke | 93 | 1686 |
| 541653 | 96488 | 96503 | Intron 1 | GGCCCAGAGGTTATAG | eekddddddddddkke | 30 | 1687 |
| 541654 | 96991 | 97006 | Intron 1 | GTCCTTAGACCCCTCA | eekddddddddddkke | 70 | 1688 |
| 541655 | 97539 | 97554 | Intron 1 | GCCCTGGCTAGAGACA | eekddddddddddkke | 39 | 1689 |
| 541656 | 98132 | 98147 | Intron 1 | CATCCAGCAGCTGGAC | eekddddddddddkke | 35 | 1690 |
| 541657 | 98833 | 98848 | Intron 1 | GACTGAGGTCATCACA | eekddddddddddkke | 60 | 1691 |
| 541658 | 99258 | 99273 | Intron 1 | GGCCAGGCACATCATG | eekddddddddddkke | 45 | 1692 |
| 541659 | 99843 | 99858 | Intron 1 | GGAGCTCATTGAGCCA | eekddddddddddkke | 36 | 1693 |
| 541660 | 100406 | 100421 | Intron 1 | GTGCCCATTGCTGTGT | eekddddddddddkke | 70 | 1694 |
| 541661 | 100742 | 100757 | Intron 1 | CCAAGTGTGGCTTCAG | eekddddddddddkke | 54 | 1695 |
| 541662 | 101305 | 101320 | Intron 1 | CCACCCTTTATACGCA | eekddddddddddkke | 87 | 1696 |
| 541663 | 101788 | 101803 | Intron 1 | CAGTAACCCCAAGGGA | eekddddddddddkke | 12 | 1697 |
| 541664 | 102649 | 102664 | Intron 1 | CCCCACCTTATATGGG | eekddddddddddkke | 9 | 1698 |
| 541665 | 103034 | 103049 | Intron 1 | AGGCCCTTTTTACATG | eekddddddddddkke | 9 | 1699 |
| 541666 | 103316 | 103331 | Intron 1 | TCAATAAGTCCCTAGG | eekddddddddddkke | 20 | 1700 |
| 541667 | 104277 | 104292 | Intron 1 | GGCATTGAGTGACTGC | eekddddddddddkke | 51 | 1701 |
| 541668 | 104679 | 104694 | Intron 1 | ATAATGCCTTCTCAGC | eekddddddddddkke | 62 | 1702 |
| 541669 | 106349 | 106364 | Intron 1 | GTGAGGCATTTAGCCC | eekddddddddddkke | 35 | 1703 |
| 541670 | 106632 | 106647 | Intron 1 | GCTCTTGTGTTGGGTA | eekddddddddddkke | 89 | 1704 |
| 541671 | 107084 | 107099 | Intron 1 | TGTGCAGGAGGTCTCA | eekddddddddddkke | 60 | 1705 |
| 541672 | 107949 | 107964 | Intron 1 | TGGAGAGTCTTGTCTC | eekddddddddddkke | 17 | 1706 |
| 541673 | 108773 | 108788 | Intron 1 | GTGACCCACCCAAGAG | eekddddddddddkke | 34 | 1707 |
| 541674 | 109336 | 109351 | Intron 1 | GTTGTAGCTAGTGTTC | eekddddddddddkke | 74 | 1708 |
| 541675 | 109849 | 109864 | Intron 1 | GCCTTAGTTTGTGCCA | eekddddddddddkke | 78 | 1709 |
| 541676 | 110427 | 110442 | Intron 1 | GCCCCAGCTGAGAATT | eekddddddddddkke | 29 | 1710 |
| 541677 | 110701 | 110716 | Intron 1 | ACAACAATCCAGGGTG | eekddddddddddkke | 61 | 1711 |
| 541678 | 110959 | 110974 | Intron 1 | CTCCCCTGGAAGTCAC | eekddddddddddkke | 59 | 1712 |
| 541679 | 111307 | 111322 | Intron 1 | GCCCTCATGGCTCAAG | eekddddddddddkke | 60 | 1713 |
| 541680 | 112499 | 112514 | Intron 1 | TCAGCAGATAGGGAGC | eekddddddddddkke | 61 | 1714 |
| 541681 | 113896 | 113911 | Intron 1 | GAATGCGGTGATCAGG | eekddddddddddkke | 29 | 1715 |
| 541682 | 117477 | 117492 | Intron 1 | CTGAGAGAATTGGCCC | eekddddddddddkke | 5 | 1716 |
| 541683 | 117740 | 117755 | Intron 1 | AGGCACATTGTTACCA | eekddddddddddkke | 26 | 1717 |
| 541684 | 118229 | 118244 | Intron 1 | GGGAGGCACTAGAGAA | eekddddddddddkke | 13 | 1718 |
| 541685 | 119269 | 119284 | Intron 1 | TACAGTAACACATCCC | eekddddddddddkke | 78 | 1719 |
| 541686 | 119688 | 119703 | Intron 1 | GAAGCTCAGCCTGATC | eekddddddddddkke | 45 | 1720 |
| 541687 | 120376 | 120391 | Intron 1 | CTTGCCTGACAACCTA | eekddddddddddkke | 53 | 1721 |
| 541688 | 120738 | 120753 | Intron 1 | GCCTACCTGCTTTTGC | eekddddddddddkke | 10 | 1722 |
| 541689 | 121242 | 121257 | Intron 1 | TTTCCCAACCACTTAG | eekddddddddddkke | 7 | 1723 |
| 541690 | 121615 | 121630 | Intron 1 | TCTCCTATTTCAGTTA | eekddddddddddkke | 23 | 1724 |
| 541691 | 121823 | 121838 | Intron 1 | GGGTGATGGATGAACT | eekddddddddddkke | 40 | 1725 |
| 541692 | 122345 | 122360 | Intron 1 | ACACTGCTGGTAGTGA | eekddddddddddkke | 0 | 1726 |
| 541693 | 122588 | 122603 | Intron 1 | ACCCAACTAGCCTGTC | eekddddddddddkke | 35 | 1727 |
| 541694 | 123152 | 123167 | Intron 1 | GAGACCTGCTGCCTGA | eekddddddddddkke | 80 | 1728 |
| 541695 | 123671 | 123686 | Intron 1 | ACATCTCTTGGGAGGT | eekddddddddddkke | 78 | 1729 |
| 541696 | 124040 | 124055 | Intron 1 | ACATAGTACCCCTCCA | eekddddddddddkke | 35 | 1730 |
| 541697 | 124430 | 124445 | Intron 1 | CTCTCAAGTACCTGCC | eekddddddddddkke | 72 | 1731 |
| 541698 | 124824 | 124839 | Intron 1 | TTTGTACCCAACCCCC | eekddddddddddkke | 15 | 1732 |
| 541699 | 125032 | 125047 | Intron 1 | AGGCCCACATAAATGC | eekddddddddddkke | 21 | 1733 |
| 541700 | 125533 | 125548 | Intron 1 | GAGCATCCCCTACACT | eekddddddddddkke | 12 | 1734 |
| 541701 | 126357 | 126372 | Intron 1 | GCTGGGCCTTTAGCTG | eekddddddddddkke | 66 | 1735 |
| 541702 | 126736 | 126751 | Intron 1 | TTGGTCAATTGGGCAG | eekddddddddddkke | 79 | 1736 |
| 541703 | 127179 | 127194 | Intron 1 | GTCTCATGAGGCCTAT | eekddddddddddkke | 60 | 1737 |
| 541704 | 127454 | 127469 | Intron 1 | GGAGGTGGGATCCCAC | eekddddddddddkke | 35 | 1738 |
| 541705 | 128467 | 128482 | Intron 1 | GCCCACTACCTAGCAC | eekddddddddddkke | 30 | 1739 |
| 541706 | 129096 | 129111 | Intron 1 | CCCAGCTGGCTGGTCG | eekddddddddddkke | 50 | 1740 |
| 541707 | 129312 | 129327 | Intron 1 | GCACCAGGTCTCCTGT | eekddddddddddkke | 7 | 1741 |
| 541708 | 129516 | 129531 | Intron 1 | GTCTAGAAGCCTAGGG | eekddddddddddkke | 23 | 1742 |
| 541709 | 129976 | 129991 | Intron 1 | GCCGGGTGTTGGTGCA | eekddddddddddkke | 50 | 1743 |
| 541710 | 130308 | 130323 | Intron 1 | TTGGTGCCTGTGTTGC | eekddddddddddkke | 49 | 1744 |
| 541711 | 130767 | 130782 | Intron 1 | TGCTTCTGATCCCTAC | eekddddddddddkke | 18 | 1745 |
| 541712 | 131286 | 131301 | Intron 1 | GTTCCCAGGAGGCTTA | eekddddddddddkke | 56 | 1746 |
| 541713 | 131676 | 131691 | Intron 1 | AGGCCCCTAGAGTCTA | eekddddddddddkke | 41 | 1747 |
| 541714 | 132292 | 132307 | Intron 1 | TGGTGTGCCCAGACTT | eekddddddddddkke | 60 | 1748 |
| 541715 | 132730 | 132745 | Intron 1 | GATGGCTAACCCACTG | eekddddddddddkke | 14 | 1749 |
| 541716 | 133101 | 133116 | Intron 1 | CCCCCAAAAGTTGCCC | eekddddddddddkke | 12 | 1750 |
| 541717 | 133522 | 133537 | Intron 1 | TAGGGTGTTCCAGATC | eekddddddddddkke | 44 | 1751 |
| 541718 | 133724 | 133739 | Intron 1 | GTACCATGAAGCTCTG | eekddddddddddkke | 67 | 1752 |
| 541719 | 134086 | 134101 | Intron 1 | CTTGGACTTGGACCAT | eekddddddddddkke | 42 | 1753 |
| 541720 | 134441 | 134456 | Intron 1 | GTGCATAGGGCCTGTC | eekddddddddddkke | 42 | 1754 |
| 541721 | 135015 | 135030 | Intron 1 | CCTCACCTGAACACCC | eekddddddddddkke | 23 | 1755 |
| 541722 | 135859 | 135874 | Intron 1 | ATGCCTCCCCGCAACT | eekddddddddddkke | 27 | 1756 |
| 541723 | 136287 | 136302 | Intron 1 | TTGTGCTTGGGTGTAC | eekddddddddddkke | 39 | 1757 |
| 541724 | 137000 | 137015 | Intron 1 | AGGCTTCATGTGAGGT | eekddddddddddkke | 86 | 1758 |

**Table 58**

| Inhibition of GHR mRNA by deoxy, MOE and cEt gapmers targeting introns 1 and 2 of SEQ ID NO: 2 | | | | | | | |
|---|---|---|---|---|---|---|---|
| ISIS NO | SEQ ID NO: 2 Start Site | SEQ ID NO: 2 Stop Site | Target Region | Sequence | Chemistry | % inhibition | SEQ ID NO |
| 541262 | 156891 | 156906 | Intron 2 | TTGGTTTGTCAATCCT | eekddddddddddkke | 95 | 1370 |
| 541725 | 137372 | 137387 | Intron 1 | TGTAAAAGGTCCTCCC | eekddddddddddkke | 53 | 1759 |
| 541726 | 137750 | 137765 | Intron 1 | GACCTGTGCAGCAGGT | eekddddddddddkke | 32 | 1760 |
| 541727 | 138783 | 138798 | Intron 1 | TCCTCTTGGAGATCCA | eekddddddddddkke | 44 | 1761 |
| 541728 | 139825 | 139840 | Intron 1 | AGGTCATAGGACTGCT | eekddddddddddkke | 73 | 1762 |
| 541729 | 140343 | 140358 | Intron 1 | GAAGGTCAGACTAGGG | eekddddddddddkke | 53 | 1763 |
| 541730 | 140686 | 140701 | Intron 1 | TCTGTAGACTGCCCAG | eekddddddddddkke | 87 | 1764 |
| 541731 | 141116 | 141131 | Intron 1 | GTCCCTCTATTCCCCT | eekddddddddddkke | 57 | 1765 |
| 541732 | 141591 | 141606 | Intron 1 | AATTGCCATGCTCCCA | eekddddddddddkke | 56 | 1766 |
| 541733 | 142113 | 142128 | Intron 1 | GATGACCTTCCTCCAA | eekddddddddddkke | 15 | 1767 |
| 541734 | 142327 | 142342 | Intron 1 | GTTTCCAGTAGCACCT | eekddddddddddkke | 82 | 1768 |
| 541735 | 143118 | 143133 | Intron 1 | GGCCTTGAGCTGATGG | eekddddddddddkke | 11 | 1769 |
| 541736 | 143836 | 143851 | Intron 1 | TATCCCTAATCAGGCT | eekddddddddddkke | 40 | 1770 |
| 541737 | 144094 | 144109 | Intron 1 | GGTGTCCACATCCCGG | eekddddddddddkke | 58 | 1771 |
| 541738 | 144558 | 144573 | Intron 1 | AGCTGGACAGGCCATA | eekddddddddddkke | 27 | 1772 |
| 541740 | 145510 | 145525 | Intron 2 | GGTAATCACCCAGAGA | eekddddddddddkke | 90 | 1773 |
| 541741 | 145937 | 145952 | Intron 2 | GCGCTAAGTCTGCTGT | eekddddddddddkke | 92 | 1774 |
| 541742 | 146320 | 146335 | Intron 2 | CCTCAAATCTTGCCCA | eekddddddddddkke | 96 | 1775 |
| 541743 | 147028 | 147043 | Intron 2 | ATCCAGACCTGGCAGA | eekddddddddddkke | 84 | 1776 |
| 541744 | 147262 | 147277 | Intron 2 | ATCCCTGCTCAAGTGC | eekddddddddddkke | 89 | 1777 |
| 541745 | 147671 | 147686 | Intron 2 | CAGGCACTCCTTGGAA | eekddddddddddkke | 93 | 1778 |
| 541746 | 148139 | 148154 | Intron 2 | AGCTGAGGTATCCCTC | eekddddddddddkke | 94 | 1779 |
| 541747 | 148564 | 148579 | Intron 2 | GGGCCCAGCAAGTCTT | eekddddddddddkke | 33 | 1780 |
| 541748 | 149069 | 149084 | Intron 2 | GTTTTGTCAGTGTGCA | eekddddddddddkke | 98 | 1781 |
| 541749 | 149491 | 149506 | Intron 2 | GTGACCTGCTGAACTC | eekddddddddddkke | 95 | 1782 |
| 541750 | 150236 | 150251 | Intron 2 | GGCTGAACTGTGCACC | eekddddddddddkke | 95 | 1783 |
| 541751 | 150748 | 150763 | Intron 2 | GGGTGGTCCCACTCCT | eekddddddddddkke | 91 | 1784 |
| 541752 | 151124 | 151139 | Intron 2 | GAGGAATCCTGGGCCC | eekddddddddddkke | 94 | 1785 |
| 541753 | 151373 | 151388 | Intron 2 | ATGACAAGCTAGGTGC | eekddddddddddkke | 81 | 1786 |
| 541754 | 151644 | 151659 | Intron 2 | TTGCCAGACAGGGCAC | eekddddddddddkke | 18 | 1787 |
| 541755 | 152373 | 152388 | Intron 2 | AGACCCCTCCCACTAT | eekddddddddddkke | 43 | 1788 |
| 541756 | 152617 | 152632 | Intron 2 | GGTGCTGGGTGACCGG | eekddddddddddkke | 91 | 1789 |
| 541757 | 153349 | 153364 | Intron 2 | GGCCAAACGGTGCCCT | eekddddddddddkke | 23 | 1790 |
| 541758 | 153918 | 153933 | Intron 2 | TGGGTGAATAGCAACC | eekddddddddddkke | 85 | 1791 |
| 541759 | 154171 | 154186 | Intron 2 | GCCCCCAAGGAAGTGA | eekddddddddddkke | 76 | 1792 |
| 541760 | 154813 | 154828 | Intron 2 | CAGGCTTCATGTGTGG | eekddddddddddkke | 92 | 1793 |
| 541761 | 155289 | 155304 | Intron 2 | CTGTCAGTGCTTTGGT | eekddddddddddkke | 52 | 1794 |
| 541762 | 156233 | 156248 | Intron 2 | GAGTACCCTGGCAGGT | eekddddddddddkke | 58 | 1795 |
| 541763 | 156847 | 156862 | Intron 2 | TAGCTAGCACCTGGGT | eekddddddddddkke | 90 | 1796 |
| 541764 | 157552 | 157567 | Intron 2 | GGCAAACCTTTGAGCC | eekddddddddddkke | 27 | 1797 |
| 541765 | 157927 | 157942 | Intron 2 | GCTATCATTGGAGCAG | eekddddddddddkke | 94 | 1798 |
| 541766 | 158542 | 158557 | Intron 2 | CCTCTGAGTACTCCCT | eekddddddddddkke | 96 | 1799 |
| 541767 | 159252 | 159267 | Intron 2 | AGCTGAAGGCAACCAG | eekddddddddddkke | 97 | 1800 |
| 541768 | 159539 | 159554 | Intron 2 | GGGCAGTTTTCCATAG | eekddddddddddkke | 89 | 1801 |
| 541769 | 159778 | 159793 | Intron 2 | GGTCCTACCTCTGACA | eekddddddddddkke | 82 | 1802 |
| 541770 | 160352 | 160367 | Intron 2 | GGCTGCCTTAGGGTGG | eekddddddddddkke | 90 | 1803 |
| 541771 | 160812 | 160827 | Intron 2 | CGCACCTCCCCCACTA | eekddddddddddkke | 15 | 1804 |
| 541772 | 161461 | 161476 | Intron 2 | GCTTATTGGTCCATGG | eekddddddddddkke | 93 | 1805 |
| 541773 | 161821 | 161836 | Intron 2 | AACCGCAGAGCCCCCA | eekddddddddddkke | 76 | 1806 |
| 541774 | 162132 | 162147 | Intron 2 | GGGCTTGTTCTGCCAA | eekddddddddddkke | 33 | 1807 |
| 541775 | 162639 | 162654 | Intron 2 | GGGACCTGCGCTGACT | eekddddddddddkke | 86 | 1808 |
| 541776 | 163024 | 163039 | Intron 2 | CTTTCACCTGGTGACT | eekddddddddddkke | 83 | 1809 |
| 541777 | 163542 | 163557 | Intron 2 | AGCTTGAGGGAGTATA | eekddddddddddkke | 52 | 1810 |
| 541778 | 164144 | 164159 | Intron 2 | GCCTGCTCAATTGAGG | eekddddddddddkke | 32 | 1811 |
| 541779 | 164570 | 164585 | Intron 2 | ATAGCAGCTGGCTGCC | eekddddddddddkke | 24 | 1812 |
| 541780 | 165419 | 165434 | Intron 2 | AAAAGCTTGGCACCCC | eekddddddddddkke | 91 | 1813 |
| 541781 | 165859 | 165874 | Intron 2 | CCTGGCAAGAAGGGCC | eekddddddddddkke | 65 | 1814 |
| 541782 | 166435 | 166450 | Intron 2 | TTAGCCCATCTATCCC | eekddddddddddkke | 82 | 1815 |
| 541783 | 166837 | 166852 | Intron 2 | GTGGTCTCCCTGTGCC | eekddddddddddkke | 90 | 1816 |
| 541784 | 167107 | 167122 | Intron 2 | AGCCCTCTCTGGCAAA | eekddddddddddkke | 38 | 1817 |
| 541785 | 168004 | 168019 | Intron 2 | TTACTGTGGCCCGAGT | eekddddddddddkke | 94 | 1818 |
| 541786 | 169062 | 169077 | Intron 2 | GTAGACTCCTAGGGTC | eekddddddddddkke | 90 | 1819 |
| 541787 | 169696 | 169711 | Intron 2 | CCTCCAGTTAGTGTGC | eekddddddddddkke | 91 | 1820 |
| 541788 | 170081 | 170096 | Intron 2 | GTGGGTGGCCAACAGG | eekddddddddddkke | 91 | 1821 |
| 541789 | 170799 | 170814 | Intron 2 | GGGATTCCCTGGTAGC | eekddddddddddkke | 77 | 1822 |
| 541790 | 171021 | 171036 | Intron 2 | GTGAGACCGGCCTTTG | eekddddddddddkke | 23 | 1823 |
| 541791 | 171530 | 171545 | Intron 2 | ACTGGCACCCACTTGG | eekddddddddddkke | 54 | 1824 |
| 541792 | 172447 | 172462 | Intron 2 | ATTGGCCTAATGCCCC | eekddddddddddkke | 76 | 1825 |
| 541793 | 172733 | 172748 | Intron 2 | AGGCTATACATTCCAG | eekddddddddddkke | 94 | 1826 |
| 541794 | 173045 | 173060 | Intron 2 | GGTGGCAGCTAGGTGG | eekddddddddddkke | 80 | 1827 |
| 541795 | 173677 | 173692 | Intron 2 | TCCACAGTTGGCACTG | eekddddddddddkke | 77 | 1828 |
| 541796 | 174128 | 174143 | Intron 2 | TGGGCCTTAGATTGTA | eekddddddddddkke | 69 | 1829 |
| 541797 | 174521 | 174536 | Intron 2 | TGTCTTCCTGGTGGCC | eekddddddddddkke | 97 | 1830 |
| 541798 | 174870 | 174885 | Intron 2 | CCCGCCTCTCCAGCAA | eekddddddddddkke | 89 | 1831 |
| 541799 | 175275 | 175290 | Intron 2 | GCAGCAGCCAATAAGT | eekddddddddddkke | 76 | 1832 |
| 541800 | 175691 | 175706 | Intron 2 | TTGTATCCTGGCCCCT | eekddddddddddkke | 80 | 1833 |
| 541801 | 176038 | 176053 | Intron 2 | GCCTCATGGGCCTTAC | eekddddddddddkke | 66 | 1834 |

**Table 59**

| Inhibition of GHR mRNA by deoxy, MOE and cEt gapmers targeting introns 2 and 3 of SEQ ID NO: 2 | | | | | | | |
|---|---|---|---|---|---|---|---|
| ISIS NO | SEQ ID NO: 2 Start Site | SEQ ID NO: 2 Stop Site | Target Region | Sequence | Chemistry | % inhibition | SEQ ID NO |
| 541262 | 156891 | 156906 | Intron 2 | TTGGTTTGTCAATCCT | eekddddddddddkke | 97 | 1370 |
| 541802 | 176619 | 176634 | Intron 2 | GGATGCCAGTCTTGGC | eekddddddddddkke | 48 | 1835 |
| 541803 | 176835 | 176850 | Intron 2 | CTGCTCTCAGTACCTC | eekddddddddddkke | 87 | 1836 |
| 541804 | 177300 | 177315 | Intron 2 | ACCCAAGAAGTCACCT | eekddddddddddkke | 93 | 1837 |
| 541805 | 177551 | 177566 | Intron 2 | GCCTCAAGCCCTACCC | eekddddddddddkke | 73 | 1838 |
| 541806 | 178066 | 178081 | Intron 2 | AGCTCCAGCCTATAGA | eekddddddddddkke | 81 | 1839 |
| 541807 | 178361 | 178376 | Intron 2 | GGTCCACATGGCCCTA | eekddddddddddkke | 90 | 1840 |
| 541808 | 178895 | 178910 | Intron 2 | CAGGCCCAGGATTGTC | eekddddddddddkke | 81 | 1841 |
| 541809 | 179444 | 179459 | Intron 2 | GGGCCTGCTTTGCAGC | eekddddddddddkke | 81 | 1842 |
| 541810 | 179863 | 179878 | Intron 2 | ACTCCTCTCTTTAGGC | eekddddddddddkke | 87 | 1843 |
| 541811 | 180524 | 180539 | Intron 2 | CTGGGTAACAGTCCTC | eekddddddddddkke | 98 | 1844 |
| 541812 | 181528 | 181543 | Intron 2 | ACTGTATGGTTTCCAC | eekddddddddddkke | 83 | 1845 |
| 541813 | 182103 | 182118 | Intron 2 | GCCAAAGATAGCTCTT | eekddddddddddkke | 94 | 1846 |
| 541814 | 182978 | 182993 | Intron 2 | GGCATTGGAAGTTGGT | eekddddddddddkke | 87 | 1847 |
| 541815 | 183193 | 183208 | Intron 2 | CCCTTCCTGACCTTAC | eekddddddddddkke | 55 | 1848 |
| 541816 | 183658 | 183673 | Intron 2 | TTACCCTCTATTCACC | eekddddddddddkke | 65 | 1849 |
| 541818 | 184501 | 184516 | Intron 2 | GGCACCCCAGGCCGGG | eekddddddddddkke | 25 | 1850 |
| 541819 | 185080 | 185095 | Intron 2 | CAGCAGCTAGTTCCCC | eekddddddddddkke | 96 | 1851 |
| 541820 | 185327 | 185342 | Intron 2 | GTGGGCACTAGTGTGT | eekddddddddddkke | 75 | 1852 |
| 541821 | 185682 | 185697 | Intron 2 | TGCCCTTGTCAGGGCA | eekddddddddddkke | 20 | 1853 |
| 541822 | 186025 | 186040 | Intron 2 | GCAGATAGGCTCAGCA | eekddddddddddkke | 98 | 1854 |
| 541823 | 186570 | 186585 | Intron 2 | CCCTAGCCCTTAGCAC | eekddddddddddkke | 44 | 1855 |
| 541824 | 186841 | 186856 | Intron 2 | ACTGGAATGGCCCTCT | eekddddddddddkke | 86 | 1856 |
| 541825 | 187176 | 187191 | Intron 2 | TTTGCTCATGCTCACA | eekddddddddddkke | 96 | 1857 |
| 541826 | 187629 | 187644 | Intron 2 | GCCTTTGTGTGTCACT | eekddddddddddkke | 99 | 1858 |
| 541827 | 187857 | 187872 | Intron 2 | TATGTGGTAGCATGTC | eekddddddddddkke | 96 | 1859 |
| 541828 | 188442 | 188457 | Intron 2 | CCCCAGGAAGTTGGCC | eekddddddddddkke | 68 | 1860 |
| 541829 | 189086 | 189101 | Intron 2 | TAGCTGTCAAGGCCCT | eekddddddddddkke | 90 | 1861 |
| 541830 | 189534 | 189549 | Intron 2 | CCTAGTCAGCCACTAG | eekddddddddddkke | 20 | 1862 |
| 541831 | 189889 | 189904 | Intron 2 | AGACTCCCCATCAGCC | eekddddddddddkke | 74 | 1863 |
| 541832 | 190172 | 190187 | Intron 2 | GTGAAGGGCCTTCATC | eekddddddddddkke | 68 | 1864 |
| 541833 | 190961 | 190976 | Intron 2 | GGTTGAGAGTCCAATG | eekddddddddddkke | 95 | 1865 |
| 541834 | 191404 | 191419 | Intron 2 | CAGCTAATTCCCTCAT | eekddddddddddkke | 79 | 1866 |
| 541835 | 191614 | 191629 | Intron 2 | TTGTGTCTCAACCCAC | eekddddddddddkke | 95 | 1867 |
| 541836 | 191999 | 192014 | Intron 2 | GGCTATGCTGCATGCT | eekddddddddddkke | 91 | 1868 |
| 541837 | 192860 | 192875 | Intron 2 | CCCCATACCCAGTGGA | eekddddddddddkke | 71 | 1869 |
| 541838 | 193460 | 193475 | Intron 2 | GGTGGTTTTCCTCCCT | eekddddddddddkke | 95 | 1870 |
| 541839 | 194144 | 194159 | Intron 2 | GAGCCTGCCCAACTTT | eekddddddddddkke | 90 | 1871 |
| 541840 | 194425 | 194440 | Intron 2 | TGATGCCCAAGAGTGA | eekddddddddddkke | 85 | 1872 |
| 541841 | 194953 | 194968 | Intron 2 | TTCCCTCTGCGAACAT | eekddddddddddkke | 96 | 1873 |
| 541842 | 195428 | 195443 | Intron 2 | GTTCCATCTCAATCCA | eekddddddddddkke | 94 | 1874 |
| 541843 | 196858 | 196873 | Intron 2 | ACGGCCACTCCACTGG | eekddddddddddkke | 44 | 1875 |
| 541844 | 197326 | 197341 | Intron 2 | TGGAAGTGGTTCCAGA | eekddddddddddkke | 90 | 1876 |
| 541845 | 197946 | 197961 | Intron 2 | TTGCCCCAGACCAACA | eekddddddddddkke | 47 | 1877 |
| 541846 | 198366 | 198381 | Intron 2 | GAGGTTGTGGAGGTGC | eekddddddddddkke | 26 | 1878 |
| 541847 | 198715 | 198730 | Intron 2 | GAGTTGCTGTGTGTGA | eekddddddddddkke | 83 | 1879 |
| 541848 | 198939 | 198954 | Intron 2 | CATGTCAGAGGTGTCC | eekddddddddddkke | 93 | 1880 |
| 541849 | 199506 | 199521 | Intron 2 | AGGTAAGGATCATGGC | eekddddddddddkke | 87 | 1881 |
| 541850 | 199816 | 199831 | Intron 2 | GTTCAGTTGCATCACG | eekddddddddddkke | 90 | 1882 |
| 541851 | 200249 | 200264 | Intron 2 | GCCCAGCTAGCCACCC | eekddddddddddkke | 68 | 1883 |
| 541852 | 201258 | 201273 | Intron 2 | CCTTAGCAGCCAGGCC | eekddddddddddkke | 86 | 1884 |
| 541853 | 202079 | 202094 | Intron 2 | GCACTTAGGGTTTTGC | eekddddddddddkke | 94 | 1885 |
| 541854 | 202382 | 202397 | Intron 2 | GTTGAACTTTCCCTAC | eekddddddddddkke | 53 | 1886 |
| 541855 | 202702 | 202717 | Intron 2 | TGACTCCTTGAGACAG | eekddddddddddkke | 83 | 1887 |
| 541856 | 203098 | 203113 | Intron 2 | TGCGCTGGCTTAGCAA | eekddddddddddkke | 59 | 1888 |
| 541857 | 203464 | 203479 | Intron 2 | GGCCTAACATCAGCAG | eekddddddddddkke | 88 | 1889 |
| 541858 | 204212 | 204227 | Intron 2 | ACTCCTCCCAGTTAGC | eekddddddddddkke | 70 | 1890 |
| 541859 | 205630 | 205645 | Intron 2 | ACCAGTGGCCAATGTC | eekddddddddddkke | 92 | 1891 |
| 541861 | 206422 | 206437 | Intron 2 | GCCTAGACACAGTAGG | eekddddddddddkke | 70 | 1892 |
| 541862 | 206749 | 206764 | Intron 2 | TATTCTCCCCCTAGGG | eekddddddddddkke | 42 | 1893 |
| 541863 | 207517 | 207532 | Intron 2 | GACGGCCTTGGGCACA | eekddddddddddkke | 96 | 1894 |
| | 210196 | 210211 | | | | | |
| 541865 | 208659 | 208674 | Intron 3 | GCAGGCTGTATTAGCA | eekddddddddddkke | 15 | 1895 |
| 541867 | 209999 | 210014 | Intron 3 | ACCCCCTATCCTGCAC | eekddddddddddkke | 58 | 1896 |
| 541868 | 210281 | 210296 | Intron 3 | TCCTCCATACCTAGAG | eekddddddddddkke | 61 | 1897 |
| | 211033 | 211048 | | | | | |
| 541869 | 210502 | 210517 | Intron 3 | GATAGGTGCCCACTGT | eekddddddddddkke | 80 | 1898 |
| 541870 | 210920 | 210935 | Intron 3 | GTCAGTTCTGGCTAGG | eekddddddddddkke | 97 | 1899 |
| 541871 | 211269 | 211284 | Intron 3 | GCCTGAACTTACAAGC | eekddddddddddkke | 68 | 1900 |
| 541872 | 211836 | 211851 | Intron 3 | ACCCTGGGCTGACCTT | eekddddddddddkke | 92 | 1901 |
| 541873 | 212606 | 212621 | Intron 3 | GGACCTGGACAAGCAA | eekddddddddddkke | 97 | 1902 |
| 541874 | 213099 | 213114 | Intron 3 | CTCCTTGCGAGAGAGG | eekddddddddddkke | 7 | 1903 |
| 541875 | 213425 | 213440 | Intron 3 | AGAGTTGACATGGGCA | eekddddddddddkke | 96 | 1904 |
| 541876 | 213846 | 213861 | Intron 3 | CACTAGGTCCCTGACC | eekddddddddddkke | 37 | 1905 |
| 541877 | 214483 | 214498 | Intron 3 | CACTCTCTTGGGCTGT | eekddddddddddkke | 94 | 1906 |
| 541878 | 214884 | 214899 | Intron 3 | AGGGACCTGCATTCCA | eekddddddddddkke | 72 | 1907 |

**Table 60**

| Inhibition of GHR mRNA by deoxy, MOE and cEt gapmers targeting introns 2 and 3 of SEQ ID NO: 2 | | | | | | | |
|---|---|---|---|---|---|---|---|
| ISIS NO | SEQ ID NO: 2 Start Site | SEQ ID NO: 2 Stop Site | Target Region | Sequence | Chemistry | % inhibition | SEQ ID NO |
| 541262 | 156891 | 156906 | Intron 2 | TTGGTTTGTCAATCCT | eekddddddddddkke | 91 | 1370 |
| 541879 | 215493 | 215508 | Intron 3 | TTCACCACCCATTGGG | eekddddddddddkke | 63 | 1908 |
| 541880 | 216192 | 216207 | Intron 3 | ATCTGGTCTGAGGGCC | eekddddddddddkke | 92 | 1909 |
| 541881 | 216458 | 216473 | Intron 3 | GACATGCAATTGACCC | eekddddddddddkke | 98 | 1910 |
| 541882 | 217580 | 217595 | Intron 3 | GTGTGCAGCAGACTGT | eekddddddddddkke | 92 | 1911 |
| 541883 | 218233 | 218248 | Intron 3 | GACAGTCCAGCTGCAA | eekddddddddddkke | 84 | 1912 |
| 541884 | 218526 | 218541 | Intron 3 | CCTGCGGCAGTGAAGA | eekddddddddddkke | 85 | 1913 |
| 541885 | 218734 | 218749 | Intron 3 | CTCTGAGGATAACCCT | eekddddddddddkke | 76 | 1914 |
| 541886 | 219342 | 219357 | Intron 3 | GTTCCCAGCTCCCCAA | eekddddddddddkke | 68 | 1915 |
| 541887 | 219618 | 219633 | Intron 3 | TAGGGTCAGTGTCCCA | eekddddddddddkke | 79 | 1916 |
| 541888 | 220039 | 220054 | Intron 3 | GGCGAGCCTCTCAGCC | eekddddddddddkke | 52 | 1917 |
| 541889 | 220393 | 220408 | Intron 3 | GACTCATCCAGGCAGT | eekddddddddddkke | 91 | 1918 |
| 541890 | 220665 | 220680 | Intron 3 | TCCCTCCCTTAGGCAC | eekddddddddddkke | 71 | 1919 |
| 541891 | 221044 | 221059 | Intron 3 | GAGGAGCCAGGCATAT | eekddddddddddkke | 80 | 1920 |
| 541892 | 221562 | 221577 | Intron 3 | CACCAACGAAGTCCCC | eekddddddddddkke | 89 | 1921 |
| 541893 | 221947 | 221962 | Intron 3 | GCTGGCAGTCACCAAA | eekddddddddddkke | 90 | 1922 |
| 541894 | 222569 | 222584 | Intron 3 | GCCCACACCATTGAGC | eekddddddddddkke | 70 | 1923 |
| 541895 | 222983 | 222998 | Intron 3 | AGTGAGATGCCCTGGT | eekddddddddddkke | 92 | 1924 |
| 541896 | 223436 | 223451 | Intron 3 | CACTGGCAGTTAGACC | eekddddddddddkke | 88 | 1925 |
| 541897 | 224107 | 224122 | Intron 3 | ACTCTGGCCACTAGTA | eekddddddddddkke | 80 | 1926 |
| 541898 | 224731 | 224746 | Intron 3 | GGTAGGGTGGCCACAT | eekddddddddddkke | 78 | 1927 |
| 541899 | 225133 | 225148 | Intron 3 | GAGCCATGTCTAGGCA | eekddddddddddkke | 18 | 1928 |
| 541900 | 225465 | 225480 | Intron 3 | CAGACTGAAACCCACC | eekddddddddddkke | 86 | 1929 |
| 541901 | 225671 | 225686 | Intron 3 | TATGGTCCAGCCACCA | eekddddddddddkke | 76 | 1930 |
| 541902 | 226110 | 226125 | Intron 3 | TACCTCCTCTGTTGGT | eekddddddddddkke | 36 | 1931 |
| 541903 | 227025 | 227040 | Intron 3 | ACACCTCAGTCATGAT | eekddddddddddkke | 92 | 1932 |
| 541904 | 227236 | 227251 | Intron 3 | AACAGGCTTCAAGAGG | eekddddddddddkke | 91 | 1933 |
| 541905 | 227485 | 227500 | Intron 3 | GTACTACTGGCCATGT | eekddddddddddkke | 73 | 1934 |
| 541906 | 227914 | 227929 | Intron 3 | CTGCAGGCGGTTGCTA | eekddddddddddkke | 60 | 1935 |
| 541907 | 228718 | 228733 | Intron 3 | GTCTGTTGCCAAGAGC | eekddddddddddkke | 95 | 1936 |
| 541908 | 229174 | 229189 | Intron 3 | CCCTGGGTCACTTAAG | eekddddddddddkke | 44 | 1937 |
| 541909 | 229423 | 229438 | Intron 3 | CCTGTCCTTGCTTGCA | eekddddddddddkke | 96 | 1938 |
| 541910 | 230042 | 230057 | Intron 3 | GCCCAGCTTATCCTAA | eekddddddddddkke | 78 | 1939 |
| 541911 | 230313 | 230328 | Intron 3 | AGTAGAGCCTTTGCCT | eekddddddddddkke | 75 | 1940 |
| 541912 | 230580 | 230595 | Intron 3 | CTGTCTCTTGGCCCAT | eekddddddddddkke | 80 | 1941 |
| 541913 | 231330 | 231345 | Intron 3 | GGCCCAAATCTTGAGT | eekddddddddddkke | 67 | 1942 |
| 541914 | 231817 | 231832 | Intron 3 | GCTTGTTACAGCACTA | eekddddddddddkke | 92 | 1943 |
| 541915 | 232088 | 232103 | Intron 3 | ACTTTGGCCCAGAGAT | eekddddddddddkke | 51 | 1944 |
| 541916 | 232884 | 232899 | Intron 3 | GCAGTCAGGTCAGCTG | eekddddddddddkke | 75 | 1945 |
| 541917 | 233210 | 233225 | Intron 3 | GCCTTGTCCTACTACC | eekddddddddddkke | 65 | 1946 |
| 541918 | 233657 | 233672 | Intron 3 | GGCTCTGCTATTGGCC | eekddddddddddkke | 59 | 1947 |
| 541919 | 233998 | 234013 | Intron 3 | CTTATAGAGCCTTGCC | eekddddddddddkke | 59 | 1948 |
| 541920 | 234296 | 234311 | Intron 3 | GGAAGGGCCCAAATAT | eekddddddddddkke | 15 | 1949 |
| 541921 | 234903 | 234918 | Intron 3 | GATCTACTCCTACTGC | eekddddddddddkke | 65 | 1950 |
| 541922 | 235313 | 235328 | Intron 3 | GTCAGCCTGTGTCTGA | eekddddddddddkke | 45 | 1951 |
| 541923 | 235770 | 235785 | Intron 3 | AGCTTCCTCCTTACAC | eekddddddddddkke | 54 | 1952 |
| 541924 | 236198 | 236213 | Intron 3 | CTGCTAAGCCCCTACC | eekddddddddddkke | 59 | 1953 |
| 541925 | 236684 | 236699 | Intron 3 | AGAGGTCAGGTGCATA | eekddddddddddkke | 77 | 1954 |
| 541926 | 237055 | 237070 | Intron 3 | TTCAGCCTGGTTGGGA | eekddddddddddkke | 71 | 1955 |
| 541927 | 237585 | 237600 | Intron 3 | GATTGATTGAGCTCCT | eekddddddddddkke | 86 | 1956 |
| 541928 | 237949 | 237964 | Intron 3 | ATGGACTCCCTAGGCT | eekddddddddddkke | 61 | 1957 |
| 541929 | 238542 | 238557 | Intron 3 | TACTCAAGGGCCCCTC | eekddddddddddkke | 67 | 1958 |
| 541930 | 245319 | 245334 | Intron 3 | GGCATATGTAGCTTGC | eekddddddddddkke | 91 | 1959 |
| 541931 | 245765 | 245780 | Intron 3 | GAGCTTAGATCTGTGC | eekddddddddddkke | 73 | 1960 |
| 541932 | 246251 | 246266 | Intron 3 | ATGCTCACGGCTGTGT | eekddddddddddkke | 81 | 1961 |
| 541933 | 246500 | 246515 | Intron 3 | ATTGAAAGGCCCATCA | eekddddddddddkke | 45 | 1962 |
| 541934 | 246936 | 246951 | Intron 3 | CAACCCAGTTTGCCGG | eekddddddddddkke | 71 | 1963 |
| 541935 | 247225 | 247240 | Intron 3 | CAGCTATTCCCTGTTT | eekddddddddddkke | 53 | 1964 |
| 541936 | 247644 | 247659 | Intron 3 | GCTGTGTCACACTTCC | eekddddddddddkke | 98 | 1965 |
| 541937 | 248223 | 248238 | Intron 3 | GTCCAAGGATCACAGC | eekddddddddddkke | 86 | 1966 |
| 541938 | 248695 | 248710 | Intron 3 | GCTACCACTAGAGCCT | eekddddddddddkke | 81 | 1967 |
| 541939 | 249494 | 249509 | Intron 3 | GTTTCAGGGCTTATGT | eekddddddddddkke | 63 | 1968 |
| 541940 | 250693 | 250708 | Intron 3 | TCCCACACCTATTGAA | eekddddddddddkke | 51 | 1969 |
| 541941 | 251622 | 251637 | Intron 3 | ACTGACTAGAGAGTCC | eekddddddddddkke | 81 | 1970 |
| 541942 | 251950 | 251965 | Intron 3 | TCCAAGGCTGATGTCC | eekddddddddddkke | 85 | 1971 |
| 541943 | 252665 | 252680 | Intron 3 | TCCCATGGTGGACATG | eekddddddddddkke | 39 | 1972 |
| 541944 | 253140 | 253155 | Intron 3 | AGTAGCTGGCAGAAGG | eekddddddddddkke | 85 | 1973 |
| 541945 | 253594 | 253609 | Intron 3 | CTGGGAGTGACTACTA | eekddddddddddkke | 77 | 1974 |
| 541946 | 254036 | 254051 | Intron 3 | TGGTATAGCTACTGGG | eekddddddddddkke | 84 | 1975 |
| 541947 | 254905 | 254920 | Intron 3 | CTGTGGTTTGGCAGGT | eekddddddddddkke | 90 | 1976 |
| 541948 | 255407 | 255422 | Intron 3 | GTTCTCACCTGAACTA | eekddddddddddkke | 65 | 1977 |
| 541949 | 255618 | 255633 | Intron 3 | ATAGGCTACTGGCAGG | eekddddddddddkke | 89 | 1978 |
| 541950 | 255992 | 256007 | Intron 3 | CCCAGCTAGCTGGAGT | eekddddddddddkke | 50 | 1979 |
| 541951 | 256428 | 256443 | Intron 3 | GGCTGGCTCTCAAAGG | eekddddddddddkke | 61 | 1980 |
| 541952 | 256689 | 256704 | Intron 3 | TGGTGATACTGTGGCA | eekddddddddddkke | 94 | 1981 |
| 541953 | 257317 | 257332 | Intron 3 | GCTGATTTTGGTGCCA | eekddddddddddkke | 92 | 1982 |
| 541954 | 257826 | 257841 | Intron 3 | GCTAATCTTGCCTCGA | eekddddddddddkke | 52 | 1983 |
| 541955 | 258407 | 258422 | Intron 3 | CACTGGTGGCTTTCAA | eekddddddddddkke | 31 | 1984 |

**Table 61**

| Inhibition of GHR mRNA by deoxy, MOE and cEt gapmers targeting intronic and exonic regions of SEQ ID NOs: 1 and 2 | | | | | | | |
|---|---|---|---|---|---|---|---|
| ISIS NO | SEQ ID NO: 1 Start Site | Target Region | Sequence | Chemistry | % inhibition | SEQ ID NO: 2 Start Site | SEQ ID NO |
| 541262 | n/a | Intron 2 | TTGGTTTGTCAATCCT | eekddddddddddkke | 93 | 156891 | 1370 |
| 541956 | n/a | Intron 3 | GTCCCCTTCTTAAGCA | eekddddddddddkke | 56 | 258980 | 1985 |
| 541957 | n/a | Intron 3 | GCCAGGCCAACTGTGG | eekddddddddddkke | 53 | 259290 | 1986 |
| 541958 | n/a | Intron 3 | GGCCCGTTATGGTGGA | eekddddddddddkke | 72 | 259500 | 1987 |
| 541959 | n/a | Intron 3 | CCTAAAGTCCAACTCC | eekddddddddddkke | 76 | 261641 | 1988 |
| 541960 | n/a | Intron 3 | CCCTATCCAGCCTTCA | eekddddddddddkke | 77 | 262021 | 1989 |
| 541961 | n/a | Intron 3 | AAGCATGGCCTCTGGC | eekddddddddddkke | 23 | 262453 | 1990 |
| 541962 | n/a | Intron 3 | TACCCTGCACCCTCCT | eekddddddddddkke | 71 | 262764 | 1991 |
| 541963 | n/a | Intron 3 | TCCTTAGTAGAATGCC | eekddddddddddkke | 82 | 263342 | 1992 |
| 541964 | n/a | Intron 3 | TTAGCCCTGGGAGCAC | eekddddddddddkke | 78 | 263913 | 1993 |
| 541965 | n/a | Intron 3 | GCTGGGTCAGGTAGCG | eekddddddddddkke | 71 | 266503 | 1994 |
| 541966 | n/a | Intron 3 | GGGAGGCTCTCAATCT | eekddddddddddkke | 75 | 266861 | 1995 |
| 541967 | n/a | Intron 3 | GTAAGTGCAGAATGCC | eekddddddddddkke | 87 | 267116 | 1996 |
| 541968 | n/a | Intron 3 | TGCCGAGGCAGGCACC | eekddddddddddkke | 33 | 267380 | 1997 |
| 541969 | n/a | Intron 3 | TCCGTGTCTAGGAGGT | eekddddddddddkke | 84 | 267865 | 1998 |
| 541970 | n/a | Intron 4 | GTCTCCCTGCATTGGA | eekddddddddddkke | 31 | 268366 | 1999 |
| 541971 | n/a | Intron 4 | CCATATCACTCTCCTC | eekddddddddddkke | 79 | 268786 | 2000 |
| 541972 | n/a | Intron 4 | CGAACACCTTGAGCCA | eekddddddddddkke | 90 | 269252 | 2001 |
| 541973 | n/a | Intron 4 | GGCCCAGCTTAAGAGG | eekddddddddddkke | 59 | 270038 | 2002 |
| 541974 | n/a | Intron 4 | CTGATACTCCTAATCC | eekddddddddddkke | 70 | 270501 | 2003 |
| 541975 | n/a | Intron 4 | GCCTGTAGGGCTGTGC | eekddddddddddkke | 82 | 270817 | 2004 |
| 541976 | n/a | Intron 4 | TGCCCTTTCTCCCTAC | eekddddddddddkke | 87 | 271216 | 2005 |
| 541977 | n/a | Intron 4 | AGTGCATGTCAGTACC | eekddddddddddkke | 75 | 271812 | 2006 |
| 541978 | n/a | Intron 4 | TGCTCCTCAGCTGTTG | eekddddddddddkke | 44 | 272631 | 2007 |
| 541979 | n/a | Intron 4 | GTTTGGGACCATCCCT | eekddddddddddkke | 41 | 272834 | 2008 |
| 541980 | n/a | Intron 4 | AGTGCTCTCTAGGGTC | eekddddddddddkke | 87 | 273257 | 2009 |
| 541981 | n/a | Intron 4 | TACAGAGAATCACCCC | eekddddddddddkke | 82 | 273651 | 2010 |
| 541982 | n/a | Intron 4 | GTCCAAGTAAGGTGCT | eekddddddddddkke | 57 | 273947 | 2011 |
| 541983 | n/a | Intron 5 | GACCTTGCAGGCTTCC | eekddddddddddkke | 87 | 274244 | 2012 |
| 541984 | n/a | Intron 5 | GGGCAAAGGATCCTCT | eekddddddddddkke | 71 | 274758 | 2013 |
| 541985 | n/a | Intron 5 | CCCATTCTGCTATCCC | eekddddddddddkke | 92 | 275198 | 2014 |
| 541986 | n/a | Intron 5 | GCTGACTAGGAGGGCT | eekddddddddddkke | 62 | 275732 | 2015 |
| 541987 | n/a | Intron 5 | CCTGTGAGGTAGTACC | eekddddddddddkke | 83 | 276309 | 2016 |
| 541988 | n/a | Intron 5 | GTCCCCCTCCAGTCTA | eekddddddddddkke | 50 | 276932 | 2017 |
| 541989 | n/a | Intron 5 | GAGGACTCAATTCCTC | eekddddddddddkke | 0 | 277149 | 2018 |
| 541990 | n/a | Intron 5 | GACAAGGTCCTTTTGG | eekddddddddddkke | 43 | 277391 | 2019 |
| 541991 | n/a | Intron 5 | GCTCTTGTGTGCACCC | eekddddddddddkke | 90 | 277730 | 2020 |
| 541992 | n/a | Intron 5 | TCACCGCCTGCACCAC | eekddddddddddkke | 75 | 278342 | 2021 |
| 541993 | n/a | Intron 5 | GGTTGCACTGTGCAAT | eekddddddddddkke | 26 | 278917 | 2022 |
| 541994 | n/a | Intron 6 | TTCCACAGGCCTCCAT | eekddddddddddkke | 64 | 279303 | 2023 |
| 541995 | n/a | Intron 6 | GCTGAGTTCCATATGC | eekddddddddddkke | 72 | 279679 | 2024 |
| 541996 | n/a | Intron 6 | GAACCGCCACCTCAGG | eekddddddddddkke | 38 | 280157 | 2025 |
| 541997 | n/a | Intron 6 | GCTCACGGTTGGAGAC | eekddddddddddkke | 42 | 280799 | 2026 |
| 541998 | n/a | Intron 6 | TGGGCTCCCATGTTCA | eekddddddddddkke | 45 | 281595 | 2027 |
| 541999 | n/a | Intron 6 | TCACTCTACCAACCTC | eekddddddddddkke | 33 | 282572 | 2028 |
| 542000 | n/a | Intron 6 | TCCTTGCTTACAGATG | eekddddddddddkke | 33 | 283079 | 2029 |
| 542001 | n/a | Intron 6 | TGATGCTAGCATTACC | eekddddddddddkke | 37 | 283653 | 2030 |
| 542002 | n/a | Intron 6 | TGGGTAACTGGCTAGT | eekddddddddddkke | 47 | 285711 | 2031 |
| 542003 | n/a | Intron 6 | AACCATTCCTCACCAA | eekddddddddddkke | 53 | 287181 | 2032 |
| 542004 | n/a | Intron 6 | GCCCTGAACAGTTGAT | eekddddddddddkke | 37 | 287895 | 2033 |
| 542005 | n/a | Intron 6 | GGCTCCTATCATACCT | eekddddddddddkke | 38 | 288943 | 2034 |
| 542006 | n/a | Intron 6 | TAGGTCTCACAACCCT | eekddddddddddkke | 10 | 289638 | 2035 |
| 542007 | n/a | Intron 6 | GTGCATTAGTCTTCCA | eekddddddddddkke | 74 | 290035 | 2036 |
| 542008 | n/a | Intron 7 | CAAAAGCCAGGTTAGC | eekddddddddddkke | 13 | 290503 | 2037 |
| 542009 | n/a | Intron 7 | CTGCTGTTGACTACCT | eekddddddddddkke | 50 | 290924 | 2038 |
| 542010 | n/a | Intron 7 | GTACCTGCCAGCTACT | eekddddddddddkke | 35 | 291807 | 2039 |
| 542011 | n/a | Exon 8-intron 8 junction | CCTACCTTTGCTGTTT | eekddddddddddkke | 12 | 292611 | 2040 |
| 542012 | n/a | Intron 8 | AGTCACCAGCCTAAGC | eekddddddddddkke | 47 | 292860 | 2041 |
| 542013 | n/a | Intron 8 | AGGCAACCTGGGAGTG | eekddddddddddkke | 52 | 293377 | 2042 |
| 542014 | n/a | Intron 8 | TGGCCTTCACAATGGC | eekddddddddddkke | 33 | 294052 | 2043 |
| 542015 | n/a | Intron 8 | GGTGAAGTGGGTTGGA | eekddddddddddkke | 27 | 294536 | 2044 |
| 542016 | n/a | Intron 8 | GCTGGTTGTCTGCTGC | eekddddddddddkke | 60 | 294931 | 2045 |
| 542017 | n/a | Intron 8 | AGTTTGTGACCCCTGC | eekddddddddddkke | 81 | 295475 | 2046 |
| 542018 | n/a | Intron 8 | CCACTCAGTGTGAATG | eekddddddddddkke | 85 | 295955 | 2047 |
| 542019 | n/a | Intron 8 | CTGGCCTCAGGGCAAT | eekddddddddddkke | 51 | 296186 | 2048 |
| 542020 | n/a | Intron 8 | GTAGACTTGGGTAGGT | eekddddddddddkke | 53 | 296680 | 2049 |
| 542022 | n/a | 3'UTR | TGGTGCTAAGCTCTCC | eekddddddddddkke | 67 | 301009 | 2050 |
| 542023 | n/a | 3'UTR | CATGCTCAAGCTGGAA | eekddddddddddkke | 47 | 301280 | 2051 |
| 542024 | 206 | Exon 2 | AAGGTCAACAGCAGCT | eekddddddddddkke | 93 | 144990 | 2052 |
| 542025 | 207 | Exon 2 | CAAGGTCAACAGCAGC | eekddddddddddkke | 85 | 144991 | 2053 |
| 542026 | 208 | Exon 2 | CCAAGGTCAACAGCAG | eekddddddddddkke | 82 | 144992 | 2054 |
| 542027 | 209 | Exon 2 | GCCAAGGTCAACAGCA | eekddddddddddkke | 84 | 144993 | 2055 |

**Table 62**

| Inhibition of GHR mRNA by deoxy, MOE and cEt gapmers targeting intronic and exonic regions of SEQ ID NOs: 1 and 2 | | | | | | | |
|---|---|---|---|---|---|---|---|
| ISIS NO | SEQ ID NO: 1 Start Site | Target Region | Sequence | Chemistry | % inhibition | SEQ NO: 2 Start Site | SEQ ID NO |
| 541262 | n/a | Intron 2 | TTGGTTTGTCAATCCT | eekddddddddddkke | 86 | 156891 | 1370 |
| 542034 | 870 | Exon 7 | TCTCACACGCACTTCA | eekddddddddddkke | 49 | 290368 | 2056 |
| 542035 | 871 | Exon 7 | ATCTCACACGCACTTC | eekddddddddddkke | 39 | 290369 | 2057 |
| 542036 | 872 | Exon 7 | GATCTCACACGCACTT | eekddddddddddkke | 50 | 290370 | 2058 |
| 542049 | n/a | Intron 1 | CTTTCATGAATCAAGC | eekddddddddddkke | 85 | 17928 | 2059 |
| 542050 | n/a | Intron 1 | TCTTTCATGAATCAAG | eekddddddddddkke | 54 | 17929 | 2060 |
| 542051 | n/a | Intron 1 | GTCTTTCATGAATCAA | eekddddddddddkke | 96 | 17930 | 2061 |
| 542052 | n/a | Intron 1 | GGTCTTTCATGAATCA | eekddddddddddkke | 98 | 17931 | 2062 |
| 542053 | n/a | Intron 1 | ATGGTCTTTCATGAAT | eekddddddddddkke | 94 | 17933 | 2063 |
| 542054 | n/a | Intron 1 | GATGGTCTTTCATGAA | eekddddddddddkke | 73 | 17934 | 2064 |
| 542055 | n/a | Intron 1 | TGATGGTCTTTCATGA | eekddddddddddkke | 83 | 17935 | 2065 |
| 542056 | n/a | Intron 1 | TATATCAATATTCTCC | eekddddddddddkke | 75 | 21821 | 2066 |
| 542057 | n/a | Intron 1 | TTATATCAATATTCTC | eekddddddddddkke | 23 | 21822 | 2067 |
| 542058 | n/a | Intron 1 | GTTATATCAATATTCT | eekddddddddddkke | 87 | 21823 | 2068 |
| 542059 | n/a | Intron 1 | TTTCTTTAGCAATAGT | eekddddddddddkke | 85 | 22519 | 2069 |
| 542060 | n/a | Intron 1 | CTTTCTTTAGCAATAG | eekddddddddddkke | 81 | 22520 | 2070 |
| 542061 | n/a | Intron 1 | GCTTTCTTTAGCAATA | eekddddddddddkke | 68 | 22521 | 2071 |
| 542062 | n/a | Intron 1 | CTCCATTAGGGTTCTG | eekddddddddddkke | 91 | 50948 | 2072 |
| 542063 | n/a | Intron 1 | TCTCCATTAGGGTTCT | eekddddddddddkke | 88 | 50949 | 2073 |
| 542064 | n/a | Intron 1 | TTCTCCATTAGGGTTC | eekddddddddddkke | 85 | 50950 | 2074 |
| 542065 | n/a | Intron 1 | GTTCTCCATTAGGGTT | eekddddddddddkke | 84 | 50951 | 2075 |
| 542066 | n/a | Intron 1 | AGGTTGGCAGACAGAC | eekddddddddddkke | 92 | 53467 | 2076 |
| 542067 | n/a | Intron 1 | CAGGTTGGCAGACAGA | eekddddddddddkke | 93 | 53468 | 2077 |
| 542068 | n/a | Intron 1 | GCAGGTTGGCAGACAG | eekddddddddddkke | 91 | 53469 | 2078 |
| 542069 | n/a | Intron 1 | CTTCTTGTGAGCTGGC | eekddddddddddkke | 95 | 64885 | 2079 |
| 542070 | n/a | Intron 1 | TCTTCTTGTGAGCTGG | eekddddddddddkke | 89 | 64886 | 2080 |
| 542071 | n/a | Intron 1 | GTCTTCTTGTGAGCTG | eekddddddddddkke | 96 | 64887 | 2081 |
| 542072 | n/a | Intron 1 | AGTCTTCTTGTGAGCT | eekddddddddddkke | 81 | 64888 | 2082 |
| 542073 | n/a | Intron 1 | TCTTCCACTCACATCC | eekddddddddddkke | 89 | 65991 | 2083 |
| 542074 | n/a | Intron 1 | CTCTTCCACTCACATC | eekddddddddddkke | 79 | 65992 | 2084 |
| 542075 | n/a | Intron 1 | TCTCTTCCACTCACAT | eekddddddddddkke | 86 | 65993 | 2085 |
| 542076 | n/a | Intron 1 | GTCTCTTCCACTCACA | eekddddddddddkke | 92 | 65994 | 2086 |
| 542077 | n/a | Intron 1 | ATAGATTTTGACTTCC | eekddddddddddkke | 86 | 72108 | 2087 |
| 542078 | n/a | Intron 1 | CATAGATTTTGACTTC | eekddddddddddkke | 42 | 72109 | 2088 |
| 542079 | n/a | Intron 1 | GCATAGATTTTGACTT | eekddddddddddkke | 66 | 72110 | 2089 |
| 542080 | n/a | Intron 1 | AAATGTCAACAGTGCA | eekddddddddddkke | 97 | 80639 | 2090 |
| 542081 | n/a | Intron 1 | CATGACTATGTTCTGG | eekddddddddddkke | 68 | 125595 | 2091 |
| 542082 | n/a | Intron 1 | ACATGACTATGTTCTG | eekddddddddddkke | 66 | 125596 | 2092 |
| 542083 | n/a | Intron 1 | CACATGACTATGTTCT | eekddddddddddkke | 74 | 125597 | 2093 |
| 542084 | n/a | Intron 2 | GAATTCTGAGCTCTGG | eekddddddddddkke | 91 | 145430 | 2094 |
| 542085 | n/a | Intron 2 | TGAATTCTGAGCTCTG | eekddddddddddkke | 94 | 145431 | 2095 |
| 542086 | n/a | Intron 2 | CTGAATTCTGAGCTCT | eekddddddddddkke | 94 | 145432 | 2096 |
| 542087 | n/a | Intron 2 | CCTGAATTCTGAGCTC | eekddddddddddkke | 93 | 145433 | 2097 |
| 542088 | n/a | Intron 2 | GCCTGAATTCTGAGCT | eekddddddddddkke | 87 | 145434 | 2098 |
| 542089 | n/a | Intron 2 | AGCCTGAATTCTGAGC | eekddddddddddkke | 84 | 145435 | 2099 |
| 542090 | n/a | Intron 2 | ATATTGTAATTCTTGG | eekddddddddddkke | 47 | 148060 | 2100 |
| 542091 | n/a | Intron 2 | GATATTGTAATTCTTG | eekddddddddddkke | 61 | 148061 | 2101 |
| 542092 | n/a | Intron 2 | TGATATTGTAATTCTT | eekddddddddddkke | 0 | 148062 | 2102 |
| 542093 | n/a | Intron 2 | CTGATATTGTAATTCT | eekddddddddddkke | 58 | 148063 | 2103 |
| 542094 | n/a | Intron 2 | CCTGATATTGTAATTC | eekddddddddddkke | 95 | 148064 | 2104 |
| 542095 | n/a | Intron 2 | GCCTGATATTGTAATT | eekddddddddddkke | 85 | 148065 | 2105 |
| 542096 | n/a | Intron 2 | TGCCTGATATTGTAAT | eekddddddddddkke | 86 | 148066 | 2106 |
| 542097 | n/a | Intron 2 | ATTATGTGCTTTGCCT | eekddddddddddkke | 86 | 148907 | 2107 |
| 542098 | n/a | Intron 2 | AATTATGTGCTTTGCC | eekddddddddddkke | 75 | 148908 | 2108 |
| 542099 | n/a | Intron 2 | CAATTATGTGCTTTGC | eekddddddddddkke | 88 | 148909 | 2109 |
| 542100 | n/a | Intron 2 | TCAATTATGTGCTTTG | eekddddddddddkke | 78 | 148910 | 2110 |
| 542101 | n/a | Intron 2 | GTCAATTATGTGCTTT | eekddddddddddkke | 97 | 148911 | 2111 |
| 542102 | n/a | Intron 2 | GCCATCACCAAACACC | eekddddddddddkke | 97 | 150973 | 2112 |
| 542103 | n/a | Intron 2 | TGCCATCACCAAACAC | eekddddddddddkke | 90 | 150974 | 2113 |
| 542104 | n/a | Intron 2 | TTGCCATCACCAAACA | eekddddddddddkke | 89 | 150975 | 2114 |
| 542105 | n/a | Intron 2 | TGGTGACTCTGCCTGA | eekddddddddddkke | 98 | 151388 | 2115 |
| 542106 | n/a | Intron 2 | CTGGTGACTCTGCCTG | eekddddddddddkke | 96 | 151389 | 2116 |
| 542107 | n/a | Intron 2 | GCTGGTGACTCTGCCT | eekddddddddddkke | 98 | 151390 | 2117 |
| 542108 | n/a | Intron 2 | TGCTGGTGACTCTGCC | eekddddddddddkke | 97 | 151391 | 2118 |
| 542109 | n/a | Intron 2 | CTGCTGGTGACTCTGC | eekddddddddddkke | 93 | 151392 | 2119 |

**Table 63**

| Inhibition of GHR mRNA by deoxy, MOE and cEt gapmers targeting introns 2 and 3 of SEQ ID NO: 2 | | | | | | | |
|---|---|---|---|---|---|---|---|
| ISIS NO | SEQ ID NO: 2 Start Site | SEQ ID NO: 2 Stop Site | Target Region | Sequence | Chemistry | % inhibition | SEQ ID NO |
| 541262 | 156891 | 156906 | Intron 2 | TTGGTTTGTCAATCCT | eekddddddddddkke | 95 | 1370 |
| 542110 | 153002 | 153017 | Intron 2 | AGTAGTCAATATTATT | eekddddddddddkke | 74 | 2120 |
| 542111 | 153003 | 153018 | Intron 2 | CAGTAGTCAATATTAT | eekddddddddddkke | 55 | 2121 |
| 542112 | 153004 | 153019 | Intron 2 | CCAGTAGTCAATATTA | eekddddddddddkke | 97 | 2122 |
| 542113 | 153922 | 153937 | Intron 2 | CCTTTGGGTGAATAGC | eekddddddddddkke | 90 | 2123 |
| 542114 | 153923 | 153938 | Intron 2 | ACCTTTGGGTGAATAG | eekddddddddddkke | 71 | 2124 |
| 542115 | 153924 | 153939 | Intron 2 | CACCTTTGGGTGAATA | eekddddddddddkke | 78 | 2125 |
| 542116 | 155595 | 155610 | Intron 2 | CAACTTGAGGACAATA | eekddddddddddkke | 89 | 2126 |
| 542118 | 155597 | 155612 | Intron 2 | CTCAACTTGAGGACAA | eekddddddddddkke | 98 | 2127 |
| 542119 | 156395 | 156410 | Intron 2 | CAGGAAGAAAGGAACC | eekddddddddddkke | 95 | 2128 |
| 542120 | 156396 | 156411 | Intron 2 | CCAGGAAGAAAGGAAC | eekddddddddddkke | 83 | 2129 |
| 542121 | 156397 | 156412 | Intron 2 | ACCAGGAAGAAAGGAA | eekddddddddddkke | 90 | 2130 |
| 542122 | 156595 | 156610 | Intron 2 | TGCAGTCATGTACACA | eekddddddddddkke | 97 | 2131 |
| 542123 | 156596 | 156611 | Intron 2 | CTGCAGTCATGTACAC | eekddddddddddkke | 90 | 2132 |
| 542124 | 156597 | 156612 | Intron 2 | TCTGCAGTCATGTACA | eekddddddddddkke | 81 | 2133 |
| 542125 | 156890 | 156905 | Intron 2 | TGGTTTGTCAATCCTT | eekddddddddddkke | 97 | 2134 |
| 542126 | 156892 | 156907 | Intron 2 | CTTGGTTTGTCAATCC | eekddddddddddkke | 99 | 2135 |
| 542127 | 157204 | 157219 | Intron 2 | GCTACAATGCACAGGA | eekddddddddddkke | 98 | 2136 |
| 542128 | 157205 | 157220 | Intron 2 | TGCTACAATGCACAGG | eekddddddddddkke | 98 | 2137 |
| 542129 | 158008 | 158023 | Intron 2 | GATATTTATTGCTGTA | eekddddddddddkke | 61 | 2138 |
| 542130 | 158009 | 158024 | Intron 2 | TGATATTTATTGCTGT | eekddddddddddkke | 41 | 2139 |
| 542131 | 158010 | 158025 | Intron 2 | CTGATATTTATTGCTG | eekddddddddddkke | 86 | 2140 |
| 542132 | 162752 | 162767 | Intron 2 | AGGGTCTTTACAAAGT | eekddddddddddkke | 69 | 2141 |
| 542133 | 162753 | 162768 | Intron 2 | CAGGGTCTTTACAAAG | eekddddddddddkke | 71 | 2142 |
| 542134 | 162754 | 162769 | Intron 2 | CCAGGGTCTTTACAAA | eekddddddddddkke | 93 | 2143 |
| 542135 | 166353 | 166368 | Intron 2 | TTCTGCAGTATCCTAG | eekddddddddddkke | 84 | 2144 |
| 542136 | 166354 | 166369 | Intron 2 | TTTCTGCAGTATCCTA | eekddddddddddkke | 88 | 2145 |
| 542137 | 166355 | 166370 | Intron 2 | GTTTCTGCAGTATCCT | eekddddddddddkke | 95 | 2146 |
| 542138 | 166356 | 166371 | Intron 2 | AGTTTCTGCAGTATCC | eekddddddddddkke | 92 | 2147 |
| 542139 | 166357 | 166372 | Intron 2 | CAGTTTCTGCAGTATC | eekddddddddddkke | 93 | 2148 |
| 542140 | 172747 | 172762 | Intron 2 | CAAATTCCAGTCCTAG | eekddddddddddkke | 73 | 2149 |
| 542141 | 172748 | 172763 | Intron 2 | CCAAATTCCAGTCCTA | eekddddddddddkke | 91 | 2150 |
| 542142 | 172749 | 172764 | Intron 2 | TCCAAATTCCAGTCCT | eekddddddddddkke | 90 | 2151 |
| 542143 | 175372 | 175387 | Intron 2 | ACCCATTTCATCCATT | eekddddddddddkke | 94 | 2152 |
| 542144 | 175373 | 175388 | Intron 2 | AACCCATTTCATCCAT | eekddddddddddkke | 93 | 2153 |
| 542145 | 175374 | 175389 | Intron 2 | GAACCCATTTCATCCA | eekddddddddddkke | 97 | 2154 |
| 542146 | 175375 | 175390 | Intron 2 | GGAACCCATTTCATCC | eekddddddddddkke | 96 | 2155 |
| 542147 | 175376 | 175391 | Intron 2 | AGGAACCCATTTCATC | eekddddddddddkke | 68 | 2156 |
| 542148 | 189120 | 189135 | Intron 2 | GCTTCATGTCTTTCTA | eekddddddddddkke | 90 | 2157 |
| 542149 | 189121 | 189136 | Intron 2 | TGCTTCATGTCTTTCT | eekddddddddddkke | 96 | 2158 |
| 542150 | 189122 | 189137 | Intron 2 | GTGCTTCATGTCTTTC | eekddddddddddkke | 97 | 2159 |
| 542151 | 189485 | 189500 | Intron 2 | TGAGCTTAGCAGTCAC | eekddddddddddkke | 92 | 2160 |
| 542152 | 189486 | 189501 | Intron 2 | ATGAGCTTAGCAGTCA | eekddddddddddkke | 95 | 2161 |
| 542153 | 189487 | 189502 | Intron 2 | CATGAGCTTAGCAGTC | eekddddddddddkke | 95 | 2162 |
| 542154 | 191143 | 191158 | Intron 2 | TACAGACATAGCTCTA | eekddddddddddkke | 91 | 2163 |
| 542155 | 191144 | 191159 | Intron 2 | ATACAGACATAGCTCT | eekddddddddddkke | 74 | 2164 |
| 542156 | 191145 | 191160 | Intron 2 | GATACAGACATAGCTC | eekddddddddddkke | 91 | 2165 |
| 542157 | 191146 | 191161 | Intron 2 | GGATACAGACATAGCT | eekddddddddddkke | 94 | 2166 |
| 542158 | 198149 | 198164 | Intron 2 | TGTGGCTTTAATTCAC | eekddddddddddkke | 71 | 2167 |
| 542159 | 198150 | 198165 | Intron 2 | ATGTGGCTTTAATTCA | eekddddddddddkke | 81 | 2168 |
| 542160 | 198151 | 198166 | Intron 2 | TATGTGGCTTTAATTC | eekddddddddddkke | 78 | 2169 |
| 542161 | 199817 | 199832 | Intron 2 | TGTTCAGTTGCATCAC | eekddddddddddkke | 91 | 2170 |
| 542162 | 199818 | 199833 | Intron 2 | GTGTTCAGTTGCATCA | eekddddddddddkke | 89 | 2171 |
| 542163 | 199819 | 199834 | Intron 2 | TGTGTTCAGTTGCATC | eekddddddddddkke | 90 | 2172 |
| 542164 | 210562 | 210577 | Intron 3 | CATCTGGATGTGAGGC | eekddddddddddkke | 90 | 2173 |
| 542165 | 210563 | 210578 | Intron 3 | ACATCTGGATGTGAGG | eekddddddddddkke | 78 | 2174 |
| 542166 | 210564 | 210579 | Intron 3 | CACATCTGGATGTGAG | eekddddddddddkke | 55 | 2175 |
| 542167 | 219020 | 219035 | Intron 3 | TCAGGTAATTTCTGGA | eekddddddddddkke | 82 | 2176 |
| 542168 | 219021 | 219036 | Intron 3 | CTCAGGTAATTTCTGG | eekddddddddddkke | 73 | 2177 |
| 542169 | 219022 | 219037 | Intron 3 | TCTCAGGTAATTTCTG | eekddddddddddkke | 40 | 2178 |
| 542170 | 225568 | 225583 | Intron 3 | TGCTTATTTACCTGGG | eekddddddddddkke | 90 | 2179 |
| 542171 | 225569 | 225584 | Intron 3 | TTGCTTATTTACCTGG | eekddddddddddkke | 90 | 2180 |
| 542172 | 225570 | 225585 | Intron 3 | TTTGCTTATTTACCTG | eekddddddddddkke | 79 | 2181 |
| 542173 | 225571 | 225586 | Intron 3 | TTTTGCTTATTTACCT | eekddddddddddkke | 32 | 2182 |
| 542174 | 229619 | 229634 | Intron 3 | ATGATGTTACTACTAC | eekddddddddddkke | 63 | 2183 |
| 542175 | 229620 | 229635 | Intron 3 | AATGATGTTACTACTA | eekddddddddddkke | 53 | 2184 |
| 542176 | 229621 | 229636 | Intron 3 | CAATGATGTTACTACT | eekddddddddddkke | 12 | 2185 |
| 542177 | 232827 | 232842 | Intron 3 | CCCCTAGAGCAATGGT | eekddddddddddkke | 76 | 2186 |
| 542178 | 232828 | 232843 | Intron 3 | CCCCCTAGAGCAATGG | eekddddddddddkke | 83 | 2187 |
| 542179 | 232829 | 232844 | Intron 3 | TCCCCCTAGAGCAATG | eekddddddddddkke | 49 | 2188 |
| 542180 | 237676 | 237691 | Intron 3 | TCAATTGCAGATGCTC | eekddddddddddkke | 88 | 2189 |
| 542181 | 237677 | 237692 | Intron 3 | CTCAATTGCAGATGCT | eekddddddddddkke | 90 | 2190 |
| 542182 | 237678 | 237693 | Intron 3 | GCTCAATTGCAGATGC | eekddddddddddkke | 81 | 2191 |
| 542183 | 237679 | 237694 | Intron 3 | AGCTCAATTGCAGATG | eekddddddddddkke | 85 | 2192 |
| 542184 | 248232 | 248247 | Intron 3 | GTATATTCAGTCCAAG | eekddddddddddkke | 90 | 2193 |
| 542185 | 248233 | 248248 | Intron 3 | AGTATATTCAGTCCAA | eekddddddddddkke | 94 | 2194 |
| 542186 | 248234 | 248249 | Intron 3 | CAGTATATTCAGTCCA | eekddddddddddkke | 97 | 2195 |

**Table 64**

| Inhibition of GHR mRNA by deoxy, MOE and cEt gapmers targeting intronic and exonic regions of SEQ ID NOs: 1 and 2 | | | | | | | |
|---|---|---|---|---|---|---|---|
| ISIS NO | SEQ ID NO: 1 Start Site | Target Region | Sequence | Chemistry | % inhibition | SEQ ID NO: 2 Start Site | SEQ ID NO |
| 541262 | n/a | Intron 2 | TTGGTTTGTCAATCCT | eekddddddddddkke | 93 | 156891 | 1370 |
| 545316 | 168 | exon 1 - intron 1 junction | ACCTCCGAGCTTCGCC | eekddddddddddkke | 80 | 3044 | 2196 |
| 545317 | 173 | exon-exon junction | GTAGGACCTCCGAGCT | eekddddddddddkke | 74 | n/a | 2197 |
| 545318 | 177 | exon-exon junction | ACCTGTAGGACCTCCG | eekddddddddddkke | 70 | n/a | 2198 |
| 545321 | 213 | Exon 2 | CAGTGCCAAGGTCAAC | eekddddddddddkke | 77 | 144997 | 2199 |
| 545322 | 225 | Exon 2 | ACTTGATCCTGCCAGT | eekddddddddddkke | 36 | 145009 | 2200 |
| 545332 | 361 | Exon 4/ Intron 3 | CTCGCTCAGGTGAACG | eekddddddddddkke | 57 | 268024 | 2201 |
| 545333 | 366 | Exon 4/ Intron 3 | AGTCTCTCGCTCAGGT | eekddddddddddkke | 88 | 268029 | 2202 |
| 545337 | 444 | Exon 4-intron 4 junction | CCTTCTGGTATAGAAC | eekddddddddddkke | 21 | 268107 | 2203 |
| 545340 | 570 | Exon 5 | GCTAGTTAGCTTGATA | eekddddddddddkke | 39 | 274130 | 2204 |
| 545343 | 626 | exon 3- exon 4 junction | TCTGGTTGCACTATTT | eekddddddddddkke | 34 | n/a | 2205 |
| 545344 | 629 | exon 3- exon 4 junction | GGATCTGGTTGCACTA | eekddddddddddkke | 30 | n/a | 2206 |
| 545345 | 632 | Exon 6 | GGTGGATCTGGTTGCA | eekddddddddddkke | 18 | 278926 | 2207 |
| 545346 | 638 | Exon 6 | GCAATGGGTGGATCTG | eekddddddddddkke | 50 | 278932 | 2208 |
| 545347 | 647 | Exon 6 | CAGTTGAGGGCAATGG | eekddddddddddkke | 71 | 278941 | 2209 |
| 545348 | 651 | Exon 6 | AGTCCAGTTGAGGGCA | eekddddddddddkke | 58 | 278945 | 2210 |
| 545349 | 655 | Exon 6 | GTAAAGTCCAGTTGAG | eekddddddddddkke | 34 | 278949 | 2211 |
| 545350 | 660 | Exon 6 | GTTCAGTAAAGTCCAG | eekddddddddddkke | 52 | 278954 | 2212 |
| 545351 | 685 | Exon 6 | CTGCATGAATCCCAGT | eekddddddddddkke | 77 | 278979 | 2213 |
| 545355 | 923 | Exon 7 | ACATAGAGCACCTCAC | eekddddddddddkke | 38 | 290421 | 2214 |
| 545356 | 926 | Exon 7 | GTTACATAGAGCACCT | eekddddddddddkke | 79 | 290424 | 2215 |
| 545357 | 929 | Exon 7 | AGTGTTACATAGAGCA | eekddddddddddkke | 70 | 290427 | 2216 |
| 545362 | 1124 | Exon 7- exon 8 junction | TCCTTGAGGAGATCTG | eekddddddddddkke | 3 | n/a | 2217 |
| 545363 | 1170 | Exon 10 | GCTATCATGAATGGCT | eekddddddddddkke | 69 | 297587 | 2218 |
| 545364 | 1180 | Exon 10 | CGGGTTTATAGCTATC | eekddddddddddkke | 58 | 297597 | 2219 |
| 545369 | 1320 | Exon 10 | ATCCTTCACCCCTAGG | eekddddddddddkke | 46 | 297737 | 2220 |
| 545370 | 1328 | Exon 10 | GAGTCGCCATCCTTCA | eekddddddddddkke | 60 | 297745 | 2221 |
| 545371 | 1332 | Exon 10 | TCCAGAGTCGCCATCC | eekddddddddddkke | 51 | 297749 | 2222 |
| 545373 | 1418 | Exon 10 | GGCTGAGCAACCTCTG | eekddddddddddkke | 80 | 297835 | 2223 |
| 545374 | 1422 | Exon 10 | CTGTGGCTGAGCAACC | eekddddddddddkke | 63 | 297839 | 2224 |
| 545380 | 1524 | Exon 10 | GATAACACTGGGCTGC | eekddddddddddkke | 60 | 297941 | 2225 |
| 545381 | 1530 | Exon 10 | TGCTTGGATAACACTG | eekddddddddddkke | 76 | 297947 | 2226 |
| 545382 | 1533 | Exon 10 | CTCTGCTTGGATAACA | eekddddddddddkke | 60 | 297950 | 2227 |
| 545386 | 1600 | Exon 10 | GCTGAATATGGGCAGC | eekddddddddddkke | 29 | 298017 | 2228 |
| 545387 | 1613 | Exon 10 | CTTGGATTGCTTAGCT | eekddddddddddkke | 59 | 298030 | 2229 |
| 545388 | 1645 | Exon 10 | CCTGGGCATAAAAGTC | eekddddddddddkke | 47 | 298062 | 2230 |
| 545392 | 1832 | Exon 10 | ACCTTGATGTGAGGAG | eekddddddddddkke | 44 | 298249 | 2231 |

**Table 65**

| Inhibition of GHR mRNA by deoxy, MOE and cEt gapmers targeting intronic and exonic regions of SEQ ID NOs: 1 and 2 | | | | | | | |
|---|---|---|---|---|---|---|---|
| ISIS NO | SEQ ID NO: 1 Start Site | Target Region | Sequence | Chemistry | % inhibition | SEQ ID NO: 2 Start Site | SEQ ID NO |
| 541262 | n/a | Intron 2 | TTGGTTTGTCAATCCT | eekddddddddddkke | 89 | 156891 | 1370 |
| 545393 | 1838 | Exon 10 | GATTCAACCTTGATGT | eekddddddddddkke | 40 | 298255 | 2232 |
| 545394 | 1844 | Exon 10 | ATGTGTGATTCAACCT | eekddddddddddkke | 80 | 298261 | 2233 |
| 545395 | 1956 | Exon 10 | TGGGACAGGCATCTCA | eekddddddddddkke | 29 | 298373 | 2234 |
| 545396 | 1961 | Exon 10 | TAGTCTGGGACAGGCA | eekddddddddddkke | 48 | 298378 | 2235 |
| 545397 | 1968 | Exon 10 | GGAGGTATAGTCTGGG | eekddddddddddkke | 61 | 298385 | 2236 |
| 545398 | 1986 | Exon 10 | GGACTGTACTATATGA | eekddddddddddkke | 48 | 298403 | 2237 |
| 545401 | 2077 | Exon 10 | TCAGTTGGTCTGTGCT | eekddddddddddkke | 60 | 298494 | 2238 |
| 545402 | 2095 | Exon 10 | GCTAAGGCATGATTTT | eekddddddddddkke | 53 | 298512 | 2239 |
| 545406 | 2665 | Exon 10 | GCCATGCTTGAAGTCT | eekddddddddddkke | 87 | 299082 | 2240 |
| 545407 | 2668 | Exon 10 | ATAGCCATGCTTGAAG | eekddddddddddkke | 70 | 299085 | 2241 |
| 545408 | 2692 | Exon 10 | ACACAGTGTGTAGTGT | eekddddddddddkke | 60 | 299109 | 2242 |
| 545409 | 2699 | Exon 10 | CTGCAGTACACAGTGT | eekddddddddddkke | 31 | 299116 | 2243 |
| 545410 | 2704 | Exon 10 | ACCAACTGCAGTACAC | eekddddddddddkke | 57 | 299121 | 2244 |
| 545411 | 2739 | Exon 10 | TAGACTGTAGTTGCTA | eekddddddddddkke | 53 | 299156 | 2245 |
| 545412 | 2747 | Exon 10 | ACCAGCTTTAGACTGT | eekddddddddddkke | 56 | 299164 | 2246 |
| 545413 | 2945 | Exon 10 | GTAAGTTGATCTGTGC | eekddddddddddkke | 79 | 299362 | 2247 |
| 545414 | 2963 | Exon 10 | TACTTCTTTTGGTGCC | eekddddddddddkke | 82 | 299380 | 2248 |
| 545416 | 3212 | Exon 10 | TCTTGTACCTTATTCC | eekddddddddddkke | 73 | 299629 | 2249 |
| 545417 | 3306 | Exon 10 | TGGTTATAGGCTGTGA | eekddddddddddkke | 90 | 299723 | 2250 |
| 545418 | 3309 | Exon 10 | GTCTGGTTATAGGCTG | eekddddddddddkke | 88 | 299726 | 2251 |
| 545419 | 3313 | Exon 10 | ATGTGTCTGGTTATAG | eekddddddddddkke | 68 | 299730 | 2252 |
| 545420 | 3317 | Exon 10 | GAGTATGTGTCTGGTT | eekddddddddddkke | 84 | 299734 | 2253 |
| 545421 | 4049 | Exon 10 | GGTCTGCGATAAATGG | eekddddddddddkke | 69 | 300466 | 2254 |
| 545429 | 4424 | Exon 10 | GCCAGACACAACTAGT | eekddddddddddkke | 59 | 300841 | 2255 |
| 545430 | 31 | Exon 1 | ACCGCCACTGTAGCAG | eekddddddddddkke | 76 | 2907 | 2256 |
| 545431 | 36 | Exon 1 | CCGCCACCGCCACTGT | eekddddddddddkke | 94 | 2912 | 2257 |
| 545432 | 103 | Exon 1 | GGGCCTCCGGCCCGCG | eekddddddddddkke | 22 | 2979 | 2258 |
| 545433 | 143 | Exon 1 | AGAGCGCGGGTTCGCG | eekddddddddddkke | 61 | 3019 | 2259 |
| 545434 | n/a | Intron 1/Exon 1 | TACTGACCCCAGTTCC | eekddddddddddkke | 68 | 3654 | 2260 |
| 545435 | n/a | Intron 1/Exon 1 | ACTCTACTGACCCCAG | eekddddddddddkke | 70 | 3658 | 2261 |
| 545436 | n/a | Intron 1/Exon 1 | GTCACTCTACTGACCC | eekddddddddddkke | 83 | 3661 | 2262 |
| 545437 | n/a | Intron 1/Exon 1 | TTCATGCGGACTGGTG | eekddddddddddkke | 68 | 3680 | 2263 |
| 545438 | n/a | Intron 3/Exon 3 | GTGAGCATGGACCCCA | eekddddddddddkke | 94 | 225436 | 2264 |
| 545439 | n/a | Intron 3/Exon 3 | TGATATGTGAGCATGG | eekddddddddddkke | 88 | 225442 | 2265 |
| 545440 | n/a | Intron 3/Exon 3 | AAGTTGGTGAGCTTCT | eekddddddddddkke | 85 | 226785 | 2266 |
| 545441 | n/a | Intron 3/Exon 3 | CCTTCAAGTTGGTGAG | eekddddddddddkke | 88 | 226790 | 2267 |
| 545442 | n/a | Intron 3/Exon 3 | GTAAGATCCTTTTGCC | eekddddddddddkke | 70 | 226883 | 2268 |
| 545443 | n/a | Intron 3/Exon 3 | CAGCTGTGCAACTTGC | eekddddddddddkke | 50 | 238345 | 2269 |
| 545444 | n/a | Intron 3/Exon 3 | GCCTTGGTAGGTAGGG | eekddddddddddkke | 68 | 238422 | 2270 |
| 545445 | n/a | Intron 3/Exon 3 | AGAGCCTTGGTAGGTA | eekddddddddddkke | 85 | 238425 | 2271 |
| 545446 | n/a | Intron 1/Exon 1 | CCCGCACAAACGCGCA | eekddddddddddkke | 10 | 3614 | 2272 |
| 545447 | n/a | Intron 1/Exon 1 | GTCTTCAAGGTCAGTT | eekddddddddddkke | 92 | 93208 | 2273 |
| 545448 | n/a | Intron 1/Exon 1 | GCCCAGTGAATTCAGC | eekddddddddddkke | 76 | 93246 | 2274 |
| 545449 | n/a | Intron 1/Exon 1 | AGATGCGCCCAGTGAA | eekddddddddddkke | 60 | 93252 | 2275 |
| 545450 | n/a | Intron 1/Exon 1 | GTAAGATGCGCCCAGT | eekddddddddddkke | 78 | 93255 | 2276 |
| 545451 | n/a | Intron 1/Exon 1 | CCAGAAGGCACTTGTA | eekddddddddddkke | 42 | 93301 | 2277 |
| 545452 | n/a | Intron 1/Exon 1 | GGAAGATTTGCAGAAC | eekddddddddddkke | 15 | 93340 | 2278 |
| 545453 | n/a | Intron 1/Exon 1 | CCTTGGTCATGGAAGA | eekddddddddddkke | 35 | 93350 | 2279 |
| 545454 | n/a | Intron 1/Exon 1 | TGACCTTGGTCATGGA | eekddddddddddkke | 55 | 93353 | 2280 |
| 545455 | n/a | Intron 1/Exon 1 | GAGGTGACCTTGGTCA | eekddddddddddkke | 70 | 93357 | 2281 |
| 545456 | n/a | Intron 1/Exon 1 | ATCCAAAGAGGTGACC | eekddddddddddkke | 41 | 93364 | 2282 |
| 545457 | n/a | Intron 1/Exon 1 | GCCAATCCAAAGAGGT | eekddddddddddkke | 56 | 93368 | 2283 |
| 545458 | n/a | Intron 1/Exon 1 | GGTCTGCCAATCCAAA | eekddddddddddkke | 79 | 93373 | 2284 |
| 545459 | n/a | Intron 1/Exon 1 | CCCTGGGTCTGCCAAT | eekddddddddddkke | 68 | 93378 | 2285 |
| 545460 | n/a | Intron 1/Exon 1 | GAGATCTCAACAAGGG | eekddddddddddkke | 52 | 93427 | 2286 |
| 545461 | n/a | Intron 1/Exon 1 | CGCCCATCACTCTTCC | eekddddddddddkke | 68 | 93988 | 2287 |
| 545462 | n/a | Intron 1/Exon 1 | CACCTGTCGCCCATCA | eekddddddddddkke | 67 | 93995 | 2288 |
| 545463 | n/a | Intron 1/Exon 1 | CATCACCTGTCGCCCA | eekddddddddddkke | 78 | 93998 | 2289 |
| 545464 | n/a | Intron 1/Exon 1 | CACCATCACCTGTCGC | eekddddddddddkke | 74 | 94001 | 2290 |
| 545465 | n/a | Intron 1/Exon 1 | AATAGTTGTCACCATC | eekddddddddddkke | 76 | 94010 | 2291 |
| 545466 | n/a | Intron 1/Exon 1 | GCCACCTTTCATGAGA | eekddddddddddkke | 58 | 94048 | 2292 |
| 545467 | n/a | Intron 2/Exon 2 | CTCTTGGAAGTAGGTA | eekddddddddddkke | 89 | 198762 | 2293 |
| 545468 | n/a | Intron 2/Exon 2 | GTTCTCTTGGAAGTAG | eekddddddddddkke | 80 | 198765 | 2294 |
| 545469 | n/a | Intron 2/Exon 2 | TAAACAGGTTGGTCTG | eekddddddddddkke | 68 | 198854 | 2295 |

### Example 8: Dose-dependent antisense inhibition of human GHR in Hep3B cells by deoxy, MOE and cEt gapmers

Gapmers from studies described above exhibiting significant *in vitro* inhibition of GHR mRNA were selected and tested at various doses in Hep3B cells. The antisense oligonucleotides were tested in a series of experiments that had similar culture conditions. The results for each experiment are presented in separate tables shown below. Cells were plated at a density of 20,000 cells per well and transfected using electroporation with 0.625 µM, 1.25 µM, 2.50 µM, 5.00 µM and 10.00 µM concentrations of antisense oligonucleotide. After a treatment period of approximately 16 hours, RNA was isolated from the cells and GHR mRNA levels were measured by quantitative real-time PCR. Human primer probe set RTS3437_MGB was used to measure mRNA levels. GHR mRNA levels were adjusted according to total RNA content, as measured by RIBOGREEN®. Results are presented as percent inhibition of GHR, relative to untreated control cells.

The half maximal inhibitory concentration (IC₅₀) of each oligonucleotide is also presented. GHR mRNA levels were significantly reduced in a dose-dependent manner in antisense oligonucleotide treated cells.

**Table 66**

| ISIS No | 0.625 µM | 1.250 µM | 2.50 µM | 5.00 µM | 10.00 µM | IC₅₀ (µM) |
|---|---|---|---|---|---|---|
| 541396 | 30 | 51 | 68 | 74 | 67 | 1.4 |
| 541262 | 55 | 87 | 90 | 94 | 97 | 0.2 |
| 541393 | 30 | 38 | 52 | 66 | 81 | 2.1 |
| 541375 | 41 | 45 | 54 | 64 | 79 | 1.6 |
| 541438 | 44 | 49 | 75 | 80 | 91 | 0.9 |
| 541428 | 35 | 32 | 56 | 78 | 88 | 1.8 |
| 541491 | 13 | 46 | 67 | 55 | 95 | 2.0 |
| 541435 | 21 | 46 | 55 | 72 | 94 | 1.9 |
| 541471 | 11 | 49 | 50 | 77 | 89 | 2.0 |
| 541430 | 24 | 44 | 56 | 57 | 79 | 2.2 |
| 541492 | 32 | 40 | 65 | 80 | 85 | 1.5 |
| 541431 | 22 | 46 | 73 | 84 | 92 | 1.5 |

**Table 67**

| ISIS No | 0.625 µM | 1.250 µM | 2.50 µM | 5.00 µM | 10.00 µM | IC₅₀ (µM) |
|---|---|---|---|---|---|---|
| 541487 | 36 | 46 | 66 | 85 | 92 | 1.3 |
| 541423 | 33 | 55 | 64 | 80 | 93 | 1.2 |
| 541452 | 37 | 60 | 79 | 87 | 94 | 0.9 |
| 541505 | 51 | 75 | 86 | 92 | 97 | 0.4 |
| 541522 | 54 | 76 | 81 | 90 | 95 | 0.3 |
| 541539 | 65 | 76 | 85 | 94 | 98 | 0.2 |
| 541503 | 54 | 65 | 80 | 93 | 97 | 0.5 |
| 541520 | 43 | 61 | 86 | 94 | 96 | 0.7 |
| 541515 | 57 | 72 | 85 | 92 | 94 | 0.3 |
| 541564 | 57 | 72 | 88 | 90 | 97 | 0.3 |
| 541554 | 43 | 65 | 81 | 89 | 93 | 0.7 |
| 541509 | 11 | 8 | 19 | 6 | 8 | >10 |
| 541584 | 59 | 65 | 84 | 91 | 96 | 0.3 |
| 541585 | 70 | 80 | 93 | 92 | 98 | 0.1 |

**Table 68**

| ISIS No | 0.625 µM | 1.250 µM | 2.50 µM | 5.00 µM | 10.00 µM | IC₅₀ (µM) |
|---|---|---|---|---|---|---|
| 541598 | 26 | 43 | 75 | 80 | 76 | 1.5 |
| 541592 | 35 | 48 | 67 | 85 | 95 | 1.2 |
| 541641 | 22 | 63 | 70 | 91 | 93 | 1.2 |
| 541590 | 27 | 59 | 70 | 94 | 95 | 1.2 |
| 541615 | 40 | 65 | 84 | 88 | 94 | 0.7 |
| 541595 | 35 | 57 | 73 | 84 | 95 | 1.0 |
| 541575 | 49 | 60 | 79 | 84 | 95 | 0.6 |
| 541571 | 41 | 50 | 76 | 80 | 94 | 1.0 |
| 541582 | 0 | 10 | 25 | 50 | 82 | 4.4 |
| 541262 | 66 | 79 | 93 | 94 | 99 | <0.6 |
| 541652 | 1 | 44 | 80 | 82 | 87 | 1.9 |
| 541670 | 29 | 40 | 63 | 79 | 89 | 1.6 |
| 541662 | 17 | 13 | 45 | 62 | 84 | 3.1 |
| 541724 | 37 | 47 | 72 | 85 | 95 | 1.2 |

**Table 69**

| ISIS No | 0.625 µM | 1.250 µM | 2.50 µM | 5.00 µM | 10.00 µM | IC₅₀ (µM) |
|---|---|---|---|---|---|---|
| 541748 | 86 | 94 | 96 | 98 | 98 | <0.6 |
| 541767 | 83 | 91 | 95 | 96 | 98 | <0.6 |
| 541797 | 78 | 89 | 93 | 97 | 99 | <0.6 |
| 541766 | 59 | 82 | 92 | 97 | 99 | <0.6 |
| 541742 | 65 | 87 | 93 | 95 | 99 | <0.6 |
| 541750 | 80 | 86 | 96 | 96 | 99 | <0.6 |
| 541262 | 79 | 88 | 93 | 97 | 97 | <0.6 |
| 541749 | 71 | 84 | 93 | 95 | 98 | <0.6 |
| 541793 | 71 | 88 | 94 | 97 | 98 | <0.6 |
| 541785 | 56 | 79 | 89 | 93 | 98 | <0.6 |
| 541746 | 34 | 61 | 85 | 94 | 97 | 0.9 |
| 541752 | 49 | 72 | 88 | 93 | 93 | <0.6 |
| 541826 | 86 | 94 | 95 | 99 | 98 | <0.6 |
| 541811 | 66 | 87 | 93 | 97 | 98 | <0.6 |

**Table 70**

| ISIS No | 0.625 µM | 1.250 µM | 2.50 µM | 5.00 µM | 10.00 µM | IC₅₀ (µM) |
|---|---|---|---|---|---|---|
| 541822 | 83 | 88 | 95 | 96 | 96 | <0.6 |
| 541870 | 77 | 87 | 95 | 97 | 98 | <0.6 |
| 541262 | 85 | 93 | 96 | 97 | 98 | <0.6 |
| 541873 | 32 | 77 | 93 | 94 | 97 | 0.7 |
| 541819 | 60 | 91 | 97 | 97 | 99 | <0.6 |
| 541841 | 86 | 91 | 95 | 96 | 97 | <0.6 |
| 541825 | 78 | 88 | 95 | 98 | 98 | <0.6 |
| 541863 | 63 | 77 | 87 | 93 | 97 | <0.6 |
| 541827 | 42 | 80 | 87 | 94 | 97 | <0.6 |
| 541875 | 77 | 84 | 93 | 96 | 97 | <0.6 |
| 541835 | 56 | 73 | 90 | 95 | 98 | <0.6 |
| 541838 | 72 | 90 | 93 | 98 | 97 | <0.6 |
| 541833 | 52 | 69 | 83 | 92 | 97 | <0.6 |
| 541813 | 47 | 75 | 86 | 95 | 97 | <0.6 |

**Table 71**

| ISIS No | 0.625 µM | 1.250 µM | 2.50 µM | 5.00 µM | 10.00 µM | IC₅₀ (µM) |
|---|---|---|---|---|---|---|
| 541853 | 74 | 79 | 88 | 93 | 91 | <0.6 |
| 541842 | 69 | 85 | 91 | 97 | 99 | <0.6 |
| 541877 | 79 | 91 | 93 | 98 | 97 | <0.6 |
| 541848 | 58 | 90 | 96 | 98 | 98 | 0.7 |
| 541804 | 23 | 81 | 89 | 95 | 95 | 0.8 |
| 541881 | 87 | 94 | 98 | 98 | 99 | <0.6 |
| 541936 | 91 | 96 | 98 | 99 | 98 | <0.6 |
| 541909 | 56 | 80 | 89 | 95 | 97 | <0.6 |
| 541907 | 75 | 91 | 95 | 97 | 98 | <0.6 |
| 541952 | 68 | 81 | 93 | 97 | 98 | <0.6 |
| 541953 | 68 | 80 | 94 | 97 | 98 | <0.6 |
| 541914 | 60 | 78 | 94 | 97 | 97 | <0.6 |
| 541880 | 56 | 74 | 89 | 94 | 95 | <0.6 |
| 541903 | 37 | 74 | 87 | 96 | 98 | 0.6 |

**Table 72**

| ISIS No | 0.625 µM | 1.250 µM | 2.50 µM | 5.00 µM | 10.00 µM | IC₅₀ (µM) |
|---|---|---|---|---|---|---|
| 541895 | 47 | 72 | 85 | 93 | 94 | <0.6 |
| 541882 | 60 | 67 | 89 | 93 | 97 | <0.6 |
| 541889 | 63 | 80 | 87 | 94 | 97 | <0.6 |
| 541904 | 26 | 78 | 23 | 89 | 93 | 1.4 |
| 545418 | 0 | 81 | 91 | 94 | 95 | 1.7 |
| 541930 | 58 | 71 | 82 | 88 | 92 | <0.6 |
| 545439 | 67 | 87 | 93 | 96 | 98 | <0.6 |
| 542024 | 15 | 58 | 78 | 87 | 90 | 1.4 |
| 541985 | 59 | 81 | 88 | 93 | 97 | <0.6 |
| 541972 | 47 | 58 | 83 | 90 | 92 | 0.6 |
| 541991 | 57 | 64 | 88 | 92 | 83 | <0.6 |
| 541980 | 33 | 50 | 76 | 72 | 93 | 1.2 |

**Table 73**

| ISIS No | 0.625 µM | 1.250 µM | 2.50 µM | 5.00 µM | 10.00 µM | IC₅₀ (µM) |
|---|---|---|---|---|---|---|
| 541264 | 26 | 44 | 64 | 79 | 89 | 1.6 |
| 541265 | 29 | 32 | 62 | 79 | 91 | 1.8 |
| 541263 | 25 | 40 | 62 | 78 | 93 | 1.7 |
| 541268 | 57 | 73 | 85 | 90 | 95 | 0.3 |
| 541266 | 15 | 33 | 46 | 66 | 90 | 2.5 |
| 542107 | 93 | 97 | 98 | 98 | 98 | <0.6 |
| 542052 | 93 | 96 | 97 | 96 | 98 | <0.6 |
| 542105 | 80 | 92 | 96 | 98 | 97 | <0.6 |
| 542102 | 94 | 96 | 96 | 97 | 98 | <0.6 |
| 542108 | 90 | 92 | 94 | 97 | 99 | <0.6 |
| 542080 | 87 | 93 | 95 | 95 | 97 | <0.6 |

**Table 74**

| ISIS No | 0.625 µM | 1.250 µM | 2.50 µM | 5.00 µM | 10.00 µM | IC₅₀ (µM) |
|---|---|---|---|---|---|---|
| 542101 | 90 | 97 | 97 | 97 | 95 | <0.6 |
| 542051 | 89 | 96 | 95 | 98 | 97 | <0.6 |
| 542106 | 83 | 93 | 96 | 96 | 98 | <0.6 |
| 542071 | 84 | 91 | 94 | 97 | 97 | <0.6 |
| 542094 | 85 | 92 | 94 | 97 | 98 | <0.6 |
| 542069 | 89 | 94 | 97 | 95 | 98 | <0.6 |
| 542086 | 83 | 94 | 96 | 97 | 98 | <0.6 |
| 542085 | 85 | 92 | 96 | 97 | 97 | <0.6 |
| 542053 | 64 | 83 | 94 | 98 | 97 | <0.6 |
| 542087 | 69 | 84 | 99 | 95 | 98 | <0.6 |
| 542109 | 87 | 94 | 96 | 98 | 98 | <0.6 |
| 542126 | 96 | 98 | 99 | 98 | 98 | <0.6 |
| 542127 | 94 | 96 | 97 | 98 | 97 | <0.6 |
| 542128 | 90 | 96 | 98 | 98 | 97 | <0.6 |

**Table 75**

| ISIS No | 0.625 µM | 1.250 µM | 2.50 µM | 5.00 µM | 10.00 µM | IC₅₀ (µM) |
|---|---|---|---|---|---|---|
| 542118 | 97 | 97 | 98 | 95 | 43 | <0.6 |
| 542186 | 93 | 96 | 98 | 99 | 98 | <0.6 |
| 542150 | 95 | 97 | 98 | 99 | 99 | <0.6 |
| 542122 | 90 | 94 | 98 | 98 | 99 | <0.6 |
| 542125 | 88 | 97 | 98 | 98 | 99 | <0.6 |
| 542145 | 90 | 96 | 98 | 99 | 99 | <0.6 |
| 542112 | 86 | 94 | 99 | 99 | 99 | <0.6 |
| 542149 | 88 | 93 | 99 | 98 | 99 | <0.6 |
| 542146 | 79 | 93 | 96 | 97 | 98 | <0.6 |
| 542153 | 87 | 94 | 97 | 98 | 99 | <0.6 |
| 542119 | 64 | 84 | 93 | 97 | 98 | <0.6 |
| 542137 | 76 | 91 | 97 | 97 | 98 | <0.6 |
| 542152 | 84 | 94 | 96 | 96 | 97 | <0.6 |
| 542157 | 83 | 95 | 98 | 99 | 98 | <0.6 |

**Table 76**

| ISIS No | 0.625 µM | 1.250 µM | 2.50 µM | 5.00 µM | 10.00 µM | IC₅₀ (µM) |
|---|---|---|---|---|---|---|
| 542185 | 82 | 93 | 96 | 96 | 94 | <0.6 |
| 542143 | 81 | 91 | 96 | 98 | 98 | <0.6 |
| 542144 | 77 | 93 | 95 | 96 | 99 | <0.6 |
| 542139 | 87 | 93 | 98 | 98 | 98 | <0.6 |
| 542134 | 83 | 90 | 90 | 95 | 96 | <0.6 |
| 545333 | 68 | 85 | 91 | 96 | 98 | <0.6 |
| 545373 | 57 | 73 | 86 | 92 | 97 | <0.6 |
| 545438 | 84 | 96 | 98 | 97 | 99 | <0.6 |
| 545431 | 77 | 91 | 93 | 97 | 98 | <0.6 |
| 545447 | 70 | 85 | 96 | 96 | 97 | <0.6 |
| 545417 | 62 | 82 | 90 | 93 | 95 | <0.6 |
| 545467 | 77 | 88 | 91 | 94 | 95 | <0.6 |
| 545441 | 63 | 82 | 92 | 94 | 96 | <0.6 |

### Example 9: Dose-dependent antisense inhibition of human GHR in Hep3B cells by deoxy, MOE and cEt gapmers

Gapmers from studies described above exhibiting significant *in vitro* inhibition of GHR mRNA were selected and tested at various doses in Hep3B cells. The antisense oligonucleotides were tested in a series of experiments that had similar culture conditions. The results for each experiment are presented in separate tables shown below. Cells were plated at a density of 20,000 cells per well and transfected using electroporation with 0.04 µM, 0.11 µM, 0.33 µM, 1.00 µM, and 3.00 µM concentrations of antisense oligonucleotide. After a treatment period of approximately 16 hours, RNA was isolated from the cells and GHR mRNA levels were measured by quantitative real-time PCR. Human primer probe set RTS3437_MGB was used to measure mRNA levels. GHR mRNA levels were adjusted according to total RNA content, as measured by RIBOGREEN®. Results are presented as percent inhibition of GHR, relative to untreated control cells.

The half maximal inhibitory concentration (IC₅₀) of each oligonucleotide is also presented. GHR mRNA levels were significantly reduced in a dose-dependent manner in antisense oligonucleotide treated cells.

**Table 77**

| ISIS No | 0.04 µM | 0.11 µM | 0.33 µM | 1.00 µM | 3.00 µM | IC₅₀ (µM) |
|---|---|---|---|---|---|---|
| 539380 | 11 | 16 | 57 | 93 | 98 | 0.2 |
| 541724 | 0 | 27 | 71 | 66 | 83 | 0.3 |
| 541748 | 28 | 40 | 71 | 90 | 97 | 0.1 |
| 541767 | 19 | 38 | 54 | 87 | 98 | 0.2 |
| 541797 | 23 | 46 | 70 | 88 | 97 | 0.1 |
| 541766 | 15 | 26 | 49 | 82 | 96 | 0.3 |
| 541742 | 17 | 28 | 41 | 80 | 95 | 0.3 |
| 541750 | 33 | 27 | 60 | 89 | 98 | 0.2 |
| 541749 | 27 | 16 | 62 | 84 | 82 | 0.2 |
| 541793 | 0 | 14 | 44 | 77 | 96 | 0.4 |
| 541785 | 4 | 11 | 39 | 75 | 95 | 0.4 |
| 541752 | 14 | 6 | 45 | 70 | 94 | 0.4 |
| 541826 | 8 | 34 | 74 | 94 | 99 | 0.2 |
| 541811 | 6 | 4 | 45 | 79 | 97 | 0.4 |
| 541822 | 9 | 29 | 67 | 89 | 97 | 0.2 |

**Table 78**

| ISIS No | 0.04 µM | 0.11 µM | 0.33 µM | 1.00 µM | 3.00 µM | IC₅₀ (µM) |
|---|---|---|---|---|---|---|
| 539380 | 0 | 16 | 47 | 82 | 98 | 0.4 |
| 541819 | 3 | 12 | 50 | 76 | 94 | 0.3 |
| 541841 | 0 | 19 | 47 | 80 | 95 | 0.3 |
| 541825 | 0 | 6 | 40 | 74 | 96 | 0.4 |
| 541827 | 5 | 26 | 48 | 76 | 95 | 0.3 |
| 541835 | 7 | 11 | 33 | 74 | 93 | 0.4 |
| 541838 | 21 | 26 | 61 | 90 | 97 | 0.2 |
| 541833 | 0 | 9 | 41 | 63 | 89 | 0.5 |
| 541813 | 0 | 17 | 28 | 65 | 92 | 0.5 |
| 541842 | 5 | 15 | 30 | 72 | 90 | 0.4 |
| 541804 | 0 | 12 | 3 | 49 | 79 | 1.1 |
| 542024 | 0 | 0 | 26 | 54 | 76 | 1.0 |
| 542107 | 15 | 45 | 78 | 92 | 99 | 0.1 |
| 542105 | 2 | 14 | 55 | 88 | 98 | 0.3 |
| 542102 | 10 | 16 | 73 | 88 | 98 | 0.2 |

**Table 79**

| ISIS No | 0.04 µM | 0.11 µM | 0.33 µM | 1.00 µM | 3.00 µM | IC₅₀ (µM) |
|---|---|---|---|---|---|---|
| 539380 | 4 | 18 | 50 | 86 | 95 | 0.3 |
| 542108 | 15 | 13 | 65 | 86 | 97 | 0.2 |
| 542101 | 17 | 40 | 68 | 92 | 98 | 0.2 |
| 542106 | 4 | 23 | 56 | 88 | 98 | 0.3 |
| 542094 | 0 | 30 | 51 | 86 | 96 | 0.3 |
| 542086 | 13 | 38 | 50 | 84 | 97 | 0.2 |
| 542085 | 0 | 27 | 57 | 90 | 98 | 0.3 |
| 542087 | 7 | 3 | 49 | 80 | 92 | 0.4 |
| 542109 | 17 | 10 | 56 | 88 | 98 | 0.3 |
| 542126 | 40 | 63 | 91 | 96 | 99 | <0.03 |
| 542127 | 27 | 47 | 69 | 93 | 97 | 0.1 |
| 542128 | 11 | 30 | 66 | 90 | 98 | 0.2 |
| 542118 | 14 | 42 | 77 | 95 | 98 | 0.1 |
| 542150 | 31 | 46 | 72 | 94 | 98 | 0.1 |
| 542122 | 13 | 14 | 59 | 90 | 97 | 0.3 |

**Table 80**

| ISIS No | 0.04 µM | 0.11 µM | 0.33 µM | 1.00 µM | 3.00 µM | IC₅₀ (µM) |
|---|---|---|---|---|---|---|
| 539380 | 0 | 2 | 50 | 86 | 97 | 0.4 |
| 542125 | 31 | 32 | 69 | 89 | 96 | 0.1 |
| 542145 | 15 | 29 | 64 | 91 | 97 | 0.2 |
| 542112 | 14 | 38 | 61 | 87 | 96 | 0.2 |
| 542149 | 9 | 37 | 63 | 90 | 97 | 0.2 |
| 542146 | 13 | 33 | 59 | 82 | 95 | 0.2 |
| 542153 | 22 | 26 | 63 | 86 | 96 | 0.2 |
| 542119 | 10 | 20 | 34 | 70 | 87 | 0.4 |
| 542137 | 3 | 19 | 47 | 77 | 95 | 0.3 |
| 542152 | 0 | 9 | 47 | 82 | 96 | 0.4 |
| 542157 | 0 | 26 | 56 | 84 | 96 | 0.3 |
| 542143 | 8 | 12 | 44 | 81 | 95 | 0.3 |
| 542144 | 0 | 21 | 42 | 75 | 95 | 0.4 |
| 542139 | 0 | 14 | 46 | 82 | 97 | 0.4 |
| 542134 | 3 | 23 | 43 | 72 | 92 | 0.4 |

**Table 81**

| ISIS No | 0.04 µM | 0.11 µM | 0.33 µM | 1.00 µM | 3.00 µM | IC₅₀ (µM) |
|---|---|---|---|---|---|---|
| 539380 | 0 | 9 | 64 | 85 | 97 | 0.3 |
| 541870 | 7 | 15 | 48 | 80 | 92 | 0.3 |
| 541262 | 0 | 29 | 63 | 90 | 98 | 0.2 |
| 541863 | 0 | 26 | 40 | 82 | 93 | 0.4 |
| 541875 | 6 | 30 | 71 | 84 | 91 | 0.2 |
| 541853 | 0 | 13 | 39 | 67 | 91 | 0.5 |
| 541877 | 0 | 26 | 41 | 79 | 94 | 0.4 |
| 541881 | 0 | 30 | 54 | 87 | 94 | 0.3 |
| 541936 | 20 | 41 | 73 | 93 | 98 | 0.1 |
| 541909 | 0 | 16 | 34 | 64 | 90 | 0.5 |
| 541907 | 6 | 31 | 59 | 84 | 96 | 0.2 |
| 541952 | 0 | 0 | 50 | 72 | 92 | 0.5 |
| 541953 | 0 | 22 | 50 | 80 | 92 | 0.4 |
| 541914 | 0 | 0 | 46 | 76 | 93 | 0.4 |
| 541880 | 0 | 13 | 48 | 79 | 89 | 0.4 |

**Table 82**

| ISIS No | 0.04 µM | 0.11 µM | 0.33 µM | 1.00 µM | 3.00 µM | IC₅₀ (µM) |
|---|---|---|---|---|---|---|
| 539380 | 0 | 5 | 53 | 78 | 94 | 0.4 |
| 541903 | 12 | 20 | 26 | 62 | 88 | 0.5 |
| 541895 | 3 | 12 | 29 | 66 | 92 | 0.5 |
| 541882 | 2 | 0 | 27 | 65 | 86 | 0.7 |
| 541889 | 12 | 12 | 47 | 68 | 87 | 0.4 |
| 541930 | 0 | 6 | 40 | 59 | 85 | 0.6 |
| 541985 | 0 | 16 | 41 | 66 | 93 | 0.4 |
| 542031 | 1 | 0 | 22 | 55 | 80 | 0.8 |
| 541972 | 0 | 1 | 23 | 46 | 83 | 0.9 |
| 541991 | 4 | 35 | 42 | 67 | 89 | 0.4 |
| 542052 | 5 | 28 | 70 | 92 | 98 | 0.2 |
| 542080 | 0 | 18 | 54 | 87 | 96 | 0.3 |
| 542051 | 0 | 18 | 52 | 86 | 97 | 0.3 |
| 542071 | 5 | 3 | 51 | 74 | 95 | 0.4 |
| 542069 | 0 | 7 | 56 | 85 | 94 | 0.3 |

**Table 83**

| ISIS No | 0.04 µM | 0.11 µM | 0.33 µM | 1.00 µM | 3.00 µM | IC₅₀ (µM) |
|---|---|---|---|---|---|---|
| 539380 | 11 | 20 | 54 | 89 | 92 | 0.3 |
| 542053 | 6 | 14 | 38 | 69 | 74 | 0.6 |
| 542186 | 14 | 43 | 70 | 90 | 98 | 0.2 |
| 542185 | 0 | 26 | 48 | 80 | 96 | 0.3 |
| 545333 | 0 | 4 | 27 | 65 | 90 | 0.6 |
| 545336 | 0 | 15 | 24 | 43 | 79 | 0.9 |
| 545373 | 0 | 2 | 9 | 42 | 86 | 1.0 |
| 545438 | 0 | 24 | 56 | 81 | 92 | 0.3 |
| 545431 | 0 | 18 | 50 | 73 | 91 | 0.4 |
| 545447 | 0 | 15 | 34 | 78 | 93 | 0.4 |
| 545417 | 0 | 11 | 39 | 66 | 87 | 0.5 |
| 545467 | 12 | 16 | 37 | 76 | 93 | 0.4 |
| 545441 | 21 | 15 | 20 | 60 | 87 | 0.6 |
| 545439 | 17 | 24 | 49 | 82 | 91 | 0.3 |

### Example 10: Dose-dependent antisense inhibition of rhesus monkey GHR in LLC-MK2 cells

Gapmers from studies described above exhibiting significant *in vitro* inhibition of GHR mRNA were selected and tested for their potency for rhesus GHR mRNA in LLC-MK2 cells. Cells were plated at a density of 20,000 cells per well and transfected using electroporation with 0.12 µM, 0.37 µM, 1.11 µM, 3.33 µM, and 10.00 µM concentrations of antisense oligonucleotide. After a treatment period of approximately 16 hours, RNA was isolated from the cells and GHR mRNA levels were measured by quantitative real-time PCR. Primer probe set RTS3437MGB was used to measure mRNA levels. GHR mRNA levels were adjusted according to total RNA content, as measured by RIBOGREEN®. Results are presented as percent inhibition of GHR, relative to untreated control cells.

The half maximal inhibitory concentration (IC₅₀) of each oligonucleotide is also presented. GHR mRNA levels were significantly reduced in a dose-dependent manner in antisense oligonucleotide treated cells.

**Table 84**

| ISIS No | Chemistry | 0.12 µM | 0.37 µM | 1.11 µM | 3.33 µM | 10.00 µM | IC₅₀ (µM) |
|---|---|---|---|---|---|---|---|
| 541262 | Deoxy, MOE and cEt | 9 | 25 | 42 | 85 | 91 | 1.1 |
| 541742 | Deoxy, MOE and cEt | 0 | 24 | 19 | 58 | 77 | 3.2 |
| 541767 | Deoxy, MOE and cEt | 6 | 10 | 30 | 68 | 88 | 2.0 |
| 541875 | Deoxy, MOE and cEt | 7 | 19 | 64 | 84 | 96 | 0.9 |
| 541881 | Deoxy, MOE and cEt | 6 | 24 | 59 | 79 | 91 | 1.0 |
| 542101 | Deoxy, MOE and cEt | 0 | 5 | 38 | 71 | 81 | 2.0 |
| 542112 | Deoxy, MOE and cEt | 5 | 17 | 33 | 67 | 76 | 2.0 |
| 542118 | Deoxy, MOE and cEt | 1 | 6 | 35 | 68 | 86 | 2.0 |
| 542125 | Deoxy, MOE and cEt | 0 | 12 | 57 | 83 | 93 | 1.0 |
| 542127 | Deoxy, MOE and cEt | 1 | 0 | 30 | 68 | 84 | 2.4 |
| 542128 | Deoxy, MOE and cEt | 12 | 0 | 26 | 58 | 83 | 2.7 |
| 542153 | Deoxy, MOE and cEt | 4 | 0 | 0 | 36 | 59 | 6.6 |
| 542185 | Deoxy, MOE and cEt | 4 | 0 | 25 | 56 | 87 | 2.5 |
| 542186 | Deoxy, MOE and cEt | 15 | 23 | 51 | 73 | 90 | 1.1 |
| 542051 | Deoxy, MOE and cEt | 5 | 19 | 40 | 81 | 94 | 1.2 |

**Table 85**

| ISIS No | Chemistry | 0.12 µM | 0.37 µM | 1.11 µM | 3.33 µM | 10.00 µM | IC₅₀ (µM) |
|---|---|---|---|---|---|---|---|
| 523723 | 5-10-5 MOE | 23 | 14 | 31 | 43 | 71 | 3.5 |
| 532254 | 5-10-5 MOE | 29 | 35 | 42 | 69 | 87 | 0.8 |
| 532401 | 5-10-5 MOE | 27 | 28 | 46 | 73 | 88 | 1.2 |
| 533932 | 5-10-5 MOE | 10 | 24 | 48 | 70 | 92 | 1.2 |
| 539376 | 3-10-4 MOE | 21 | 8 | 8 | 35 | 81 | 4.3 |
| 539399 | 3-10-4 MOE | 2 | 10 | 14 | 18 | 57 | 8.3 |
| 539404 | 3-10-4 MOE | 39 | 12 | 25 | 27 | 57 | 7.7 |
| 539416 | 3-10-4 MOE | 24 | 35 | 44 | 79 | 89 | 1.0 |
| 539432 | 3-10-4 MOE | 9 | 29 | 42 | 73 | 89 | 1.2 |
| 541262 | Deoxy, MOE and cEt | 0 | 43 | 63 | 88 | 94 | 0.8 |
| 541742 | Deoxy, MOE and cEt | 3 | 19 | 35 | 56 | 85 | 1.9 |
| 541767 | Deoxy, MOE and cEt | 3 | 24 | 39 | 64 | 86 | 1.6 |
| 545439 | Deoxy, MOE and cEt | 19 | 15 | 43 | 74 | 80 | 1.7 |
| 545447 | Deoxy, MOE and cEt | 25 | 34 | 58 | 80 | 90 | 0.6 |

### Example 11: Dose-dependent antisense inhibition of GHR in cynomolgus primary hepatocytes

Gapmers from studies described above exhibiting significant *in vitro* inhibition of GHR mRNA were selected and tested for their potency for GHR mRNA in cynomolgus monkey primary hepatocytes. Cells were plated at a density of 20,000 cells per well and transfected using electroporation with 0.12 µM, 0.37 µM, 1.11 µM, 3.33 µM, and 10.00 µM concentrations of antisense oligonucleotide. After a treatment period of approximately 16 hours, RNA was isolated from the cells and GHR mRNA levels were measured by quantitative real-time PCR. Primer probe set RTS3437MGB was used to measure mRNA levels. GHR mRNA levels were adjusted according to total RNA content, as measured by RIBOGREEN®. Results are presented as percent inhibition of GHR, relative to untreated control cells.

The half maximal inhibitory concentration (IC₅₀) of each oligonucleotide is also presented. GHR mRNA levels were significantly reduced in a dose-dependent manner in antisense oligonucleotide treated cells.

**Table 86**

| ISIS No | Chemistry | 0.12 µM | 0.37 µM | 1.11 µM | 3.33 µM | 10.00 µM | IC₅₀ (µM) |
|---|---|---|---|---|---|---|---|
| 541262 | Deoxy, MOE and cEt | 40 | 52 | 75 | 92 | 98 | 0.3 |
| 541742 | Deoxy, MOE and cEt | 40 | 57 | 51 | 91 | 96 | 0.2 |
| 541767 | Deoxy, MOE and cEt | 36 | 59 | 60 | 78 | 91 | 0.4 |
| 541875 | Deoxy, MOE and cEt | 54 | 76 | 88 | 95 | 95 | <0.1 |
| 541881 | Deoxy, MOE and cEt | 53 | 75 | 85 | 98 | 98 | <0.1 |
| 542101 | Deoxy, MOE and cEt | 38 | 55 | 78 | 89 | 97 | 0.2 |
| 542112 | Deoxy, MOE and cEt | 28 | 50 | 74 | 89 | 96 | 0.4 |
| 542118 | Deoxy, MOE and cEt | 20 | 45 | 69 | 84 | 91 | 0.5 |
| 542125 | Deoxy, MOE and cEt | 33 | 62 | 77 | 92 | 97 | 0.3 |
| 542127 | Deoxy, MOE and cEt | 30 | 50 | 65 | 86 | 92 | 0.4 |
| 542128 | Deoxy, MOE and cEt | 25 | 40 | 52 | 80 | 93 | 0.7 |
| 542153 | Deoxy, MOE and cEt | 10 | 31 | 51 | 73 | 85 | 1.0 |
| 542185 | Deoxy, MOE and cEt | 12 | 45 | 65 | 85 | 93 | 0.6 |
| 542186 | Deoxy, MOE and cEt | 36 | 54 | 74 | 90 | 96 | 0.3 |
| 542051 | Deoxy, MOE and cEt | 9 | 29 | 32 | 32 | 42 | >10 |

**Table 87**

| ISIS No | Chemistry | 0.12 µM | 0.37 µM | 1.11 µM | 3.33 µM | 10.00 µM | IC₅₀ (µM) |
|---|---|---|---|---|---|---|---|
| 523435 | 5-10-5 MOE | 35 | 47 | 61 | 74 | 85 | 0.5 |
| 523723 | 5-10-5 MOE | 4 | 16 | 40 | 66 | 86 | 1.8 |
| 532254 | 5-10-5 MOE | 14 | 15 | 24 | 16 | 9 | >10 |
| 532401 | 5-10-5 MOE | 37 | 54 | 73 | 88 | 94 | 0.3 |
| 533932 | 5-10-5 MOE | 23 | 40 | 69 | 78 | 86 | 0.6 |
| 539376 | 3-10-4 MOE | 3 | 0 | 44 | 65 | 91 | 2.0 |
| 539399 | 3-10-4 MOE | 0 | 0 | 9 | 42 | 67 | 5.0 |
| 539404 | 3-10-4 MOE | 0 | 0 | 26 | 52 | 71 | 3.5 |
| 539416 | 3-10-4 MOE | 8 | 29 | 62 | 89 | 93 | 0.7 |
| 539432 | 3-10-4 MOE | 0 | 24 | 55 | 85 | 93 | 0.9 |
| 541262 | Deoxy, MOE and cEt | 23 | 52 | 73 | 92 | 96 | 0.4 |
| 541742 | Deoxy, MOE and cEt | 15 | 51 | 73 | 86 | 97 | 0.5 |
| 541767 | Deoxy, MOE and cEt | 19 | 20 | 39 | 68 | 81 | 1.8 |
| 545439 | Deoxy, MOE and cEt | 0 | 0 | 30 | 61 | 90 | 2.4 |
| 545447 | Deoxy, MOE and cEt | 0 | 17 | 17 | 19 | 27 | >10 |

### Example 12: Dose-dependent antisense inhibition of GHR in Hep3B cells

Gapmers from studies described above exhibiting significant *in vitro* inhibition of GHR mRNA were selected and tested for their potency for GHR mRNA at various doses in Hep3B cells. Cells were plated at a density of 20,000 cells per well and transfected using electroporation with 0.12 µM, 0.37 µM, 1.11 µM, 3.33 µM, and 10.00 µM concentrations of antisense oligonucleotide. After a treatment period of approximately 16 hours, RNA was isolated from the cells and GHR mRNA levels were measured by quantitative real-time PCR. Human primer probe set RTS3437_MGB was used to measure mRNA levels. GHR mRNA levels were adjusted according to total RNA content, as measured by RIBOGREEN®. Results are presented as percent inhibition of GHR, relative to untreated control cells.

The half maximal inhibitory concentration (IC₅₀) of each oligonucleotide is also presented. GHR mRNA levels were significantly reduced in a dose-dependent manner in antisense oligonucleotide treated cells.

**Table 88**

| ISIS No | 0.12 µM | 0.37 µM | 1.11 µM | 3.33 µM | 10.00 µM | IC₅₀ (µM) |
|---|---|---|---|---|---|---|
| 541262 | 25 | 43 | 76 | 85 | 94 | 0.5 |
| 541742 | 32 | 55 | 76 | 88 | 97 | 0.3 |
| 541767 | 29 | 56 | 83 | 89 | 97 | 0.3 |
| 541875 | 38 | 68 | 84 | 93 | 94 | 0.1 |
| 541881 | 32 | 57 | 81 | 94 | 97 | 0.3 |
| 542051 | 34 | 66 | 83 | 95 | 98 | 0.2 |
| 542101 | 25 | 55 | 85 | 95 | 98 | 0.3 |
| 542112 | 18 | 56 | 83 | 95 | 98 | 0.4 |
| 542118 | 42 | 61 | 88 | 95 | 97 | 0.1 |
| 542125 | 30 | 63 | 87 | 95 | 98 | 0.2 |
| 542127 | 50 | 70 | 91 | 91 | 98 | 0.1 |
| 542128 | 38 | 63 | 88 | 96 | 98 | 0.2 |
| 542153 | 37 | 59 | 85 | 94 | 97 | 0.2 |
| 542185 | 44 | 51 | 76 | 89 | 96 | 0.2 |
| 542186 | 46 | 59 | 84 | 95 | 97 | 0.1 |

**Table 89**

| ISIS No | 0.12 µM | 0.37 µM | 1.11 µM | 3.33 µM | 10.00 µM | IC₅₀ (µM) |
|---|---|---|---|---|---|---|
| 523435 | 9 | 26 | 49 | 78 | 93 | 1.0 |
| 523723 | 7 | 16 | 39 | 72 | 90 | 1.4 |
| 532254 | 36 | 46 | 69 | 86 | 94 | 0.4 |
| 532401 | 25 | 54 | 71 | 86 | 91 | 0.4 |
| 533932 | 8 | 47 | 69 | 80 | 94 | 0.7 |
| 539376 | 26 | 31 | 54 | 73 | 86 | 0.8 |
| 539399 | 23 | 43 | 72 | 89 | 94 | 0.5 |
| 539404 | 30 | 60 | 88 | 95 | 98 | 0.2 |
| 539416 | 30 | 59 | 84 | 93 | 98 | 0.3 |
| 539432 | 35 | 62 | 88 | 95 | 98 | 0.2 |
| 541262 | 43 | 60 | 84 | 89 | 98 | 0.2 |
| 541742 | 23 | 53 | 73 | 84 | 97 | 0.4 |
| 541767 | 22 | 49 | 74 | 85 | 92 | 0.4 |
| 545439 | 41 | 69 | 88 | 95 | 96 | 0.1 |
| 545447 | 31 | 47 | 63 | 74 | 82 | 0.5 |

### Example 13: Dose-dependent antisense inhibition of GHR in cynomolgus primary hepatocytes

Gapmers from studies described above exhibiting significant *in vitro* inhibition of GHR mRNA were selected and tested at various doses in cynomolgous monkey primary hepatocytes. Cells were plated at a density of 35,000 cells per well and transfected using electroporation with 0.04 µM, 0.12 µM, 0.37 µM, 1.11 µM, 3.33 µM, and 10.00 µM concentrations of antisense oligonucleotide. After a treatment period of approximately 16 hours, RNA was isolated from the cells and GHR mRNA levels were measured by quantitative real-time PCR. Primer probe set RTS3437_MGB was used to measure mRNA levels. GHR mRNA levels were adjusted according to total RNA content, as measured by RIBOGREEN®. Results are presented as percent inhibition of GHR, relative to untreated control cells.

The half maximal inhibitory concentration (IC₅₀) of each oligonucleotide is also presented. GHR mRNA levels were significantly reduced in a dose-dependent manner in antisense oligonucleotide treated cells.

**Table 90**

| ISIS No | 0.04 µM | 0.12 µM | 0.37 µM | 1.11 µM | 3.33 µM | 10.00 µM | IC₅₀ (µM) |
|---|---|---|---|---|---|---|---|
| 541767 | 8 | 17 | 29 | 48 | 59 | 58 | 0.4 |
| 541875 | 20 | 39 | 48 | 51 | 55 | 58 | 0.2 |
| 541881 | 23 | 36 | 49 | 60 | 56 | 58 | 0.1 |
| 542112 | 23 | 21 | 35 | 42 | 54 | 68 | 0.5 |
| 542118 | 19 | 14 | 26 | 38 | 54 | 59 | 0.8 |
| 542153 | 17 | 20 | 27 | 39 | 46 | 52 | 2.2 |
| 542185 | 20 | 23 | 27 | 46 | 39 | 56 | 2.0 |
| 532254 | 1 | 20 | 23 | 11 | 1 | 23 | >10 |
| 532401 | 0 | 15 | 24 | 39 | 47 | 55 | 1.6 |
| 523723 | 0 | 0 | 7 | 24 | 49 | 54 | 2.0 |

### Example 14: Comparative analysis of dose-dependent antisense inhibition of GHR in Hep3B cells

ISIS 532401 was compared with specific antisense oligonucleotides disclosed in US 2006/0178325 by testing at various doses in Hep3B cells. The oligonucleotides were selected based on the potency demonstrated in studies described in the application. Cells were plated at a density of 20,000 cells per well and transfected using electroporation with 0.11 µM, 0.33 µM, 1.00 µM, 1.11 µM, 3.00 µM, and 9.00 µM concentrations of antisense oligonucleotide. After a treatment period of approximately 16 hours, RNA was isolated from the cells and GHR mRNA levels were measured by quantitative real-time PCR. Human primer probe set RTS3437_MGB was used to measure mRNA levels. GHR mRNA levels were adjusted according to total RNA content, as measured by RIBOGREEN®. Results are presented as percent inhibition of GHR, relative to untreated control cells.

The half maximal inhibitory concentration (IC₅₀) of each oligonucleotide is also presented. The results indicate that ISIS 532401 was markedly more potent than the most potent oligonucleotides of US 2006/0178325.

**Table 91**

| ISIS No | 0.11 µM | 0.33 µM | 1.00 µM | 3.00 µM | 9.00 µM | IC₅₀ (µM) |
|---|---|---|---|---|---|---|
| 227452 | 11 | 12 | 46 | 73 | 92 | 1.4 |
| 227488 | 26 | 25 | 39 | 76 | 88 | 1.2 |
| 272309 | 16 | 14 | 39 | 66 | 91 | 1.6 |
| 272322 | 13 | 20 | 44 | 70 | 86 | 1.4 |
| 272328 | 22 | 20 | 24 | 43 | 56 | 5.7 |
| 272338 | 22 | 24 | 52 | 71 | 85 | 1.1 |
| 532401 | 34 | 53 | 72 | 87 | 94 | 0.3 |

### Example 15: Tolerability of 5-10-5 MOE gapmers targeting human GHR in CD1 mice

CD1® mice (Charles River, MA) are a multipurpose mice model, frequently utilized for safety and efficacy testing. The mice were treated with ISIS antisense oligonucleotides selected from studies described above and evaluated for changes in the levels of various plasma chemistry markers.

### Treatment

Groups of eight- to ten-week old male CD1 mice were injected subcutaneously twice a week for 6 weeks with 50 mg/kg of ISIS oligonucleotides (100 mg/kg/week dose). One group of male CD1 mice was injected subcutaneously twice a week for 6 weeks with PBS. Mice were euthanized 48 hours after the last dose, and organs and plasma were harvested for further analysis.

### Plasma chemistry markers

To evaluate the effect of ISIS oligonucleotides on liver and kidney function, plasma levels of transaminases, bilirubin, creatinine, and BUN were measured using an automated clinical chemistry analyzer (Hitachi Olympus AU400e, Melville, NY). The results are presented in Table 92. ISIS oligonucleotides that caused changes in the levels of any of the liver or kidney function markers outside the expected range for antisense oligonucleotides were excluded in further studies.

**Table 92**

| Plasma chemistry markers in CD1 mice plasma at week 6 | | | | | |
|---|---|---|---|---|---|
| | ALT (IU/L) | AST (IU/L) | Bilirubin (mg/dL) | Creatinine (mg/dL) | BUN (mg/dL) |
| PBS | 31 | 50 | 0.28 | 0.15 | 28 |
| ISIS 523271 | 366 | 285 | 0.18 | 0.11 | 29 |
| ISIS 523324 | 222 | 139 | 0.19 | 0.10 | 31 |
| ISIS 523604 | 2106 | 1157 | 0.41 | 0.06 | 48 |
| ISIS 532254 | 66 | 84 | 0.11 | 0.10 | 27 |
| ISIS 533121 | 176 | 155 | 0.19 | 0.09 | 27 |
| ISIS 533161 | 1094 | 904 | 0.23 | 0.07 | 29 |
| ISIS 533178 | 78 | 83 | 0.18 | 0.08 | 28 |
| ISIS 533234 | 164 | 147 | 0.21 | 0.09 | 26 |

### Hematology assays

Blood obtained from all mice groups were sent to Antech Diagnostics for hematocrit (HCT) measurements and analysis, as well as measurements of the various blood cells, such as WBC, RBC, and platelets, and total hemoglobin content. The results are presented in Table 93. ISIS oligonucleotides that caused changes in the levels of any of the hematology markers outside the expected range for antisense oligonucleotides were excluded in further studies.

**Table 93**

| Hematology markers in CD1 mice plasma at week 6 | | | | | |
|---|---|---|---|---|---|
| | HCT (%) | Hemoglobin (g/dL) | RBC (10⁶/µL) | WBC (10³/µL) | Platelets (10³/µL) |
| PBS | 45 | 13 | 8.2 | 4.1 | 689 |
| ISIS 523271 | 42 | 12 | 7.9 | 4.5 | 1181 |
| ISIS 523324 | 39 | 11 | 7.5 | 7.9 | 980 |
| ISIS 523604 | 33 | 10 | 6.9 | 14.1 | 507 |
| ISIS 532254 | 35 | 10 | 6.9 | 7.2 | 861 |
| ISIS 533121 | 39 | 12 | 7.9 | 8.4 | 853 |
| ISIS 533161 | 49 | 14 | 9.3 | 9.0 | 607 |
| ISIS 533178 | 44 | 13 | 8.5 | 6.9 | 765 |
| ISIS 533234 | 42 | 12 | 7.8 | 9.2 | 1045 |

### Example 16: Tolerability of 5-10-5 MOE gapmers targeting human GHR in CD1 mice

CD1® mice were treated with ISIS antisense oligonucleotides selected from studies described above and evaluated for changes in the levels of various plasma chemistry markers.

### Treatment

Groups of eight- to ten-week old male CD1 mice were injected subcutaneously twice a week for 6 weeks with 50 mg/kg of ISIS oligonucleotide (100 mg/kg/week dose). One group of male CD1 mice was injected subcutaneously twice a week for 6 weeks with PBS. Mice were euthanized 48 hours after the last dose, and organs and plasma were harvested for further analysis.

### Plasma chemistry markers

To evaluate the effect of ISIS oligonucleotides on liver and kidney function, plasma levels of transaminases, bilirubin, creatinine, and BUN were measured using an automated clinical chemistry analyzer (Hitachi Olympus AU400e, Melville, NY). The results are presented in Table 94. ISIS oligonucleotides that caused changes in the levels of any of the liver or kidney function markers outside the expected range for antisense oligonucleotides were excluded in further studies.

**Table 94**

| Plasma chemistry markers in CD1 mice plasma at week 6 | | | | | |
|---|---|---|---|---|---|
| | ALT (IU/L) | AST (IU/L) | Bilirubin (mg/dL) | Creatinine (mg/dL) | BUN (mg/dL) |
| PBS | 30 | 59 | 0.26 | 0.14 | 20 |
| ISIS 523715 | 636 | 505 | 0.24 | 0.14 | 22 |
| ISIS 523723 | 57 | 80 | 0.20 | 0.16 | 23 |
| ISIS 523726 | 165 | 167 | 0.18 | 0.15 | 23 |
| ISIS 523736 | 140 | 177 | 0.20 | 0.15 | 23 |
| ISIS 523747 | 96 | 108 | 0.17 | 0.14 | 23 |
| ISIS 523789 | 45 | 74 | 0.20 | 0.15 | 22 |
| ISIS 532395 | 64 | 111 | 0.23 | 0.12 | 21 |
| ISIS 532401 | 47 | 88 | 0.21 | 0.17 | 22 |
| ISIS 532411 | 225 | 426 | 0.17 | 0.16 | 22 |
| ISIS 532420 | 60 | 99 | 0.21 | 0.12 | 25 |
| ISIS 532468 | 319 | 273 | 0.15 | 0.14 | 21 |
| ISIS 533932 | 62 | 81 | 0.18 | 0.14 | 21 |

### Hematology assays

Blood obtained from all mice groups were sent to Antech Diagnostics for hematocrit (HCT) measurements and analysis, as well as measurements of the various blood cells, such as WB), RBC, and platelets, and total hemoglobin content. The results are presented in Table 95. ISIS oligonucleotides that caused changes in the levels of any of the hematology markers outside the expected range for antisense oligonucleotides were excluded in further studies.

**Table 95**

| Hematology markers in CD1 mice plasma at week 6 | | | | | |
|---|---|---|---|---|---|
| | HCT (%) | Hemoglobin (g/dL) | RBC (10⁶/µL) | WBC (10³/µL) | Platelets (10³/µL) |
| PBS | 43 | 13 | 8.1 | 3.3 | 1047 |
| ISIS 523715 | 40 | 12 | 8.1 | 4.2 | 1153 |
| ISIS 523723 | 35 | 11 | 6.8 | 2.9 | 1154 |
| ISIS 523726 | 32 | 10 | 6.8 | 5.8 | 1056 |
| ISIS 523736 | 35 | 11 | 7.1 | 3.6 | 1019 |
| ISIS 523747 | 37 | 11 | 7.7 | 2.8 | 1146 |
| ISIS 523789 | 37 | 11 | 7.3 | 2.5 | 1033 |
| ISIS 532395 | 37 | 11 | 7.4 | 4.5 | 890 |
| ISIS 532401 | 36 | 11 | 7.1 | 3.7 | 1175 |
| ISIS 532411 | 27 | 8 | 5.3 | 3.2 | 641 |
| ISIS 532420 | 35 | 11 | 7.0 | 3.3 | 1101 |
| ISIS 532468 | 36 | 11 | 7.4 | 4.0 | 1043 |
| ISIS 533932 | 36 | 11 | 7.2 | 3.8 | 981 |

### Example 17: Tolerability of 3-10-4 MOE gapmers targeting human GHR in CD1 mice

CD1® mice were treated with ISIS antisense oligonucleotides selected from studies described above and evaluated for changes in the levels of various plasma chemistry markers.

### Treatment

Groups of eight- to ten-week old male CD1 mice were injected subcutaneously twice a week for 6 weeks with 50 mg/kg of ISIS oligonucleotide (100 mg/kg/week dose). One group of male CD1 mice was injected subcutaneously twice a week for 6 weeks with PBS. Mice were euthanized 48 hours after the last dose, and organs and plasma were harvested for further analysis.

### Plasma chemistry markers

To evaluate the effect of ISIS oligonucleotides on liver and kidney function, plasma levels of transaminases, bilirubin, creatinine, and BUN were measured using an automated clinical chemistry analyzer (Hitachi Olympus AU400e, Melville, NY). The results are presented in Table 96. ISIS oligonucleotides that caused changes in the levels of any of the liver or kidney function markers outside the expected range for antisense oligonucleotides were excluded in further studies.

**Table 96**

| Plasma chemistry markers in CD1 mice plasma at week 6 | | | | | |
|---|---|---|---|---|---|
| | ALT (IU/L) | AST (IU/L) | Bilirubin (mg/dL) | Creatinine (mg/dL) | BUN (mg/dL) |
| PBS | 48 | 63 | 0.20 | 0.13 | 28 |
| ISIS 539302 | 204 | 192 | 0.15 | 0.15 | 24 |
| ISIS 539321 | 726 | 455 | 0.17 | 0.12 | 27 |
| ISIS 539360 | 3287 | 2495 | 0.58 | 0.13 | 22 |
| ISIS 539361 | 310 | 226 | 0.17 | 0.11 | 21 |
| ISIS 539376 | 77 | 75 | 0.14 | 0.12 | 27 |
| ISIS 539379 | 134 | 136 | 0.16 | 0.13 | 24 |
| ISIS 539380 | 180 | 188 | 0.14 | 0.12 | 23 |
| ISIS 539383 | 80 | 81 | 0.15 | 0.12 | 25 |
| ISIS 539399 | 119 | 127 | 0.13 | 0.12 | 24 |
| ISIS 539401 | 1435 | 1172 | 0.24 | 0.11 | 24 |
| ISIS 539403 | 1543 | 883 | 0.18 | 0.12 | 26 |
| ISIS 539404 | 75 | 109 | 0.16 | 0.13 | 23 |
| ISIS 539416 | 100 | 107 | 0.19 | 0.15 | 26 |
| ISIS 539432 | 55 | 64 | 0.20 | 0.14 | 22 |
| ISIS 539433 | 86 | 91 | 0.12 | 0.13 | 22 |

### Hematology assays

Blood obtained from all mice groups were sent to Antech Diagnostics for hematocrit (HCT) measurements and analysis, as well as measurements of the various blood cells, such as WBC, RBC, and platelets, and total hemoglobin content. The results are presented in Table 97. ISIS oligonucleotides that caused changes in the levels of any of the hematology markers outside the expected range for antisense oligonucleotides were excluded in further studies.

**Table 97**

| Hematology markers in CD1 mice plasma at week 6 | | | | | |
|---|---|---|---|---|---|
| | HCT (%) | Hemoglobin (g/dL) | RBC (10⁶/µL) | WBC (10³/µL) | Platelets (10³/µL) |
| PBS | 46 | 13 | 8.5 | 6 | 954 |
| ISIS 539302 | 40 | 11 | 8.1 | 13 | 830 |
| ISIS 539321 | 39 | 11 | 7.8 | 16 | 723 |
| ISIS 539360 | 49 | 14 | 9.0 | 14 | 671 |
| ISIS 539361 | 45 | 13 | 8.5 | 9 | 893 |
| ISIS 539376 | 42 | 12 | 7.7 | 6 | 988 |
| ISIS 539379 | 42 | 12 | 8.1 | 7 | 795 |
| ISIS 539380 | 38 | 10 | 7.7 | 8 | 950 |
| ISIS 539383 | 45 | 12 | 8.4 | 8 | 795 |
| ISIS 539399 | 41 | 12 | 8.0 | 10 | 895 |
| ISIS 539401 | 41 | 11 | 8.2 | 9 | 897 |
| ISIS 539403 | 33 | 9 | 6.2 | 13 | 1104 |
| ISIS 539404 | 42 | 12 | 8.4 | 7 | 641 |
| ISIS 539416 | 41 | 11 | 7.5 | 5 | 686 |
| ISIS 539432 | 44 | 12 | 8.0 | 6 | 920 |
| ISIS 539433 | 40 | 11 | 7.4 | 6 | 987 |

### Example 18: Tolerability of deoxy, MOE and cEt gapmers targeting human GHR in CD1 mice

CD1® mice were treated with ISIS antisense oligonucleotides selected from studies described above and evaluated for changes in the levels of various plasma chemistry markers.

### Treatment

Groups of eight- to ten-week old male CD1 mice were injected subcutaneously twice a week for 6 weeks with 25 mg/kg of ISIS oligonucleotide (50 mg/kg/week dose). One group of male CD1 mice was injected subcutaneously twice a week for 6 weeks with PBS. Mice were euthanized 48 hours after the last dose, and organs and plasma were harvested for further analysis.

### Plasma chemistry markers

To evaluate the effect of ISIS oligonucleotides on liver and kidney function, plasma levels of transaminases, bilirubin, creatinine, and BUN were measured using an automated clinical chemistry analyzer (Hitachi Olympus AU400e, Melville, NY). The results are presented in Table 98. ISIS oligonucleotides that caused changes in the levels of any of the liver or kidney function markers outside the expected range for antisense oligonucleotides were excluded in further studies.

**Table 98**

| Plasma chemistry markers in CD1 mice plasma at week 6 | | | | | |
|---|---|---|---|---|---|
| | ALT (IU/L) | AST (IU/L) | Bilirubin (mg/dL) | Creatinine (mg/dL) | BUN (mg/dL) |
| PBS | 36 | 71 | 0.22 | 0.18 | 22 |
| ISIS 541262 | 115 | 133 | 0.21 | 0.18 | 21 |
| ISIS 541724 | 543 | 531 | 0.34 | 0.17 | 21 |
| ISIS 541742 | 44 | 71 | 0.18 | 0.16 | 21 |
| ISIS 541748 | 269 | 582 | 0.16 | 0.15 | 22 |
| ISIS 541749 | 626 | 491 | 0.20 | 0.20 | 22 |
| ISIS 541750 | 1531 | 670 | 0.20 | 0.18 | 23 |
| ISIS 541766 | 2107 | 1139 | 0.21 | 0.21 | 23 |
| ISIS 541767 | 42 | 62 | 0.21 | 0.17 | 20 |
| ISIS 541822 | 493 | 202 | 0.13 | 0.16 | 22 |
| ISIS 541826 | 889 | 398 | 0.21 | 0.14 | 17 |
| ISIS 541838 | 266 | 172 | 0.16 | 0.15 | 20 |
| ISIS 541870 | 445 | 272 | 0.23 | 0.16 | 23 |
| ISIS 541875 | 103 | 114 | 0.20 | 0.15 | 20 |
| ISIS 541907 | 940 | 725 | 0.16 | 0.19 | 35 |
| ISIS 541991 | 1690 | 1733 | 0.31 | 0.20 | 23 |

### Hematology assays

Blood obtained from all mice groups were sent to Antech Diagnostics for hematocrit (HCT) measurements and analysis, as well as measurements of the various blood cells, such as WBC, RBC, and platelets, and total hemoglobin content. The results are presented in Table 99. ISIS oligonucleotides that caused changes in the levels of any of the hematology markers outside the expected range for antisense oligonucleotides were excluded in further studies.

**Table 99**

| Hematology markers in CD1 mice plasma at week 6 | | | | | |
|---|---|---|---|---|---|
| | HCT (%) | Hemoglobin (g/dL) | RBC (10⁶/µL) | WBC (10³/µL) | Platelets (10³/µL) |
| PBS | 37 | 11 | 7 | 3 | 1083 |
| ISIS 541262 | 38 | 11 | 7 | 6 | 1010 |
| ISIS 541724 | 52 | 16 | 10 | 9 | 940 |
| ISIS 541742 | 47 | 14 | 9 | 6 | 1134 |
| ISIS 541748 | 41 | 12 | 8 | 7 | 941 |
| ISIS 541749 | 41 | 12 | 8 | 5 | 1142 |
| ISIS 541750 | 42 | 12 | 8 | 4 | 1409 |
| ISIS 541766 | 39 | 11 | 7 | 7 | 989 |
| ISIS 541767 | 46 | 14 | 9 | 2 | 994 |
| ISIS 541822 | 42 | 12 | 8 | 3 | 1190 |
| ISIS 541826 | 41 | 12 | 8 | 10 | 1069 |
| ISIS 541838 | 44 | 13 | 8 | 6 | 1005 |
| ISIS 541870 | 38 | 11 | 7 | 8 | 1020 |
| ISIS 541875 | 44 | 13 | 8 | 6 | 1104 |
| ISIS 541907 | 40 | 11 | 8 | 9 | 1271 |
| ISIS 541991 | 34 | 10 | 6 | 6 | 1274 |

### Example 19: Tolerability of deoxy, MOE and cEt gapmers targeting human GHR in CD1 mice

CD1® mice were treated with ISIS antisense oligonucleotides selected from studies described above and evaluated for changes in the levels of various plasma chemistry markers. The 3-10-4 MOE gapmer ISIS 539376 was also included in the study.

### Treatment

Groups of eight- to ten-week old male CD1 mice were injected subcutaneously twice a week for 6 weeks with 25 mg/kg of ISIS oligonucleotide (50 mg/kg/week dose). One group of male CD1 mice was injected subcutaneously twice a week for 6 weeks with PBS. Mice were euthanized 48 hours after the last dose, and organs and plasma were harvested for further analysis.

### Plasma chemistry markers

To evaluate the effect of ISIS oligonucleotides on liver and kidney function, plasma levels of transaminases, bilirubin, creatinine, and BUN were measured using an automated clinical chemistry analyzer (Hitachi Olympus AU400e, Melville, NY). The results are presented in Table 100. ISIS oligonucleotides that caused changes in the levels of any of the liver or kidney function markers outside the expected range for antisense oligonucleotides were excluded in further studies.

**Table 100**

| Plasma chemistry markers in CD1 mice plasma at week 6 | | | | | |
|---|---|---|---|---|---|
| | ALT (IU/L) | AST (IU/L) | Bilirubin (mg/dL) | Creatinine (mg/dL) | BUN (mg/dL) |
| PBS | 43 | 66 | 0.21 | 0.11 | 20 |
| ISIS 541881 | 63 | 109 | 0.28 | 0.13 | 23 |
| ISIS 541936 | 3260 | 2108 | 0.40 | 0.13 | 24 |
| ISIS 542051 | 97 | 119 | 0.23 | 0.12 | 23 |
| ISIS 542052 | 454 | 236 | 0.23 | 0.12 | 25 |
| ISIS 542069 | 293 | 211 | 0.23 | 0.13 | 27 |
| ISIS 542085 | 91 | 87 | 0.18 | 0.10 | 21 |
| ISIS 542086 | 137 | 133 | 0.24 | 0.10 | 23 |
| ISIS 542094 | 86 | 143 | 0.23 | 0.13 | 21 |
| ISIS 542101 | 46 | 74 | 0.19 | 0.10 | 21 |
| ISIS 542102 | 4920 | 2432 | 2.30 | 0.15 | 29 |
| ISIS 542105 | 1255 | 575 | 0.35 | 0.13 | 21 |
| ISIS 542106 | 3082 | 2295 | 3.42 | 0.17 | 23 |
| ISIS 542107 | 4049 | 3092 | 0.50 | 0.14 | 20 |
| ISIS 542108 | 1835 | 859 | 0.32 | 0.11 | 21 |
| ISIS 539376 | 40 | 79 | 0.27 | 0.08 | 22 |

### Hematology assays

Blood obtained from all mice groups were sent to Antech Diagnostics for hematocrit (HCT) measurements and analysis, as well as measurements of the various blood cells, such as WBC, RBC, and total hemoglobin content. The results are presented in Table 101. ISIS oligonucleotides that caused changes in the levels of any of the hematology markers outside the expected range for antisense oligonucleotides were excluded in further studies.

**Table 101**

| Hematology markers in CD1 mice plasma at week 6 | | | | |
|---|---|---|---|---|
| | HCT (%) | Hemoglobin (g/dL) | RBC (10⁶/µL) | WBC (10³/µL) |
| PBS | 46 | 13 | 8 | 6 |
| ISIS 541881 | 53 | 15 | 10 | 7 |
| ISIS 541936 | 41 | 11 | 8 | 18 |
| ISIS 542051 | 49 | 14 | 9 | 8 |
| ISIS 542052 | 46 | 13 | 9 | 9 |
| ISIS 542069 | 43 | 13 | 8 | 7 |
| ISIS 542085 | 38 | 11 | 7 | 5 |
| ISIS 542086 | 49 | 14 | 9 | 9 |
| ISIS 542094 | 36 | 10 | 6 | 5 |
| ISIS 542101 | 44 | 13 | 9 | 5 |
| ISIS 542102 | 27 | 7 | 5 | 25 |
| ISIS 542105 | 42 | 12 | 8 | 7 |
| ISIS 542106 | 37 | 10 | 7 | 14 |
| ISIS 542107 | 41 | 12 | 7 | 17 |
| ISIS 542108 | 51 | 14 | 8 | 10 |
| ISIS 539376 | 49 | 14 | 10 | 5 |

### Example 20: Tolerability of deoxy, MOE and cEt gapmers targeting human GHR in CD1 mice

CD1® mice were treated with ISIS antisense oligonucleotides selected from studies described above and evaluated for changes in the levels of various plasma chemistry markers.

### Treatment

Groups of eight- to ten-week old male CD1 mice were injected subcutaneously twice a week for 6 weeks with 25 mg/kg of ISIS oligonucleotide (50 mg/kg/week dose). One group of male CD1 mice was injected subcutaneously twice a week for 6 weeks with PBS. Mice were euthanized 48 hours after the last dose, and organs and plasma were harvested for further analysis.

### Plasma chemistry markers

To evaluate the effect of ISIS oligonucleotides on liver and kidney function, plasma levels of transaminases, bilirubin, creatinine, and BUN were measured using an automated clinical chemistry analyzer (Hitachi Olympus AU400e, Melville, NY). The results are presented in Table 102. ISIS oligonucleotides that caused changes in the levels of any of the liver or kidney function markers outside the expected range for antisense oligonucleotides were excluded in further studies.

**Table 102**

| Plasma chemistry markers in CD1 mice plasma at week 6 | | | | | |
|---|---|---|---|---|---|
| | ALT (IU/L) | AST (IU/L) | Bilirubin (mg/dL) | Creatinine (mg/dL) | BUN (mg/dL) |
| PBS | 51 | 63 | 0.3 | 0.14 | 27 |
| ISIS 542109 | 3695 | 2391 | 0.8 | 0.19 | 24 |
| ISIS 542112 | 119 | 104 | 0.3 | 0.16 | 28 |
| ISIS 542118 | 66 | 86 | 0.3 | 0.15 | 26 |
| ISIS 542122 | 1112 | 350 | 0.3 | 0.16 | 27 |
| ISIS 542125 | 79 | 92 | 0.2 | 0.13 | 26 |
| ISIS 542126 | 381 | 398 | 0.5 | 0.14 | 23 |
| ISIS 542127 | 54 | 85 | 0.3 | 0.16 | 26 |
| ISIS 542128 | 55 | 89 | 0.2 | 0.12 | 24 |
| ISIS 542145 | 834 | 671 | 0.3 | 0.11 | 24 |
| ISIS 542146 | 163 | 107 | 0.2 | 0.14 | 30 |
| ISIS 542149 | 974 | 752 | 0.3 | 0.12 | 26 |
| ISIS 542150 | 2840 | 2126 | 2.4 | 0.17 | 23 |
| ISIS 542153 | 53 | 75 | 0.2 | 0.14 | 28 |
| ISIS 542157 | 137 | 122 | 0.3 | 0.13 | 25 |
| ISIS 542185 | 57 | 72 | 0.2 | 0.11 | 23 |
| ISIS 542186 | 62 | 84 | 0.2 | 0.12 | 24 |
| ISIS 545431 | 2622 | 1375 | 3.0 | 0.15 | 28 |
| ISIS 545438 | 1710 | 1000 | 0.3 | 0.14 | 26 |
| ISIS 545439 | 70 | 117 | 0.2 | 0.12 | 28 |
| ISIS 545447 | 141 | 108 | 0.3 | 0.13 | 26 |

### Hematology assays

Blood obtained from all mice groups were sent to Antech Diagnostics for hematocrit (HCT) measurements and analysis, as well as measurements of the various blood cells, such as WBC, RBC, and total hemoglobin content. The results are presented in Table 103. ISIS oligonucleotides that caused changes in the levels of any of the hematology markers outside the expected range for antisense oligonucleotides were excluded in further studies.

**Table 103**

| Hematology markers in CD1 mice plasma at week 6 | | | | | |
|---|---|---|---|---|---|
| | HCT (%) | Hemoglobin (g/dL) | RBC (10⁶/µL) | WBC (10³/µL) | Platelets (10³/µL) |
| PBS | 40 | 12 | 7 | 6 | 1210 |
| ISIS 542109 | 47 | 13 | 9 | 16 | 1244 |
| ISIS 542112 | 50 | 13 | 8 | 7 | 1065 |
| ISIS 542118 | 42 | 12 | 8 | 8 | 1120 |
| ISIS 542122 | 37 | 11 | 7 | 7 | 1064 |
| ISIS 542125 | 42 | 13 | 8 | 7 | 1063 |
| ISIS 542126 | 34 | 10 | 7 | 9 | 1477 |
| ISIS 542127 | 41 | 12 | 7 | 7 | 1144 |
| ISIS 542128 | 40 | 12 | 7 | 6 | 1196 |
| ISIS 542145 | 42 | 12 | 8 | 8 | 1305 |
| ISIS 542146 | 45 | 13 | 8 | 7 | 1310 |
| ISIS 542149 | 33 | 10 | 6 | 12 | 903 |
| ISIS 542150 | 27 | 7 | 5 | 18 | 1202 |
| ISIS 542153 | 46 | 13 | 8 | 5 | 1130 |
| ISIS 542157 | 44 | 12 | 9 | 6 | 791 |
| ISIS 542185 | 45 | 13 | 8 | 3 | 1031 |
| ISIS 542186 | 44 | 12 | 8 | 6 | 985 |
| ISIS 545431 | 28 | 7 | 6 | 13 | 2609 |
| ISIS 545438 | 40 | 11 | 8 | 8 | 1302 |
| ISIS 545439 | 48 | 13 | 9 | 4 | 857 |
| ISIS 545447 | 45 | 13 | 9 | 9 | 964 |

### Example 21: Tolerability of MOE gapmers targeting human GHR in Sprague-Dawley rats

Sprague-Dawley rats are a multipurpose model used for safety and efficacy evaluations. The rats were treated with ISIS antisense oligonucleotides from the studies described in the Examples above and evaluated for changes in the levels of various plasma chemistry markers.

### Treatment

Male Sprague-Dawley rats were maintained on a 12-hour light/dark cycle and fed ad libitum with Purina normal rat chow, diet 5001. Groups of 4 Sprague-Dawley rats each were injected subcutaneously twice a week for 6 weeks with 50 mg/kg of ISIS oligonucleotide (100 mg/kg weekly dose). Forty eight hours after the last dose, rats were euthanized and organs and plasma were harvested for further analysis.

### Liver function

To evaluate the effect of ISIS oligonucleotides on hepatic function, plasma levels of transaminases were measured using an automated clinical chemistry analyzer (Hitachi Olympus AU400e, Melville, NY). Plasma levels of ALT (alanine transaminase) and AST (aspartate transaminase) were measured and the results are presented in Table 104 expressed in IU/L. Plasma levels of bilirubin were also measured using the same clinical chemistry analyzer and the results are also presented in Table 104 expressed in mg/dL. ISIS oligonucleotides that caused changes in the levels of any markers of liver function outside the expected range for antisense oligonucleotides were excluded in further studies.

**Table 104**

| Liver function markers in Sprague-Dawley rats | | | |
|---|---|---|---|
| | ALT (IU/L) | AST (IU/L) | Bilirubin (mg/dL) |
| PBS | 69 | 90 | 0.15 |
| ISIS 523723 | 79 | 123 | 0.12 |
| ISIS 523789 | 71 | 105 | 0.15 |
| ISIS 532254 | 67 | 97 | 0.14 |
| ISIS 532401 | 61 | 77 | 0.12 |
| ISIS 532420 | 102 | 127 | 0.17 |
| ISIS 533178 | 157 | 219 | 0.34 |
| ISIS 533234 | 71 | 90 | 0.11 |
| ISIS 533932 | 58 | 81 | 0.12 |
| ISIS 539376 | 75 | 101 | 0.14 |
| ISIS 539380 | 86 | 128 | 0.16 |
| ISIS 539383 | 64 | 94 | 0.14 |
| ISIS 539399 | 52 | 95 | 0.14 |
| ISIS 539404 | 88 | 118 | 0.13 |
| ISIS 539416 | 63 | 104 | 0.14 |
| ISIS 539432 | 63 | 90 | 0.13 |
| ISIS 539433 | 69 | 92 | 0.13 |

### Kidney function

To evaluate the effect of ISIS oligonucleotides on kidney function, plasma levels of blood urea nitrogen (BUN) and creatinine were measured using an automated clinical chemistry analyzer (Hitachi Olympus AU400e, Melville, NY). Results are presented in Table 105, expressed in mg/dL. ISIS oligonucleotides that caused changes in the levels of any of the kidney function markers outside the expected range for antisense oligonucleotides were excluded in further studies.

**Table 105**

| Kidney function markers (mg/dL) in Sprague-Dawley rats | | |
|---|---|---|
| | BUN | Creatinine |
| PBS | 24 | 0.32 |
| ISIS 523723 | 20 | 0.39 |
| ISIS 523789 | 19 | 0.37 |
| ISIS 532254 | 21 | 0.43 |
| ISIS 532401 | 17 | 0.36 |
| ISIS 532420 | 20 | 0.31 |
| ISIS 533178 | 20 | 0.43 |
| ISIS 533234 | 22 | 0.41 |
| ISIS 533932 | 19 | 0.43 |
| ISIS 539376 | 19 | 0.36 |
| ISIS 539380 | 18 | 0.35 |
| ISIS 539383 | 19 | 0.35 |
| ISIS 539399 | 18 | 0.39 |
| ISIS 539404 | 23 | 0.39 |
| ISIS 539416 | 17 | 0.39 |
| ISIS 539432 | 20 | 0.39 |
| ISIS 539433 | 20 | 0.34 |

### Hematology assays

Blood obtained from all rat groups were sent to Antech Diagnostics for hematocrit (HCT) measurements and analysis, as well as measurements of the various blood cells, such as WBC, RBC, and total hemoglobin content. The results are presented in Table 106. ISIS oligonucleotides that caused changes in the levels of any of the hematology markers outside the expected range for antisense oligonucleotides were excluded in further studies.

**Table 106**

| Hematology markers in Sprague-Dawley rats | | | | | |
|---|---|---|---|---|---|
| | HCT (%) | Hemoglobin (g/dL) | RBC (10⁶/µL) | WBC (10³/µL) | Platelets (10³/µL) |
| PBS | 46 | 15 | 8 | 11 | 1078 |
| ISIS 523723 | 38 | 12 | 7 | 19 | 626 |
| ISIS 523789 | 38 | 12 | 8 | 12 | 702 |
| ISIS 532254 | 36 | 12 | 7 | 11 | 547 |
| ISIS 532401 | 42 | 14 | 8 | 12 | 858 |
| ISIS 532420 | 37 | 12 | 7 | 17 | 542 |
| ISIS 533178 | 37 | 12 | 7 | 15 | 1117 |
| ISIS 533234 | 38 | 12 | 7 | 8 | 657 |
| ISIS 533932 | 40 | 13 | 7 | 9 | 999 |
| ISIS 539376 | 43 | 14 | 9 | 8 | 910 |
| ISIS 539380 | 33 | 11 | 5 | 6 | 330 |
| ISIS 539383 | 39 | 13 | 7 | 10 | 832 |
| ISIS 539399 | 37 | 11 | 7 | 4 | 603 |
| ISIS 539404 | 37 | 12 | 7 | 6 | 639 |
| ISIS 539416 | 33 | 11 | 6 | 9 | 601 |
| ISIS 539432 | 44 | 14 | 9 | 10 | 810 |
| ISIS 539433 | 38 | 12 | 7 | 9 | 742 |

### Organ weights

Liver, heart, spleen and kidney weights were measured at the end of the study, and are presented in Table 107. ISIS oligonucleotides that caused any changes in organ weights outside the expected range for antisense oligonucleotides were excluded from further studies.

**Table 107**

| Organ weights (g) | | | | |
|---|---|---|---|---|
| | Heart | Liver | Spleen | Kidney |
| PBS | 0.35 | 3.6 | 0.2 | 0.8 |
| ISIS 523723 | 0.31 | 4.9 | 0.7 | 0.8 |
| ISIS 523789 | 0.34 | 4.8 | 0.6 | 0.8 |
| ISIS 532254 | 0.32 | 5.0 | 0.6 | 1.0 |
| ISIS 532401 | 0.32 | 3.8 | 0.4 | 0.8 |
| ISIS 532420 | 0.29 | 4.6 | 0.7 | 1.0 |
| ISIS 533178 | 0.34 | 5.2 | 0.7 | 0.9 |
| ISIS 533234 | 0.30 | 4.4 | 0.6 | 1.0 |
| ISIS 533932 | 0.31 | 3.9 | 0.5 | 0.9 |
| ISIS 539376 | 0.29 | 4.4 | 0.4 | 0.8 |
| ISIS 539380 | 0.31 | 6.3 | 1.6 | 1.2 |
| ISIS 539383 | 0.31 | 4.5 | 0.6 | 1.0 |
| ISIS 539399 | 0.31 | 4.5 | 0.8 | 1.0 |
| ISIS 539404 | 0.34 | 4.9 | 0.6 | 1.0 |
| ISIS 539416 | 0.32 | 4.7 | 0.7 | 0.9 |
| ISIS 539432 | 0.30 | 3.8 | 0.4 | 0.8 |
| ISIS 539433 | 0.28 | 4.1 | 0.7 | 1.0 |

### Example 22: Tolerability of deoxy, MOE, and cEt gapmers targeting human GHR in Sprague-Dawley rats

Sprague-Dawley rats were treated with ISIS antisense oligonucleotides from the studies described in the Examples above and evaluated for changes in the levels of various plasma chemistry markers.

### Treatment

Male Sprague-Dawley rats were maintained on a 12-hour light/dark cycle and fed ad libitum with Purina normal rat chow, diet 5001. Groups of 4 Sprague-Dawley rats each were injected subcutaneously once a week for 6 weeks with 50 mg/kg of ISIS oligonucleotide (50 mg/kg weekly dose). Two groups of rats were injected subcutaneously once a week for 6 weeks with PBS. Forty eight hours after the last dose, rats were euthanized and organs and plasma were harvested for further analysis.

### Liver function

To evaluate the effect of ISIS oligonucleotides on hepatic function, plasma levels of transaminases were measured using an automated clinical chemistry analyzer (Hitachi Olympus AU400e, Melville, NY). Plasma levels of ALT and AST were measured and the results are presented in Table 108 expressed in IU/L. Plasma levels of bilirubin were also measured using the same clinical chemistry analyzer and the results are also presented in Table 108 expressed in mg/dL. ISIS oligonucleotides that caused changes in the levels of any markers of liver function outside the expected range for antisense oligonucleotides were excluded in further studies.

**Table 108**

| Liver function markers in Sprague-Dawley rats | | | |
|---|---|---|---|
| | ALT (IU/L) | AST (IU/L) | Bilirubin (mg/dL) |
| PBS group 1 | 34 | 56 | 0.08 |
| PBS group 2 | 37 | 54 | 0.09 |
| ISIS 541881 | 53 | 77 | 0.12 |
| ISIS 542051 | 61 | 96 | 0.09 |
| ISIS 542101 | 64 | 214 | 0.10 |
| ISIS 542112 | 46 | 72 | 0.10 |
| ISIS 542118 | 42 | 60 | 0.08 |
| ISIS 542125 | 39 | 67 | 0.10 |
| ISIS 542127 | 56 | 75 | 0.12 |
| ISIS 542128 | 45 | 71 | 0.12 |
| ISIS 542153 | 44 | 69 | 0.11 |
| ISIS 542185 | 44 | 93 | 0.09 |
| ISIS 542186 | 51 | 107 | 0.12 |
| ISIS 545439 | 41 | 73 | 0.10 |
| ISIS 545447 | 103 | 114 | 0.10 |
| ISIS 541262 | 106 | 133 | 0.12 |
| ISIS 541742 | 56 | 102 | 0.11 |
| ISIS 541767 | 53 | 69 | 0.09 |
| ISIS 541875 | 70 | 133 | 0.08 |

### Kidney function

To evaluate the effect of ISIS oligonucleotides on kidney function, plasma levels of blood urea nitrogen (BUN) and creatinine were measured using an automated clinical chemistry analyzer (Hitachi Olympus AU400e, Melville, NY). Results are presented in Table 109, expressed in mg/dL. ISIS oligonucleotides that caused changes in the levels of any of the kidney function markers outside the expected range for antisense oligonucleotides were excluded in further studies.

**Table 109**

| Kidney function markers (mg/dL) in Sprague-Dawley rats | | |
|---|---|---|
| | BUN | Creatinine |
| PBS group 1 | 16 | 0.2 |
| PBS group 2 | 15 | 0.2 |
| ISIS 541881 | 22 | 0.3 |
| ISIS 542051 | 18 | 0.2 |
| ISIS 542101 | 22 | 0.3 |
| ISIS 542112 | 18 | 0.2 |
| ISIS 542118 | 18 | 0.3 |
| ISIS 542125 | 18 | 0.3 |
| ISIS 542127 | 19 | 0.3 |
| ISIS 542128 | 18 | 0.3 |
| ISIS 542153 | 17 | 0.3 |
| ISIS 542185 | 19 | 0.3 |
| ISIS 542186 | 19 | 0.3 |
| ISIS 545439 | 16 | 0.2 |
| ISIS 545447 | 16 | 0.2 |
| ISIS 541262 | 21 | 0.4 |
| ISIS 541742 | 19 | 0.2 |
| ISIS 541767 | 15 | 0.2 |
| ISIS 541875 | 16 | 0.2 |

### Hematology assays

Blood obtained from all rat groups were sent to Antech Diagnostics for hematocrit (HCT) measurements and analysis, as well as measurements of the various blood cells, such as WBC, RBC, and total hemoglobin content. The results are presented in Table 110. ISIS oligonucleotides that caused changes in the levels of any of the hematology markers outside the expected range for antisense oligonucleotides were excluded in further studies.

**Table 110**

| Hematology markers in Sprague-Dawley rats | | | | | |
|---|---|---|---|---|---|
| | HCT (%) | Hemoglobin (g/dL) | RBC (10⁶/µL) | WBC (10³/µL) | Platelets (10³/µL) |
| PBS group 1 | 43 | 14 | 7 | 7 | 775 |
| PBS group 2 | 49 | 15 | 8 | 8 | 1065 |
| ISIS 541881 | 41 | 13 | 8 | 6 | 553 |
| ISIS 542051 | 39 | 13 | 7 | 9 | 564 |
| ISIS 542101 | 37 | 12 | 7 | 15 | 603 |
| ISIS 542112 | 45 | 14 | 8 | 10 | 587 |
| ISIS 542118 | 47 | 15 | 8 | 7 | 817 |
| ISIS 542125 | 41 | 13 | 7 | 7 | 909 |
| ISIS 542127 | 44 | 14 | 8 | 10 | 872 |
| ISIS 542128 | 44 | 14 | 8 | 7 | 679 |
| ISIS 542153 | 48 | 15 | 8 | 7 | 519 |
| ISIS 542185 | 44 | 14 | 8 | 9 | 453 |
| ISIS 542186 | 44 | 14 | 8 | 12 | 433 |
| ISIS 545439 | 40 | 12 | 7 | 11 | 733 |
| ISIS 545447 | 43 | 13 | 8 | 9 | 843 |
| ISIS 541262 | 46 | 14 | 8 | 17 | 881 |
| ISIS 541742 | 47 | 15 | 8 | 10 | 813 |
| ISIS 541767 | 53 | 16 | 9 | 9 | 860 |
| ISIS 541875 | 42 | 13 | 7 | 9 | 840 |

### Organ weights

Liver, heart, spleen and kidney weights were measured at the end of the study, and are presented in Table 111. ISIS oligonucleotides that caused any changes in organ weights outside the expected range for antisense oligonucleotides were excluded from further studies.

**Table 111**

| Organ weights (g) | | | | |
|---|---|---|---|---|
| | Heart | Liver | Spleen | Kidney |
| PBS group 1 | 0.4 | 3.7 | 0.2 | 0.9 |
| PBS group 2 | 0.3 | 3.2 | 0.2 | 0.7 |
| ISIS 541881 | 0.4 | 3.4 | 0.4 | 0.9 |
| ISIS 542051 | 0.4 | 3.8 | 0.4 | 1.0 |
| ISIS 542101 | 0.3 | 4.2 | 0.6 | 1.1 |
| ISIS 542112 | 0.3 | 3.7 | 0.4 | 0.8 |
| ISIS 542118 | 0.4 | 3.6 | 0.2 | 0.8 |
| ISIS 542125 | 0.4 | 3.7 | 0.3 | 1.1 |
| ISIS 542127 | 0.3 | 4.2 | 0.3 | 0.8 |
| ISIS 542128 | 0.3 | 3.5 | 0.3 | 0.8 |
| ISIS 542153 | 0.3 | 3.5 | 0.3 | 0.8 |
| ISIS 542185 | 0.4 | 3.8 | 0.4 | 0.9 |
| ISIS 542186 | 0.3 | 3.8 | 0.6 | 0.9 |
| ISIS 545439 | 0.4 | 4.1 | 0.3 | 0.9 |
| ISIS 545447 | 0.4 | 3.4 | 0.3 | 1.1 |
| ISIS 541262 | 0.3 | 3.4 | 0.3 | 2.0 |
| ISIS 541742 | 0.3 | 3.8 | 0.3 | 0.8 |
| ISIS 541767 | 0.3 | 3.4 | 0.2 | 0.8 |
| ISIS 541875 | 0.3 | 5.2 | 0.4 | 1.0 |

### Example 23: Effect of ISIS antisense oligonucleotides targeting human GHR in cynomolgus monkeys

Cynomolgus monkeys were treated with ISIS antisense oligonucleotides selected from studies described in the Examples above. Antisense oligonucleotide efficacy and tolerability, as well as their pharmacokinetic profile in the liver and kidney, were evaluated.

At the time this study was undertaken, the cynomolgus monkey genomic sequence was not available in the National Center for Biotechnology Information (NCBI) database; therefore, cross-reactivity with the cynomolgus monkey gene sequence could not be confirmed. Instead, the sequences of the ISIS antisense oligonucleotides used in the cynomolgus monkeys was compared to a rhesus monkey sequence for homology. It is expected that ISIS oligonucleotides with homology to the rhesus monkey sequence are fully cross-reactive with the cynomolgus monkey sequence as well. The human antisense oligonucleotides tested are cross-reactive with the rhesus genomic sequence (GENBANK Accession No. NW_001120958.1 truncated from nucleotides 4410000 to 4720000, designated herein as SEQ ID NO: 2296). The greater the complementarity between the human oligonucleotide and the rhesus monkey sequence, the more likely the human oligonucleotide can cross-react with the rhesus monkey sequence. The start and stop sites of each oligonucleotide to SEQ ID NO: 2296 is presented in Table 112. "Start site" indicates the 5'-most nucleotide to which the gapmer is targeted in the rhesus monkey gene sequence.

**Table 112**

| Antisense oligonucleotides complementary to the rhesus GHR genomic sequence (SEQ ID NO: 2296) | | | | |
|---|---|---|---|---|
| ISIS No | Target Start Site | Target Stop Site | Chemistry | SEQ ID NO |
| 523723 | 149071 | 149090 | 5-10-5 MOE | 918 |
| 532254 | 64701 | 64720 | 5-10-5 MOE | 479 |
| 532401 | 147560 | 147579 | 5-10-5 MOE | 703 |
| 541767 | 152700 | 152715 | Deoxy, MOE and cEt | 1800 |
| 541875 | 210099 | 210114 | Deoxy, MOE and cEt | 1904 |
| 542112 | 146650 | 146665 | Deoxy, MOE and cEt | 2122 |
| 542118 | 149074 | 149089 | Deoxy, MOE and cEt | 2127 |
| 542185 | 245782 | 245797 | Deoxy, MOE and cEt | 2194 |

### Study 1

Prior to the study, the monkeys were kept in quarantine during which the animals were observed daily for general health. The monkeys were 2-4 years old and weighed between 2 and 4 kg. Nine groups of 5 randomly assigned male cynomolgus monkeys each were injected subcutaneously with ISIS oligonucleotide or PBS using a stainless steel dosing needle and syringe of appropriate size into the intracapsular region and outer thigh of the monkeys. The monkeys were dosed three times (days 1, 4, and 7) for the first week, and then subsequently once a week for 12 weeks with 40 mg/kg of ISIS oligonucleotide. A control group of 5 cynomolgus monkeys was injected with PBS in a similar manner and served as the control group.

During the study period, the monkeys were observed twice daily for signs of illness or distress. Any animal experiencing more than momentary or slight pain or distress due to the treatment, injury or illness was treated by the veterinary staff with approved analgesics or agents to relieve the pain after consultation with the Study Director. Any animal in poor health or in a possible moribund condition was identified for further monitoring and possible euthanasia. Scheduled euthanasia of the animals was conducted on day 86 by exsanguination after ketamine/xylazine-induced anesthesia and administration of sodium pentobarbital. The protocols described in the Example were approved by the Institutional Animal Care and Use Committee (IACUC).

### Target Reduction

### RNA analysis

On day 86, RNA was extracted from liver, white adipose tissue (WAT) and kidney for real-time PCR analysis of measurement of mRNA expression of GHR. Results are presented as percent inhibition of mRNA, relative to PBS control, normalized with RIBOGREEN®. 'n.d.' indicates that the data for that particular oligonucleotide was not measured. As shown in Table 113, treatment with ISIS antisense oligonucleotides resulted in significant reduction of GHR mRNA in comparison to the PBS control. Specifically, treatment with ISIS 532401 resulted in significant reduction of mRNA expression in all tissues.

The expression of the growth hormone-responsive gene, ALS was also measured in liver, kidney and adipose tissue. Treatment with ISIS 532401 resulted in ALS RNA expression reduction in liver by 44 ± 9 %, correlating with GHR levels. There was no reduction observed in adipose tissue. The expression of IGF1 in the liver was also measured. Treatment with ISIS 532401 resulted in IGF1 RNA expression reduction in liver by 71 ± 10 %, correlating with GHR levels.

**Table 113**

| Percent inhibition of GHR mRNA in the cynomolgus monkey liver relative to the PBS control | | | |
|---|---|---|---|
| ISIS No | Liver | Kidney | WAT |
| 532401 | 60 | 47 | 59 |
| 532254 | 63 | 65 | n.d. |
| 523723 | 38 | 0 | n.d. |
| 542112 | 61 | 60 | 36 |
| 542118 | 0 | 22 | 27 |
| 542185 | 66 | 53 | n.d. |
| 541767 | 0 | 14 | n.d. |
| 541875 | 34 | 77 | n.d. |

### Protein analysis

Approximately 1 mL of blood was collected from all available animals at day 85 and placed in tubes containing the potassium salt of EDTA. The tubes were centrifuged (3000 rpm for 10 min at room temperature) to obtain plasma. Plasma levels of IGF-1 and GH were measured in the plasma. The results are presented in Table 114. The results indicate that treatment with ISIS oligonucleotides resulted in reduced IGF-1 protein levels.

Plasma levels of IGF1 after treatment with ISIS 532401 are also presented in Table 115 and demonstrate the effect of antisense inhibition of GHR in reducing IGF1 levels at day 7 and day 85.

**Table 114**

| Plasma protein levels in the cynomolgus monkey | | |
|---|---|---|
| | IGF-1 (% baseline) | GH (ng/mL) |
| PBS | 121 | 19 |
| 532401 | 57 | 39 |
| 532254 | 51 | 26 |
| 523723 | 77 | 16 |
| 542112 | 46 | 48 |
| 542118 | 97 | 6 |
| 542185 | 59 | 32 |
| 541767 | 101 | 22 |
| 541875 | 45 | 47 |

**Table 115**

| Plasma IGF1 levels in the cynomolgus monkey | | |
|---|---|---|
| | Day 7 | Day 85 |
| PBS | 458 | 643 |
| ISIS 532401 | 326 | 263 |

### Tolerability studies

### Body and organ weight measurements

To evaluate the effect of ISIS oligonucleotides on the overall health of the animals, body and organ weights were measured. Body weights were measured on day 84 and are presented in Table 115. Organ weights were measured on day 86 and the data is also presented in Table 115. The results indicate that effect of treatment with antisense oligonucleotides on body and organ weights was within the expected range for antisense oligonucleotides. Specifically, treatment with ISIS 532401 was well tolerated in terms of the body and organ weights of the monkeys.

**Table 115**

| Final body and organ weights in cynomolgus monkey | | | | |
|---|---|---|---|---|
| | Body Wt (kg) | Spleen (g) | Kidney (g) | Liver (g) |
| PBS | 2.7 | 2.8 | 12.3 | 56.7 |
| 532401 | 2.6 | 4.0 | 11.5 | 58.5 |
| 532254 | 2.6 | 4.8 | 15.4 | 69.5 |
| 523723 | 2.8 | 3.1 | 14.8 | 69.4 |
| 542112 | 2.6 | 3.5 | 13.6 | 60.0 |
| 542118 | 2.7 | 2.7 | 11.9 | 58.6 |
| 542185 | 2.6 | 5.5 | 17.2 | 68.5 |
| 541767 | 2.8 | 5.1 | 11.7 | 65.1 |
| 541875 | 2.8 | 5.5 | 13.2 | 55.0 |

### Liver function

To evaluate the effect of ISIS oligonucleotides on hepatic function, blood samples were collected from all the study groups. The blood samples were collected via femoral venipuncture, 48 hrs post-dosing. The monkeys were fasted overnight prior to blood collection. Blood was collected in tubes containing K₂-EDTA anticoagulant, which were centrifuged to obtain plasma. Levels of various liver function markers were measured using a Toshiba 200FR NEO chemistry analyzer (Toshiba Co., Japan). Plasma levels of ALT and AST and bilirubin were measured. The Tables below present the results for ALT and AST levels at various time points. The results indicate that antisense oligonucleotides had no effect on liver function outside the expected range for antisense oligonucleotides. Specifically, treatment with ISIS 532401 was well tolerated in terms of the liver function in monkeys.

**Table 116**

| ALT levels (IU/L) in cynomolgus monkey | | | |
|---|---|---|---|
| | Day 16 | Day 44 | Day 86 |
| PBS | 46 | 37 | 40 |
| ISIS 532401 | 63 | 59 | 88 |
| ISIS 532254 | 62 | 46 | 56 |
| ISIS 523723 | 50 | 77 | 86 |
| ISIS 542112 | 53 | 54 | 60 |
| ISIS 542118 | 38 | 41 | 52 |
| ISIS 542185 | 58 | 59 | 91 |
| ISIS 541767 | 56 | 45 | 46 |
| ISIS 541875 | 70 | 54 | 71 |

**Table 117**

| AST levels (IU/L) in cynomolgus monkey | | | |
|---|---|---|---|
| | Day 16 | Day 44 | Day 86 |
| PBS | 58 | 40 | 45 |
| ISIS 532401 | 47 | 48 | 61 |
| ISIS 532254 | 71 | 81 | 98 |
| ISIS 523723 | 56 | 61 | 73 |
| ISIS 542112 | 58 | 65 | 89 |
| ISIS 542118 | 41 | 40 | 46 |
| ISIS 542185 | 61 | 63 | 98 |
| ISIS 541767 | 52 | 39 | 63 |
| ISIS 541875 | 70 | 50 | 70 |

### Kidney function

To evaluate the effect of ISIS oligonucleotides on kidney function, blood samples were collected from all the study groups. The blood samples were collected via femoral venipuncture, 48 hrs post-dosing. The monkeys were fasted overnight prior to blood collection. Blood was collected in tubes containing K₂-EDTA anticoagulant, which were centrifuged to obtain plasma. Levels of BUN and creatinine were measured using a Toshiba 200FR NEO chemistry analyzer (Toshiba Co., Japan). The Tables below present the results for BUN and creatinine levels at various time points.

The plasma chemistry data indicate that most of the ISIS oligonucleotides did not have any effect on the kidney function outside the expected range for antisense oligonucleotides. Specifically, treatment with ISIS 532401 was well tolerated in terms of the kidney function of the monkeys.

**Table 118**

| BUN levels (mg/dL) in cynomolgus monkey | | | |
|---|---|---|---|
| | Day 16 | Day 44 | Day 86 |
| PBS | 29 | 26 | 26 |
| ISIS 532401 | 27 | 27 | 27 |
| ISIS 532254 | 21 | 22 | 25 |
| ISIS 523723 | 25 | 24 | 22 |
| ISIS 542112 | 26 | 24 | 24 |
| ISIS 542118 | 29 | 27 | 29 |
| ISIS 542185 | 22 | 21 | 22 |
| ISIS 541767 | 29 | 24 | 24 |
| ISIS 541875 | 29 | 24 | 21 |

**Table 119**

| Creatinine levels (mg/dL) in cynomolgus monkey | | | |
|---|---|---|---|
| | Day 16 | Day 44 | Day 86 |
| PBS | 0.9 | 0.8 | 0.9 |
| ISIS 532401 | 1.1 | 1.0 | 1.1 |
| ISIS 532254 | 1.0 | 1.0 | 1.0 |
| ISIS 523723 | 1.0 | 1.0 | 1.0 |
| ISIS 542112 | 1.0 | 0.9 | 1.0 |
| ISIS 542118 | 0.9 | 0.9 | 0.9 |
| ISIS 542185 | 1.0 | 0.9 | 0.9 |
| ISIS 541767 | 1.1 | 0.9 | 0.9 |
| ISIS 541875 | 1.2 | 1.1 | 1.1 |

### Hematology

To evaluate any effect of ISIS oligonucleotides in cynomolgus monkeys on hematologic parameters, blood samples of approximately 1.3 mL of blood was collected from each of the available study animals in tubes containing K₂-EDTA. Samples were analyzed for red blood cell (RBC) count, white blood cells (WBC) count, individual white blood cell counts, such as that of monocytes, neutrophils, lymphocytes, as well as for platelet count, hemoglobin content and hematocrit, using an ADVIA120 hematology analyzer (Bayer, USA). The Table below presents the results for platelet count at various time points, 'n/a' indicates that the data for that time point is not available.

The data indicate the oligonucleotides did not cause any changes in hematologic parameters outside the expected range for antisense oligonucleotides at this dose. Specifically, treatment with ISIS 532401 was well tolerated in terms of the hematologic parameters of the monkeys.

**Table 120**

| Platelet count (x 10³/µL) in cynomolgus monkey | | | |
|---|---|---|---|
| | Day 30 | Day 58 | Day 86 |
| PBS | 538 | 464 | 403 |
| ISIS 532401 | 493 | 465 | 395 |
| ISIS 532254 | 334 | 328 | 306 |
| ISIS 523723 | 352 | 304 | 268 |
| ISIS 542112 | 454 | 430 | 368 |
| ISIS 542118 | 418 | 379 | 377 |
| ISIS 542185 | 370 | 303 | 296 |
| ISIS 541767 | 435 | 326 | 325 |
| ISIS 541875 | 437 | 359 | n/a |

### C-reactive protein and complement C3 level analysis

To evaluate any inflammatory effect of ISIS oligonucleotides in cynomolgus monkeys, blood samples were taken for analysis. The monkeys were fasted overnight prior to blood collection. Approximately 1.5 mL of blood was collected from each animal and put into tubes without anticoagulant for serum separation. The tubes were kept at room temperature for a minimum of 90 min and then centrifuged at 3,000 rpm for 10 min at room temperature to obtain serum. C-reactive protein (CRP), which is synthesized in the liver and which serves as a marker of inflammation, was measured using a Toshiba 200FR NEO chemistry analyzer (Toshiba Co., Japan). The Tables below present the results for CRP and C3 levels at various time points. The results indicate that treatment with ISIS 532401 did not cause inflammation in monkeys.

**Table 121**

| CRP (mg/L) in cynomolgus monkey | | | |
|---|---|---|---|
| | Day 16 | Day 44 | Day 86 |
| PBS | 3.8 | 2.0 | 2.0 |
| ISIS 532401 | 2.3 | 1.7 | 1.9 |
| ISIS 532254 | 2.1 | 3.2 | 5.9 |
| ISIS 523723 | 6.1 | 4.5 | 4.4 |
| ISIS 542112 | 2.5 | 2.7 | 2.7 |
| ISIS 542118 | 2.2 | 2.8 | 2.1 |
| ISIS 542185 | 2.3 | 11.9 | 9.3 |
| ISIS 541767 | 1.9 | 1.5 | 6.7 |
| ISIS 541875 | 4.9 | 3.5 | 8.4 |

**Table 122**

| C3 (mg/dL) on day 85 (24 hours after dosing) in cynomolgus monkey | |
|---|---|
| | C3 |
| PBS | 114 |
| ISIS 532401 | 96 |
| ISIS 532254 | 100 |
| ISIS 523723 | 87 |
| ISIS 542112 | 100 |
| ISIS 542118 | 110 |
| ISIS 542185 | 98 |
| ISIS 541767 | 99 |
| ISIS 541875 | 81 |

### Measurement of oligonucleotide concentration

The concentration of the full-length oligonucleotide in the liver and the kidney of the monkeys was measured. The method used is a modification of previously published methods (Leeds et al., 1996; Geary et al., 1999) which consist of a phenol-chloroform (liquid-liquid) extraction followed by a solid phase extraction. An internal standard (ISIS 355868, a 27-mer 2'-O-methoxyethyl modified phosphorothioate oligonucleotide, GCGTTTGCTCTTCTTCTTGCGTTTTTT, designated herein as SEQ ID NO: 2300) was added prior to extraction. Tissue sample concentrations were calculated using calibration curves, with a lower limit of quantitation (LLOQ) of approximately 1.14 µg/g. Half-lives were then calculated using WinNonlin software (PHARSIGHT).

The results are presented in Table 123, expressed as µg/g of tissue, as well as the ratio of concentration in kidney versus liver.

**Table 123**

| Oligonucleotide concentration in the liver and kidney of cynomolgus monkeys | | | | |
|---|---|---|---|---|
| | Chemistry | Liver | Kidney | ratio |
| ISIS 532401 | 5-10-5 MOE | 725 | 2154 | 3.0 |
| ISIS 532254 | 5-10-5 MOE | 911 | 4467 | 4.9 |
| ISIS 523723 | 5-10-5 MOE | 657 | 3093 | 4.7 |
| ISIS 542112 | 3-10-3 cEt/MOE | 491 | 2863 | 5.8 |
| ISIS 542118 | 3-10-3 cEt/MOE | 429 | 1222 | 2.8 |
| ISIS 542185 | 3-10-3 cEt/MOE | 432 | 3126 | 7.2 |
| ISIS 541767 | 3-10-3 cEt/MOE | 280 | 994 | 3.5 |
| ISIS 541875 | 3-10-3 cEt/MOE | 766 | 3892 | 5.1 |

### Study 2

One group of 5 randomly assigned male cynomolgus monkeys was injected subcutaneously with ISIS 532401 or PBS using a stainless steel dosing needle and syringe of appropriate size into the intracapsular region and outer thigh of the monkeys. The monkeys were dosed a loading dose per week (days 1, 3, 5, and 7) for the first week, and then subsequently once a week (days 14, 21, 28, 35, 42, 49, 56, 63, 70, 77, 84, and 91) with 40 mg/kg of ISIS 532401. A control group of 5 cynomolgus monkeys was injected with PBS in a similar manner and served as the control group.

### Target Reduction

### RNA analysis

On day 93, RNA was extracted from liver, white adipose tissue (WAT) and muscle for real-time PCR analysis of measurement of mRNA expression of GHR. Treatment with ISIS 532401 resulted in significant reduction of GHR mRNA in liver and white adipose tissue.

The expression of the growth hormone-responsive gene, ALS was also measured in the liver. Treatment with ISIS 532401 resulted in ALS RNA expression reduction in liver by 38%, correlating with GHR levels. 'n.d.' indicates that the levels were not checked in that particular tissue. The expression of IGF1 in the liver, muscle and fat tissues was also measured. Treatment with ISIS 532401 resulted in IGF1 RNA expression reduction in liver and in the WAT, correlating with GHR levels.

**Table 124**

| Effect of treatment with ISIS 532401 on mRNA levels (% inhibition compared to the PBS control) in the cynomolgus monkey | | | |
|---|---|---|---|
| | Liver | WAT | Muscle |
| GHR | 64 | 75 | 21 |
| ALS | 38 | n.d. | n.d. |
| IGF1 | 73 | 56 | 35 |

### Protein analysis

Plasma levels of IGF-1 and GH were measured in the plasma. The results are presented in the Table below. The results indicate that treatment with ISIS 532401 resulted in reduced IGF-1 protein levels. There was no increase in plasma growth hormone levels.

**Table 125**

| Plasma IGF1 levels (ng/mL) in the cynomolgus monkey | | | |
|---|---|---|---|
| | Day 7 | Day 49 | Day 91 |
| PBS | 625 | 776 | 850 |
| ISIS 532401 | 378 | 455 | 363 |

**Table 126**

| Plasma growth hormone levels (ng/mL) in the cynomolgus monkey | | | |
|---|---|---|---|
| | Day 7 | Day 49 | Day 91 |
| PBS | 25 | 27 | 33 |
| ISIS 532401 | 16 | 13 | 17 |

### Example 24: Measurement of viscosity of ISIS antisense oligonucleotides targeting human GHR

The viscosity of select antisense oligonucleotides from the study described in the Examples above was measured with the aim of screening out antisense oligonucleotides which have a viscosity more than 40 cP. Oligonucleotides having a viscosity greater than 40 cP would be too viscous to be administered to any subject.

ISIS oligonucleotides (32-35 mg) were weighed into a glass vial, 120 µL of water was added and the antisense oligonucleotide was dissolved into solution by heating the vial at 50°C. Part of (75 µL) the pre-heated sample was pipetted to a micro-viscometer (Cambridge). The temperature of the micro-viscometer was set to 25 °C and the viscosity of the sample was measured. Another part (20 µL) of the pre-heated sample was pipetted into 10 mL of water for UV reading at 260 nM at 85°C (Cary UV instrument). The results are presented in Table 127 and indicate that all the antisense oligonucleotides solutions are optimal in their viscosity under the criterion stated above.

**Table 127**

| Viscosity of ISIS antisense oligonucleotides targeting human GHR | | |
|---|---|---|
| ISIS No. | Chemistry | Viscosity (cP) |
| 523723 | 5-10-5 MOE | 8 |
| 532254 | 5-10-5 MOE | 22 |
| 532401 | 5-10-5 MOE | 12 |
| 541767 | Deoxy, MOE and cEt | 13 |
| 541875 | Deoxy, MOE and cEt | 33 |
| 542112 | Deoxy, MOE and cEt | 10 |
| 542118 | Deoxy, MOE and cEt | 14 |
| 542185 | Deoxy, MOE and cEt | 17 |

### Example 25: Effect of antisense inhibition of GHR in mice

In order to confirm the effect of antisense inhibition of GHR in the primate model, an ISIS oligonucleotide targeting murine GHR was employed to replicate the result in a mouse model.

ISIS 563223 (GAGACTTTTCCTTGTACACA, designated herein as SEQ ID NO: 2301) is a 5-10-5 MOE gapmer murine antisense oligonucleotide targeting murine GHR (GENBANK Accession No; NM_010284.2, designated herein as SEQ ID NO: 2302) at target start site 3230. A group of male and female CD1 mice were injected with a loading dose (on days 1, 3, 5, and 7) on the first week and subsequently with a once weekly dose (on days 14, 21, 28, 35, 42, 49, 56, 63, 70, 77, 84, and 91) with 40 mg/kg of ISIS 563223. One group of CD1 mice was injected in a similar manner with PBS. Mice were euthanized 48 hours after the last dose, and organs and plasma were harvested for further analysis.

### mRNA expression

Liver mRNA expression of GHR, GHBP, IGF1, and ALS were measured. The results are presented in Table 128. Antisense inhibition of GHR resulted in inhibition of GHBP, IGF1 and ALS gene expression levels.

**Table 128**

| mRNA expression (% Inhibition) in CD1 mice liver | | |
|---|---|---|
| | % (in male mice) | % (in female mice) |
| GHR | 98 | 96 |
| GHBP | 74 | 66 |
| IGF1 | 60 | 48 |
| ALS | 84 | 74 |

### Protein expression

Plasma levels of IGF1 and growth hormone were measured. The results are presented in Table 129. Antisense inhibition of GHR resulted in decrease in IGF1 levels, and had no effect on growth hormone levels.

**Table 129**

| IGF1 protein levels (ng/mL) in CD1 mice liver | | |
|---|---|---|
| | in male mice | in female mice |
| PBS | 949 | 1002 |
| ISIS 563223 | 439 | 740 |

**Table 130**

| Growth hormone protein levels (ng/mL) in CD1 mice liver | | |
|---|---|---|
| | in male mice | in female mice |
| PBS | 3.3 ± 2.2 | 2.2 ± 1.3 |
| ISIS 563223 | 5.6 ± 6.7 | 2.9 ± 1.7 |

## Claims

1. A compound comprising a modified oligonucleotide consisting of 18 to 30 linked nucleosides in length, wherein the modified oligonucleotide has a nucleobase sequence comprising a portion of at least 18 contiguous nucleobases 100% complementary to an equal length portion of nucleobases 153921-153940 of a growth hormone receptor nucleic acid having the nucleobase sequence of SEQ ID NO: 2, wherein the nucleobase sequence of the modified oligonucleotide is at least 90% complementary to SEQ ID NO: 2, wherein the compound reduces the amount or activity of the growth hormone receptor nucleic acid.

2. The compound of claim 1, wherein the modified oligonucleotide is 100% complementary to SEQ ID NO: 2.

3. The compound of any preceding claim, wherein the modified oligonucleotide has a nucleobase sequence comprising the sequence recited in SEQ ID NO: 703, optionally wherein the modified oligonucleotide consists of 20 linked nucleosides and has a nucleobase sequence consisting of the sequence recited in SEQ ID NO: 703.

4. The compound of any one of claims 1-3, wherein the modified oligonucleotide comprises at least one modified sugar and wherein the at least one modified sugar optionally comprises a 2'-O-methoxyethyl group or is a bicyclic sugar, such as a bicyclic sugar that comprises a 4'-CH(CH3)-O-2' group or a bicyclic sugar that comprises a 4'-CH2-O-2' or 4'-(CH2)2-O-2'group.

5. The compound of any one of claims 1-4, wherein the modified oligonucleotide comprises at least one modified internucleoside linkage and wherein the modified internucleoside linkage is optionally a phosphorothioate internucleoside linkage.

6. The compound of any one of claims 1-5, wherein the modified oligonucleotide comprises at least one modified nucleobase and wherein the modified nucleobase is optionally 5-methylcytosine.

7. The compound of any one of claims 1-6, wherein the modified oligonucleotide comprises:
a gap segment consisting of linked deoxynucleosides;
a 5' wing segment consisting of linked nucleosides; and
a 3' wing segment consisting of linked nucleosides;
wherein the gap segment is positioned between the 5' wing segment and the 3' wing segment and wherein each nucleoside of each wing segment comprises a modified sugar.

8. The compound of any preceding claim, wherein the modified oligonucleotide consists of 20 linked nucleosides having a nucleobase sequence consisting of the sequence recited in SEQ ID NO: 703, and wherein the modified oligonucleotide comprises:
a gap segment consisting of ten linked deoxynucleosides;
a 5' wing segment consisting of five linked nucleosides; and
a 3' wing segment consisting of five linked nucleosides;
wherein the gap segment is positioned between the 5' wing segment and the 3' wing segment; wherein each nucleoside of each wing segment comprises a 2'-O-methoxyethyl sugar; wherein at least one internucleoside linkage is a phosphorothioate linkage; and wherein each cytosine is a 5 -methylcytosine.

9. The compound of any one of claims 1-8, wherein the compound is single-stranded.

10. A composition comprising the compound of any one of claims 1-9 or salt thereof and at least one of a pharmaceutically acceptable carrier or diluent.

11. The compound of any one of claims 1-9, or the composition of claim 10, for use in therapy.

12. The compound or composition for use of claim 11, for use in treating or preventing a disease associated with excess growth hormone in a human, wherein the disease associated with excess growth hormone is optionally acromegaly.

13. The compound or composition for use according to claim 12, wherein treating the acromegaly reduces IGF-1 levels.

14. The compound or composition of any one of claims 1-10, for use in a method of reducing growth hormone receptor (GHR) levels in a human, said method comprising administering to the human a therapeutically effective amount of the compound or composition, thereby reducing GHR levels in the human, optionally wherein the human has a disease associated with excess growth hormone, such as acromegaly.

## Patentansprüche

1. Verbindung, umfassend ein modifiziertes Oligonukleotid, bestehend aus 18 bis 30 verknüpften Nukleosiden Länge, wobei das modifizierte Oligonukleotid eine Nukleobasensequenz aufweist, umfassend einen Teil von mindestens 18 zusammenhängenden Nukleobasen, die 100% komplementär sind zu einem gleich langen Teil der Nukleobasen 153921-153940 einer Nukleinsäure eines Wachstumshormonrezeptors mit der Nukleobasensequenz von SEQ ID NO: 2, wobei die Nukleobasensequenz des modifizierten Oligonukleotids mindestens 90% komplementär ist zu SEQ ID NO: 2, wobei die Verbindung die Menge oder Aktivität der Nukleinsäure des Wachstumshormonrezeptors reduziert.

2. Verbindung nach Anspruch 1, wobei das modifizierte Oligonukleotid zu SEQ ID NO: 2 zu 100% komplementär ist.

3. Verfahren nach einem vorhergehenden Anspruch, wobei das modifizierte Oligonukleotid eine Nukleobasensequenz aufweist, die die in SEQ ID NO: 703 aufgeführte Sequenz umfasst, wobei das modifizierte Oligonukleotid gegebenenfalls aus 20 verknüpften Nukleosiden besteht und eine Nukleobasensequenz aufweist, die aus der in SEQ ID NO: 703 aufgeführten Sequenz besteht.

4. Verbindung nach einem der Ansprüche 1 bis 3, wobei das modifizierte Oligonukleotid mindestens einen modifizierten Zucker umfasst und wobei der mindestens eine modifizierte Zucker gegebenenfalls eine 2'-0-Methoxyethylgruppe umfasst oder ein bicyclischer Zucker ist, wie ein bicyclischer Zucker, der eine 4'-CH(CH3)-O-2'-Gruppe umfasst, oder ein bicyclischer Zucker, der eine 4'-CH2-O-2'- oder 4'-(CH2)2-O-2'-Gruppé umfasst.

5. Verbindung nach einem der Ansprüche 1 bis 4, wobei das modifizierte Oligonukleotid mindestens eine modifizierte Internukleosidbindung umfasst, und wobei die modifizierte Internukleosidbindung gegebenenfalls eine Phosphorthioat-Internukleosidbindung ist.

6. Verbindung nach einem der Ansprüche 1 bis 5, wobei das modifizierte Oligonukleotid mindestens eine modifizierte Nukleobase umfasst, und wobei die modifizierte Nukleobase gegebenenfalls 5-Methylcytosin ist.

7. Verbindung nach einem der Ansprüche 1 bis 6, wobei das modifizierte Oligonukleotid umfasst:
ein Lückensegment, das aus verknüpften Desoxynukleosiden besteht;
ein 5'-Flügelsegment, das aus verknüpften Nukleosiden besteht; und
ein 3'-Flügelsegment, das aus verknüpften Nukleosiden besteht;
wobei das Lückensegment zwischen dem 5'-Flügelsegment und dem 3'-Flügelsegment positioniert ist und wobei jedes Nukleosid jedes Flügelsegments einen modifizierten Zucker umfasst.

8. Verbindung nach einem vorhergehenden Anspruch, wobei das modifizierte Oligonukleotid aus 20 verknüpften Nukleosiden mit einer Nukleobasensequenz besteht, die aus der in SEQ ID NO: 703 aufgeführten Sequenz besteht, und wobei das modifizierte Oligonukleotid umfasst:
ein Lückensegment, das aus zehn verknüpften Desoxynukleosiden besteht;
ein 5'-Flügelsegment, das aus fünf verknüpften Nukleosiden besteht; und
ein 3'-Flügelsegment, das aus fünf verknüpften Nukleosiden besteht;
wobei das Lückensegment zwischen dem 5'-Flügelsegment und dem 3'-Flügelsegment positioniert ist; wobei jedes Nukleosid jedes Flügelsegments einen 2'-O-Methoxyethylzucker umfasst; wobei mindestens eine Internukleosidbindung eine Phosphorthioatbindung ist und wobei jedes Cytosin ein 5-Methylcytosin ist.

9. Verbindung nach einem der Ansprüche 1 bis 8, wobei die Verbindung einzelsträngig ist.

10. Zusammensetzung, umfassend die Verbindung nach einem der Ansprüche 1 bis 9 oder ein Salz davon und mindestens ein(en) von einem pharmazeutisch akzeptablen Träger oder einem pharmazeutisch akzeptablen Verdünnungsmittel.

11. Verbindung nach einem der Ansprüche 1 bis 9 oder Zusammensetzung nach Anspruch 10 zur Verwendung bei der Therapie.

12. Verbindung oder Zusammensetzung zur Verwendung nach Anspruch 11, zur Verwendung bei der Behandlung oder Prävention einer mit überschüssigem Wachstumshormon einhergehenden Krankheit bei einem Menschen, wobei die mit überschüssigem Wachstumshormon einhergehende Krankheit gegebenenfalls Akromegalie ist.

13. Verbindung oder Zusammensetzung zur Verwendung nach Anspruch 12, wobei die Behandlung der Akromegalie die IGF-1-Spiegel verringert.

14. Verbindung oder Zusammensetzung nach einem der Ansprüche 1 bis 10 zur Verwendung bei einem Verfahren zum Verringern der Wachstumshormonrezeptor-(GHR)-Spiegel bei einem Menschen, wobei das Verfahren das Verabreichen einer therapeutisch wirksamen Menge der Verbindung oder Zusammensetzung an den Menschen umfasst, wodurch die GHR-Spiegel bei dem Menschen verringert werden, wobei der Mensch gegebenenfalls eine Krankheit hat, die mit überschüssigem Wachstumshormon einhergeht, wie Akromegalie.

## Revendications

1. Composé comprenant un oligonucléotide modifié constitué d'une longueur de 18 à 30 nucléosides liés, dans lequel l'oligonucléotide modifié a une séquence de nucléobases comprenant une partie d'au moins 18 nucléobases contiguës 100 % complémentaire d'une partie de longueur égale de nucléobases 153921-153940 d'un acide nucléique de récepteur de l'hormone de croissance ayant la séquence de nucléobases de SEQ ID n° : 2, dans lequel la séquence de nucléobases de l'oligonucléotide modifié est au moins à 90 % complémentaire de SEQ ID n° : 2, le composé réduisant la quantité ou l'activité de l'acide nucléique du récepteur de l'hormone de croissance.

2. Composé selon la revendication 1, dans lequel l'oligonucléotide modifié est 100 % complémentaire de SEQ ID n° : 2.

3. Composé selon une quelconque revendication précédente, dans lequel l'oligonucléotide modifié a une séquence de nucléobases comprenant la séquence indiquée dans SEQ ID n° : 703, éventuellement dans lequel l'oligonucléotide modifié est constitué de 20 nucléosides liés et a une séquence de nucléobases constituée de la séquence indiquée dans SEQ ID n° : 703.

4. Composé selon l'une quelconque des revendications 1-3, dans lequel l'oligonucléotide modifié comprend au moins un sucre modifié et dans lequel l'au moins un sucre modifié comprend éventuellement un groupe 2'-0-méthoxyéthyle ou est un sucre bicyclique, tel qu'un sucre bicyclique qui comprend un groupe 4'-CH(CH₃)-O-2' ou un sucre bicyclique qui comprend un groupe 4'-CH₂-O-2' ou 4'- (CH₂) ₂-O-2' .

5. Composé selon l'une quelconque des revendications 1-4, dans lequel l'oligonucléotide modifié comprend au moins une liaison internucléosidique modifiée et dans lequel la liaison internucléosidique modifiée est éventuellement une liaison internucléosidique phosphorothioate.

6. Composé selon l'une quelconque des revendications 1-5, dans lequel l'oligonucléotide modifié comprend au moins une nucléobase modifiée et dans lequel la nucléobase modifiée est éventuellement la 5-méthylcytosine.

7. Composé selon l'une quelconque des revendications 1-6, dans lequel l'oligonucléotide modifié comprend :
un segment de brèche constitué de désoxynucléosides liés ;
un segment d'aile 5' constitué de nucléosides liés ; et
un segment d'aile 3' constitué de nucléosides liés ; dans lequel le segment de brèche est situé entre le segment d'aile 5' et le segment d'aile 3' et dans lequel chaque nucléoside de chaque segment d'aile comprend un sucre modifié.

8. Composé selon une quelconque revendication précédente, dans lequel l'oligonucléotide modifié est constitué de 20 nucléosides liés ayant une séquence de nucléobases constituée de la séquence indiquée dans SEQ ID n° : 703 et dans lequel l'oligonucléotide modifié comprend :
un segment de brèche constitué de dix désoxynucléosides liés ;
un segment d'aile 5' constitué de cinq nucléosides liés ; et
un segment d'aile 3' constitué de cinq nucléosides liés ;
dans lequel le segment de brèche est situé entre le segment d'aile 5' et le segment d'aile 3' ; dans lequel chaque nucléoside de chaque segment d'aile comprend un 2'-O-méthoxyéthyl-sucre ; dans lequel au moins une liaison internucléosidique est une liaison phosphorothioate ; et dans lequel chaque cytosine est une 5-méthylcytosine.

9. Composé selon l'une quelconque des revendications 1-8, le composé étant simple brin.

10. Composition comprenant le composé selon l'une quelconque des revendications 1-9 ou un sel de celui-ci et au moins un vecteur ou diluant pharmaceutiquement acceptable.

11. Composé selon l'une quelconque des revendications 1-9 ou composition selon la revendication 10, destinés à être utilisés en thérapie.

12. Composé ou composition destinés à être utilisés selon la revendication 11, destinés à être utilisés en traitement ou prévention d'une maladie associée à un excès d'hormone de croissance chez un être humain, la maladie associée à un excès d'hormone de croissance étant éventuellement l'acromégalie.

13. Composé ou composition destinés à être utilisés selon la revendication 12, le traitement de l'acromégalie réduisant les taux d'IGF-1.

14. Composé ou composition selon l'une quelconque des revendications 1-10, destinés à être utilisés dans une méthode de réduction de taux de récepteur de l'hormone de croissance (GHR) chez un être humain, ladite méthode comprenant l'administration à l'être humain d'une quantité thérapeutiquement efficace du composé ou de la composition, ce qui réduit ainsi les taux de GHR chez l'être humain, éventuellement l'être humain ayant une maladie associée à un excès d'hormone de croissance, telle que l'acromégalie.
